# EUROPEAN PATENT SPECIFICATION

(11) **EP 2 382 320 B1**
(45) Date of publication and mention of the grant of the patent: **18.04.2018**
(21) Application number: 10702930.8
(22) Date of filing: 21.01.2010
(51) Int. Cl.: C12P 13/04, C12P 13/08

(54) **PROCESS FOR PRODUCING L-AMINO ACIDS EMPLOYING BACTERIA OF THE ENTEROBACTERIACEA FAMILY IN A CULTURE MEDIUM WITH CONTROLLED GLYCEROL CONCENTRATION**
VERFAHREN ZUR HERSTELLUNG VON L-AMINOSÄUREN UNTER VERWENDUNG VON BAKTERIEN DER FAMILIE DER ENTEROBACTERIACEA IN EINEM KULTURMEDIUM MIT KONTROLLIERTER GLYZERIN-KONZENTRATION
PROCÉDÉ DE PRODUCTION D'ACIDES L-AMINÉS EN UTILISANT DES BACTÉRIES DE LA FAMILLE DES ENTEROBACTERIACEAE DANS UN MILIEU DE CULTURE À CONCENTRATION CONTRÔLÉE EN GLYCÉRINE

(30) Priority: 23.01.2009 JP 2009013123; 27.04.2009 US 172948 P; 13.11.2009 JP 2009259654
(43) Date of publication of application: 02.11.2011
(73) Proprietor: Ajinomoto Co., Inc., Tokyo 104-8315 (JP)
(72) Inventor: NAKAHARA, Yuichi, Kawasaki-shi Kanagawa 210-8681 (JP); HAGIWARA, Yusuke, Kawasaki-shi Kanagawa 210-8681 (JP); NAGAI, Yuri, Kawasaki-shi Kanagawa 210-8681 (JP); UEDA, Takuji, Kawasaki-shi Kanagawa 210-8681 (JP); HAYASHI, Kazuyuki, Kawasaki-shi Kanagawa 210-8681 (JP); YANO, Yuki, Kawasaki-shi Kanagawa 210-8681 (JP)
(74) Representative: Hoffmann Eitle
(86) International application number: PCT/JP2010/051071
(87) International publication number: WO 2010/084995

(56) References cited:
- EP-A1- 2 113 570
- WO-A1-2008/107277

### SEQUENCE LISTING

<110> Ajinomoto Co., Inc.
<120> A method for producing an L-amino acid
<130> D070-C9111
<150> JP2009-013123
   <151> 2009-01-23
<150> US61/172948
   <151> 2009-04-27
<150> JP2009-259654
   <151> 2009-11-13
<160> 94
<170> PatentIn version 3.5
<210> 1
   <211> 1104
   <212> DNA
   <213> Escherichia coli
<220>
   <221> CDS
   <222> (1).. (1104)
<400> 1
   atg gac cgc att att caa tca ccg ggt aaa tac atc cag ggc gct gat       48
   Met Asp Arg Ile Ile Gln Ser Pro Gly Lys Tyr Ile Gln Gly Ala Asp
   1               5                   10                  15
   gtg att aat cgt ctg ggc gaa tac ctg aag ccg ctg gca gaa cgc tgg       96
   Val Ile Asn Arg Leu Gly Glu Tyr Leu Lys Pro Leu Ala Glu Arg Trp
               20                  25                  30
   tta gtg gtg ggt gac aaa ttt gtt tta ggt ttt gct caa tcc act gtc      144
   Leu Val Val Gly Asp Lys Phe Val Leu Gly Phe Ala Gln Ser Thr Val
           35                  40                  45
   gag aaa agc ttt aaa gat gct gga ctg gta gta gaa att gcg ccg ttt      192
   Glu Lys Ser Phe Lys Asp Ala Gly Leu Val Val Glu Ile Ala Pro Phe
       50                  55                  60
   ggc ggt gaa tgt tcg caa aat gag atc gac cgt ctg cgt ggc atc gcg      240
   Gly Gly Glu Cys Ser Gln Asn Glu Ile Asp Arg Leu Arg Gly Ile Ala
   65                  70                  75                  80
   gag act gcg cag tgt ggc gca att ctc ggt atc ggt ggc gga aaa acc      288
   Glu Thr Ala Gln Cys Gly Ala Ile Leu Gly Ile Gly Gly Gly Lys Thr
                   85                  90                  95
   ctc gat act gcc aaa gca ctg gca cat ttc atg ggt gtt ccg gta gcg      336
   Leu Asp Thr Ala Lys Ala Leu Ala His Phe Met Gly Val Pro Val Ala
               100                 105                 110
   atc gca ccg act atc gcc tct acc gat gca ccg tgc agc gca ttg tct      384
   Ile Ala Pro Thr Ile Ala Ser Thr Asp Ala Pro Cys Ser Ala Leu Ser
           115                 120                 125
   gtt atc tac acc gat gag ggt gag ttt gac cgc tat ctg ctg ttg cca      432
   Val Ile Tyr Thr Asp Glu Gly Glu Phe Asp Arg Tyr Leu Leu Leu Pro
       130                 135                 140
   aat aac ccg aat atg gtc att gtc gac acc aaa atc gtc gct ggc gca      480
   Asn Asn Pro Asn Met Val Ile Val Asp Thr Lys Ile Val Ala Gly Ala
   145                 150                 155                 160
   cct gca cgt ctg tta gcg gcg ggt atc ggc gat gcg ctg gca acc tgg      528
   Pro Ala Arg Leu Leu Ala Ala Gly Ile Gly Asp Ala Leu Ala Thr Trp
                   165                 170                 175
   ttt gaa gcg cgt gcc tgc tct cgt agc ggc gcg acc acc atg gcg ggc      576
   Phe Glu Ala Arg Ala Cys Ser Arg Ser Gly Ala Thr Thr Met Ala Gly
               180                 185                 190
   ggc aag tgc acc cag gct gcg ctg gca ctg gct gaa ctg tgc tac aac      624
   Gly Lys Cys Thr Gln Ala Ala Leu Ala Leu Ala Glu Leu Cys Tyr Asn
           195                 200                 205
   acc ctg ctg gaa gaa ggc gaa aaa gcg atg ctt gct gcc gaa cag cat      672
   Thr Leu Leu Glu Glu Gly Glu Lys Ala Met Leu Ala Ala Glu Gln His
       210                 215                 220
   gta gtg act ccg gcg ctg gag cgc gtg att gaa gcg aac acc tat ttg      720
   Val Val Thr Pro Ala Leu Glu Arg Val Ile Glu Ala Asn Thr Tyr Leu
   225                 230                 235                 240
   agc ggt gtt ggt ttt gaa agt ggt ggt ctg gct gcg gcg cac gca gtg      768
   Ser Gly Val Gly Phe Glu Ser Gly Gly Leu Ala Ala Ala His Ala Val
                   245                 250                 255
   cat aac ggc ctg acc gct atc ccg gac gcg cat cac tat tat cac ggt      816
   His Asn Gly Leu Thr Ala Ile Pro Asp Ala His His Tyr Tyr His Gly
               260                 265                 270
   gaa aaa gtg gca ttc ggt acg ctg acg cag ctg gtt ctg gaa aat gcg      864
   Glu Lys Val Ala Phe Gly Thr Leu Thr Gln Leu Val Leu Glu Asn Ala
           275                 280                 285
   ccg gtg gag gaa atc gaa acc gta gct gcc ctt agc cat gcg gta ggt      912
   Pro Val Glu Glu Ile Glu Thr Val Ala Ala Leu Ser His Ala Val Gly
       290                 295                 300
   ttg cca ata act ctc gct caa ctg gat att aaa gaa gat gtc ccg gcg      960
   Leu Pro Ile Thr Leu Ala Gln Leu Asp Ile Lys Glu Asp Val Pro Ala
   305                 310                 315                 320
   aaa atg cga att gtg gca gaa gcg gca tgt gca gaa ggt gaa acc att     1008
   Lys Met Arg Ile Val Ala Glu Ala Ala Cys Ala Glu Gly Glu Thr Ile
                   325                 330                 335
   cac aac atg cct ggc ggc gcg acg cca gat cag gtt tac gcc gct ctg     1056
   His Asn Met Pro Gly Gly Ala Thr Pro Asp Gln Val Tyr Ala Ala Leu
               340                 345                 350
   ctg gta gcc gac cag tac ggt cag cgt ttc ctg caa gag tgg gaa taa     1104
   Leu Val Ala Asp Gln Tyr Gly Gln Arg Phe Leu Gln Glu Trp Glu
           355                 360                 365
<210> 2
   <211> 367
   <212> PRT
   <213> Escherichia coli
<400> 2
   Met Asp Arg Ile Ile Gln Ser Pro Gly Lys Tyr Ile Gln Gly Ala Asp
   1               5                   10                  15
   Val Ile Asn Arg Leu Gly Glu Tyr Leu Lys Pro Leu Ala Glu Arg Trp
               20                  25                  30
   Leu Val Val Gly Asp Lys Phe Val Leu Gly Phe Ala Gln Ser Thr Val
           35                  40                  45
   Glu Lys Ser Phe Lys Asp Ala Gly Leu Val Val Glu Ile Ala Pro Phe
       50                  55                  60
   Gly Gly Glu Cys Ser Gln Asn Glu Ile Asp Arg Leu Arg Gly Ile Ala
   65                  70                  75                  80
   Glu Thr Ala Gln Cys Gly Ala Ile Leu Gly Ile Gly Gly Gly Lys Thr
                   85                  90                  95
   Leu Asp Thr Ala Lys Ala Leu Ala His Phe Met Gly Val Pro Val Ala
               100                 105                 110
   Ile Ala Pro Thr Ile Ala Ser Thr Asp Ala Pro Cys Ser Ala Leu Ser
           115                 120                 125
   Val Ile Tyr Thr Asp Glu Gly Glu Phe Asp Arg Tyr Leu Leu Leu Pro
       130                 135                 140
   Asn Asn Pro Asn Met Val Ile Val Asp Thr Lys Ile Val Ala Gly Ala
   145                 150                 155                 160
   Pro Ala Arg Leu Leu Ala Ala Gly Ile Gly Asp Ala Leu Ala Thr Trp
                   165                 170                 175
   Phe Glu Ala Arg Ala Cys Ser Arg Ser Gly Ala Thr Thr Met Ala Gly
               180                 185                 190
   Gly Lys Cys Thr Gln Ala Ala Leu Ala Leu Ala Glu Leu Cys Tyr Asn
           195                 200                 205
   Thr Leu Leu Glu Glu Gly Glu Lys Ala Met Leu Ala Ala Glu Gln His
       210                 215                 220
   Val Val Thr Pro Ala Leu Glu Arg Val Ile Glu Ala Asn Thr Tyr Leu
   225                 230                 235                 240
   Ser Gly Val Gly Phe Glu Ser Gly Gly Leu Ala Ala Ala His Ala Val
                   245                 250                 255
   His Asn Gly Leu Thr Ala Ile Pro Asp Ala His His Tyr Tyr His Gly
               260                 265                 270
   Glu Lys Val Ala Phe Gly Thr Leu Thr Gln Leu Val Leu Glu Asn Ala
           275                 280                 285
   Pro Val Glu Glu Ile Glu Thr Val Ala Ala Leu Ser His Ala Val Gly
       290                 295                 300
   Leu Pro Ile Thr Leu Ala Gln Leu Asp Ile Lys Glu Asp Val Pro Ala
   305                 310                 315                 320
   Lys Met Arg Ile Val Ala Glu Ala Ala Cys Ala Glu Gly Glu Thr Ile
                   325                 330                 335
   His Asn Met Pro Gly Gly Ala Thr Pro Asp Gln Val Tyr Ala Ala Leu
               340                 345                 350
   Leu Val Ala Asp Gln Tyr Gly Gln Arg Phe Leu Gln Glu Trp Glu
           355                 360                 365
<210> 3
   <211> 1755
   <212> DNA
   <213> Saccharomyces cerevisiae
<220>
   <221> CDS
   <222> (1).. (1755)
<400> 3
   atg tcc gct aaa tcg ttt gaa gtc aca gat cca gtc aat tca agt ctc      48
   Met Ser Ala Lys Ser Phe Glu Val Thr Asp Pro Val Asn Ser Ser Leu
   1               5                   10                  15
   aaa ggg ttt gcc ctt gct aac ccc tcc att acg ctg gtc cct gaa gaa      96
   Lys Gly Phe Ala Leu Ala Asn Pro Ser Ile Thr Leu Val Pro Glu Glu
               20                  25                  30
   aaa att ctc ttc aga aag acc gat tcc gac aag atc gca tta att tct     144
   Lys Ile Leu Phe Arg Lys Thr Asp Ser Asp Lys Ile Ala Leu Ile Ser
           35                  40                  45
   ggt ggt ggt agt gga cat gaa cct aca cac gcc ggt ttc att ggt aag     192
   Gly Gly Gly Ser Gly His Glu Pro Thr His Ala Gly Phe Ile Gly Lys
       50                  55                  60
   ggt atg ttg agt ggc gcc gtg gtt ggc gaa att ttt gca tcc cct tca     240
   Gly Met Leu Ser Gly Ala Val Val Gly Glu Ile Phe Ala Ser Pro Ser
   65                  70                  75                  80
   aca aaa cag att tta aat gca atc cgt tta gtc aat gaa aat gcg tct     288
   Thr Lys Gln Ile Leu Asn Ala Ile Arg Leu Val Asn Glu Asn Ala Ser
                   85                  90                  95
   ggc gtt tta ttg att gtg aag aac tac aca ggt gat gtt ttg cat ttt     336
   Gly Val Leu Leu Ile Val Lys Asn Tyr Thr Gly Asp Val Leu His Phe
               100                 105                 110
   ggt ctg tcc gct gag aga gca aga gcc ttg ggt att aac tgc cgc gtt     384
   Gly Leu Ser Ala Glu Arg Ala Arg Ala Leu Gly Ile Asn Cys Arg Val
           115                 120                 125
   gct gtc ata ggt gat gat gtt gca gtt ggc aga gaa aag ggt ggt atg     432
   Ala Val Ile Gly Asp Asp Val Ala Val Gly Arg Glu Lys Gly Gly Met
       130                 135                 140
   gtt ggt aga aga gca ttg gca ggt acc gtt ttg gtt cat aag att gta     480
   Val Gly Arg Arg Ala Leu Ala Gly Thr Val Leu Val His Lys Ile Val
   145                 150                 155                 160
   ggt gcc ttc gca gaa gaa tat tct agt aag tat ggc tta gac ggt aca     528
   Gly Ala Phe Ala Glu Glu Tyr Ser Ser Lys Tyr Gly Leu Asp Gly Thr
                   165                 170                 175
   gct aaa gtg gct aaa att atc aac gac aat ttg gtg acc att gga tct     576
   Ala Lys Val Ala Lys Ile Ile Asn Asp Asn Leu Val Thr Ile Gly Ser
               180                 185                 190
   tct tta gac cat tgt aaa gtt cct ggc agg aaa ttc gaa agt gaa tta     624
   Ser Leu Asp His Cys Lys Val Pro Gly Arg Lys Phe Glu Ser Glu Leu
           195                 200                 205
   aac gaa aaa caa atg gaa ttg ggt atg ggt att cat aac gaa cct ggt     672
   Asn Glu Lys Gln Met Glu Leu Gly Met Gly Ile His Asn Glu Pro Gly
       210                 215                 220
   gtg aaa gtt tta gac cct att cct tct acc gaa gac ttg atc tcc aag     720
   Val Lys Val Leu Asp Pro Ile Pro Ser Thr Glu Asp Leu Ile Ser Lys
   225                 230                 235                 240
   tat atg cta cca aaa cta ttg gat cca aac gat aag gat aga gct ttt     768
   Tyr Met Leu Pro Lys Leu Leu Asp Pro Asn Asp Lys Asp Arg Ala Phe
                   245                 250                 255
   gta aag ttt gat gaa gat gat gaa gtt gtc ttg tta gtt aac aat ctc     816
   Val Lys Phe Asp Glu Asp Asp Glu Val Val Leu Leu Val Asn Asn Leu
               260                 265                 270
   ggc ggt gtt tct aat ttt gtt att agt tct atc act tcc aaa act acg     864
   Gly Gly Val Ser Asn Phe Val Ile Ser Ser Ile Thr Ser Lys Thr Thr
               275                 280                 285
   gat ttc tta aag gaa aat tac aac ata acc ccg gtt caa aca att gct      912
   Asp Phe Leu Lys Glu Asn Tyr Asn Ile Thr Pro Val Gln Thr Ile Ala
           290                 295                 300
   ggc aca ttg atg acc tcc ttc aat ggt aat ggg ttc agt atc aca tta      960
   Gly Thr Leu Met Thr Ser Phe Asn Gly Asn Gly Phe Ser Ile Thr Leu
   305                 310                 315                 320
   cta aac gcc act aag gct aca aag gct ttg caa tct gat ttt gag gag     1008
   Leu Asn Ala Thr Lys Ala Thr Lys Ala Leu Gln Ser Asp Phe Glu Glu
                   325                 330                 335
   atc aaa tca gta cta gac ttg ttg aac gca ttt acg aac gca ccg ggc     1056
   Ile Lys Ser Val Leu Asp Leu Leu Asn Ala Phe Thr Asn Ala Pro Gly
               340                 345                 350
   tgg cca att gca gat ttt gaa aag act tct gcc cca tct gtt aac gat     1104
   Trp Pro Ile Ala Asp Phe Glu Lys Thr Ser Ala Pro Ser Val Asn Asp
           355                 360                 365
   gac ttg tta cat aat gaa gta aca gca aag gcc gtc ggt acc tat gac     1152
   Asp Leu Leu His Asn Glu Val Thr Ala Lys Ala Val Gly Thr Tyr Asp
       370                 375                 380
   ttt gac aag ttt gct gag tgg atg aag agt ggt gct gaa caa gtt atc     1200
   Phe Asp Lys Phe Ala Glu Trp Met Lys Ser Gly Ala Glu Gln Val Ile
   385                 390                 395                 400
   aag agc gaa ccg cac att acg gaa cta gac aat caa gtt ggt gat ggt     1248
   Lys Ser Glu Pro His Ile Thr Glu Leu Asp Asn Gln Val Gly Asp Gly
                   405                 410                 415
   gat tgt ggt tac act tta gtg gca gga gtt aaa ggc atc acc gaa aac     1296
   Asp Cys Gly Tyr Thr Leu Val Ala Gly Val Lys Gly Ile Thr Glu Asn
               420                 425                 430
   ctt gac aag ctg tcg aag gac tca tta tct cag gcg gtt gcc caa att     1344
   Leu Asp Lys Leu Ser Lys Asp Ser Leu Ser Gln Ala Val Ala Gln Ile
           435                 440                 445
   tca gat ttc att gaa ggc tca atg gga ggt act tct ggt ggt tta tat     1392
   Ser Asp Phe Ile Glu Gly Ser Met Gly Gly Thr Ser Gly Gly Leu Tyr
       450                 455                 460
   tct att ctt ttg tcg ggt ttt tca cac gga tta att cag gtt tgt aaa     1440
   Ser Ile Leu Leu Ser Gly Phe Ser His Gly Leu Ile Gln Val Cys Lys
   465                 470                 475                 480
   tca aag gat gaa ccc gtc act aag gaa att gtg gct aag tca ctc gga     1488
   Ser Lys Asp Glu Pro Val Thr Lys Glu Ile Val Ala Lys Ser Leu Gly
                   485                 490                 495
   att gca ttg gat act tta tac aaa tat aca aag gca agg aag gga tca     1536
   Ile Ala Leu Asp Thr Leu Tyr Lys Tyr Thr Lys Ala Arg Lys Gly Ser
               500                 505                 510
   tcc acc atg att gat gct tta gaa cca ttc gtt aaa gaa ttt act gca     1584
   Ser Thr Met Ile Asp Ala Leu Glu Pro Phe Val Lys Glu Phe Thr Ala
           515                 520                 525
   tct aag gat ttc aat aag gcg gta aaa gct gca gag gaa ggt gct aaa     1632
   Ser Lys Asp Phe Asn Lys Ala Val Lys Ala Ala Glu Glu Gly Ala Lys
       530                 535                 540
   tcc act gct aca ttc gag gcc aaa ttt ggc aga gct tcg tat gtc ggc     1680
   Ser Thr Ala Thr Phe Glu Ala Lys Phe Gly Arg Ala Ser Tyr Val Gly
   545                 550                 555                 560
   gat tca tct caa gta gaa gat cct ggt gca gta ggc cta tgt gag ttt     1728
   Asp Ser Ser Gln Val Glu Asp Pro Gly Ala Val Gly Leu Cys Glu Phe
                   565                 570                 575
   ttg aag ggg gtt caa agc gcc ttg taa 1755
   Leu Lys Gly Val Gln Ser Ala Leu
   580
<210> 4
   <211> 584
   <212> PRT
   <213> Saccharomyces cerevisiae
<400> 4
   Met Ser Ala Lys Ser Phe Glu Val Thr Asp Pro Val Asn Ser Ser Leu
   1 5 10 15
   Lys Gly Phe Ala Leu Ala Asn Pro Ser Ile Thr Leu Val Pro Glu Glu
   20 25 30
   Lys Ile Leu Phe Arg Lys Thr Asp Ser Asp Lys Ile Ala Leu Ile Ser
   35 40 45
   Gly Gly Gly Ser Gly His Glu Pro Thr His Ala Gly Phe Ile Gly Lys
   50 55 60
   Gly Met Leu Ser Gly Ala Val Val Gly Glu Ile Phe Ala Ser Pro Ser
   65 70 75 80
   Thr Lys Gln Ile Leu Asn Ala Ile Arg Leu Val Asn Glu Asn Ala Ser
   85 90 95
   Gly Val Leu Leu Ile Val Lys Asn Tyr Thr Gly Asp Val Leu His Phe
   100 105 110
   Gly Leu Ser Ala Glu Arg Ala Arg Ala Leu Gly Ile Asn Cys Arg Val
   115 120 125
   Ala Val Ile Gly Asp Asp Val Ala Val Gly Arg Glu Lys Gly Gly Met
   130 135 140
   Val Gly Arg Arg Ala Leu Ala Gly Thr Val Leu Val His Lys Ile Val
   145 150 155 160
   Gly Ala Phe Ala Glu Glu Tyr Ser Ser Lys Tyr Gly Leu Asp Gly Thr
   165 170 175
   Ala Lys Val Ala Lys Ile Ile Asn Asp Asn Leu Val Thr Ile Gly Ser
   180 185 190
   Ser Leu Asp His Cys Lys Val Pro Gly Arg Lys Phe Glu Ser Glu Leu
   195 200 205
   Asn Glu Lys Gln Met Glu Leu Gly Met Gly Ile His Asn Glu Pro Gly
   210 215 220
   Val Lys Val Leu Asp Pro Ile Pro Ser Thr Glu Asp Leu Ile Ser Lys
   225 230 235 240
   Tyr Met Leu Pro Lys Leu Leu Asp Pro Asn Asp Lys Asp Arg Ala Phe
   245 250 255
   Val Lys Phe Asp Glu Asp Asp Glu Val Val Leu Leu Val Asn Asn Leu
   260 265 270
   Gly Gly Val Ser Asn Phe Val Ile Ser Ser Ile Thr Ser Lys Thr Thr
   275 280 285
   Asp Phe Leu Lys Glu Asn Tyr Asn Ile Thr Pro Val Gln Thr Ile Ala
   290 295 300
   Gly Thr Leu Met Thr Ser Phe Asn Gly Asn Gly Phe Ser Ile Thr Leu
   305 310 315 320
   Leu Asn Ala Thr Lys Ala Thr Lys Ala Leu Gln Ser Asp Phe Glu Glu
   325 330 335
   Ile Lys Ser Val Leu Asp Leu Leu Asn Ala Phe Thr Asn Ala Pro Gly
   340 345 350
   Trp Pro Ile Ala Asp Phe Glu Lys Thr Ser Ala Pro Ser Val Asn Asp
   355 360 365
   Asp Leu Leu His Asn Glu Val Thr Ala Lys Ala Val Gly Thr Tyr Asp
   370 375 380
   Phe Asp Lys Phe Ala Glu Trp Met Lys Ser Gly Ala Glu Gln Val Ile
   385 390 395 400
   Lys Ser Glu Pro His Ile Thr Glu Leu Asp Asn Gln Val Gly Asp Gly
   405 410 415
   Asp Cys Gly Tyr Thr Leu Val Ala Gly Val Lys Gly Ile Thr Glu Asn
   420 425 430
   Leu Asp Lys Leu Ser Lys Asp Ser Leu Ser Gln Ala Val Ala Gln Ile
   435 440 445
   Ser Asp Phe Ile Glu Gly Ser Met Gly Gly Thr Ser Gly Gly Leu Tyr
   450 455 460
   Ser Ile Leu Leu Ser Gly Phe Ser His Gly Leu Ile Gln Val Cys Lys
   465 470 475 480
   Ser Lys Asp Glu Pro Val Thr Lys Glu Ile Val Ala Lys Ser Leu Gly
   485 490 495
   Ile Ala Leu Asp Thr Leu Tyr Lys Tyr Thr Lys Ala Arg Lys Gly Ser
   500 505 510
   Ser Thr Met Ile Asp Ala Leu Glu Pro Phe Val Lys Glu Phe Thr Ala
   515 520 525
   Ser Lys Asp Phe Asn Lys Ala Val Lys Ala Ala Glu Glu Gly Ala Lys
   530 535 540
   Ser Thr Ala Thr Phe Glu Ala Lys Phe Gly Arg Ala Ser Tyr Val Gly
   545 550 555 560
   Asp Ser Ser Gln Val Glu Asp Pro Gly Ala Val Gly Leu Cys Glu Phe
   565 570 575
   Leu Lys Gly Val Gln Ser Ala Leu
   580
<210> 5
   <211> 1695
   <212> DNA
   <213> Agrobacterium tumefaciens
<220>
   <221> CDS
   <222> (1).. (1695)
<400> 5
   atg aag aag ctc atc aac gat cca tcc acc gtt gtc cgg gac atg ctg 48
   Met Lys Lys Leu Ile Asn Asp Pro Ser Thr Val Val Arg Asp Met Leu
   1 5 10 15
   gag ggc atc gtg gcg ctc agc ccc gaa acc att ctg ctt cag gat gag 96
   Glu Gly Ile Val Ala Leu Ser Pro Glu Thr Ile Leu Leu Gln Asp Glu
   20 25 30
   aac gtg gtc ata agg tcc ggc ctg cct gaa gcg gaa aag cgc aag gtt 144
   Asn Val Val Ile Arg Ser Gly Leu Pro Glu Ala Glu Lys Arg Lys Val
   35 40 45
   gca gtg ctt tcg ggc ggt ggc agc gga cat gag ccg gcc cac gcc ggt 192
   Ala Val Leu Ser Gly Gly Gly Ser Gly His Glu Pro Ala His Ala Gly
   50 55 60
   tat gtc ggc acg ggc atg ttg acg gtt gcg gtg gcg ggc gat gtc ttc 240
   Tyr Val Gly Thr Gly Met Leu Thr Val Ala Val Ala Gly Asp Val Phe
   65                  70                  75                  80
   act tcg ccg agc acc gac gcg gtt ctc gcc ggc atc agg gcc gcg gcc      288
   Thr Ser Pro Ser Thr Asp Ala Val Leu Ala Gly Ile Arg Ala Ala Ala
                   85                  90                  95
   ggc cct gcc ggt gcg ctg gtc atc gtc aag aac tat acc ggc gac cgg      336
   Gly Pro Ala Gly Ala Leu Val Ile Val Lys Asn Tyr Thr Gly Asp Arg
               100                 105                 110
   ctg aat ttc ggc ttg gcg gcg gag ctg gcg agg gcc gaa gga atc cct      384
   Leu Asn Phe Gly Leu Ala Ala Glu Leu Ala Arg Ala Glu Gly Ile Pro
           115                 120                 125
   gtc gag atc gtt gtc gtc gcc gac gac gtt gcc ttg aag gat acg gtt      432
   Val Glu Ile Val Val Val Ala Asp Asp Val Ala Leu Lys Asp Thr Val
       130                 135                 140
   ccc gcc gag cgc cgc cgc ggg att gcg ggg acg gtg ctc gtg cac aag      480
   Pro Ala Glu Arg Arg Arg Gly Ile Ala Gly Thr Val Leu Val His Lys
   145                 150                 155                 160
   ctc gcg ggg gcc gca gcg gaa aag ggc ctt cct ctt caa gag gtg gcc      528
   Leu Ala Gly Ala Ala Ala Glu Lys Gly Leu Pro Leu Gln Glu Val Ala
                   165                 170                 175
   cgc atc gcc cgg gac gct gcc gcc aag cta tct tcc atg ggt gtc tcg      576
   Arg Ile Ala Arg Asp Ala Ala Ala Lys Leu Ser Ser Met Gly Val Ser
               180                 185                 190
   ctg gga tcc tgc acg ctg ccg gct gtc ggc aag ccg ggc ttc gtg ctt      624
   Leu Gly Ser Cys Thr Leu Pro Ala Val Gly Lys Pro Gly Phe Val Leu
           195                 200                 205
   ggc gag acc gaa atc gaa gtc ggg ctc ggt atc cat ggc gag cag ggc      672
   Gly Glu Thr Glu Ile Glu Val Gly Leu Gly Ile His Gly Glu Gln Gly
       210                 215                 220
   gtg cag cgg atg ccc att gcc tcg gct gac gcg ctc gtg cag ctg gtg      720
   Val Gln Arg Met Pro Ile Ala Ser Ala Asp Ala Leu Val Gln Leu Val
   225                 230                 235                 240
   atc gaa acg atc gaa gcc gac ggc aag ctc gcc ggc ggc aat cgc gtc      768
   Ile Glu Thr Ile Glu Ala Asp Gly Lys Leu Ala Gly Gly Asn Arg Val
                   245                 250                 255
   gct ctg ctg gtc aac ggc ctg ggg gca acg ccg ccg atg gaa ctc gcc      816
   Ala Leu Leu Val Asn Gly Leu Gly Ala Thr Pro Pro Met Glu Leu Ala
               260                 265                 270
   atc gtc gca cgg tcg gca gtc gcg cgg ctg gag gcg aaa ggc atc gtc      864
   Ile Val Ala Arg Ser Ala Val Ala Arg Leu Glu Ala Lys Gly Ile Val
           275                 280                 285
   gtg gaa cgt gcc tgg gcc ggc acc ttc ctt tca gcc ctc gat atg ccc      912
   Val Glu Arg Ala Trp Ala Gly Thr Phe Leu Ser Ala Leu Asp Met Pro
       290                 295                 300
   ggg ttt tcg ttg tcg gtc atg cag gtt gac gac gca gcg ctc agc ctc      960
   Gly Phe Ser Leu Ser Val Met Gln Val Asp Asp Ala Ala Leu Ser Leu
   305                 310                 315                 320
   atc gac gcg cca acc gag gct ggc gca tgg ccg cgc ggc ggt gcg gtg     1008
   Ile Asp Ala Pro Thr Glu Ala Gly Ala Trp Pro Arg Gly Gly Ala Val
                   325                 330                 335
   aac cgc aag cgg gtt ctg cct tcg gca aac gcc gaa aag acc gtg gtt     1056
   Asn Arg Lys Arg Val Leu Pro Ser Ala Asn Ala Glu Lys Thr Val Val
               340                 345                 350
   gcg aca aac aag atg acg gcg gcc ggc gag cgg ctt cgt tcg ggc gcg     1104
   Ala Thr Asn Lys Met Thr Ala Ala Gly Glu Arg Leu Arg Ser Gly Ala
           355                 360                 365
   gaa cgg tcc gcg aga gcc ctg atc gct gcg gag ccc agg ctg acg caa      1152
   Glu Arg Ser Ala Arg Ala Leu Ile Ala Ala Glu Pro Arg Leu Thr Gln
       370                 375                 380
   ctc gat agc gtt gca ggt gac ggc gac ctc ggc gcc agc atg gtg cgt      1200
   Leu Asp Ser Val Ala Gly Asp Gly Asp Leu Gly Ala Ser Met Val Arg
   385                 390                 395                 400
   ggc ggc gag gcg atc ctt gcg ctg ccg aaa gag agt ttc ggc gac gtc      1248
   Gly Gly Glu Ala Ile Leu Ala Leu Pro Lys Glu Ser Phe Gly Asp Val
                   405                 410                 415
   tcc gat gga ttg atg gcg atg gcc aat gcg atg cgc aag gcc atc ggc      1296
   Ser Asp Gly Leu Met Ala Met Ala Asn Ala Met Arg Lys Ala Ile Gly
               420                 425                 430
   gga agc tcg ggg ccg ttc tat gcg aca ggc ctc atg cgc gct tcg cga      1344
   Gly Ser Ser Gly Pro Phe Tyr Ala Thr Gly Leu Met Arg Ala Ser Arg
           435                 440                 445
   cag ctg gca ggg atc gat gag cca gcg gcc cag cag atg gcg gaa gca      1392
   Gln Leu Ala Gly Ile Asp Glu Pro Ala Ala Gln Gln Met Ala Glu Ala
       450                 455                 460
   ttc gtg gcg gct gtt gcg gcg gtc tcg gaa ctt ggc ggt gcg aaa ccg      1440
   Phe Val Ala Ala Val Ala Ala Val Ser Glu Leu Gly Gly Ala Lys Pro
   465                 470                 475                 480
   ggc gat cgc acg atg atc gat gcg ctt tat ccg gca gcg aaa acc ttc      1488
   Gly Asp Arg Thr Met Ile Asp Ala Leu Tyr Pro Ala Ala Lys Thr Phe
                   485                 490                 495
   agg gac aag ctt gtg aca ggc gct tcg gca gaa gaa gcc tgg caa tcc      1536
   Arg Asp Lys Leu Val Thr Gly Ala Ser Ala Glu Glu Ala Trp Gln Ser
               500                 505                 510
   gcg gtg gcg gcg ggc gag gtg ggc gca gag gcg aca gcg tcc atg aag      1584
   Ala Val Ala Ala Gly Glu Val Gly Ala Glu Ala Thr Ala Ser Met Lys
           515                 520                 525
   cca cgg ctc ggg cgc gca agt tac ctc ggt gag cgg gcc gtc ggc cat      1632
   Pro Arg Leu Gly Arg Ala Ser Tyr Leu Gly Glu Arg Ala Val Gly His
       530                 535                 540
   ccc gat ggg ggc gcg gtc gcc gtt ggc atc tgg ctc aaa gct atc gag      1680
   Pro Asp Gly Gly Ala Val Ala Val Gly Ile Trp Leu Lys Ala Ile Glu
   545                 550                 555                 560
   gct gcg atc tcg tga                                                  1695
   Ala Ala Ile Ser
<210> 6
   <211> 564
   <212> PRT
   <213> Agrobacterium tumefaciens
<400> 6
   Met Lys Lys Leu Ile Asn Asp Pro Ser Thr Val Val Arg Asp Met Leu
   1               5                   10                  15
   Glu Gly Ile Val Ala Leu Ser Pro Glu Thr Ile Leu Leu Gln Asp Glu
               20                  25                  30
   Asn Val Val Ile Arg Ser Gly Leu Pro Glu Ala Glu Lys Arg Lys Val
           35                  40                  45
   Ala Val Leu Ser Gly Gly Gly Ser Gly His Glu Pro Ala His Ala Gly
           50                  55                  60
   Tyr Val Gly Thr Gly Met Leu Thr Val Ala Val Ala Gly Asp Val Phe
   65                  70                  75                  80
   Thr Ser Pro Ser Thr Asp Ala Val Leu Ala Gly Ile Arg Ala Ala Ala
                   85                  90                  95
   Gly Pro Ala Gly Ala Leu Val Ile Val Lys Asn Tyr Thr Gly Asp Arg
               100                 105                 110
   Leu Asn Phe Gly Leu Ala Ala Glu Leu Ala Arg Ala Glu Gly Ile Pro
           115                 120                 125
   Val Glu Ile Val Val Val Ala Asp Asp Val Ala Leu Lys Asp Thr Val
       130                 135                 140
   Pro Ala Glu Arg Arg Arg Gly Ile Ala Gly Thr Val Leu Val His Lys
   145                 150                 155                 160
   Leu Ala Gly Ala Ala Ala Glu Lys Gly Leu Pro Leu Gln Glu Val Ala
                   165                 170                 175
   Arg Ile Ala Arg Asp Ala Ala Ala Lys Leu Ser Ser Met Gly Val Ser
               180                 185                 190
   Leu Gly Ser Cys Thr Leu Pro Ala Val Gly Lys Pro Gly Phe Val Leu
           195                 200                 205
   Gly Glu Thr Glu Ile Glu Val Gly Leu Gly Ile His Gly Glu Gln Gly
       210                 215                 220
   Val Gln Arg Met Pro Ile Ala Ser Ala Asp Ala Leu Val Gln Leu Val
   225                 230                 235                 240
   Ile Glu Thr Ile Glu Ala Asp Gly Lys Leu Ala Gly Gly Asn Arg Val
                   245                 250                 255
   Ala Leu Leu Val Asn Gly Leu Gly Ala Thr Pro Pro Met Glu Leu Ala
               260                 265                 270
   Ile Val Ala Arg Ser Ala Val Ala Arg Leu Glu Ala Lys Gly Ile Val
           275                 280                 285
   Val Glu Arg Ala Trp Ala Gly Thr Phe Leu Ser Ala Leu Asp Met Pro
       290                 295                 300
   Gly Phe Ser Leu Ser Val Met Gln Val Asp Asp Ala Ala Leu Ser Leu
   305                 310                 315                 320
   Ile Asp Ala Pro Thr Glu Ala Gly Ala Trp Pro Arg Gly Gly Ala Val
                   325                 330                 335
   Asn Arg Lys Arg Val Leu Pro Ser Ala Asn Ala Glu Lys Thr Val Val
               340                 345                 350
   Ala Thr Asn Lys Met Thr Ala Ala Gly Glu Arg Leu Arg Ser Gly Ala
           355                 360                 365
   Glu Arg Ser Ala Arg Ala Leu Ile Ala Ala Glu Pro Arg Leu Thr Gln
       370                 375                 380
   Leu Asp Ser Val Ala Gly Asp Gly Asp Leu Gly Ala Ser Met Val Arg
   385                 390                 395                 400
   Gly Gly Glu Ala Ile Leu Ala Leu Pro Lys Glu Ser Phe Gly Asp Val
                   405                 410                 415
   Ser Asp Gly Leu Met Ala Met Ala Asn Ala Met Arg Lys Ala Ile Gly
               420                 425                 430
   Gly Ser Ser Gly Pro Phe Tyr Ala Thr Gly Leu Met Arg Ala Ser Arg
           435                 440                 445
   Gln Leu Ala Gly Ile Asp Glu Pro Ala Ala Gln Gln Met Ala Glu Ala
       450                 455                 460
   Phe Val Ala Ala Val Ala Ala Val Ser Glu Leu Gly Gly Ala Lys Pro
   465                 470                 475                 480
   Gly Asp Arg Thr Met Ile Asp Ala Leu Tyr Pro Ala Ala Lys Thr Phe
                   485                 490                 495
   Arg Asp Lys Leu Val Thr Gly Ala Ser Ala Glu Glu Ala Trp Gln Ser
               500                 505                 510
   Ala Val Ala Ala Gly Glu Val Gly Ala Glu Ala Thr Ala Ser Met Lys
           515                 520                 525
   Pro Arg Leu Gly Arg Ala Ser Tyr Leu Gly Glu Arg Ala Val Gly His
       530                 535                 540
   Pro Asp Gly Gly Ala Val Ala Val Gly Ile Trp Leu Lys Ala Ile Glu
   545                 550                 555                 560
   Ala Ala Ile Ser
<210> 7
   <211> 1659
   <212> DNA
   <213> Citrobacter freundii
<220>
   <221> CDS
   <222> (1).. (1659)
<400> 7
   atg tct caa ttc ttt ttt aac caa cgc acc cat ctt gtg agc gac gtc       48
   Met Ser Gln Phe Phe Phe Asn Gln Arg Thr His Leu Val Ser Asp Val
   1               5                   10                  15
   atc gac ggg gcg att atc gcc agc cca tgg aat aac ctg gcg cgt ctg       96
   Ile Asp Gly Ala Ile Ile Ala Ser Pro Trp Asn Asn Leu Ala Arg Leu
               20                  25                  30
   gaa agc gat ccg gcc att cgc atc gtg gtc cgt cgt gac ctt aat aaa      144
   Glu Ser Asp Pro Ala Ile Arg Ile Val Val Arg Arg Asp Leu Asn Lys
           35                  40                  45
   aat aac gta gcg gtc att tcc ggc ggc ggt tcg gga cac gaa ccc gcg      192
   Asn Asn Val Ala Val Ile Ser Gly Gly Gly Ser Gly His Glu Pro Ala
       50                  55                  60
   cac gtt ggg ttt atc ggt aaa ggc atg cta acc gct gcg gtc tgc ggc      240
   His Val Gly Phe Ile Gly Lys Gly Met Leu Thr Ala Ala Val Cys Gly
   65                  70                  75                  80
   gac gtt ttc gcc tcc ccg agc gtg gat gct gta ctg acc gcg att cag      288
   Asp Val Phe Ala Ser Pro Ser Val Asp Ala Val Leu Thr Ala Ile Gln
                   85                  90                  95
   gcg gtg acc ggt gag gct ggc tgt ttg ttg att gtg aaa aac tac acc      336
   Ala Val Thr Gly Glu Ala Gly Cys Leu Leu Ile Val Lys Asn Tyr Thr
               100                 105                 110
   ggt gac cgt ctt aat ttc ggt ctc gcc gcc gag aag gcg cgt cgc ctt      384
   Gly Asp Arg Leu Asn Phe Gly Leu Ala Ala Glu Lys Ala Arg Arg Leu
           115                 120                 125
   ggc tat aac gtt gaa atg ctg att gtc ggc gac gac atc tcc ctg ccg      432
   Gly Tyr Asn Val Glu Met Leu Ile Val Gly Asp Asp Ile Ser Leu Pro
       130                 135                 140
   gat aac aaa cac cca cgt ggc att gcg gga act atc ctg gtg cat aaa      480
   Asp Asn Lys His Pro Arg Gly Ile Ala Gly Thr Ile Leu Val His Lys
   145                 150                 155                 160
   atc gca ggc tat ttt gcc gaa cgc ggc tat aac ctc gcc acc gtc ctg      528
   Ile Ala Gly Tyr Phe Ala Glu Arg Gly Tyr Asn Leu Ala Thr Val Leu
                   165                 170                 175
   cgt gaa gcg cag tac gca gcc agc aac acc ttt agc ctg ggc gta gcg      576
   Arg Glu Ala Gln Tyr Ala Ala Ser Asn Thr Phe Ser Leu Gly Val Ala
               180                 185                 190
   ctt tcc agc tgt cat ctg ccg caa gaa acc gac gca gcc cct cgt cat      624
   Leu Ser Ser Cys His Leu Pro Gln Glu Thr Asp Ala Ala Pro Arg His
           195                 200                 205
   cat ccg ggt cat gcg gag ctg ggt atg gga att cac ggc gaa cca ggc      672
   His Pro Gly His Ala Glu Leu Gly Met Gly Ile His Gly Glu Pro Gly
       210                 215                 220
   gca tcg gtt atc gac acc caa aac agt gcg caa gtg gta aac ctg atg      720
   Ala Ser Val Ile Asp Thr Gln Asn Ser Ala Gln Val Val Asn Leu Met
   225                 230                 235                 240
   gtg gat aaa ctg ctg gcc gcc ctg cct gaa acc ggt cgt ctg gcg gtg      768
   Val Asp Lys Leu Leu Ala Ala Leu Pro Glu Thr Gly Arg Leu Ala Val
                   245                 250                 255
   atg att aat aat ctt ggc ggc gtt tcc gtg gcc gaa atg gcc atc atc      816
   Met Ile Asn Asn Leu Gly Gly Val Ser Val Ala Glu Met Ala Ile Ile
               260                 265                 270
   acc cgc gaa ctc gcc agc agc ccg ctg cac tcg cgt atc gac tgg cta      864
   Thr Arg Glu Leu Ala Ser Ser Pro Leu His Ser Arg Ile Asp Trp Leu
           275                 280                 285
   att ggc ccg gcc tcg ctg gtc acc gcg ctg gat atg aaa ggc ttc tca      912
   Ile Gly Pro Ala Ser Leu Val Thr Ala Leu Asp Met Lys Gly Phe Ser
       290                 295                 300
   ctg acg gcc atc gtg ctg gaa gag agc atc gaa aaa gca ctg ctc acc      960
   Leu Thr Ala Ile Val Leu Glu Glu Ser Ile Glu Lys Ala Leu Leu Thr
   305                 310                 315                 320
   gaa gtg gaa acc agc aac tgg ccg acg ccg gtc cca ccg cgt gaa atc     1008
   Glu Val Glu Thr Ser Asn Trp Pro Thr Pro Val Pro Pro Arg Glu Ile
                   325                 330                 335
   acc tgc gta gtg tca tct cac gct agc gcc cgc gtg gaa ttc cag cct     1056
   Thr Cys Val Val Ser Ser His Ala Ser Ala Arg Val Glu Phe Gln Pro
               340                 345                 350
   tcg gca aac gcc ctg gtg gcc ggg att gtg gag ctg gtc acc gca acc     1104
   Ser Ala Asn Ala Leu Val Ala Gly Ile Val Glu Leu Val Thr Ala Thr
           355                 360                 365
   ctt tcc gat ctg gag act cat ctg aat gcg ctg gac gcc aaa gtc ggc     1152
   Leu Ser Asp Leu Glu Thr His Leu Asn Ala Leu Asp Ala Lys Val Gly
       370                 375                 380
   gat ggc gat acc ggt tcg acc ttt gcc gcc gcg gcg cgt gaa att gcc     1200
   Asp Gly Asp Thr Gly Ser Thr Phe Ala Ala Ala Ala Arg Glu Ile Ala
   385                 390                 395                 400
   agc ctg ctg cat cgc cag cag ctg ccg ctg aat aac ctt gcc acg ctg     1248
   Ser Leu Leu His Arg Gln Gln Leu Pro Leu Asn Asn Leu Ala Thr Leu
                   405                 410                 415
   ttc gcg ctg att ggc gaa cgt ctg acc gtg gtg atg ggc ggt tcc agc     1296
   Phe Ala Leu Ile Gly Glu Arg Leu Thr Val Val Met Gly Gly Ser Ser
               420                 425                 430
   ggt gtg ctg atg tca atc ttc ttt acc gcc gcc ggg cag aaa ctg gaa     1344
   Gly Val Leu Met Ser Ile Phe Phe Thr Ala Ala Gly Gln Lys Leu Glu
           435                 440                 445
   cag ggc gct aac gtt gtc gaa gcg cta aat acg ggg ctg gcg cag atg     1392
   Gln Gly Ala Asn Val Val Glu Ala Leu Asn Thr Gly Leu Ala Gln Met
       450                 455                 460
   aag ttc tac ggc ggc gca gac gaa ggc gat cgc acg atg att gat gcg     1440
   Lys Phe Tyr Gly Gly Ala Asp Glu Gly Asp Arg Thr Met Ile Asp Ala
   465                 470                 475                 480
   ctg caa ccg gcc ctg acc tcg ctg ctc gca cag ccg aaa aat ctg cag      1488
   Leu Gln Pro Ala Leu Thr Ser Leu Leu Ala Gln Pro Lys Asn Leu Gln
                   485                 490                 495
   gcc gca ttc gac gcc gcg caa gcg gga gcc gaa cga acc tgt ttg tcg      1536
   Ala Ala Phe Asp Ala Ala Gln Ala Gly Ala Glu Arg Thr Cys Leu Ser
               500                 505                 510
   agc aaa gcc aat gcg ggt cgc gca tcg tat ctg agc agc gaa agc ctg      1584
   Ser Lys Ala Asn Ala Gly Arg Ala Ser Tyr Leu Ser Ser Glu Ser Leu
           515                 520                 525
   ctc gga aat atg gac ccc ggc gcg cag cgc cta gcg atg gtg ttt aaa      1632
   Leu Gly Asn Met Asp Pro Gly Ala Gln Arg Leu Ala Met Val Phe Lys
       530                 535                 540
   gcg cta gcg gag agt gag ctg ggc taa                                  1659
   Ala Leu Ala Glu Ser Glu Leu Gly
   545                 550
<210> 8
   <211> 552
   <212> PRT
   <213> Citrobacter freundii
<400> 8
   Met Ser Gln Phe Phe Phe Asn Gln Arg Thr His Leu Val Ser Asp Val
   1               5                   10                  15
   Ile Asp Gly Ala Ile Ile Ala Ser Pro Trp Asn Asn Leu Ala Arg Leu
               20                  25                  30
   Glu Ser Asp Pro Ala Ile Arg Ile Val Val Arg Arg Asp Leu Asn Lys
           35                  40                  45
   Asn Asn Val Ala Val Ile Ser Gly Gly Gly Ser Gly His Glu Pro Ala
       50                  55                  60
   His Val Gly Phe Ile Gly Lys Gly Met Leu Thr Ala Ala Val Cys Gly
   65                  70                  75                  80
   Asp Val Phe Ala Ser Pro Ser Val Asp Ala Val Leu Thr Ala Ile Gln
                   85                  90                  95
   Ala Val Thr Gly Glu Ala Gly Cys Leu Leu Ile Val Lys Asn Tyr Thr
               100                 105                 110
   Gly Asp Arg Leu Asn Phe Gly Leu Ala Ala Glu Lys Ala Arg Arg Leu
           115                 120                 125
   Gly Tyr Asn Val Glu Met Leu Ile Val Gly Asp Asp Ile Ser Leu Pro
       130                 135                 140
   Asp Asn Lys His Pro Arg Gly Ile Ala Gly Thr Ile Leu Val His Lys
   145                 150                 155                 160
   Ile Ala Gly Tyr Phe Ala Glu Arg Gly Tyr Asn Leu Ala Thr Val Leu
                   165                 170                 175
   Arg Glu Ala Gln Tyr Ala Ala Ser Asn Thr Phe Ser Leu Gly Val Ala
               180                 185                 190
   Leu Ser Ser Cys His Leu Pro Gln Glu Thr Asp Ala Ala Pro Arg His
           195                 200                 205
   His Pro Gly His Ala Glu Leu Gly Met Gly Ile His Gly Glu Pro Gly
       210                 215                 220
   Ala Ser Val Ile Asp Thr Gln Asn Ser Ala Gln Val Val Asn Leu Met
   225                 230                 235                 240
   Val Asp Lys Leu Leu Ala Ala Leu Pro Glu Thr Gly Arg Leu Ala Val
                   245                 250                 255
   Met Ile Asn Asn Leu Gly Gly Val Ser Val Ala Glu Met Ala Ile Ile
               260                 265                 270
   Thr Arg Glu Leu Ala Ser Ser Pro Leu His Ser Arg Ile Asp Trp Leu
           275                 280                 285
   Ile Gly Pro Ala Ser Leu Val Thr Ala Leu Asp Met Lys Gly Phe Ser
       290                 295                 300
   Leu Thr Ala Ile Val Leu Glu Glu Ser Ile Glu Lys Ala Leu Leu Thr
   305                 310                 315                 320
   Glu Val Glu Thr Ser Asn Trp Pro Thr Pro Val Pro Pro Arg Glu Ile
                   325                 330                 335
   Thr Cys Val Val Ser Ser His Ala Ser Ala Arg Val Glu Phe Gln Pro
               340                 345                 350
   Ser Ala Asn Ala Leu Val Ala Gly Ile Val Glu Leu Val Thr Ala Thr
           355                 360                 365
   Leu Ser Asp Leu Glu Thr His Leu Asn Ala Leu Asp Ala Lys Val Gly
       370                 375                 380
   Asp Gly Asp Thr Gly Ser Thr Phe Ala Ala Ala Ala Arg Glu Ile Ala
   385                 390                 395                 400
   Ser Leu Leu His Arg Gln Gln Leu Pro Leu Asn Asn Leu Ala Thr Leu
                   405                 410                 415
   Phe Ala Leu Ile Gly Glu Arg Leu Thr Val Val Met Gly Gly Ser Ser
               420                 425                 430
   Gly Val Leu Met Ser Ile Phe Phe Thr Ala Ala Gly Gln Lys Leu Glu
           435                 440                 445
   Gln Gly Ala Asn Val Val Glu Ala Leu Asn Thr Gly Leu Ala Gln Met
       450                 455                 460
   Lys Phe Tyr Gly Gly Ala Asp Glu Gly Asp Arg Thr Met Ile Asp Ala
   465                 470                 475                 480
   Leu Gln Pro Ala Leu Thr Ser Leu Leu Ala Gln Pro Lys Asn Leu Gln
                   485                 490                 495
   Ala Ala Phe Asp Ala Ala Gln Ala Gly Ala Glu Arg Thr Cys Leu Ser
               500                 505                 510
   Ser Lys Ala Asn Ala Gly Arg Ala Ser Tyr Leu Ser Ser Glu Ser Leu
           515                 520                 525
   Leu Gly Asn Met Asp Pro Gly Ala Gln Arg Leu Ala Met Val Phe Lys
       530                 535                 540
   Ala Leu Ala Glu Ser Glu Leu Gly
   545                 550
<210> 9
   <211> 1740
   <212> DNA
   <213> Artificial sequence
<220>
   <223> attR-cat-attL-PtacM-SD-spacer
<400> 9
   tctagacgct caagttagta taaaaaagct gaacgagaaa cgtaaaatga tataaatatc     60
   aatatattaa attagatttt gcataaaaaa cagactacat aatactgtaa aacacaacat    120
   atgcagtcac tatgaatcaa ctacttagat ggtattagtg acctgtaaca gactgcagtg    180
   gtcgaaaaaa aaagcccgca ctgtcaggtg cgggcttttt tctgtgttaa gcttcgacga    240
   atttctgcca ttcatccgct tattatcact tattcaggcg tagcaccagg cgtttaaggg     300
   caccaataac tgccttaaaa aaattacgcc ccgccctgcc actcatcgca gtactgttgt     360
   aattcattaa gcattctgcc gacatggaag ccatcacaga cggcatgatg aacctgaatc     420
   gccagcggca tcagcacctt gtcgccttgc gtataatatt tgcccatggt gaaaacgggg     480
   gcgaagaagt tgtccatatt ggccacgttt aaatcaaaac tggtgaaact cacccaggga     540
   ttggctgaga cgaaaaacat attctcaata aaccctttag ggaaataggc caggttttca     600
   ccgtaacacg ccacatcttg cgaatatatg tgtagaaact gccggaaatc gtcgtggtat     660
   tcactccaga gcgatgaaaa cgtttcagtt tgctcatgga aaacggtgta acaagggtga     720
   acactatccc atatcaccag ctcaccgtct ttcattgcca tacggaattc cggatgagca     780
   ttcatcaggc gggcaagaat gtgaataaag gccggataaa acttgtgctt atttttcttt     840
   acggtcttta aaaaggccgt aatatccagc tgaacggtct ggttataggt acattgagca     900
   actgactgaa atgcctcaaa atgttcttta cgatgccatt gggatatatc aacggtggta     960
   tatccagtga tttttttctc cattttagct tccttagctc ctgaaaatct cggatccggc    1020
   caagctagct tggctctagc tagagcgccc ggttgacgct gctagtgtta cctagcgatt    1080
   tgtatcttac tgcatgttac ttcatgttgt caatacctgt ttttcgtgcg acttatcagg    1140
   ctgtctactt atccggagat ccacaggacg ggtgtggtcg ccatgatcgc gtagtcgata    1200
   gtggctccaa gtagcgaagc gagcaggact gggcggcggc caaagcggtc ggacagtgct    1260
   ccgagaacgg gtgcgcatag aaattgcatc aacgcatata gcgctagcag cacgccatag    1320
   tgactggcga tgctgtcgga atggacgata tcccgcaaga ggcccggcag taccggcata    1380
   accaagccta tgcctacagc atccagggtg acggtgccga ggatgacgat gagcgcattg    1440
   ttagatttca tacacggtgc ctgactgcgt tagcaattta actgtgataa actaccgcat    1500
   taaagcttat cgatgataag ctgtcaaaca tgagaattcg aaatcaaata atgattttat    1560
   tttgactgat agtgacctgt tcgttgcaac aaattgataa gcaatgcttt tttataatgc    1620
   caacttagta taaaaaagca ggcttcaaga tctctcccca tccccctgtt cacaattaat    1680
   catcggctcg tataatgtgt ggaattgtga gcggataaca atttcacaca ggagactgcc    1740
<210> 10
   <211> 80
   <212> DNA
   <213> Artificial sequence
<220>
   <223> PtacM
<220>
   <221> promoter
   <222> (1).. (80)
   <223> PtacM
<400> 10
   ctccccatcc ccctgttcac aattaatcat cggctcgtat aatgtgtgga attgtgagcg       60
   gataacaatt tcacacagga                                                   80
<210> 11
   <211> 2844
   <212> DNA
   <213> Artificial sequence
<220>
   <223> PtacMg1dA::Cm
<400> 11
   tctagacgct caagttagta taaaaaagct gaacgagaaa cgtaaaatga tataaatatc       60
   aatatattaa attagatttt gcataaaaaa cagactacat aatactgtaa aacacaacat    120
   atgcagtcac tatgaatcaa ctacttagat ggtattagtg acctgtaaca gactgcagtg    180
   gtcgaaaaaa aaagcccgca ctgtcaggtg cgggcttttt tctgtgttaa gcttcgacga    240
   atttctgcca ttcatccgct tattatcact tattcaggcg tagcaccagg cgtttaaggg    300
   caccaataac tgccttaaaa aaattacgcc ccgccctgcc actcatcgca gtactgttgt    360
   aattcattaa gcattctgcc gacatggaag ccatcacaga cggcatgatg aacctgaatc    420
   gccagcggca tcagcacctt gtcgccttgc gtataatatt tgcccatggt gaaaacgggg    480
   gcgaagaagt tgtccatatt ggccacgttt aaatcaaaac tggtgaaact cacccaggga    540
   ttggctgaga cgaaaaacat attctcaata aaccctttag ggaaataggc caggttttca    600
   ccgtaacacg ccacatcttg cgaatatatg tgtagaaact gccggaaatc gtcgtggtat    660
   tcactccaga gcgatgaaaa cgtttcagtt tgctcatgga aaacggtgta acaagggtga    720
   acactatccc atatcaccag ctcaccgtct ttcattgcca tacggaattc cggatgagca    780
   ttcatcaggc gggcaagaat gtgaataaag gccggataaa acttgtgctt atttttcttt    840
   acggtcttta aaaaggccgt aatatccagc tgaacggtct ggttataggt acattgagca    900
   actgactgaa atgcctcaaa atgttcttta cgatgccatt gggatatatc aacggtggta    960
   tatccagtga tttttttctc cattttagct tccttagctc ctgaaaatct cggatccggc   1020
   caagctagct tggctctagc tagagcgccc ggttgacgct gctagtgtta cctagcgatt   1080
   tgtatcttac tgcatgttac ttcatgttgt caatacctgt ttttcgtgcg acttatcagg   1140
   ctgtctactt atccggagat ccacaggacg ggtgtggtcg ccatgatcgc gtagtcgata   1200
   gtggctccaa gtagcgaagc gagcaggact gggcggcggc caaagcggtc ggacagtgct   1260
   ccgagaacgg gtgcgcatag aaattgcatc aacgcatata gcgctagcag cacgccatag   1320
   tgactggcga tgctgtcgga atggacgata tcccgcaaga ggcccggcag taccggcata   1380
   accaagccta tgcctacagc atccagggtg acggtgccga ggatgacgat gagcgcattg   1440
   ttagatttca tacacggtgc ctgactgcgt tagcaattta actgtgataa actaccgcat   1500
   taaagcttat cgatgataag ctgtcaaaca tgagaattcg aaatcaaata atgattttat   1560
   tttgactgat agtgacctgt tcgttgcaac aaattgataa gcaatgcttt tttataatgc   1620
   caacttagta taaaaaagca ggcttcaaga tctctcccca tccccctgtt cacaattaat   1680
   catcggctcg tataatgtgt ggaattgtga gcggataaca atttcacaca ggagactgcc   1740
   atggaccgca ttattcaatc accgggtaaa tacatccagg gcgctgatgt gattaatcgt   1800
   ctgggcgaat acctgaagcc gctggcagaa cgctggttag tggtgggtga caaatttgtt   1860
   ttaggttttg ctcaatccac tgtcgagaaa agctttaaag atgctggact ggtagtagaa   1920
   attgcgccgt ttggcggtga atgttcgcaa aatgagatcg accgtctgcg tggcatcgcg   1980
   gagactgcgc agtgtggcgc aattctcggt atcggtggcg gaaaaaccct cgatactgcc   2040
   aaagcactgg cacatttcat gggtgttccg gtagcgatcg caccgactat cgcctctacc   2100
   gatgcaccgt gcagcgcatt gtctgttatc tacaccgatg agggtgagtt tgaccgctat   2160
   ctgctgttgc caaataaccc gaatatggtc attgtcgaca ccaaaatcgt cgctggcgca   2220
   cctgcacgtc tgttagcggc gggtatcggc gatgcgctgg caacctggtt tgaagcgcgt   2280
   gcctgctctc gtagcggcgc gaccaccatg gcgggcggca agtgcaccca ggctgcgctg   2340
   gcactggctg aactgtgcta caacaccctg ctggaagaag gcgaaaaagc gatgcttgct   2400
   gccgaacagc atgtagtgac tccggcgctg gagcgcgtga ttgaagcgaa cacctatttg   2460
   agcggtgttg gttttgaaag tggtggtctg gctgcggcgc acgcagtgca taacggcctg   2520
   accgctatcc cggacgcgca tcactattat cacggtgaaa aagtggcatt cggtacgctg   2580
   acgcagctgg ttctggaaaa tgcgccggtg gaggaaatcg aaaccgtagc tgcccttagc   2640
   catgcggtag gtttgccaat aactctcgct caactggata ttaaagaaga tgtcccggcg   2700
   aaaatgcgaa ttgtggcaga agcggcatgt gcagaaggtg aaaccattca caacatgcct   2760
   ggcggcgcga cgccagatca ggtttacgcc gctctgctgg tagccgacca gtacggtcag   2820
   cgtttcctgc aagagtggga ataa                                          2844
<210> 12
   <211> 77
   <212> DNA
   <213> Artificial sequence
<220>
   <223> atL-ptac-gldA
<400> 12
   acatcagcgc cctggatgta tttacccggt gattgaataa tgcggtccat ggcagtctcc 60
   tgtgtgaaat tgttatc 77
<210> 13
   <211> 77
   <212> DNA
   <213> Artificial sequence
<220>
   <223> atL-Ptac-fsaB1
<400> 13
   aacgccgcct ctgccgacgc tatcgccagc ctgctgcaac atgaactgga actgtaaatc 60
   tagacgctca agttagt 77
<210> 14
   <211> 50
   <212> DNA
   <213> Artificial sequence
<220>
   <223> pMW-dak1F
<400> 14
   tgattacgcc aagcttagga ggttaaatgt ccgctaaatc gtttgaagtc 50
<210> 15
   <211> 52
   <212> DNA
   <213> Artificial sequence
<220>
   <223> pMW-dak1R
<400> 15
   atcctctaga gtcgacgcgg ccgctactta caaggcgctt tgaaccccct tc 52
<210> 16
   <211> 846
   <212> DNA
   <213> Escherichia coli
<220>
   <221> CDS
   <222> (1)..(846)
<400> 16
   atg agt caa aca tca acc ttg aaa ggc cag tgc att gct gaa ttc ctc 48
   Met Ser Gln Thr Ser Thr Leu Lys Gly Gln Cys Ile Ala Glu Phe Leu
   1               5                   10                  15
   ggt acc ggg ttg ttg att ttc ttc ggt gtg ggt tgc gtt gca gca cta     96
   Gly Thr Gly Leu Leu Ile Phe Phe Gly Val Gly Cys Val Ala Ala Leu
               20                  25                  30
   aaa gtc gct ggt gcg tct ttt ggt cag tgg gaa atc agt gtc att tgg    144
   Lys Val Ala Gly Ala Ser Phe Gly Gln Trp Glu Ile Ser Val Ile Trp
           35                  40                  45
   gga ctg ggg gtg gca atg gcc atc tac ctg acc gca ggg gtt tcc ggc    192
   Gly Leu Gly Val Ala Met Ala Ile Tyr Leu Thr Ala Gly Val Ser Gly
       50                  55                  60
   gcg cat ctt aat ccc gct gtt acc att gca ttg tgg ctg ttt gcc tgt    240
   Ala His Leu Asn Pro Ala Val Thr Ile Ala Leu Trp Leu Phe Ala Cys
   65                  70                  75                  80
   ttc gac aag cgc aaa gtt att cct ttt atc gtt tca caa gtt gcc ggc    288
   Phe Asp Lys Arg Lys Val Ile Pro Phe Ile Val Ser Gln Val Ala Gly
                   85                  90                  95
   gct ttc tgt gct gcg gct tta gtt tac ggg ctt tac tac aat tta ttt    336
   Ala Phe Cys Ala Ala Ala Leu Val Tyr Gly Leu Tyr Tyr Asn Leu Phe
               100                 105                 110
   ttc gac ttc gag cag act cat cac att gtt cgc ggc agc gtt gaa agt    384
   Phe Asp Phe Glu Gln Thr His His Ile Val Arg Gly Ser Val Glu Ser
           115                 120                 125
   gtt gat ctg gct ggc act ttc tct act tac cct aat cct cat atc aat    432
   Val Asp Leu Ala Gly Thr Phe Ser Thr Tyr Pro Asn Pro His Ile Asn
       130                 135                 140
   ttt gtg cag gct ttc gca gtt gag atg gtg att acc gct att ctg atg    480
   Phe Val Gln Ala Phe Ala Val Glu Met Val Ile Thr Ala Ile Leu Met
   145                 150                 155                 160
   ggg ctg atc ctg gcg tta acg gac gat ggc aac ggt gta cca cgc ggc    528
   Gly Leu Ile Leu Ala Leu Thr Asp Asp Gly Asn Gly Val Pro Arg Gly
                   165                 170                 175
   cct ttg gct ccc ttg ctg att ggt cta ctg att gcg gtc att ggc gca    576
   Pro Leu Ala Pro Leu Leu Ile Gly Leu Leu Ile Ala Val Ile Gly Ala
               180                 185                 190
   tct atg ggc cca ttg aca ggt ttt gcc atg aac cca gcg cgt gac ttc    624
   Ser Met Gly Pro Leu Thr Gly Phe Ala Met Asn Pro Ala Arg Asp Phe
           195                 200                 205
   ggt ccg aaa gtc ttt gcc tgg ctg gcg ggc tgg ggc aat gtc gcc ttt    672
   Gly Pro Lys Val Phe Ala Trp Leu Ala Gly Trp Gly Asn Val Ala Phe
       210                 215                 220
   acc ggc ggc aga gac att cct tac ttc ctg gtg ccg ctt ttc ggc cct    720
   Thr Gly Gly Arg Asp Ile Pro Tyr Phe Leu Val Pro Leu Phe Gly Pro
   225                 230                 235                 240
   atc gtt ggc gcg att gta ggt gca ttt gcc tac cgc aaa ctg att ggt    768
   Ile Val Gly Ala Ile Val Gly Ala Phe Ala Tyr Arg Lys Leu Ile Gly
                   245                 250                 255
   cgc cat ttg cct tgc gat atc tgt gtt gtg gaa gaa aag gaa acc aca    816
   Arg His Leu Pro Cys Asp Ile Cys Val Val Glu Glu Lys Glu Thr Thr
               260                 265                 270
   act cct tca gaa caa aaa gct tcg ctg taa                            846
   Thr Pro Ser Glu Gln Lys Ala Ser Leu
           275                 280
<210> 17
   <211> 281
   <212> PRT
   <213> Escherichia coli
<400> 17
   Met Ser Gln Thr Ser Thr Leu Lys Gly Gln Cys Ile Ala Glu Phe Leu
   1               5                   10                  15
   Gly Thr Gly Leu Leu Ile Phe Phe Gly Val Gly Cys Val Ala Ala Leu
               20                  25                  30
   Lys Val Ala Gly Ala Ser Phe Gly Gln Trp Glu Ile Ser Val Ile Trp
           35                  40                  45
   Gly Leu Gly Val Ala Met Ala Ile Tyr Leu Thr Ala Gly Val Ser Gly
       50                  55                  60
   Ala His Leu Asn Pro Ala Val Thr Ile Ala Leu Trp Leu Phe Ala Cys
   65                  70                  75                  80
   Phe Asp Lys Arg Lys Val Ile Pro Phe Ile Val Ser Gln Val Ala Gly
                   85                  90                  95
   Ala Phe Cys Ala Ala Ala Leu Val Tyr Gly Leu Tyr Tyr Asn Leu Phe
               100                 105                 110
   Phe Asp Phe Glu Gln Thr His His Ile Val Arg Gly Ser Val Glu Ser
           115                 120                 125
   Val Asp Leu Ala Gly Thr Phe Ser Thr Tyr Pro Asn Pro His Ile Asn
       130                 135                 140
   Phe Val Gln Ala Phe Ala Val Glu Met Val Ile Thr Ala Ile Leu Met
   145                 150                 155                 160
   Gly Leu Ile Leu Ala Leu Thr Asp Asp Gly Asn Gly Val Pro Arg Gly
                   165                 170                 175
   Pro Leu Ala Pro Leu Leu Ile Gly Leu Leu Ile Ala Val Ile Gly Ala
               180                 185                 190
   Ser Met Gly Pro Leu Thr Gly Phe Ala Met Asn Pro Ala Arg Asp Phe
           195                 200                 205
   Gly Pro Lys Val Phe Ala Trp Leu Ala Gly Trp Gly Asn Val Ala Phe
       210                 215                 220
   Thr Gly Gly Arg Asp Ile Pro Tyr Phe Leu Val Pro Leu Phe Gly Pro
   225                 230                 235                 240
   Ile Val Gly Ala Ile Val Gly Ala Phe Ala Tyr Arg Lys Leu Ile Gly
                   245                 250                 255
   Arg His Leu Pro Cys Asp Ile Cys Val Val Glu Glu Lys Glu Thr Thr
               260                 265                 270
   Thr Pro Ser Glu Gln Lys Ala Ser Leu
           275                 280
<210> 18
   <211> 768
   <212> DNA
   <213> Escherichia coli
<220>
   <221> CDS
   <222> (1).. (768)
<400> 18
   atg cga cat cct tta gtg atg ggt aac tgg aaa ctg aac ggc agc cgc 48
   Met Arg His Pro Leu Val Met Gly Asn Trp Lys Leu Asn Gly Ser Arg
   1               5                   10                  15
   cac atg gtt cac gag ctg gtt tct aac ctg cgt aaa gag ctg gca ggt      96
   His Met Val His Glu Leu Val Ser Asn Leu Arg Lys Glu Leu Ala Gly
               20                  25                  30
   gtt gct ggc tgt gcg gtt gca atc gca cca ccg gaa atg tat atc gat     144
   Val Ala Gly Cys Ala Val Ala Ile Ala Pro Pro Glu Met Tyr Ile Asp
           35                  40                  45
   atg gcg aag cgc gaa gct gaa ggc agc cac atc atg ctg ggt gcg caa     192
   Met Ala Lys Arg Glu Ala Glu Gly Ser His Ile Met Leu Gly Ala Gln
       50                  55                  60
   aac gtg gac ctg aac ctg tcc ggc gca ttc acc ggt gaa acc tct gct     240
   Asn Val Asp Leu Asn Leu Ser Gly Ala Phe Thr Gly Glu Thr Ser Ala
   65                  70                  75                  80
   gct atg ctg aaa gac atc ggc gca cag tac atc atc atc ggt cac tct     288
   Ala Met Leu Lys Asp Ile Gly Ala Gln Tyr Ile Ile Ile Gly His Ser
                   85                  90                  95
   gaa cgt cgt act tac cac aaa gaa tct gac gaa ctg atc gcg aaa aaa     336
   Glu Arg Arg Thr Tyr His Lys Glu Ser Asp Glu Leu Ile Ala Lys Lys
               100                 105                 110
   ttc gcg gtg ctg aaa gag cag ggc ctg act ccg gtt ctg tgc atc ggt     384
   Phe Ala Val Leu Lys Glu Gln Gly Leu Thr Pro Val Leu Cys Ile Gly
           115                 120                 125
   gaa acc gaa gct gaa aat gaa gcg ggc aaa act gaa gaa gtt tgc gca     432
   Glu Thr Glu Ala Glu Asn Glu Ala Gly Lys Thr Glu Glu Val Cys Ala
       130                 135                 140
   cgt cag atc gac gcg gta ctg aaa act cag ggt gct gcg gca ttc gaa     480
   Arg Gln Ile Asp Ala Val Leu Lys Thr Gln Gly Ala Ala Ala Phe Glu
   145                 150                 155                 160
   ggt gcg gtt atc gct tac gaa cct gta tgg gca atc ggt act ggc aaa     528
   Gly Ala Val Ile Ala Tyr Glu Pro Val Trp Ala Ile Gly Thr Gly Lys
                   165                 170                 175
   tct gca act ccg gct cag gca cag gct gtt cac aaa ttc atc cgt gac     576
   Ser Ala Thr Pro Ala Gln Ala Gln Ala Val His Lys Phe Ile Arg Asp
               180                 185                 190
   cac atc gct aaa gtt gac gct aac atc gct gaa caa gtg atc att cag     624
   His Ile Ala Lys Val Asp Ala Asn Ile Ala Glu Gln Val Ile Ile Gln
           195                 200                 205
   tac ggc ggc tct gta aac gcg tct aac gct gca gaa ctg ttt gct cag     672
   Tyr Gly Gly Ser Val Asn Ala Ser Asn Ala Ala Glu Leu Phe Ala Gln
       210                 215                 220
   ccg gat atc gac ggc gcg ctg gtt ggt ggt gct tct ctg aaa gct gac     720
   Pro Asp Ile Asp Gly Ala Leu Val Gly Gly Ala Ser Leu Lys Ala Asp
   225                 230                 235                 240
   gcc ttc gca gta atc gtt aaa gct gca gaa gcg gct aaa cag gct taa     768
   Ala Phe Ala Val Ile Val Lys Ala Ala Glu Ala Ala Lys Gln Ala
                   245                 250                 255
<210> 19
   <211> 255
   <212> PRT
   <213> Escherichia coli
<400> 19
   Met Arg His Pro Leu Val Met Gly Asn Trp Lys Leu Asn Gly Ser Arg
   1               5                   10                  15
   His Met Val His Glu Leu Val Ser Asn Leu Arg Lys Glu Leu Ala Gly
               20                  25                  30
   Val Ala Gly Cys Ala Val Ala Ile Ala Pro Pro Glu Met Tyr Ile Asp
           35                  40                  45
   Met Ala Lys Arg Glu Ala Glu Gly Ser His Ile Met Leu Gly Ala Gln
       50                  55                  60
   Asn Val Asp Leu Asn Leu Ser Gly Ala Phe Thr Gly Glu Thr Ser Ala
   65                  70                  75                  80
   Ala Met Leu Lys Asp Ile Gly Ala Gln Tyr Ile Ile Ile Gly His Ser
                   85                  90                  95
   Glu Arg Arg Thr Tyr His Lys Glu Ser Asp Glu Leu Ile Ala Lys Lys
               100                 105                 110
   Phe Ala Val Leu Lys Glu Gln Gly Leu Thr Pro Val Leu Cys Ile Gly
           115                 120                 125
   Glu Thr Glu Ala Glu Asn Glu Ala Gly Lys Thr Glu Glu Val Cys Ala
       130                 135                 140
   Arg Gln Ile Asp Ala Val Leu Lys Thr Gln Gly Ala Ala Ala Phe Glu
   145                 150                 155                 160
   Gly Ala Val Ile Ala Tyr Glu Pro Val Trp Ala Ile Gly Thr Gly Lys
                   165                 170                 175
   Ser Ala Thr Pro Ala Gln Ala Gln Ala Val His Lys Phe Ile Arg Asp
               180                 185                 190
   His Ile Ala Lys Val Asp Ala Asn Ile Ala Glu Gln Val Ile Ile Gln
           195                 200                 205
   Tyr Gly Gly Ser Val Asn Ala Ser Asn Ala Ala Glu Leu Phe Ala Gln
       210                 215                 220
   Pro Asp Ile Asp Gly Ala Leu Val Gly Gly Ala Ser Leu Lys Ala Asp
   225                 230                 235                 240
   Ala Phe Ala Val Ile Val Lys Ala Ala Glu Ala Ala Lys Gln Ala
                   245                 250                 255
<210> 20
   <211> 1080
   <212> DNA
   <213> Escherichia coli
<220>
   <221> CDS
   <222> (1).. (1080)
<400> 20
   atg tct aag att ttt gat ttc gta aaa cct ggc gta atc act ggt gat      48
   Met Ser Lys Ile Phe Asp Phe Val Lys Pro Gly Val Ile Thr Gly Asp
   1               5                   10                  15
   gac gta cag aaa gtt ttc cag gta gca aaa gaa aac aac ttc gca ctg      96
   Asp Val Gln Lys Val Phe Gln Val Ala Lys Glu Asn Asn Phe Ala Leu
               20                  25                  30
   cca gca gta aac tgc gtc ggt act gac tcc atc aac gcc gta ctg gaa     144
   Pro Ala Val Asn Cys Val Gly Thr Asp Ser Ile Asn Ala Val Leu Glu
           35                  40                  45
   acc gct gct aaa gtt aaa gcg ccg gtt atc gtt cag ttc tcc aac ggt     192
   Thr Ala Ala Lys Val Lys Ala Pro Val Ile Val Gln Phe Ser Asn Gly
           50                  55                  60
   ggt gct tcc ttt atc gct ggt aaa ggc gtg aaa tct gac gtt ccg cag      240
   Gly Ala Ser Phe Ile Ala Gly Lys Gly Val Lys Ser Asp Val Pro Gln
   65                  70                  75                  80
   ggt gct gct atc ctg ggc gcg atc tct ggt gcg cat cac gtt cac cag      288
   Gly Ala Ala Ile Leu Gly Ala Ile Ser Gly Ala His His Val His Gln
                   85                  90                  95
   atg gct gaa cat tat ggt gtt ccg gtt atc ctg cac act gac cac tgc      336
   Met Ala Glu His Tyr Gly Val Pro Val Ile Leu His Thr Asp His Cys
               100                 105                 110
   gcg aag aaa ctg ctg ccg tgg atc gac ggt ctg ttg gac gcg ggt gaa      384
   Ala Lys Lys Leu Leu Pro Trp Ile Asp Gly Leu Leu Asp Ala Gly Glu
           115                 120                 125
   aaa cac ttc gca gct acc ggt aag ccg ctg ttc tct tct cac atg atc      432
   Lys His Phe Ala Ala Thr Gly Lys Pro Leu Phe Ser Ser His Met Ile
       130                 135                 140
   gac ctg tct gaa gaa tct ctg caa gag aac atc gaa atc tgc tct aaa      480
   Asp Leu Ser Glu Glu Ser Leu Gln Glu Asn Ile Glu Ile Cys Ser Lys
   145                 150                 155                 160
   tac ctg gag cgc atg tcc aaa atc ggc atg act ctg gaa atc gaa ctg      528
   Tyr Leu Glu Arg Met Ser Lys Ile Gly Met Thr Leu Glu Ile Glu Leu
                   165                 170                 175
   ggt tgc acc ggt ggt gaa gaa gac ggc gtg gac aac agc cac atg gac      576
   Gly Cys Thr Gly Gly Glu Glu Asp Gly Val Asp Asn Ser His Met Asp
               180                 185                 190
   gct tct gca ctg tac acc cag ccg gaa gac gtt gat tac gca tac acc      624
   Ala Ser Ala Leu Tyr Thr Gln Pro Glu Asp Val Asp Tyr Ala Tyr Thr
           195                 200                 205
   gaa ctg agc aaa atc agc ccg cgt ttc acc atc gca gcg tcc ttc ggt      672
   Glu Leu Ser Lys Ile Ser Pro Arg Phe Thr Ile Ala Ala Ser Phe Gly
       210                 215                 220
   aac gta cac ggt gtt tac aag ccg ggt aac gtg gtt ctg act ccg acc      720
   Asn Val His Gly Val Tyr Lys Pro Gly Asn Val Val Leu Thr Pro Thr
   225                 230                 235                 240
   atc ctg cgt gat tct cag gaa tat gtt tcc aag aaa cac aac ctg ccg      768
   Ile Leu Arg Asp Ser Gln Glu Tyr Val Ser Lys Lys His Asn Leu Pro
                   245                 250                 255
   cac aac agc ctg aac ttc gta ttc cac ggt ggt tcc ggt tct act gct      816
   His Asn Ser Leu Asn Phe Val Phe His Gly Gly Ser Gly Ser Thr Ala
               260                 265                 270
   cag gaa atc aaa gac tcc gta agc tac ggc gta gta aaa atg aac atc      864
   Gln Glu Ile Lys Asp Ser Val Ser Tyr Gly Val Val Lys Met Asn Ile
           275                 280                 285
   gat acc gat acc caa tgg gca acc tgg gaa ggc gtt ctg aac tac tac      912
   Asp Thr Asp Thr Gln Trp Ala Thr Trp Glu Gly Val Leu Asn Tyr Tyr
       290                 295                 300
   aaa gcg aac gaa gct tat ctg cag ggt cag ctg ggt aac ccg aaa ggc      960
   Lys Ala Asn Glu Ala Tyr Leu Gln Gly Gln Leu Gly Asn Pro Lys Gly
   305                 310                 315                 320
   gaa gat cag ccg aac aag aaa tac tac gat ccg cgc gta tgg ctg cgt     1008
   Glu Asp Gln Pro Asn Lys Lys Tyr Tyr Asp Pro Arg Val Trp Leu Arg
                   325                 330                 335
   gcc ggt cag act tcg atg atc gct cgt ctg gag aaa gca ttc cag gaa     1056
   Ala Gly Gln Thr Ser Met Ile Ala Arg Leu Glu Lys Ala Phe Gln Glu
               340                 345                 350
   ctg aac gcg atc gac gtt ctg taa                                    1080
   Leu Asn Ala Ile Asp Val Leu
           355
<210> 21
   <211> 359
   <212> PRT
   <213> Escherichia coli
<400> 21
   Met Ser Lys Ile Phe Asp Phe Val Lys Pro Gly Val Ile Thr Gly Asp
   1               5                   10                  15
   Asp Val Gln Lys Val Phe Gln Val Ala Lys Glu Asn Asn Phe Ala Leu
               20                  25                  30
   Pro Ala Val Asn Cys Val Gly Thr Asp Ser Ile Asn Ala Val Leu Glu
           35                  40                  45
   Thr Ala Ala Lys Val Lys Ala Pro Val Ile Val Gln Phe Ser Asn Gly
       50                  55                  60
   Gly Ala Ser Phe Ile Ala Gly Lys Gly Val Lys Ser Asp Val Pro Gln
   65                  70                  75                  80
   Gly Ala Ala Ile Leu Gly Ala Ile Ser Gly Ala His His Val His Gln
                   85                  90                  95
   Met Ala Glu His Tyr Gly Val Pro Val Ile Leu His Thr Asp His Cys
               100                 105                 110
   Ala Lys Lys Leu Leu Pro Trp Ile Asp Gly Leu Leu Asp Ala Gly Glu
           115                 120                 125
   Lys His Phe Ala Ala Thr Gly Lys Pro Leu Phe Ser Ser His Met Ile
       130                 135                 140
   Asp Leu Ser Glu Glu Ser Leu Gln Glu Asn Ile Glu Ile Cys Ser Lys
   145                 150                 155                 160
   Tyr Leu Glu Arg Met Ser Lys Ile Gly Met Thr Leu Glu Ile Glu Leu
                   165                 170                 175
   Gly Cys Thr Gly Gly Glu Glu Asp Gly Val Asp Asn Ser His Met Asp
               180                 185                 190
   Ala Ser Ala Leu Tyr Thr Gln Pro Glu Asp Val Asp Tyr Ala Tyr Thr
           195                 200                 205
   Glu Leu Ser Lys Ile Ser Pro Arg Phe Thr Ile Ala Ala Ser Phe Gly
       210                 215                 220
   Asn Val His Gly Val Tyr Lys Pro Gly Asn Val Val Leu Thr Pro Thr
   225                 230                 235                 240
   Ile Leu Arg Asp Ser Gln Glu Tyr Val Ser Lys Lys His Asn Leu Pro
                   245                 250                 255
   His Asn Ser Leu Asn Phe Val Phe His Gly Gly Ser Gly Ser Thr Ala
               260                 265                 270
   Gln Glu Ile Lys Asp Ser Val Ser Tyr Gly Val Val Lys Met Asn Ile
           275                 280                 285
   Asp Thr Asp Thr Gln Trp Ala Thr Trp Glu Gly Val Leu Asn Tyr Tyr
       290                 295                 300
   Lys Ala Asn Glu Ala Tyr Leu Gln Gly Gln Leu Gly Asn Pro Lys Gly
   305                 310                 315                 320
   Glu Asp Gln Pro Asn Lys Lys Tyr Tyr Asp Pro Arg Val Trp Leu Arg
                   325                 330                 335
   Ala Gly Gln Thr Ser Met Ile Ala Arg Leu Glu Lys Ala Phe Gln Glu
               340                 345                 350
   Leu Asn Ala Ile Asp Val Leu
   355
<210> 22
   <211> 1011
   <212> DNA
   <213> Escherichia coli
<220>
   <221> CDS
   <222> (1).. (1011)
<400> 22
   atg aga cga gaa ctt gcc atc gaa ttt tcc cgc gtc acc gaa tca gcg      48
   Met Arg Arg Glu Leu Ala Ile Glu Phe Ser Arg Val Thr Glu Ser Ala
   1               5                   10                  15
   gcg ctg gct ggc tac aaa tgg tta gga cgc ggc gat aaa aac acc gcg      96
   Ala Leu Ala Gly Tyr Lys Trp Leu Gly Arg Gly Asp Lys Asn Thr Ala
               20                  25                  30
   gac ggc gcg gcg gta aac gcc atg cgt att atg ctc aac cag gtc aac     144
   Asp Gly Ala Ala Val Asn Ala Met Arg Ile Met Leu Asn Gln Val Asn
           35                  40                  45
   att gac ggc acc atc gtc att ggt gaa ggt gaa atc gac gaa gca ccg     192
   Ile Asp Gly Thr Ile Val Ile Gly Glu Gly Glu Ile Asp Glu Ala Pro
       50                  55                  60
   atg ctc tac att ggt gaa aaa gtc ggt act ggt cgc ggc gac gcg gta     240
   Met Leu Tyr Ile Gly Glu Lys Val Gly Thr Gly Arg Gly Asp Ala Val
   65                  70                  75                  80
   gat att gct gtt gat ccg att gaa ggc acg cgc atg acg gcg atg ggc     288
   Asp Ile Ala Val Asp Pro Ile Glu Gly Thr Arg Met Thr Ala Met Gly
                   85                  90                  95
   cag gct aac gcg ctg gcg gtg ctg gca gta ggc gat aaa ggc tgc ttc     336
   Gln Ala Asn Ala Leu Ala Val Leu Ala Val Gly Asp Lys Gly Cys Phe
               100                 105                 110
   ctc aat gcg ccg gat atg tat atg gag aag ctg att gtc ggg ccg gga     384
   Leu Asn Ala Pro Asp Met Tyr Met Glu Lys Leu Ile Val Gly Pro Gly
           115                 120                 125
   gcc aaa ggc acc att gat ctg aac ctg ccg ctg gcg gat aac ctg cgc     432
   Ala Lys Gly Thr Ile Asp Leu Asn Leu Pro Leu Ala Asp Asn Leu Arg
       130                 135                 140
   aat gta gcg gcg gcg ctc ggc aaa ccg ttg agc gaa ctg acg gta acg     480
   Asn Val Ala Ala Ala Leu Gly Lys Pro Leu Ser Glu Leu Thr Val Thr
   145                 150                 155                 160
   att ctg gct aaa cca cgc cac gat gcc gtt atc gct gaa atg cag caa     528
   Ile Leu Ala Lys Pro Arg His Asp Ala Val Ile Ala Glu Met Gln Gln
                   165                 170                 175
   ctc ggc gta cgc gta ttt gct att ccg gac ggc gac gtt gcg gcc tca     576
   Leu Gly Val Arg Val Phe Ala Ile Pro Asp Gly Asp Val Ala Ala Ser
               180                 185                 190
   att ctc acc tgt atg cca gac agc gaa gtt gac gtg ctg tac ggt att     624
   Ile Leu Thr Cys Met Pro Asp Ser Glu Val Asp Val Leu Tyr Gly Ile
           195                 200                 205
   ggt ggc gcg ccg gaa ggc gta gtt tct gcg gcg gtg atc cgc gca tta     672
   Gly Gly Ala Pro Glu Gly Val Val Ser Ala Ala Val Ile Arg Ala Leu
       210                 215                 220
   gat ggc gac atg aac ggt cgt ctg ctg gcg cgt cat gac gtc aaa ggc       720
   Asp Gly Asp Met Asn Gly Arg Leu Leu Ala Arg His Asp Val Lys Gly
   225                 230                 235                 240
   gac aac gaa gag aat cgt cgc att ggc gag cag gag ctg gca cgc tgc       768
   Asp Asn Glu Glu Asn Arg Arg Ile Gly Glu Gln Glu Leu Ala Arg Cys
                   245                 250                 255
   aaa gcg atg ggc atc gaa gcc ggt aaa gta ttg cgc ctg ggc gat atg       816
   Lys Ala Met Gly Ile Glu Ala Gly Lys Val Leu Arg Leu Gly Asp Met
               260                 265                 270
   gcg cgc agc gat aac gtc atc ttc tct gcc acc ggt att acc aaa ggc       864
   Ala Arg Ser Asp Asn Val Ile Phe Ser Ala Thr Gly Ile Thr Lys Gly
           275                 280                 285
   gat ctg ctg gaa ggc att agc cgc aaa ggc aat atc gcg act acc gaa       912
   Asp Leu Leu Glu Gly Ile Ser Arg Lys Gly Asn Ile Ala Thr Thr Glu
       290                 295                 300
   acg ctg ctg atc cgc ggc aag tca cgc acc att cgc cgc att cag tcc       960
   Thr Leu Leu Ile Arg Gly Lys Ser Arg Thr Ile Arg Arg Ile Gln Ser
   305                 310                 315                 320
   atc cac tat ctg gat cgc aaa gac ccg gaa atg cag gtg cac atc ctc      1008
   Ile His Tyr Leu Asp Arg Lys Asp Pro Glu Met Gln Val His Ile Leu
                   325                 330                 335
   tga                                                                  1011
<210> 23
   <211> 336
   <212> PRT
   <213> Escherichia coli
<400> 23
   Met Arg Arg Glu Leu Ala Ile Glu Phe Ser Arg Val Thr Glu Ser Ala
   1               5                   10                  15
   Ala Leu Ala Gly Tyr Lys Trp Leu Gly Arg Gly Asp Lys Asn Thr Ala
               20                  25                  30
   Asp Gly Ala Ala Val Asn Ala Met Arg Ile Met Leu Asn Gln Val Asn
           35                  40                  45
   Ile Asp Gly Thr Ile Val Ile Gly Glu Gly Glu Ile Asp Glu Ala Pro
       50                  55                  60
   Met Leu Tyr Ile Gly Glu Lys Val Gly Thr Gly Arg Gly Asp Ala Val
   65                  70                  75                  80
   Asp Ile Ala Val Asp Pro Ile Glu Gly Thr Arg Met Thr Ala Met Gly
                   85                  90                  95
   Gln Ala Asn Ala Leu Ala Val Leu Ala Val Gly Asp Lys Gly Cys Phe
               100                 105                 110
   Leu Asn Ala Pro Asp Met Tyr Met Glu Lys Leu Ile Val Gly Pro Gly
           115                 120                 125
   Ala Lys Gly Thr Ile Asp Leu Asn Leu Pro Leu Ala Asp Asn Leu Arg
       130                 135                 140
   Asn Val Ala Ala Ala Leu Gly Lys Pro Leu Ser Glu Leu Thr Val Thr
   145                 150                 155                 160
   Ile Leu Ala Lys Pro Arg His Asp Ala Val Ile Ala Glu Met Gln Gln
                   165                 170                 175
   Leu Gly Val Arg Val Phe Ala Ile Pro Asp Gly Asp Val Ala Ala Ser
               180                 185                 190
   Ile Leu Thr Cys Met Pro Asp Ser Glu Val Asp Val Leu Tyr Gly Ile
           195                 200                 205
   Gly Gly Ala Pro Glu Gly Val Val Ser Ala Ala Val Ile Arg Ala Leu
       210                 215                 220
   Asp Gly Asp Met Asn Gly Arg Leu Leu Ala Arg His Asp Val Lys Gly
   225                 230                 235                 240
   Asp Asn Glu Glu Asn Arg Arg Ile Gly Glu Gln Glu Leu Ala Arg Cys
                   245                 250                 255
   Lys Ala Met Gly Ile Glu Ala Gly Lys Val Leu Arg Leu Gly Asp Met
               260                 265                 270
   Ala Arg Ser Asp Asn Val Ile Phe Ser Ala Thr Gly Ile Thr Lys Gly
           275                 280                 285
   Asp Leu Leu Glu Gly Ile Ser Arg Lys Gly Asn Ile Ala Thr Thr Glu
       290                 295                 300
   Thr Leu Leu Ile Arg Gly Lys Ser Arg Thr Ile Arg Arg Ile Gln Ser
   305                 310                 315                 320
   Ile His Tyr Leu Asp Arg Lys Asp Pro Glu Met Gln Val His Ile Leu
                   325                 330                 335
<210> 24
   <211> 1509
   <212> DNA
   <213> Escherichia coli
<220>
   <221> CDS
   <222> (1).. (1509)
<400> 24
   atg act gaa aaa aaa tat atc gtt gcg ctc gac cag ggc acc acc agc        48
   Met Thr Glu Lys Lys Tyr Ile Val Ala Leu Asp Gln Gly Thr Thr Ser
   1               5                   10                  15
   tcc cgc gcg gtc gta atg gat cac gat gcc aat atc att agc gtg tcg        96
   Ser Arg Ala Val Val Met Asp His Asp Ala Asn Ile Ile Ser Val Ser
               20                  25                  30
   cag cgc gaa ttt gag caa atc tac cca aaa cca ggt tgg gta gaa cac       144
   Gln Arg Glu Phe Glu Gln Ile Tyr Pro Lys Pro Gly Trp Val Glu His
           35                  40                  45
   gac cca atg gaa atc tgg gcc acc caa agc tcc acg ctg gta gaa gtg       192
   Asp Pro Met Glu Ile Trp Ala Thr Gln Ser Ser Thr Leu Val Glu Val
       50                  55                  60
   ctg gcg aaa gcc gat atc agt tcc gat caa att gca gct atc ggt att       240
   Leu Ala Lys Ala Asp Ile Ser Ser Asp Gln Ile Ala Ala Ile Gly Ile
   65                  70                  75                  80
   acg aac cag cgt gaa acc act att gtc tgg gaa aaa gaa acc ggc aag       288
   Thr Asn Gln Arg Glu Thr Thr Ile Val Trp Glu Lys Glu Thr Gly Lys
                   85                  90                  95
   cct atc tat aac gcc att gtc tgg cag tgc cgt cgt acc gca gaa atc       336
   Pro Ile Tyr Asn Ala Ile Val Trp Gln Cys Arg Arg Thr Ala Glu Ile
               100                 105                 110
   tgc gag cat tta aaa cgt gac ggt tta gaa gat tat atc cgc agc aat       384
   Cys Glu His Leu Lys Arg Asp Gly Leu Glu Asp Tyr Ile Arg Ser Asn
           115                 120                 125
   acc ggt ctg gtg att gac ccg tac ttt tct ggc acc aaa gtg aag tgg       432
   Thr Gly Leu Val Ile Asp Pro Tyr Phe Ser Gly Thr Lys Val Lys Trp
       130                 135                 140
   atc ctc gac cat gtg gaa ggc tct cgc gag cgt gca cgt cgt ggt gaa      480
   Ile Leu Asp His Val Glu Gly Ser Arg Glu Arg Ala Arg Arg Gly Glu
   145                 150                 155                 160
   ttg ctg ttt ggt acg gtt gat acg tgg ctt atc tgg aaa atg act cag      528
   Leu Leu Phe Gly Thr Val Asp Thr Trp Leu Ile Trp Lys Met Thr Gln
                   165                 170                 175
   ggc cgt gtc cat gtg acc gat tac acc aac gcc tct cgt acc atg ttg      576
   Gly Arg Val His Val Thr Asp Tyr Thr Asn Ala Ser Arg Thr Met Leu
               180                 185                 190
   ttc aac atc cat acc ctg gac tgg gac gac aaa atg ctg gaa gtg ctg      624
   Phe Asn Ile His Thr Leu Asp Trp Asp Asp Lys Met Leu Glu Val Leu
           195                 200                 205
   gat att ccg cgc gag atg ctg cca gaa gtg cgt cgt tct tcc gaa gta      672
   Asp Ile Pro Arg Glu Met Leu Pro Glu Val Arg Arg Ser Ser Glu Val
       210                 215                 220
   tac ggt cag act aac att ggc ggc aaa ggc ggc acg cgt att cca atc      720
   Tyr Gly Gln Thr Asn Ile Gly Gly Lys Gly Gly Thr Arg Ile Pro Ile
   225                 230                 235                 240
   tcc ggg atc gcc ggt gac cag cag gcc gcg ctg ttt ggt cag ttg tgc      768
   Ser Gly Ile Ala Gly Asp Gln Gln Ala Ala Leu Phe Gly Gln Leu Cys
                   245                 250                 255
   gtg aaa gaa ggg atg gcg aag aac acc tat ggc act ggc tgc ttt atg      816
   Val Lys Glu Gly Met Ala Lys Asn Thr Tyr Gly Thr Gly Cys Phe Met
               260                 265                 270
   ctg atg aac act ggc gag aaa gcg gtg aaa tca gaa aac ggc ctg ctg      864
   Leu Met Asn Thr Gly Glu Lys Ala Val Lys Ser Glu Asn Gly Leu Leu
           275                 280                 285
   acc acc atc gcc tgc ggc ccg act ggc gaa gtg aac tat gcg ttg gaa      912
   Thr Thr Ile Ala Cys Gly Pro Thr Gly Glu Val Asn Tyr Ala Leu Glu
       290                 295                 300
   ggt gcg gtg ttt atg gca ggc gca tcc att cag tgg ctg cgc gat gaa      960
   Gly Ala Val Phe Met Ala Gly Ala Ser Ile Gln Trp Leu Arg Asp Glu
   305                 310                 315                 320
   atg aag ttg att aac gac gcc tac gat tcc gaa tat ttc gcc acc aaa     1008
   Met Lys Leu Ile Asn Asp Ala Tyr Asp Ser Glu Tyr Phe Ala Thr Lys
                   325                 330                 335
   gtg caa aac acc aat ggt gtg tat gtg gtt ccg gca ttt acc ggg ctg     1056
   Val Gln Asn Thr Asn Gly Val Tyr Val Val Pro Ala Phe Thr Gly Leu
               340                 345                 350
   ggt gcg ccg tac tgg gac ccg tat gcg cgc ggg gcg att ttc ggt ctg     1104
   Gly Ala Pro Tyr Trp Asp Pro Tyr Ala Arg Gly Ala Ile Phe Gly Leu
           355                 360                 365
   act cgt ggg gtg aac gct aac cac att ata cgc gcg acg ctg gag tct     1152
   Thr Arg Gly Val Asn Ala Asn His Ile Ile Arg Ala Thr Leu Glu Ser
       370                 375                 380
   att gct tat cag acg cgt gac gtg ctg gaa gcg atg cag gcc gac tct     1200
   Ile Ala Tyr Gln Thr Arg Asp Val Leu Glu Ala Met Gln Ala Asp Ser
   385                 390                 395                 400
   ggt atc cgt ctg cac gcc ctg cgc gtg gat ggt ggc gca gta gca aac     1248
   Gly Ile Arg Leu His Ala Leu Arg Val Asp Gly Gly Ala Val Ala Asn
                   405                 410                 415
   aat ttc ctg atg cag ttc cag tcc gat att ctc ggc acc cgc gtt gag     1296
   Asn Phe Leu Met Gln Phe Gln Ser Asp Ile Leu Gly Thr Arg Val Glu
               420                 425                 430
   cgc ccg gaa gtg cgc gaa gtc acc gca ttg ggt gcg gcc tat ctc gca      1344
   Arg Pro Glu Val Arg Glu Val Thr Ala Leu Gly Ala Ala Tyr Leu Ala
           435                 440                 445
   ggc ctg gcg gtt ggc ttc tgg cag aac ctc gac gag ctg caa gag aaa      1392
   Gly Leu Ala Val Gly Phe Trp Gln Asn Leu Asp Glu Leu Gln Glu Lys
       450                 455                 460
   gcg gtg att gag cgc gag ttc cgt cca ggc atc gaa acc act gag cgt      1440
   Ala Val Ile Glu Arg Glu Phe Arg Pro Gly Ile Glu Thr Thr Glu Arg
   465                 470                 475                 480
   aat tac cgt tac gca ggc tgg aaa aaa gcg gtt aaa cgc gcg atg gcg      1488
   Asn Tyr Arg Tyr Ala Gly Trp Lys Lys Ala Val Lys Arg Ala Met Ala
                   485                 490                 495
   tgg gaa gaa cac gac gaa taa                                          1509
   Trp Glu Glu His Asp Glu
               500
<210> 25
   <211> 502
   <212> PRT
   <213> Escherichia coli
<400> 25
   Met Thr Glu Lys Lys Tyr Ile Val Ala Leu Asp Gln Gly Thr Thr Ser
   1               5                   10                  15
   Ser Arg Ala Val Val Met Asp His Asp Ala Asn Ile Ile Ser Val Ser
               20                  25                  30
   Gln Arg Glu Phe Glu Gln Ile Tyr Pro Lys Pro Gly Trp Val Glu His
           35                  40                  45
   Asp Pro Met Glu Ile Trp Ala Thr Gln Ser Ser Thr Leu Val Glu Val
       50                  55                  60
   Leu Ala Lys Ala Asp Ile Ser Ser Asp Gln Ile Ala Ala Ile Gly Ile
   65                  70                  75                  80
   Thr Asn Gln Arg Glu Thr Thr Ile Val Trp Glu Lys Glu Thr Gly Lys
                   85                  90                  95
   Pro Ile Tyr Asn Ala Ile Val Trp Gln Cys Arg Arg Thr Ala Glu Ile
               100                 105                 110
   Cys Glu His Leu Lys Arg Asp Gly Leu Glu Asp Tyr Ile Arg Ser Asn
           115                 120                 125
   Thr Gly Leu Val Ile Asp Pro Tyr Phe Ser Gly Thr Lys Val Lys Trp
       130                 135                 140
   Ile Leu Asp His Val Glu Gly Ser Arg Glu Arg Ala Arg Arg Gly Glu
   145                 150                 155                 160
   Leu Leu Phe Gly Thr Val Asp Thr Trp Leu Ile Trp Lys Met Thr Gln
                   165                 170                 175
   Gly Arg Val His Val Thr Asp Tyr Thr Asn Ala Ser Arg Thr Met Leu
               180                 185                 190
   Phe Asn Ile His Thr Leu Asp Trp Asp Asp Lys Met Leu Glu Val Leu
           195                 200                 205
   Asp Ile Pro Arg Glu Met Leu Pro Glu Val Arg Arg Ser Ser Glu Val
       210                 215                 220
   Tyr Gly Gln Thr Asn Ile Gly Gly Lys Gly Gly Thr Arg Ile Pro Ile
   225                 230                 235                 240
   Ser Gly Ile Ala Gly Asp Gln Gln Ala Ala Leu Phe Gly Gln Leu Cys
                   245                 250                 255
   Val Lys Glu Gly Met Ala Lys Asn Thr Tyr Gly Thr Gly Cys Phe Met
               260                 265                 270
   Leu Met Asn Thr Gly Glu Lys Ala Val Lys Ser Glu Asn Gly Leu Leu
           275                 280                 285
   Thr Thr Ile Ala Cys Gly Pro Thr Gly Glu Val Asn Tyr Ala Leu Glu
       290                 295                 300
   Gly Ala Val Phe Met Ala Gly Ala Ser Ile Gln Trp Leu Arg Asp Glu
   305                 310                 315                 320
   Met Lys Leu Ile Asn Asp Ala Tyr Asp Ser Glu Tyr Phe Ala Thr Lys
                   325                 330                 335
   Val Gln Asn Thr Asn Gly Val Tyr Val Val Pro Ala Phe Thr Gly Leu
               340                 345                 350
   Gly Ala Pro Tyr Trp Asp Pro Tyr Ala Arg Gly Ala Ile Phe Gly Leu
           355                 360                 365
   Thr Arg Gly Val Asn Ala Asn His Ile Ile Arg Ala Thr Leu Glu Ser
       370                 375                 380
   Ile Ala Tyr Gln Thr Arg Asp Val Leu Glu Ala Met Gln Ala Asp Ser
   385                 390                 395                 400
   Gly Ile Arg Leu His Ala Leu Arg Val Asp Gly Gly Ala Val Ala Asn
                   405                 410                 415
   Asn Phe Leu Met Gln Phe Gln Ser Asp Ile Leu Gly Thr Arg Val Glu
               420                 425                 430
   Arg Pro Glu Val Arg Glu Val Thr Ala Leu Gly Ala Ala Tyr Leu Ala
           435                 440                 445
   Gly Leu Ala Val Gly Phe Trp Gln Asn Leu Asp Glu Leu Gln Glu Lys
       450                 455                 460
   Ala Val Ile Glu Arg Glu Phe Arg Pro Gly Ile Glu Thr Thr Glu Arg
   465                 470                 475                 480
   Asn Tyr Arg Tyr Ala Gly Trp Lys Lys Ala Val Lys Arg Ala Met Ala
                   485                 490                 495
   Trp Glu Glu His Asp Glu
               500
<210> 26
   <211> 1629
   <212> DNA
   <213> Escherichia coli
<220>
   <221> CDS
   <222> (1).. (1629)
<400> 26
   atg aaa act cgc gac tcg caa tca agt gac gtg att atc att ggc ggc      48
   Met Lys Thr Arg Asp Ser Gln Ser Ser Asp Val Ile Ile Ile Gly Gly
   1               5                   10                  15
   ggc gca acg gga gcc ggg att gcc cgc gac tgt gcc ctg cgc ggg ctg      96
   Gly Ala Thr Gly Ala Gly Ile Ala Arg Asp Cys Ala Leu Arg Gly Leu
               20                  25                  30
   cgc gtg att ttg gtt gag cgc cac gac atc gca acc ggt gcc acc ggg     144
   Arg Val Ile Leu Val Glu Arg His Asp Ile Ala Thr Gly Ala Thr Gly
           35                  40                  45
   cgt aac cac ggc ctg ctg cac agc ggt gcg cgc tat gcg gta acc gat     192
   Arg Asn His Gly Leu Leu His Ser Gly Ala Arg Tyr Ala Val Thr Asp
       50                  55                  60
   gcg gaa tcg gcc cgc gaa tgc att agt gaa aac cag atc ctg aaa cgc      240
   Ala Glu Ser Ala Arg Glu Cys Ile Ser Glu Asn Gln Ile Leu Lys Arg
   65                  70                  75                  80
   att gca cgt cac tgc gtt gaa cca acc aac ggc ctg ttt atc acc ctg      288
   Ile Ala Arg His Cys Val Glu Pro Thr Asn Gly Leu Phe Ile Thr Leu
                   85                  90                  95
   ccg gaa gat gac ctc tcc ttc cag gcc act ttt att cgc gcc tgc gaa      336
   Pro Glu Asp Asp Leu Ser Phe Gln Ala Thr Phe Ile Arg Ala Cys Glu
               100                 105                 110
   gaa gca ggg atc agc gca gaa gct ata gac ccg cag caa gcg cgc att      384
   Glu Ala Gly Ile Ser Ala Glu Ala Ile Asp Pro Gln Gln Ala Arg Ile
           115                 120                 125
   atc gaa cct gcc gtt aac ccg gca ctg att ggc gcg gtg aaa gtt ccg      432
   Ile Glu Pro Ala Val Asn Pro Ala Leu Ile Gly Ala Val Lys Val Pro
       130                 135                 140
   gat ggc acc gtt gat cca ttt cgt ctg acc gca gca aac atg ctg gat      480
   Asp Gly Thr Val Asp Pro Phe Arg Leu Thr Ala Ala Asn Met Leu Asp
   145                 150                 155                 160
   gcc aaa gaa cac ggt gcc gtt atc ctt acc gct cat gaa gtc acg ggg      528
   Ala Lys Glu His Gly Ala Val Ile Leu Thr Ala His Glu Val Thr Gly
                   165                 170                 175
   ctg att cgt gaa ggc gcg acg gtg tgc ggt gtt cgt gta cgt aac cat      576
   Leu Ile Arg Glu Gly Ala Thr Val Cys Gly Val Arg Val Arg Asn His
               180                 185                 190
   ctc acc ggc gaa act cag gcc ctt cat gca cct gtc gtg gtt aat gcc      624
   Leu Thr Gly Glu Thr Gln Ala Leu His Ala Pro Val Val Val Asn Ala
           195                 200                 205
   gct ggg atc tgg ggg caa cac att gcc gaa tat gcc gat ctg cgc att      672
   Ala Gly Ile Trp Gly Gln His Ile Ala Glu Tyr Ala Asp Leu Arg Ile
       210                 215                 220
   cgc atg ttc ccg gcg aaa gga tcg ctg ctg atc atg gat cac cgc att      720
   Arg Met Phe Pro Ala Lys Gly Ser Leu Leu Ile Met Asp His Arg Ile
   225                 230                 235                 240
   aac cag cat gtg atc aac cgc tgc cgt aaa cct tcc gac gcc gat att      768
   Asn Gln His Val Ile Asn Arg Cys Arg Lys Pro Ser Asp Ala Asp Ile
                   245                 250                 255
   ctg gtg cct ggc gat acc att tcg ctg att ggt acc acc tct tta cgt      816
   Leu Val Pro Gly Asp Thr Ile Ser Leu Ile Gly Thr Thr Ser Leu Arg
               260                 265                 270
   att gat tac aac gag att gac gat aat cga gtg acg gca gaa gag gtt      864
   Ile Asp Tyr Asn Glu Ile Asp Asp Asn Arg Val Thr Ala Glu Glu Val
           275                 280                 285
   gat att ctg ctg cgt gaa ggg gaa aaa ctg gcc ccc gtg atg gcg aaa      912
   Asp Ile Leu Leu Arg Glu Gly Glu Lys Leu Ala Pro Val Met Ala Lys
       290                 295                 300
   acg cgc att ttg cgg gcc tat tct ggc gtg cgc ccg ctg gtt gcc agc      960
   Thr Arg Ile Leu Arg Ala Tyr Ser Gly Val Arg Pro Leu Val Ala Ser
   305                 310                 315                 320
   gat gac gac ccg agc gga cgt aac gtc agc cgt ggc atc gtg ctg ctc     1008
   Asp Asp Asp Pro Ser Gly Arg Asn Val Ser Arg Gly Ile Val Leu Leu
                   325                 330                 335
   gac cat gct gaa cgc gat ggt ctg gac gga ttt atc acc atc acc ggt     1056
   Asp His Ala Glu Arg Asp Gly Leu Asp Gly Phe Ile Thr Ile Thr Gly
               340                 345                 350
   ggc aaa ctg atg acc tat cgg ctg atg gct gaa tgg gct acc gac gcg      1104
   Gly Lys Leu Met Thr Tyr Arg Leu Met Ala Glu Trp Ala Thr Asp Ala
           355                 360                 365
   gta tgc cgc aaa ctg ggc aac acg cgc ccc tgt acg act gcc gat ctg      1152
   Val Cys Arg Lys Leu Gly Asn Thr Arg Pro Cys Thr Thr Ala Asp Leu
       370                 375                 380
   gca ctg cct ggt tca caa gaa ccc gct gaa gtt acc ttg cgt aaa gtc      1200
   Ala Leu Pro Gly Ser Gln Glu Pro Ala Glu Val Thr Leu Arg Lys Val
   385                 390                 395                 400
   atc tcc ctg cct gcc ccg ctg cgc ggt tct gcg gtt tat cgt cat ggc      1248
   Ile Ser Leu Pro Ala Pro Leu Arg Gly Ser Ala Val Tyr Arg His Gly
                   405                 410                 415
   gat cgc acg cct gcc tgg ctg agc gaa ggc cgt ctg cac cgt agc ctg      1296
   Asp Arg Thr Pro Ala Trp Leu Ser Glu Gly Arg Leu His Arg Ser Leu
               420                 425                 430
   gta tgt gag tgc gaa gcg gta act gcg ggt gaa gtg cag tac gcg gta      1344
   Val Cys Glu Cys Glu Ala Val Thr Ala Gly Glu Val Gln Tyr Ala Val
           435                 440                 445
   gaa aat tta aac gtt aat agc ctg ctg gat tta cgc cgt cgt acc cgt      1392
   Glu Asn Leu Asn Val Asn Ser Leu Leu Asp Leu Arg Arg Arg Thr Arg
       450                 455                 460
   gtg ggg atg ggc acc tgc cag ggc gaa ctc tgc gcc tgc cgc gct gcc      1440
   Val Gly Met Gly Thr Cys Gln Gly Glu Leu Cys Ala Cys Arg Ala Ala
   465                 470                 475                 480
   gga ctg ctg caa cgt ttt aac gtc acg acg tcc gcg caa tct atc gag      1488
   Gly Leu Leu Gln Arg Phe Asn Val Thr Thr Ser Ala Gln Ser Ile Glu
                   485                 490                 495
   caa ctt tcc acc ttc ctt aac gaa cgc tgg aaa ggc gtg caa ccc atc      1536
   Gln Leu Ser Thr Phe Leu Asn Glu Arg Trp Lys Gly Val Gln Pro Ile
               500                 505                 510
   gcc tgg gga gat gca ctg cgc gaa agc gaa ttt acc cgc tgg gtt tat      1584
   Ala Trp Gly Asp Ala Leu Arg Glu Ser Glu Phe Thr Arg Trp Val Tyr
           515                 520                 525
   cag gga ttg tgt ggt ctg gag aag gag cag aaa gat gcg ctt tga          1629
   Gln Gly Leu Cys Gly Leu Glu Lys Glu Gln Lys Asp Ala Leu
       530                 535                 540
<210> 27
   <211> 542
   <212> PRT
   <213> Escherichia coli
<400> 27
   Met Lys Thr Arg Asp Ser Gln Ser Ser Asp Val Ile Ile Ile Gly Gly
   1               5                   10                  15
   Gly Ala Thr Gly Ala Gly Ile Ala Arg Asp Cys Ala Leu Arg Gly Leu
               20                  25                  30
   Arg Val Ile Leu Val Glu Arg His Asp Ile Ala Thr Gly Ala Thr Gly
           35                  40                  45
   Arg Asn His Gly Leu Leu His Ser Gly Ala Arg Tyr Ala Val Thr Asp
       50                  55                  60
   Ala Glu Ser Ala Arg Glu Cys Ile Ser Glu Asn Gln Ile Leu Lys Arg
   65                  70                  75                  80
   Ile Ala Arg His Cys Val Glu Pro Thr Asn Gly Leu Phe Ile Thr Leu
   85 90 95
   Pro Glu Asp Asp Leu Ser Phe Gln Ala Thr Phe Ile Arg Ala Cys Glu
   100 105 110
   Glu Ala Gly Ile Ser Ala Glu Ala Ile Asp Pro Gln Gln Ala Arg Ile
   115 120 125
   Ile Glu Pro Ala Val Asn Pro Ala Leu Ile Gly Ala Val Lys Val Pro
   130 135 140
   Asp Gly Thr Val Asp Pro Phe Arg Leu Thr Ala Ala Asn Met Leu Asp
   145 150 155 160
   Ala Lys Glu His Gly Ala Val Ile Leu Thr Ala His Glu Val Thr Gly
   165 170 175
   Leu Ile Arg Glu Gly Ala Thr Val Cys Gly Val Arg Val Arg Asn His
   180 185 190
   Leu Thr Gly Glu Thr Gln Ala Leu His Ala Pro Val Val Val Asn Ala
   195 200 205
   Ala Gly Ile Trp Gly Gln His Ile Ala Glu Tyr Ala Asp Leu Arg Ile
   210 215 220
   Arg Met Phe Pro Ala Lys Gly Ser Leu Leu Ile Met Asp His Arg Ile
   225 230 235 240
   Asn Gln His Val Ile Asn Arg Cys Arg Lys Pro Ser Asp Ala Asp Ile
   245 250 255
   Leu Val Pro Gly Asp Thr Ile Ser Leu Ile Gly Thr Thr Ser Leu Arg
   260 265 270
   Ile Asp Tyr Asn Glu Ile Asp Asp Asn Arg Val Thr Ala Glu Glu Val
   275 280 285
   Asp Ile Leu Leu Arg Glu Gly Glu Lys Leu Ala Pro Val Met Ala Lys
   290 295 300
   Thr Arg Ile Leu Arg Ala Tyr Ser Gly Val Arg Pro Leu Val Ala Ser
   305 310 315 320
   Asp Asp Asp Pro Ser Gly Arg Asn Val Ser Arg Gly Ile Val Leu Leu
   325 330 335
   Asp His Ala Glu Arg Asp Gly Leu Asp Gly Phe Ile Thr Ile Thr Gly
   340 345 350
   Gly Lys Leu Met Thr Tyr Arg Leu Met Ala Glu Trp Ala Thr Asp Ala
   355 360 365
   Val Cys Arg Lys Leu Gly Asn Thr Arg Pro Cys Thr Thr Ala Asp Leu
   370 375 380
   Ala Leu Pro Gly Ser Gln Glu Pro Ala Glu Val Thr Leu Arg Lys Val
   385 390 395 400
   Ile Ser Leu Pro Ala Pro Leu Arg Gly Ser Ala Val Tyr Arg His Gly
   405 410 415
   Asp Arg Thr Pro Ala Trp Leu Ser Glu Gly Arg Leu His Arg Ser Leu
   420 425 430
   Val Cys Glu Cys Glu Ala Val Thr Ala Gly Glu Val Gln Tyr Ala Val
   435 440 445
   Glu Asn Leu Asn Val Asn Ser Leu Leu Asp Leu Arg Arg Arg Thr Arg
   450 455 460
   Val Gly Met Gly Thr Cys Gln Gly Glu Leu Cys Ala Cys Arg Ala Ala
   465 470 475 480
   Gly Leu Leu Gln Arg Phe Asn Val Thr Thr Ser Ala Gln Ser Ile Glu
   485 490 495
   Gln Leu Ser Thr Phe Leu Asn Glu Arg Trp Lys Gly Val Gln Pro Ile
   500 505 510
   Ala Trp Gly Asp Ala Leu Arg Glu Ser Glu Phe Thr Arg Trp Val Tyr
               515                 520                 525
   Gln Gly Leu Cys Gly Leu Glu Lys Glu Gln Lys Asp Ala Leu
           530                 535                 540
<210> 28
   <211> 1260
   <212> DNA
   <213> Escherichia coli
<220>
   <221> CDS
   <222> (1).. (1260)
<400> 28
   atg cgc ttt gat act gtc att atg ggc ggc ggc ctc gcc gga tta ctc      48
   Met Arg Phe Asp Thr Val Ile Met Gly Gly Gly Leu Ala Gly Leu Leu
   1               5                   10                  15
   tgt ggc ctg caa ctg caa aaa cac ggc ctg cgc tgt gcc att gtc act      96
   Cys Gly Leu Gln Leu Gln Lys His Gly Leu Arg Cys Ala Ile Val Thr
               20                  25                  30
   cgt ggt caa agc gca ctg cat ttc tca tcc gga tcg ctg gat ttg ctg     144
   Arg Gly Gln Ser Ala Leu His Phe Ser Ser Gly Ser Leu Asp Leu Leu
           35                  40                  45
   agc cat ctg cca gat ggt caa ccg gtg aca gac att cac agt gga ctg     192
   Ser His Leu Pro Asp Gly Gln Pro Val Thr Asp Ile His Ser Gly Leu
       50                  55                  60
   gaa tct ttg cgt cag cag gca cca gcc cat cct tac tcc ctt ctc gag     240
   Glu Ser Leu Arg Gln Gln Ala Pro Ala His Pro Tyr Ser Leu Leu Glu
   65                  70                  75                  80
   cca caa cgc gtg ctc gat ctc gct tgc cag gcg cag gca tta atc gct     288
   Pro Gln Arg Val Leu Asp Leu Ala Cys Gln Ala Gln Ala Leu Ile Ala
                   85                  90                  95
   gaa agc ggt gcg caa ttg cag ggc agc gta gaa ctt gct cac cag cgg     336
   Glu Ser Gly Ala Gln Leu Gln Gly Ser Val Glu Leu Ala His Gln Arg
               100                 105                 110
   gtt acg ccg ctc ggc act ctg cgc tct acc tgg cta agt tcg cca gaa     384
   Val Thr Pro Leu Gly Thr Leu Arg Ser Thr Trp Leu Ser Ser Pro Glu
           115                 120                 125
   gtc ccc gtc tgg ccg ctg ccc gcg aag aaa ata tgt gta gtg gga att     432
   Val Pro Val Trp Pro Leu Pro Ala Lys Lys Ile Cys Val Val Gly Ile
       130                 135                 140
   agc ggc ctg atg gat ttt cag gcg cac ctt gcg gca gct tcg ttg cgt     480
   Ser Gly Leu Met Asp Phe Gln Ala His Leu Ala Ala Ala Ser Leu Arg
   145                 150                 155                 160
   gaa ctc ggc ctt gcc gtt gaa acc gca gaa ata gag ctg ccg gaa ctg     528
   Glu Leu Gly Leu Ala Val Glu Thr Ala Glu Ile Glu Leu Pro Glu Leu
                   165                 170                 175
   gat gtg ctg cgc aat aac gcc acc gaa ttt cgc gcg gtg aat atc gcc     576
   Asp Val Leu Arg Asn Asn Ala Thr Glu Phe Arg Ala Val Asn Ile Ala
               180                 185                 190
   cgt ttc ctt gat aat gaa gaa aac tgg ccg ctg tta ctt gat gcg ctt     624
   Arg Phe Leu Asp Asn Glu Glu Asn Trp Pro Leu Leu Leu Asp Ala Leu
           195                 200                 205
   att cct gtc gcc aat acc tgc gaa atg atc ctg atg ccc gcc tgc ttc     672
   Ile Pro Val Ala Asn Thr Cys Glu Met Ile Leu Met Pro Ala Cys Phe
       210                 215                 220
   ggt ctg gcc gat gac aaa ctg tgg cgt tgg ttg aat gaa aaa cta cct      720
   Gly Leu Ala Asp Asp Lys Leu Trp Arg Trp Leu Asn Glu Lys Leu Pro
   225                 230                 235                 240
   tgt tca ctg atg ctt ttg cca acg ctg ccg cct tcc gtg ctg ggc att      768
   Cys Ser Leu Met Leu Leu Pro Thr Leu Pro Pro Ser Val Leu Gly Ile
                   245                 250                 255
   cgt ctg caa aac cag tta cag cgc cag ttt gtg cgc cag ggt ggc gtg      816
   Arg Leu Gln Asn Gln Leu Gln Arg Gln Phe Val Arg Gln Gly Gly Val
               260                 265                 270
   tgg atg ccg ggc gat gaa gtg aaa aaa gtg acc tgt aaa aat ggc gta      864
   Trp Met Pro Gly Asp Glu Val Lys Lys Val Thr Cys Lys Asn Gly Val
           275                 280                 285
   gtg aac gaa atc tgg acc cgc aat cac gcc gat att ccg cta cgt cca      912
   Val Asn Glu Ile Trp Thr Arg Asn His Ala Asp Ile Pro Leu Arg Pro
       290                 295                 300
   cgt ttc gcg gtt ctc gcc agc ggc agt ttc ttt agt ggc gga ctg gta      960
   Arg Phe Ala Val Leu Ala Ser Gly Ser Phe Phe Ser Gly Gly Leu Val
   305                 310                 315                 320
   gcg gaa cgt aac ggc att cga gag ccg att ctc ggc ctt gat gtg cta     1008
   Ala Glu Arg Asn Gly Ile Arg Glu Pro Ile Leu Gly Leu Asp Val Leu
                   325                 330                 335
   caa acc gcc acg cgg ggt gaa tgg tat aag gga gat ttt ttt gcg ccg     1056
   Gln Thr Ala Thr Arg Gly Glu Trp Tyr Lys Gly Asp Phe Phe Ala Pro
               340                 345                 350
   caa ccg tgg cag cag ttc ggt gta acc act gat gag acg cta cgc ccg     1104
   Gln Pro Trp Gln Gln Phe Gly Val Thr Thr Asp Glu Thr Leu Arg Pro
           355                 360                 365
   tca cag gca ggg caa acc att gaa aac ctg ttt gcc atc ggt tcg gtg     1152
   Ser Gln Ala Gly Gln Thr Ile Glu Asn Leu Phe Ala Ile Gly Ser Val
       370                 375                 380
   ctg ggc gga ttt gat ccc atc gcc cag gga tgc ggc ggc ggt gtt tgt     1200
   Leu Gly Gly Phe Asp Pro Ile Ala Gln Gly Cys Gly Gly Gly Val Cys
   385                 390                 395                 400
   gcc gtc agt gct tta cat gcc gct caa cag att gcc caa cgc gca gga     1248
   Ala Val Ser Ala Leu His Ala Ala Gln Gln Ile Ala Gln Arg Ala Gly
                   405                 410                 415
   ggc caa caa tga                                                     1260
   Gly Gln Gln
<210> 29
   <211> 419
   <212> PRT
   <213> Escherichia coli
<400> 29
   Met Arg Phe Asp Thr Val Ile Met Gly Gly Gly Leu Ala Gly Leu Leu
   1               5                   10                  15
   Cys Gly Leu Gln Leu Gln Lys His Gly Leu Arg Cys Ala Ile Val Thr
               20                  25                  30
   Arg Gly Gln Ser Ala Leu His Phe Ser Ser Gly Ser Leu Asp Leu Leu
           35                  40                  45
   Ser His Leu Pro Asp Gly Gln Pro Val Thr Asp Ile His Ser Gly Leu
   50 55 60
   Glu Ser Leu Arg Gln Gln Ala Pro Ala His Pro Tyr Ser Leu Leu Glu
   65 70 75 80
   Pro Gln Arg Val Leu Asp Leu Ala Cys Gln Ala Gln Ala Leu Ile Ala
   85 90 95
   Glu Ser Gly Ala Gln Leu Gln Gly Ser Val Glu Leu Ala His Gln Arg
   100 105 110
   Val Thr Pro Leu Gly Thr Leu Arg Ser Thr Trp Leu Ser Ser Pro Glu
   115 120 125
   Val Pro Val Trp Pro Leu Pro Ala Lys Lys Ile Cys Val Val Gly Ile
   130 135 140
   Ser Gly Leu Met Asp Phe Gln Ala His Leu Ala Ala Ala Ser Leu Arg
   145 150 155 160
   Glu Leu Gly Leu Ala Val Glu Thr Ala Glu Ile Glu Leu Pro Glu Leu
   165 170 175
   Asp Val Leu Arg Asn Asn Ala Thr Glu Phe Arg Ala Val Asn Ile Ala
   180 185 190
   Arg Phe Leu Asp Asn Glu Glu Asn Trp Pro Leu Leu Leu Asp Ala Leu
   195 200 205
   Ile Pro Val Ala Asn Thr Cys Glu Met Ile Leu Met Pro Ala Cys Phe
   210 215 220
   Gly Leu Ala Asp Asp Lys Leu Trp Arg Trp Leu Asn Glu Lys Leu Pro
   225 230 235 240
   Cys Ser Leu Met Leu Leu Pro Thr Leu Pro Pro Ser Val Leu Gly Ile
   245 250 255
   Arg Leu Gln Asn Gln Leu Gln Arg Gln Phe Val Arg Gln Gly Gly Val
   260 265 270
   Trp Met Pro Gly Asp Glu Val Lys Lys Val Thr Cys Lys Asn Gly Val
   275 280 285
   Val Asn Glu Ile Trp Thr Arg Asn His Ala Asp Ile Pro Leu Arg Pro
   290 295 300
   Arg Phe Ala Val Leu Ala Ser Gly Ser Phe Phe Ser Gly Gly Leu Val
   305 310 315 320
   Ala Glu Arg Asn Gly Ile Arg Glu Pro Ile Leu Gly Leu Asp Val Leu
   325 330 335
   Gln Thr Ala Thr Arg Gly Glu Trp Tyr Lys Gly Asp Phe Phe Ala Pro
   340 345 350
   Gln Pro Trp Gln Gln Phe Gly Val Thr Thr Asp Glu Thr Leu Arg Pro
   355 360 365
   Ser Gln Ala Gly Gln Thr Ile Glu Asn Leu Phe Ala Ile Gly Ser Val
   370 375 380
   Leu Gly Gly Phe Asp Pro Ile Ala Gln Gly Cys Gly Gly Gly Val Cys
   385 390 395 400
   Ala Val Ser Ala Leu His Ala Ala Gln Gln Ile Ala Gln Arg Ala Gly
   405 410 415
   Gly Gln Gln
<210> 30
   <211> 1191
   <212> DNA
   <213> Escherichia coli
<220>
   <221> CDS
   <222> (1).. (1191)
<400> 30
   atg aat gac acc agc ttc gaa aac tgc att aag tgc acc gtc tgc acc        48
   Met Asn Asp Thr Ser Phe Glu Asn Cys Ile Lys Cys Thr Val Cys Thr
   1               5                   10                  15
   acc gcc tgc ccg gtg agc cgg gtg aat ccc ggt tat cca ggg cca aaa        96
   Thr Ala Cys Pro Val Ser Arg Val Asn Pro Gly Tyr Pro Gly Pro Lys
               20                  25                  30
   caa gcc ggg ccg gat ggc gag cgt ctg cgt ttg aaa gat ggc gca ctg        144
   Gln Ala Gly Pro Asp Gly Glu Arg Leu Arg Leu Lys Asp Gly Ala Leu
           35                  40                  45
   tat gac gag gcg ctg aaa tat tgc atc aac tgc aaa cgt tgt gaa gtc        192
   Tyr Asp Glu Ala Leu Lys Tyr Cys Ile Asn Cys Lys Arg Cys Glu Val
       50                  55                  60
   gcc tgc ccg tcc gat gtg aag att ggc gat att atc cag cgc gcg cgg        240
   Ala Cys Pro Ser Asp Val Lys Ile Gly Asp Ile Ile Gln Arg Ala Arg
   65                  70                  75                  80
   gcg aaa tat gac acc acg cgc ccg tcg ctg cgt aat ttt gtg ttg agt        288
   Ala Lys Tyr Asp Thr Thr Arg Pro Ser Leu Arg Asn Phe Val Leu Ser
                   85                  90                  95
   cat acc gac ctg atg ggt agc gtt tcc acg ccg ttc gca cca atc gtc        336
   His Thr Asp Leu Met Gly Ser Val Ser Thr Pro Phe Ala Pro Ile Val
               100                 105                 110
   aac acc gct acc tcg ctg aaa ccg gtg cgg cag ctg ctt gat gcg gcg        384
   Asn Thr Ala Thr Ser Leu Lys Pro Val Arg Gln Leu Leu Asp Ala Ala
           115                 120                 125
   tta aaa atc gat cat cgc cgc acg cta ccg aaa tac tcc ttc ggc acg        432
   Leu Lys Ile Asp His Arg Arg Thr Leu Pro Lys Tyr Ser Phe Gly Thr
       130                 135                 140
   ttc cgt cgc tgg tat cgc agc gtg gcg gct cag caa gca caa tat aaa        480
   Phe Arg Arg Trp Tyr Arg Ser Val Ala Ala Gln Gln Ala Gln Tyr Lys
   145                 150                 155                 160
   gac cag gtc gct ttc ttt cac ggc tgc ttc gtt aac tac aac cat ccg        528
   Asp Gln Val Ala Phe Phe His Gly Cys Phe Val Asn Tyr Asn His Pro
                   165                 170                 175
   cag tta ggt aaa gat tta att aaa gtg ctc aac gca atg ggt acc ggt        576
   Gln Leu Gly Lys Asp Leu Ile Lys Val Leu Asn Ala Met Gly Thr Gly
               180                 185                 190
   gta caa ctg ctc agc aaa gaa aaa tgc tgc ggc gta ccg cta atc gcc        624
   Val Gln Leu Leu Ser Lys Glu Lys Cys Cys Gly Val Pro Leu Ile Ala
           195                 200                 205
   aac ggc ttt acc gat aaa gca cgc aaa cag gca att acg aat gta gag        672
   Asn Gly Phe Thr Asp Lys Ala Arg Lys Gln Ala Ile Thr Asn Val Glu
       210                 215                 220
   tcg atc cgc gaa gct gtg gga gta aaa ggc att ccg gtg att gcc acc        720
   Ser Ile Arg Glu Ala Val Gly Val Lys Gly Ile Pro Val Ile Ala Thr
   225                 230                 235                 240
   tcc tca acc tgt aca ttt gcc ctg cgc gac gaa tac ccg gaa gtg ctg        768
   Ser Ser Thr Cys Thr Phe Ala Leu Arg Asp Glu Tyr Pro Glu Val Leu
                   245                 250                 255
   aat gtc gac aac aaa ggc ttg cgc gat cat atc gaa ctg gca acc cgc        816
   Asn Val Asp Asn Lys Gly Leu Arg Asp His Ile Glu Leu Ala Thr Arg
               260                 265                 270
   tgg ctg tgg cgc aag ctg gac gaa ggc aaa acg tta ccg ctg aaa ccg      864
   Trp Leu Trp Arg Lys Leu Asp Glu Gly Lys Thr Leu Pro Leu Lys Pro
           275                 280                 285
   ctg ccg ctg aaa gtg gtt tat cac act ccg tgc cat atg gaa aaa atg      912
   Leu Pro Leu Lys Val Val Tyr His Thr Pro Cys His Met Glu Lys Met
       290                 295                 300
   ggc tgg acg ctc tac acc ctg gag ctg ttg cgt aac atc ccg ggg ctt      960
   Gly Trp Thr Leu Tyr Thr Leu Glu Leu Leu Arg Asn Ile Pro Gly Leu
   305                 310                 315                 320
   gag tta acg gtg ctg gat tcc cag tgc tgc ggt att gcg ggt act tac     1008
   Glu Leu Thr Val Leu Asp Ser Gln Cys Cys Gly Ile Ala Gly Thr Tyr
                   325                 330                 335
   ggt ttc aaa aaa gag aac tac ccc acc tca caa gcc atc ggc gca cca     1056
   Gly Phe Lys Lys Glu Asn Tyr Pro Thr Ser Gln Ala Ile Gly Ala Pro
               340                 345                 350
   ctg ttc cgc cag ata gaa gaa agc ggc gca gat ctg gtg gtc acc gac     1104
   Leu Phe Arg Gln Ile Glu Glu Ser Gly Ala Asp Leu Val Val Thr Asp
           355                 360                 365
   tgc gaa acc tgt aaa tgg cag att gag atg tcc aca agt ctt cgc tgc     1152
   Cys Glu Thr Cys Lys Trp Gln Ile Glu Met Ser Thr Ser Leu Arg Cys
       370                 375                 380
   gaa cat ccg att acg cta ctg gcc cag gcg ctg gct taa                 1191
   Glu His Pro Ile Thr Leu Leu Ala Gln Ala Leu Ala
   385                 390                 395
<210> 31
   <211> 396
   <212> PRT
   <213> Escherichia coli
<400> 31
   Met Asn Asp Thr Ser Phe Glu Asn Cys Ile Lys Cys Thr Val Cys Thr
   1               5                   10                  15
   Thr Ala Cys Pro Val Ser Arg Val Asn Pro Gly Tyr Pro Gly Pro Lys
               20                  25                  30
   Gln Ala Gly Pro Asp Gly Glu Arg Leu Arg Leu Lys Asp Gly Ala Leu
           35                  40                  45
   Tyr Asp Glu Ala Leu Lys Tyr Cys Ile Asn Cys Lys Arg Cys Glu Val
       50                  55                  60
   Ala Cys Pro Ser Asp Val Lys Ile Gly Asp Ile Ile Gln Arg Ala Arg
   65                  70                  75                  80
   Ala Lys Tyr Asp Thr Thr Arg Pro Ser Leu Arg Asn Phe Val Leu Ser
                   85                  90                  95
   His Thr Asp Leu Met Gly Ser Val Ser Thr Pro Phe Ala Pro Ile Val
               100                 105                 110
   Asn Thr Ala Thr Ser Leu Lys Pro Val Arg Gln Leu Leu Asp Ala Ala
           115                 120                 125
   Leu Lys Ile Asp His Arg Arg Thr Leu Pro Lys Tyr Ser Phe Gly Thr
       130                 135                 140
   Phe Arg Arg Trp Tyr Arg Ser Val Ala Ala Gln Gln Ala Gln Tyr Lys
   145                 150                 155                 160
   Asp Gln Val Ala Phe Phe His Gly Cys Phe Val Asn Tyr Asn His Pro
                   165                 170                 175
   Gln Leu Gly Lys Asp Leu Ile Lys Val Leu Asn Ala Met Gly Thr Gly
   180 185 190
   Val Gln Leu Leu Ser Lys Glu Lys Cys Cys Gly Val Pro Leu Ile Ala
   195 200 205
   Asn Gly Phe Thr Asp Lys Ala Arg Lys Gln Ala Ile Thr Asn Val Glu
   210 215 220
   Ser Ile Arg Glu Ala Val Gly Val Lys Gly Ile Pro Val Ile Ala Thr
   225 230 235 240
   Ser Ser Thr Cys Thr Phe Ala Leu Arg Asp Glu Tyr Pro Glu Val Leu
   245 250 255
   Asn Val Asp Asn Lys Gly Leu Arg Asp His Ile Glu Leu Ala Thr Arg
   260 265 270
   Trp Leu Trp Arg Lys Leu Asp Glu Gly Lys Thr Leu Pro Leu Lys Pro
   275 280 285
   Leu Pro Leu Lys Val Val Tyr His Thr Pro Cys His Met Glu Lys Met
   290 295 300
   Gly Trp Thr Leu Tyr Thr Leu Glu Leu Leu Arg Asn Ile Pro Gly Leu
   305 310 315 320
   Glu Leu Thr Val Leu Asp Ser Gln Cys Cys Gly Ile Ala Gly Thr Tyr
   325 330 335
   Gly Phe Lys Lys Glu Asn Tyr Pro Thr Ser Gln Ala Ile Gly Ala Pro
   340 345 350
   Leu Phe Arg Gln Ile Glu Glu Ser Gly Ala Asp Leu Val Val Thr Asp
   355 360 365
   Cys Glu Thr Cys Lys Trp Gln Ile Glu Met Ser Thr Ser Leu Arg Cys
   370 375 380
   Glu His Pro Ile Thr Leu Leu Ala Gln Ala Leu Ala
   385 390 395
<210> 32
   <211> 1506
   <212> DNA
   <213> Escherichia coli
<220>
   <221> CDS
   <222> (1).. (1506)
<400> 32
   atg gaa acc aaa gat ctg att gtg ata ggg ggc ggc atc aat ggt gct 48
   Met Glu Thr Lys Asp Leu Ile Val Ile Gly Gly Gly Ile Asn Gly Ala
   1 5 10 15
   ggt atc gcg gca gac gcc gct gga cgc ggt tta tcc gtg ctg atg ctg 96
   Gly Ile Ala Ala Asp Ala Ala Gly Arg Gly Leu Ser Val Leu Met Leu
   20 25 30
   gag gcg cag gat ctc gct tgc gcg acc tct tcc gcc agt tca aaa ctc 144
   Glu Ala Gln Asp Leu Ala Cys Ala Thr Ser Ser Ala Ser Ser Lys Leu
   35 40 45
   att cac ggt ggc ctg cgc tac ctt gag cac tat gaa ttc cgc ctg gtc 192
   Ile His Gly Gly Leu Arg Tyr Leu Glu His Tyr Glu Phe Arg Leu Val
   50 55 60
   agc gag gcg ctg gct gaa cgt gaa gtg ctg ctg aaa atg gcc ccg cat 240
   Ser Glu Ala Leu Ala Glu Arg Glu Val Leu Leu Lys Met Ala Pro His
   65 70 75 80
   atc gcc ttc ccg atg cgt ttt cgc ctg cca cat cgt ccg cat ctg cgc      288
   Ile Ala Phe Pro Met Arg Phe Arg Leu Pro His Arg Pro His Leu Arg
                   85                  90                  95
   ccg gcg tgg atg att cgc att ggt ctg ttt atg tac gat cat ctg ggt      336
   Pro Ala Trp Met Ile Arg Ile Gly Leu Phe Met Tyr Asp His Leu Gly
               100                 105                 110
   aaa cgc acc agc ttg ccg gga tca act ggt ttg cgt ttt ggc gca aat      384
   Lys Arg Thr Ser Leu Pro Gly Ser Thr Gly Leu Arg Phe Gly Ala Asn
           115                 120                 125
   tca gtg tta aaa ccg gaa att aag cgc gga ttc gaa tat tct gac tgt      432
   Ser Val Leu Lys Pro Glu Ile Lys Arg Gly Phe Glu Tyr Ser Asp Cys
       130                 135                 140
   tgg gta gac gac gcc cgt ctg gta ctc gcc aac gcc cag atg gtg gtg      480
   Trp Val Asp Asp Ala Arg Leu Val Leu Ala Asn Ala Gln Met Val Val
   145                 150                 155                 160
   cgt aaa ggc ggc gaa gtg ctt act cgg act cgc gcc acc tct gct cgc      528
   Arg Lys Gly Gly Glu Val Leu Thr Arg Thr Arg Ala Thr Ser Ala Arg
                   165                 170                 175
   cgc gaa aac ggc ctg tgg att gtg gaa gcg gaa gat atc gat acc ggc      576
   Arg Glu Asn Gly Leu Trp Ile Val Glu Ala Glu Asp Ile Asp Thr Gly
               180                 185                 190
   aaa aaa tat agc tgg caa gcg cgc ggc ttg gtt aac gcc acc ggc ccg      624
   Lys Lys Tyr Ser Trp Gln Ala Arg Gly Leu Val Asn Ala Thr Gly Pro
           195                 200                 205
   tgg gtg aaa cag ttc ttc gac gac ggg atg cat ctg cct tcg cct tat      672
   Trp Val Lys Gln Phe Phe Asp Asp Gly Met His Leu Pro Ser Pro Tyr
       210                 215                 220
   ggc att cgc ctg atc aaa ggc agc cat att gtg gtg ccg cgc gtg cat      720
   Gly Ile Arg Leu Ile Lys Gly Ser His Ile Val Val Pro Arg Val His
   225                 230                 235                 240
   acc cag aag caa gcc tac att ctg caa aac gaa gat aaa cgt att gtg      768
   Thr Gln Lys Gln Ala Tyr Ile Leu Gln Asn Glu Asp Lys Arg Ile Val
                   245                 250                 255
   ttc gtg atc ccg tgg atg gac gag ttt tcc atc atc ggc act acc gat      816
   Phe Val Ile Pro Trp Met Asp Glu Phe Ser Ile Ile Gly Thr Thr Asp
               260                 265                 270
   gtc gag tac aaa ggc gat ccg aaa gcg gtg aag att gaa gag agt gaa      864
   Val Glu Tyr Lys Gly Asp Pro Lys Ala Val Lys Ile Glu Glu Ser Glu
           275                 280                 285
   atc aat tac ctg ctg aat gtg tat aac acg cac ttt aaa aag cag tta      912
   Ile Asn Tyr Leu Leu Asn Val Tyr Asn Thr His Phe Lys Lys Gln Leu
       290                 295                 300
   agc cgt gac gat atc gtc tgg acc tac tcc ggt gtg cgt ccg ctg tgt      960
   Ser Arg Asp Asp Ile Val Trp Thr Tyr Ser Gly Val Arg Pro Leu Cys
   305                 310                 315                 320
   gat gat gag tcc gac tcg ccg cag gct att acc cgt gat tac acc ctt     1008
   Asp Asp Glu Ser Asp Ser Pro Gln Ala Ile Thr Arg Asp Tyr Thr Leu
                   325                 330                 335
   gat att cat gat gaa aat ggc aaa gca ccg ctg ctg tcg gta ttc ggc     1056
   Asp Ile His Asp Glu Asn Gly Lys Ala Pro Leu Leu Ser Val Phe Gly
               340                 345                 350
   ggt aag ctg acc acc tac cga aaa ctg gcg gaa cat gcg ctg gaa aaa     1104
   Gly Lys Leu Thr Thr Tyr Arg Lys Leu Ala Glu His Ala Leu Glu Lys
           355                 360                 365
   cta acg ccg tat tat cag ggt att ggc ccg gca tgg acg aaa gag agt     1152
   Leu Thr Pro Tyr Tyr Gln Gly Ile Gly Pro Ala Trp Thr Lys Glu Ser
       370                 375                 380
   gtg cta ccg ggt ggc gcc att gaa ggc gac cgc gac gat tat gcc gct      1200
   Val Leu Pro Gly Gly Ala Ile Glu Gly Asp Arg Asp Asp Tyr Ala Ala
   385                 390                 395                 400
   cgc ctg cgc cgc cgc tat ccg ttc ctg act gaa tcg ctg gcg cgt cat      1248
   Arg Leu Arg Arg Arg Tyr Pro Phe Leu Thr Glu Ser Leu Ala Arg His
                   405                 410                 415
   tac gct cgc act tac ggc agc aac agc gag ctg ctg ctc ggc aat gcg      1296
   Tyr Ala Arg Thr Tyr Gly Ser Asn Ser Glu Leu Leu Leu Gly Asn Ala
               420                 425                 430
   gga acg gta agc gat ctc ggg gaa gat ttc ggt cat gag ttc tac gaa      1344
   Gly Thr Val Ser Asp Leu Gly Glu Asp Phe Gly His Glu Phe Tyr Glu
           435                 440                 445
   gcg gag ctg aaa tac ctg gtg gat cac gaa tgg gtc cgc cgc gcc gac      1392
   Ala Glu Leu Lys Tyr Leu Val Asp His Glu Trp Val Arg Arg Ala Asp
       450                 455                 460
   gac gcc ctg tgg cgt cgc aca aaa caa ggc atg tgg cta aat gcg gat      1440
   Asp Ala Leu Trp Arg Arg Thr Lys Gln Gly Met Trp Leu Asn Ala Asp
   465                 470                 475                 480
   caa caa tct cgt gtg agt cag tgg ctg gtg gag tat acg cag cag agg      1488
   Gln Gln Ser Arg Val Ser Gln Trp Leu Val Glu Tyr Thr Gln Gln Arg
                   485                 490                 495
   tta tcg ctg gcg tcg taa                                              1506
   Leu Ser Leu Ala Ser
               500
<210> 33
   <211> 501
   <212> PRT
   <213> Escherichia coli
<400> 33
   Met Glu Thr Lys Asp Leu Ile Val Ile Gly Gly Gly Ile Asn Gly Ala
   1               5                   10                  15
   Gly Ile Ala Ala Asp Ala Ala Gly Arg Gly Leu Ser Val Leu Met Leu
               20                  25                  30
   Glu Ala Gln Asp Leu Ala Cys Ala Thr Ser Ser Ala Ser Ser Lys Leu
           35                  40                  45
   Ile His Gly Gly Leu Arg Tyr Leu Glu His Tyr Glu Phe Arg Leu Val
       50                  55                  60
   Ser Glu Ala Leu Ala Glu Arg Glu Val Leu Leu Lys Met Ala Pro His
   65                  70                  75                  80
   Ile Ala Phe Pro Met Arg Phe Arg Leu Pro His Arg Pro His Leu Arg
                   85                  90                  95
   Pro Ala Trp Met Ile Arg Ile Gly Leu Phe Met Tyr Asp His Leu Gly
               100                 105                 110
   Lys Arg Thr Ser Leu Pro Gly Ser Thr Gly Leu Arg Phe Gly Ala Asn
           115                 120                 125
   Ser Val Leu Lys Pro Glu Ile Lys Arg Gly Phe Glu Tyr Ser Asp Cys
       130                 135                 140
   Trp Val Asp Asp Ala Arg Leu Val Leu Ala Asn Ala Gln Met Val Val
   145                 150                 155                 160
   Arg Lys Gly Gly Glu Val Leu Thr Arg Thr Arg Ala Thr Ser Ala Arg
                       165                 170                 175
   Arg Glu Asn Gly Leu Trp Ile Val Glu Ala Glu Asp Ile Asp Thr Gly
                   180                 185                 190
   Lys Lys Tyr Ser Trp Gln Ala Arg Gly Leu Val Asn Ala Thr Gly Pro
               195                 200                 205
   Trp Val Lys Gln Phe Phe Asp Asp Gly Met His Leu Pro Ser Pro Tyr
       210                 215                 220
   Gly Ile Arg Leu Ile Lys Gly Ser His Ile Val Val Pro Arg Val His
   225                 230                 235                 240
   Thr Gln Lys Gln Ala Tyr Ile Leu Gln Asn Glu Asp Lys Arg Ile Val
                   245                 250                 255
   Phe Val Ile Pro Trp Met Asp Glu Phe Ser Ile Ile Gly Thr Thr Asp
               260                 265                 270
   Val Glu Tyr Lys Gly Asp Pro Lys Ala Val Lys Ile Glu Glu Ser Glu
           275                 280                 285
   Ile Asn Tyr Leu Leu Asn Val Tyr Asn Thr His Phe Lys Lys Gln Leu
       290                 295                 300
   Ser Arg Asp Asp Ile Val Trp Thr Tyr Ser Gly Val Arg Pro Leu Cys
   305                 310                 315                 320
   Asp Asp Glu Ser Asp Ser Pro Gln Ala Ile Thr Arg Asp Tyr Thr Leu
                   325                 330                 335
   Asp Ile His Asp Glu Asn Gly Lys Ala Pro Leu Leu Ser Val Phe Gly
               340                 345                 350
   Gly Lys Leu Thr Thr Tyr Arg Lys Leu Ala Glu His Ala Leu Glu Lys
           355                 360                 365
   Leu Thr Pro Tyr Tyr Gln Gly Ile Gly Pro Ala Trp Thr Lys Glu Ser
       370                 375                 380
   Val Leu Pro Gly Gly Ala Ile Glu Gly Asp Arg Asp Asp Tyr Ala Ala
   385                 390                 395                 400
   Arg Leu Arg Arg Arg Tyr Pro Phe Leu Thr Glu Ser Leu Ala Arg His
                   405                 410                 415
   Tyr Ala Arg Thr Tyr Gly Ser Asn Ser Glu Leu Leu Leu Gly Asn Ala
               420                 425                 430
   Gly Thr Val Ser Asp Leu Gly Glu Asp Phe Gly His Glu Phe Tyr Glu
           435                 440                 445
   Ala Glu Leu Lys Tyr Leu Val Asp His Glu Trp Val Arg Arg Ala Asp
       450                 455                 460
   Asp Ala Leu Trp Arg Arg Thr Lys Gln Gly Met Trp Leu Asn Ala Asp
   465                 470                 475                 480
   Gln Gln Ser Arg Val Ser Gln Trp Leu Val Glu Tyr Thr Gln Gln Arg
                   485                 490                 495
   Leu Ser Leu Ala Ser
               500
<210> 34
   <211> 1071
   <212> DNA
   <213> Escherichia coli
<220>
   <221> CDS
   <222> (1).. (1071)
<400> 34
   atg aaa aaa ttg atc aat gat gtg caa gac gta ctg gac gaa caa ctg 48
   Met Lys Lys Leu Ile Asn Asp Val Gln Asp Val Leu Asp Glu Gln Leu
   1 5 10 15
   gca gga ctg gcg aaa gcg cat cca tcg ctg aca ctg cat cag gat ccg 96
   Ala Gly Leu Ala Lys Ala His Pro Ser Leu Thr Leu His Gln Asp Pro
   20 25 30
   gtg tat gtc acc cga gct gat gcc cct gtt gca gga aaa gtc gcc ctg 144
   Val Tyr Val Thr Arg Ala Asp Ala Pro Val Ala Gly Lys Val Ala Leu
   35 40 45
   ctg tcg ggt ggc ggc agc gga cac gag ccg atg cac tgt ggt tat atc 192
   Leu Ser Gly Gly Gly Ser Gly His Glu Pro Met His Cys Gly Tyr Ile
   50 55 60
   ggt cag ggg atg ctt tcg ggg gcc tgt ccg ggc gaa att ttc acc tca 240
   Gly Gln Gly Met Leu Ser Gly Ala Cys Pro Gly Glu Ile Phe Thr Ser
   65 70 75 80
   ccg acg ccc gat aaa atc ttt gaa tgc gcc atg caa gtt gat ggc ggc 288
   Pro Thr Pro Asp Lys Ile Phe Glu Cys Ala Met Gln Val Asp Gly Gly
   85 90 95
   gaa ggt gta ctg ttg att atc aaa aat tac acc ggc gat att ctt aac 336
   Glu Gly Val Leu Leu Ile Ile Lys Asn Tyr Thr Gly Asp Ile Leu Asn
   100 105 110
   ttt gaa aca gcg acc gag tta ctg cac gat agc ggc gta aaa gtg acc 384
   Phe Glu Thr Ala Thr Glu Leu Leu His Asp Ser Gly Val Lys Val Thr
   115 120 125
   act gtg gtc att gat gac gac gtt gcg gta aaa gac agt ctt tat act 432
   Thr Val Val Ile Asp Asp Asp Val Ala Val Lys Asp Ser Leu Tyr Thr
   130 135 140
   gcc ggg cga cgc ggc gtt gcc aac acc gta tta att gaa aaa ctc gta 480
   Ala Gly Arg Arg Gly Val Ala Asn Thr Val Leu Ile Glu Lys Leu Val
   145 150 155 160
   ggc gca gcg gcg gag cgt ggc gac tca ctg gac gcc tgt gcg gaa ctg 528
   Gly Ala Ala Ala Glu Arg Gly Asp Ser Leu Asp Ala Cys Ala Glu Leu
   165 170 175
   ggg cgt aag ctg aat aat caa ggc cac tca ata ggt atc gct ctc ggt 576
   Gly Arg Lys Leu Asn Asn Gln Gly His Ser Ile Gly Ile Ala Leu Gly
   180 185 190
   gcc tgt acc gtt cct gcc gcg ggc aaa cct tct ttt acc ctg gcg gat 624
   Ala Cys Thr Val Pro Ala Ala Gly Lys Pro Ser Phe Thr Leu Ala Asp
   195 200 205
   aat gag atg gag ttt ggc gtc ggc att cat ggt gag ccg ggt att gac 672
   Asn Glu Met Glu Phe Gly Val Gly Ile His Gly Glu Pro Gly Ile Asp
   210 215 220
   cgc cgc ccc ttc tct tcc ctt gat caa acc gtc gat gaa atg ttc gac 720
   Arg Arg Pro Phe Ser Ser Leu Asp Gln Thr Val Asp Glu Met Phe Asp
   225 230 235 240
   acc ctg ctg gta aat ggc tca tac cat cgc act ttg cgt ttc tgg gat 768
   Thr Leu Leu Val Asn Gly Ser Tyr His Arg Thr Leu Arg Phe Trp Asp
   245 250 255
   tat caa caa ggc agt tgg cag gaa gaa caa caa acc aaa caa ccg ctc 816
   Tyr Gln Gln Gly Ser Trp Gln Glu Glu Gln Gln Thr Lys Gln Pro Leu
   260 265 270
   cag tct ggc gat cgg gtg att gcg ctg gtt aac aat ctt ggc gca act 864
   Gln Ser Gly Asp Arg Val Ile Ala Leu Val Asn Asn Leu Gly Ala Thr
   275 280 285
   ccg ctt tct gag ctg tac ggc gtc tat aac cgc ctg acc aca cgt tgc 912
   Pro Leu Ser Glu Leu Tyr Gly Val Tyr Asn Arg Leu Thr Thr Arg Cys
       290                 295                 300
   cag caa gcg gga ttg act atc gaa cgt aat tta att ggc gcg tac tgc       960
   Gln Gln Ala Gly Leu Thr Ile Glu Arg Asn Leu Ile Gly Ala Tyr Cys
   305                 310                 315                 320
   acc tca ctg gat atg acc ggt ttc tca atc acc tta ctg aaa gtt gat      1008
   Thr Ser Leu Asp Met Thr Gly Phe Ser Ile Thr Leu Leu Lys Val Asp
                   325                 330                 335
   gac gaa acg ctg gca ctc tgg gac gcc ccg gtc cac acc ccg gcc ctt      1056
   Asp Glu Thr Leu Ala Leu Trp Asp Ala Pro Val His Thr Pro Ala Leu
               340                 345                 350
   aac tgg ggt aaa taa                                                  1071
   Asn Trp Gly Lys
           355
<210> 35
   <211> 356
   <212> PRT
   <213> Escherichia coli
<400> 35
   Met Lys Lys Leu Ile Asn Asp Val Gln Asp Val Leu Asp Glu Gln Leu
   1               5                   10                  15
   Ala Gly Leu Ala Lys Ala His Pro Ser Leu Thr Leu His Gln Asp Pro
               20                  25                  30
   Val Tyr Val Thr Arg Ala Asp Ala Pro Val Ala Gly Lys Val Ala Leu
           35                  40                  45
   Leu Ser Gly Gly Gly Ser Gly His Glu Pro Met His Cys Gly Tyr Ile
       50                  55                  60
   Gly Gln Gly Met Leu Ser Gly Ala Cys Pro Gly Glu Ile Phe Thr Ser
   65                  70                  75                  80
   Pro Thr Pro Asp Lys Ile Phe Glu Cys Ala Met Gln Val Asp Gly Gly
                   85                  90                  95
   Glu Gly Val Leu Leu Ile Ile Lys Asn Tyr Thr Gly Asp Ile Leu Asn
               100                 105                 110
   Phe Glu Thr Ala Thr Glu Leu Leu His Asp Ser Gly Val Lys Val Thr
           115                 120                 125
   Thr Val Val Ile Asp Asp Asp Val Ala Val Lys Asp Ser Leu Tyr Thr
       130                 135                 140
   Ala Gly Arg Arg Gly Val Ala Asn Thr Val Leu Ile Glu Lys Leu Val
   145                 150                 155                 160
   Gly Ala Ala Ala Glu Arg Gly Asp Ser Leu Asp Ala Cys Ala Glu Leu
                   165                 170                 175
   Gly Arg Lys Leu Asn Asn Gln Gly His Ser Ile Gly Ile Ala Leu Gly
               180                 185                 190
   Ala Cys Thr Val Pro Ala Ala Gly Lys Pro Ser Phe Thr Leu Ala Asp
           195                 200                 205
   Asn Glu Met Glu Phe Gly Val Gly Ile His Gly Glu Pro Gly Ile Asp
       210                 215                 220
   Arg Arg Pro Phe Ser Ser Leu Asp Gln Thr Val Asp Glu Met Phe Asp
   225                 230                 235                 240
   Thr Leu Leu Val Asn Gly Ser Tyr His Arg Thr Leu Arg Phe Trp Asp
                   245                 250                 255
   Tyr Gln Gln Gly Ser Trp Gln Glu Glu Gln Gln Thr Lys Gln Pro Leu
               260                 265                 270
   Gln Ser Gly Asp Arg Val Ile Ala Leu Val Asn Asn Leu Gly Ala Thr
           275                 280                 285
   Pro Leu Ser Glu Leu Tyr Gly Val Tyr Asn Arg Leu Thr Thr Arg Cys
       290                 295                 300
   Gln Gln Ala Gly Leu Thr Ile Glu Arg Asn Leu Ile Gly Ala Tyr Cys
   305                 310                 315                 320
   Thr Ser Leu Asp Met Thr Gly Phe Ser Ile Thr Leu Leu Lys Val Asp
                   325                 330                 335
   Asp Glu Thr Leu Ala Leu Trp Asp Ala Pro Val His Thr Pro Ala Leu
               340                 345                 350
   Asn Trp Gly Lys
           355
<210> 36
   <211> 633
   <212> DNA
   <213> Escherichia coli
<220>
   <221> CDS
   <222> (1).. (633)
<400> 36
   atg tca ctg agc aga act caa att gtt aac tgg ctc act cgt tgt ggc       48
   Met Ser Leu Ser Arg Thr Gln Ile Val Asn Trp Leu Thr Arg Cys Gly
   1               5                   10                  15
   gat att ttc agc acc gag agc gag tat ctt acc gga ctg gat cgc gaa       96
   Asp Ile Phe Ser Thr Glu Ser Glu Tyr Leu Thr Gly Leu Asp Arg Glu
               20                  25                  30
   att ggc gat gct gac cac ggg cta aat atg aac cga ggc ttt agc aaa      144
   Ile Gly Asp Ala Asp His Gly Leu Asn Met Asn Arg Gly Phe Ser Lys
           35                  40                  45
   gtg gtg gaa aaa ctc cct gct atc gca gat aaa gat atc ggt ttc att      192
   Val Val Glu Lys Leu Pro Ala Ile Ala Asp Lys Asp Ile Gly Phe Ile
       50                  55                  60
   ctc aag aat acc ggt atg acg ctg ctt tcc agc gtc ggt ggt gcc agt      240
   Leu Lys Asn Thr Gly Met Thr Leu Leu Ser Ser Val Gly Gly Ala Ser
   65                  70                  75                  80
   ggt ccg ctg ttc ggt acc ttc ttt atc cgc gcc gca cag gcg acc cag      288
   Gly Pro Leu Phe Gly Thr Phe Phe Ile Arg Ala Ala Gln Ala Thr Gln
                   85                  90                  95
   gca cgg caa agc ctg aca ctg gaa gag ctt tat cag atg ttc cgc gat      336
   Ala Arg Gln Ser Leu Thr Leu Glu Glu Leu Tyr Gln Met Phe Arg Asp
               100                 105                 110
   ggc gcg gac ggc gta atc agt cgc ggg aaa gcc gaa cct ggc gat aaa      384
   Gly Ala Asp Gly Val Ile Ser Arg Gly Lys Ala Glu Pro Gly Asp Lys
           115                 120                 125
   acc atg tgt gat gtg tgg gtg ccg gtg gtg gaa tcg tta cgt cag tcc      432
   Thr Met Cys Asp Val Trp Val Pro Val Val Glu Ser Leu Arg Gln Ser
       130                 135                 140
   agc gag caa aat ctc tct gtt ccg gtg gcg ctc gaa gct gcc agt agc      480
   Ser Glu Gln Asn Leu Ser Val Pro Val Ala Leu Glu Ala Ala Ser Ser
   145                 150                 155                 160
   atc gcc gaa tcc gct gca caa agt acg att acg atg caa gcc cgc aaa      528
   Ile Ala Glu Ser Ala Ala Gln Ser Thr Ile Thr Met Gln Ala Arg Lys
                   165                 170                 175
   ggc cgc gcc agt tat ctc ggt gaa cgc agt att ggt cac cag gat ccc      576
   Gly Arg Ala Ser Tyr Leu Gly Glu Arg Ser Ile Gly His Gln Asp Pro
               180                 185                 190
   ggc gcg acc tcg gtg atg ttt atg atg caa atg ttg gcg tta gcc gca      624
   Gly Ala Thr Ser Val Met Phe Met Met Gln Met Leu Ala Leu Ala Ala
           195                 200                 205
   aaa gag taa                                                          633
   Lys Glu
       210
<210> 37
   <211> 210
   <212> PRT
   <213> Escherichia coli
<400> 37
   Met Ser Leu Ser Arg Thr Gln Ile Val Asn Trp Leu Thr Arg Cys Gly
   1               5                   10                  15
   Asp Ile Phe Ser Thr Glu Ser Glu Tyr Leu Thr Gly Leu Asp Arg Glu
               20                  25                  30
   Ile Gly Asp Ala Asp His Gly Leu Asn Met Asn Arg Gly Phe Ser Lys
           35                  40                  45
   Val Val Glu Lys Leu Pro Ala Ile Ala Asp Lys Asp Ile Gly Phe Ile
       50                  55                  60
   Leu Lys Asn Thr Gly Met Thr Leu Leu Ser Ser Val Gly Gly Ala Ser
   65                  70                  75                  80
   Gly Pro Leu Phe Gly Thr Phe Phe Ile Arg Ala Ala Gln Ala Thr Gln
                   85                  90                  95
   Ala Arg Gln Ser Leu Thr Leu Glu Glu Leu Tyr Gln Met Phe Arg Asp
               100                 105                 110
   Gly Ala Asp Gly Val Ile Ser Arg Gly Lys Ala Glu Pro Gly Asp Lys
           115                 120                 125
   Thr Met Cys Asp Val Trp Val Pro Val Val Glu Ser Leu Arg Gln Ser
       130                 135                 140
   Ser Glu Gln Asn Leu Ser Val Pro Val Ala Leu Glu Ala Ala Ser Ser
   145                 150                 155                 160
   Ile Ala Glu Ser Ala Ala Gln Ser Thr Ile Thr Met Gln Ala Arg Lys
                   165                 170                 175
   Gly Arg Ala Ser Tyr Leu Gly Glu Arg Ser Ile Gly His Gln Asp Pro
               180                 185                 190
   Gly Ala Thr Ser Val Met Phe Met Met Gln Met Leu Ala Leu Ala Ala
           195                 200                 205
   Lys Glu
       210
<210> 38
   <211> 1419
   <212> DNA
   <213> Escherichia coli
<220>
   <221> CDS
   <222> (1).. (1419)
<400> 38
   atg gta aac ctg gtc ata gtt tca cat agc agc cga ctg gga gaa ggt      48
   Met Val Asn Leu Val Ile Val Ser His Ser Ser Arg Leu Gly Glu Gly
   1               5                   10                  15
   gtc ggt gaa tta gcc cgt cag atg tta atg agt gat agt tgt aaa atc      96
   Val Gly Glu Leu Ala Arg Gln Met Leu Met Ser Asp Ser Cys Lys Ile
               20                  25                  30
   gcc att gcc gcg gga att gac gat cca caa aat ccc att ggt acc gat     144
   Ala Ile Ala Ala Gly Ile Asp Asp Pro Gln Asn Pro Ile Gly Thr Asp
           35                  40                  45
   gcc gtc aaa gtg atg gag gcc atc gaa tct gtt gct gat gcc gac cat     192
   Ala Val Lys Val Met Glu Ala Ile Glu Ser Val Ala Asp Ala Asp His
       50                  55                  60
   gtg ctg gtc atg atg gat atg ggt agc gca tta ttg agt gct gaa act     240
   Val Leu Val Met Met Asp Met Gly Ser Ala Leu Leu Ser Ala Glu Thr
   65                  70                  75                  80
   gcg ctg gaa ttg ctg gct ccc gag atc gcc gca aaa gta cgt ttg tgt     288
   Ala Leu Glu Leu Leu Ala Pro Glu Ile Ala Ala Lys Val Arg Leu Cys
                   85                  90                  95
   gct gcg ccg ttg gtc gaa ggt aca ctg gca gca acg gtc agc gcg gcc     336
   Ala Ala Pro Leu Val Glu Gly Thr Leu Ala Ala Thr Val Ser Ala Ala
               100                 105                 110
   tcg ggg gcg gat atc gac aaa gtt atc ttt gac gcc atg cat gcg ctg     384
   Ser Gly Ala Asp Ile Asp Lys Val Ile Phe Asp Ala Met His Ala Leu
           115                 120                 125
   gaa gcc aaa cgt gaa caa ctg ggt tta ccg tcc tcc gac act gaa atc     432
   Glu Ala Lys Arg Glu Gln Leu Gly Leu Pro Ser Ser Asp Thr Glu Ile
       130                 135                 140
   tct gac aca tgt cct gcg tac gat gaa gaa gcc cgt tct ctg gcg gtg     480
   Ser Asp Thr Cys Pro Ala Tyr Asp Glu Glu Ala Arg Ser Leu Ala Val
   145                 150                 155                 160
   gtc ata aaa aac cgt aac ggc ctg cat gta cgt ccg gcc tcc cgg ctg     528
   Val Ile Lys Asn Arg Asn Gly Leu His Val Arg Pro Ala Ser Arg Leu
                   165                 170                 175
   gtt tat acc tta tcg aca ttt aat gcc gat atg ttg ctg gaa aaa aac     576
   Val Tyr Thr Leu Ser Thr Phe Asn Ala Asp Met Leu Leu Glu Lys Asn
               180                 185                 190
   ggc aaa tgc gtc aca cca gag agt att aac cag att gcg tta cta caa     624
   Gly Lys Cys Val Thr Pro Glu Ser Ile Asn Gln Ile Ala Leu Leu Gln
           195                 200                 205
   gtt cgc tat aac gat acg ctg cgc ctg att gcg aaa ggg cca gaa gct     672
   Val Arg Tyr Asn Asp Thr Leu Arg Leu Ile Ala Lys Gly Pro Glu Ala
       210                 215                 220
   gaa gag gca ctg atc gct ttc cgt cag ctg gct gaa gat aac ttt ggt     720
   Glu Glu Ala Leu Ile Ala Phe Arg Gln Leu Ala Glu Asp Asn Phe Gly
   225                 230                 235                 240
   gaa acg gag gaa gtc gct cca cct act ctg cgt ccc gtt ccg cct gtt     768
   Glu Thr Glu Glu Val Ala Pro Pro Thr Leu Arg Pro Val Pro Pro Val
                   245                 250                 255
   tcg ggt aaa gcc ttt tat tat caa cca gtt tta tgt acg gta cag gca     816
   Ser Gly Lys Ala Phe Tyr Tyr Gln Pro Val Leu Cys Thr Val Gln Ala
                   260                 265                 270
   aaa tca acc ctg acc gtg gaa gaa gaa caa gat cga tta cgc cag gct      864
   Lys Ser Thr Leu Thr Val Glu Glu Glu Gln Asp Arg Leu Arg Gln Ala
               275                 280                 285
   att gac ttc acg tta tta gat ctg atg acg tta aca gcg aaa gca gaa      912
   Ile Asp Phe Thr Leu Leu Asp Leu Met Thr Leu Thr Ala Lys Ala Glu
           290                 295                 300
   gcc agc ggg ctt gac gat att gcc gca atc ttt tct ggt cac cat aca      960
   Ala Ser Gly Leu Asp Asp Ile Ala Ala Ile Phe Ser Gly His His Thr
   305                 310                 315                 320
   ctg tta gat gat ccg gaa ctg ctg gcg gcg gca agc gaa ctc ctt cag     1008
   Leu Leu Asp Asp Pro Glu Leu Leu Ala Ala Ala Ser Glu Leu Leu Gln
                   325                 330                 335
   cat gaa cat tgc acg gca gaa tat gcc tgg cag caa gtt ctt aaa gaa     1056
   His Glu His Cys Thr Ala Glu Tyr Ala Trp Gln Gln Val Leu Lys Glu
               340                 345                 350
   ctt agc cag caa tac cag caa ctg gat gat gaa tat cta caa gct cgc     1104
   Leu Ser Gln Gln Tyr Gln Gln Leu Asp Asp Glu Tyr Leu Gln Ala Arg
           355                 360                 365
   tat att gat gtg gac gat ctt ctg cat cgc acc ctg gtc cac ctg acc     1152
   Tyr Ile Asp Val Asp Asp Leu Leu His Arg Thr Leu Val His Leu Thr
       370                 375                 380
   caa acg aaa gaa gaa ctc ccg cag ttt aac tcg cca act att cta ctg     1200
   Gln Thr Lys Glu Glu Leu Pro Gln Phe Asn Ser Pro Thr Ile Leu Leu
   385                 390                 395                 400
   gcg gag aac att tat cct tcc aca gta ctg caa ctg gat ccg gcg gtt     1248
   Ala Glu Asn Ile Tyr Pro Ser Thr Val Leu Gln Leu Asp Pro Ala Val
                   405                 410                 415
   gta aaa ggt atc tgc ctt agc gcc gga agt ccg gta tcc cac agc gcc     1296
   Val Lys Gly Ile Cys Leu Ser Ala Gly Ser Pro Val Ser His Ser Ala
               420                 425                 430
   cta atc gcc cgt gaa ctg ggg att ggc tgg att tgc cag cag ggt gag     1344
   Leu Ile Ala Arg Glu Leu Gly Ile Gly Trp Ile Cys Gln Gln Gly Glu
           435                 440                 445
   aaa ctg tat gcg ata caa cca gaa gaa acg cta acg ctg gac gtt aaa     1392
   Lys Leu Tyr Ala Ile Gln Pro Glu Glu Thr Leu Thr Leu Asp Val Lys
       450                 455                 460
   acg caa cgt ttc aac cgt cag ggt taa                                 1419
   Thr Gln Arg Phe Asn Arg Gln Gly
   465                 470
<210> 39
   <211> 472
   <212> PRT
   <213> Escherichia coli
<400> 39
   Met Val Asn Leu Val Ile Val Ser His Ser Ser Arg Leu Gly Glu Gly
   1               5                   10                  15
   Val Gly Glu Leu Ala Arg Gln Met Leu Met Ser Asp Ser Cys Lys Ile
               20                  25                  30
   Ala Ile Ala Ala Gly Ile Asp Asp Pro Gln Asn Pro Ile Gly Thr Asp
           35                  40                  45
   Ala Val Lys Val Met Glu Ala Ile Glu Ser Val Ala Asp Ala Asp His
   50 55 60
   Val Leu Val Met Met Asp Met Gly Ser Ala Leu Leu Ser Ala Glu Thr
   65 70 75 80
   Ala Leu Glu Leu Leu Ala Pro Glu Ile Ala Ala Lys Val Arg Leu Cys
   85 90 95
   Ala Ala Pro Leu Val Glu Gly Thr Leu Ala Ala Thr Val Ser Ala Ala
   100 105 110
   Ser Gly Ala Asp Ile Asp Lys Val Ile Phe Asp Ala Met His Ala Leu
   115 120 125
   Glu Ala Lys Arg Glu Gln Leu Gly Leu Pro Ser Ser Asp Thr Glu Ile
   130 135 140
   Ser Asp Thr Cys Pro Ala Tyr Asp Glu Glu Ala Arg Ser Leu Ala Val
   145 150 155 160
   Val Ile Lys Asn Arg Asn Gly Leu His Val Arg Pro Ala Ser Arg Leu
   165 170 175
   Val Tyr Thr Leu Ser Thr Phe Asn Ala Asp Met Leu Leu Glu Lys Asn
   180 185 190
   Gly Lys Cys Val Thr Pro Glu Ser Ile Asn Gln Ile Ala Leu Leu Gln
   195 200 205
   Val Arg Tyr Asn Asp Thr Leu Arg Leu Ile Ala Lys Gly Pro Glu Ala
   210 215 220
   Glu Glu Ala Leu Ile Ala Phe Arg Gln Leu Ala Glu Asp Asn Phe Gly
   225 230 235 240
   Glu Thr Glu Glu Val Ala Pro Pro Thr Leu Arg Pro Val Pro Pro Val
   245 250 255
   Ser Gly Lys Ala Phe Tyr Tyr Gln Pro Val Leu Cys Thr Val Gln Ala
   260 265 270
   Lys Ser Thr Leu Thr Val Glu Glu Glu Gln Asp Arg Leu Arg Gln Ala
   275 280 285
   Ile Asp Phe Thr Leu Leu Asp Leu Met Thr Leu Thr Ala Lys Ala Glu
   290 295 300
   Ala Ser Gly Leu Asp Asp Ile Ala Ala Ile Phe Ser Gly His His Thr
   305 310 315 320
   Leu Leu Asp Asp Pro Glu Leu Leu Ala Ala Ala Ser Glu Leu Leu Gln
   325 330 335
   His Glu His Cys Thr Ala Glu Tyr Ala Trp Gln Gln Val Leu Lys Glu
   340 345 350
   Leu Ser Gln Gln Tyr Gln Gln Leu Asp Asp Glu Tyr Leu Gln Ala Arg
   355 360 365
   Tyr Ile Asp Val Asp Asp Leu Leu His Arg Thr Leu Val His Leu Thr
   370 375 380
   Gln Thr Lys Glu Glu Leu Pro Gln Phe Asn Ser Pro Thr Ile Leu Leu
   385 390 395 400
   Ala Glu Asn Ile Tyr Pro Ser Thr Val Leu Gln Leu Asp Pro Ala Val
   405 410 415
   Val Lys Gly Ile Cys Leu Ser Ala Gly Ser Pro Val Ser His Ser Ala
   420 425 430
   Leu Ile Ala Arg Glu Leu Gly Ile Gly Trp Ile Cys Gln Gln Gly Glu
   435 440 445
   Lys Leu Tyr Ala Ile Gln Pro Glu Glu Thr Leu Thr Leu Asp Val Lys
   450 455 460
   Thr Gln Arg Phe Asn Arg Gln Gly
   465 470
<210> 40
   <211> 1776
   <212> DNA
   <213> Schizosaccharomyces pombe
<220>
   <221> CDS
   <222> (1).. (1776)
<400> 40
   atg tct gta aaa caa ttt gta tct gaa gga cat ata gta cgc cca tac       48
   Met Ser Val Lys Gln Phe Val Ser Glu Gly His Ile Val Arg Pro Tyr
   1               5                   10                  15
   ttg ctt gga ctt gct aga agt aac cct ggc cta act gtt att gaa cat       96
   Leu Leu Gly Leu Ala Arg Ser Asn Pro Gly Leu Thr Val Ile Glu His
               20                  25                  30
   gat cgt gta att tac agg aca gcc tct gcc ccg ggt tct ggt gat ccg      144
   Asp Arg Val Ile Tyr Arg Thr Ala Ser Ala Pro Gly Ser Gly Asp Pro
           35                  40                  45
   cct aaa gta act tta gtg tct ggt ggt ggt agt ggg cac gag cct aca      192
   Pro Lys Val Thr Leu Val Ser Gly Gly Gly Ser Gly His Glu Pro Thr
       50                  55                  60
   cat gct ggt ttt gtt ggt gat gga gcc ttg gac gcc gta gct tgt gga      240
   His Ala Gly Phe Val Gly Asp Gly Ala Leu Asp Ala Val Ala Cys Gly
   65                  70                  75                  80
   gac att ttt gct tct ccc tct act aaa caa att tac tct gct ctc aaa      288
   Asp Ile Phe Ala Ser Pro Ser Thr Lys Gln Ile Tyr Ser Ala Leu Lys
                   85                  90                  95
   gct gtt gca tct cct aag gga act tta att att gtc aaa aat tac aca      336
   Ala Val Ala Ser Pro Lys Gly Thr Leu Ile Ile Val Lys Asn Tyr Thr
               100                 105                 110
   ggt gat att att cat ttt ggc ttg gca gct gag aga gcc aag gca gct      384
   Gly Asp Ile Ile His Phe Gly Leu Ala Ala Glu Arg Ala Lys Ala Ala
           115                 120                 125
   gga atg aac gtt gag ctt gtt gcc gta gga gat gat gtt tct gta ggc      432
   Gly Met Asn Val Glu Leu Val Ala Val Gly Asp Asp Val Ser Val Gly
       130                 135                 140
   aaa aag aga ggt gca ctc gta gga cga aga ggt tta gga gcc act gtg      480
   Lys Lys Arg Gly Ala Leu Val Gly Arg Arg Gly Leu Gly Ala Thr Val
   145                 150                 155                 160
   cta gta cac aaa att gcg gga tct gca gca gca tta gga ctg gat ctg      528
   Leu Val His Lys Ile Ala Gly Ser Ala Ala Ala Leu Gly Leu Asp Leu
                   165                 170                 175
   cat caa gtt gct caa gtg gcc caa tca gtg att gac aat gct gct acc      576
   His Gln Val Ala Gln Val Ala Gln Ser Val Ile Asp Asn Ala Ala Thr
               180                 185                 190
   ata gca gct tca ttg gat cac tgc gcc gtt ccc ggt cgc aaa ttt gaa      624
   Ile Ala Ala Ser Leu Asp His Cys Ala Val Pro Gly Arg Lys Phe Glu
           195                 200                 205
   aca aac ttg ggt cca gac gag tat gag att gga atg ggt att cat aac      672
   Thr Asn Leu Gly Pro Asp Glu Tyr Glu Ile Gly Met Gly Ile His Asn
       210                 215                 220
   gag cct gga acc ttc aaa tca tca cca ctt cca tct att ccc gag cta      720
   Glu Pro Gly Thr Phe Lys Ser Ser Pro Leu Pro Ser Ile Pro Glu Leu
   225                 230                 235                 240
   gtt aca gag atg ctt tcg att ttg ttt ggt gag aaa aac ccg gac aat       768
   Val Thr Glu Met Leu Ser Ile Leu Phe Gly Glu Lys Asn Pro Asp Asn
                   245                 250                 255
   agt ttc gta gag ttt tct tca aaa gac gat gtt att ctt cta gta aac       816
   Ser Phe Val Glu Phe Ser Ser Lys Asp Asp Val Ile Leu Leu Val Asn
               260                 265                 270
   aat atg ggt ggt atg tcc aac tta gag ttg gga tat gct act gaa gta       864
   Asn Met Gly Gly Met Ser Asn Leu Glu Leu Gly Tyr Ala Thr Glu Val
           275                 280                 285
   gtt tcg gaa caa tta gcc aaa cga ggt atc att cct aaa aga acc atg       912
   Val Ser Glu Gln Leu Ala Lys Arg Gly Ile Ile Pro Lys Arg Thr Met
       290                 295                 300
   tct ggt act ttt gta acc gca ttg aac gga ccg ggt ttt gga att aca       960
   Ser Gly Thr Phe Val Thr Ala Leu Asn Gly Pro Gly Phe Gly Ile Thr
   305                 310                 315                 320
   ttg gtc aat gct tct aag gct act cca gat att ttc aaa tat ttt gac      1008
   Leu Val Asn Ala Ser Lys Ala Thr Pro Asp Ile Phe Lys Tyr Phe Asp
                   325                 330                 335
   ttg cca aca act gct agt gga tgg aac gtt tct tat cat aac gca aag      1056
   Leu Pro Thr Thr Ala Ser Gly Trp Asn Val Ser Tyr His Asn Ala Lys
               340                 345                 350
   gac tgg gag gtt ttg gct gac ggc aag gtg cca aca gct ccc gct ttg      1104
   Asp Trp Glu Val Leu Ala Asp Gly Lys Val Pro Thr Ala Pro Ala Leu
           355                 360                 365
   gag cat acc cgt aat gag aag cac agc ggt gta aag gct gac cca aag      1152
   Glu His Thr Arg Asn Glu Lys His Ser Gly Val Lys Ala Asp Pro Lys
       370                 375                 380
   atg ttt act aaa att tta aaa gct gcc gtt gac gct atc aat gaa ttt      1200
   Met Phe Thr Lys Ile Leu Lys Ala Ala Val Asp Ala Ile Asn Glu Phe
   385                 390                 395                 400
   gag cca aag aca act tgg tac gat acg att gca gga gat ggt gat tgt      1248
   Glu Pro Lys Thr Thr Trp Tyr Asp Thr Ile Ala Gly Asp Gly Asp Cys
                   405                 410                 415
   gga aca acc ctt gtg aat ggt gga gag gcc atc ata aag gct att aat      1296
   Gly Thr Thr Leu Val Asn Gly Gly Glu Ala Ile Ile Lys Ala Ile Asn
               420                 425                 430
   gat aaa tca att cga ttg gat gat ggt gta aat ggt att gac gat ttg      1344
   Asp Lys Ser Ile Arg Leu Asp Asp Gly Val Asn Gly Ile Asp Asp Leu
           435                 440                 445
   gcc tat att gtt gag gat tcg atg gga ggt aca tcg ggt ggt ctt tac      1392
   Ala Tyr Ile Val Glu Asp Ser Met Gly Gly Thr Ser Gly Gly Leu Tyr
       450                 455                 460
   tcg att tat ttg tct gcg cta gcc aaa gga gtt cgt gag tca ggc gat      1440
   Ser Ile Tyr Leu Ser Ala Leu Ala Lys Gly Val Arg Glu Ser Gly Asp
   465                 470                 475                 480
   tcc gaa tta tcc gtg cat acg ttt gcg ttt gca agc aag tat gca ctt      1488
   Ser Glu Leu Ser Val His Thr Phe Ala Phe Ala Ser Lys Tyr Ala Leu
                   485                 490                 495
   gac gct ctt ttc aaa tac act agg gcc cgt aaa gga ttc cgt act ctg      1536
   Asp Ala Leu Phe Lys Tyr Thr Arg Ala Arg Lys Gly Phe Arg Thr Leu
               500                 505                 510
   atc gat gct att caa cca ttt gtt gaa act tta aat gaa ggt aag gga      1584
   Ile Asp Ala Ile Gln Pro Phe Val Glu Thr Leu Asn Glu Gly Lys Gly
           515                 520                 525
   ctt gat gct gct gcg aaa gct gcc acg gaa ggt tct gaa caa act aga      1632
   Leu Asp Ala Ala Ala Lys Ala Ala Thr Glu Gly Ser Glu Gln Thr Arg
       530                 535                 540
   aaa atg gat gct gtc gtt gga aga gcg tct tat gtt gct aaa gag gaa      1680
   Lys Met Asp Ala Val Val Gly Arg Ala Ser Tyr Val Ala Lys Glu Glu
   545                 550                 555                 560
   ctt cat aag ctt gat agt gag gga ggc tta ccg gat cct ggt gcc ttt      1728
   Leu His Lys Leu Asp Ser Glu Gly Gly Leu Pro Asp Pro Gly Ala Phe
                   565                 570                 575
   gcg tta gcg gcg atc ttg aaa gca att gtt gag gct agt gaa cat taa      1776
   Ala Leu Ala Ala Ile Leu Lys Ala Ile Val Glu Ala Ser Glu His
               580                 585                 590
<210> 41
   <211> 591
   <212> PRT
   <213> Schizosaccharomyces pombe
<400> 41
   Met Ser Val Lys Gln Phe Val Ser Glu Gly His Ile Val Arg Pro Tyr
   1               5                   10                  15
   Leu Leu Gly Leu Ala Arg Ser Asn Pro Gly Leu Thr Val Ile Glu His
               20                  25                  30
   Asp Arg Val Ile Tyr Arg Thr Ala Ser Ala Pro Gly Ser Gly Asp Pro
           35                  40                  45
   Pro Lys Val Thr Leu Val Ser Gly Gly Gly Ser Gly His Glu Pro Thr
       50                  55                  60
   His Ala Gly Phe Val Gly Asp Gly Ala Leu Asp Ala Val Ala Cys Gly
   65                  70                  75                  80
   Asp Ile Phe Ala Ser Pro Ser Thr Lys Gln Ile Tyr Ser Ala Leu Lys
                   85                  90                  95
   Ala Val Ala Ser Pro Lys Gly Thr Leu Ile Ile Val Lys Asn Tyr Thr
               100                 105                 110
   Gly Asp Ile Ile His Phe Gly Leu Ala Ala Glu Arg Ala Lys Ala Ala
           115                 120                 125
   Gly Met Asn Val Glu Leu Val Ala Val Gly Asp Asp Val Ser Val Gly
       130                 135                 140
   Lys Lys Arg Gly Ala Leu Val Gly Arg Arg Gly Leu Gly Ala Thr Val
   145                 150                 155                 160
   Leu Val His Lys Ile Ala Gly Ser Ala Ala Ala Leu Gly Leu Asp Leu
                   165                 170                 175
   His Gln Val Ala Gln Val Ala Gln Ser Val Ile Asp Asn Ala Ala Thr
               180                 185                 190
   Ile Ala Ala Ser Leu Asp His Cys Ala Val Pro Gly Arg Lys Phe Glu
           195                 200                 205
   Thr Asn Leu Gly Pro Asp Glu Tyr Glu Ile Gly Met Gly Ile His Asn
       210                 215                 220
   Glu Pro Gly Thr Phe Lys Ser Ser Pro Leu Pro Ser Ile Pro Glu Leu
   225                 230                 235                 240
   Val Thr Glu Met Leu Ser Ile Leu Phe Gly Glu Lys Asn Pro Asp Asn
                   245                 250                 255
   Ser Phe Val Glu Phe Ser Ser Lys Asp Asp Val Ile Leu Leu Val Asn
               260                 265                 270
   Asn Met Gly Gly Met Ser Asn Leu Glu Leu Gly Tyr Ala Thr Glu Val
           275                 280                 285
   Val Ser Glu Gln Leu Ala Lys Arg Gly Ile Ile Pro Lys Arg Thr Met
       290                 295                 300
   Ser Gly Thr Phe Val Thr Ala Leu Asn Gly Pro Gly Phe Gly Ile Thr
   305                 310                 315                 320
   Leu Val Asn Ala Ser Lys Ala Thr Pro Asp Ile Phe Lys Tyr Phe Asp
                   325                 330                 335
   Leu Pro Thr Thr Ala Ser Gly Trp Asn Val Ser Tyr His Asn Ala Lys
               340                 345                 350
   Asp Trp Glu Val Leu Ala Asp Gly Lys Val Pro Thr Ala Pro Ala Leu
           355                 360                 365
   Glu His Thr Arg Asn Glu Lys His Ser Gly Val Lys Ala Asp Pro Lys
       370                 375                 380
   Met Phe Thr Lys Ile Leu Lys Ala Ala Val Asp Ala Ile Asn Glu Phe
   385                 390                 395                 400
   Glu Pro Lys Thr Thr Trp Tyr Asp Thr Ile Ala Gly Asp Gly Asp Cys
                   405                 410                 415
   Gly Thr Thr Leu Val Asn Gly Gly Glu Ala Ile Ile Lys Ala Ile Asn
               420                 425                 430
   Asp Lys Ser Ile Arg Leu Asp Asp Gly Val Asn Gly Ile Asp Asp Leu
           435                 440                 445
   Ala Tyr Ile Val Glu Asp Ser Met Gly Gly Thr Ser Gly Gly Leu Tyr
       450                 455                 460
   Ser Ile Tyr Leu Ser Ala Leu Ala Lys Gly Val Arg Glu Ser Gly Asp
   465                 470                 475                 480
   Ser Glu Leu Ser Val His Thr Phe Ala Phe Ala Ser Lys Tyr Ala Leu
                   485                 490                 495
   Asp Ala Leu Phe Lys Tyr Thr Arg Ala Arg Lys Gly Phe Arg Thr Leu
               500                 505                 510
   Ile Asp Ala Ile Gln Pro Phe Val Glu Thr Leu Asn Glu Gly Lys Gly
           515                 520                 525
   Leu Asp Ala Ala Ala Lys Ala Ala Thr Glu Gly Ser Glu Gln Thr Arg
       530                 535                 540
   Lys Met Asp Ala Val Val Gly Arg Ala Ser Tyr Val Ala Lys Glu Glu
   545                 550                 555                 560
   Leu His Lys Leu Asp Ser Glu Gly Gly Leu Pro Asp Pro Gly Ala Phe
                   565                 570                 575
   Ala Leu Ala Ala Ile Leu Lys Ala Ile Val Glu Ala Ser Glu His
               580                 585                 590
<210> 42
   <211> 1830
   <212> DNA
   <213> Pichia angusta
<220>
   <221> CDS
   <222> (1).. (1830)
<400> 42
   atg tcg agc aaa cac tgg aat tac aag caa gac ctg gtc cat gcg cac      48
   Met Ser Ser Lys His Trp Asn Tyr Lys Gln Asp Leu Val His Ala His
   1               5                   10                  15
   ctc aaa ggc ctg tgt cat gcc aat cca gac ctc caa ttc atc gaa tcc      96
   Leu Lys Gly Leu Cys His Ala Asn Pro Asp Leu Gln Phe Ile Glu Ser
               20                  25                  30
   gag cgt gtg gtg atc aac aag cac tcc aag ccc gac aag gtg atg atc      144
   Glu Arg Val Val Ile Asn Lys His Ser Lys Pro Asp Lys Val Met Ile
           35                  40                  45
   cta tct ggt gga ggg tct ggc cac gag cca ttg cac gct ggc ttt gtt      192
   Leu Ser Gly Gly Gly Ser Gly His Glu Pro Leu His Ala Gly Phe Val
       50                  55                  60
   ggt gaa ggc tgt ttg gac gtt gga gtg gct ggt ttt gtt ttc gcc tcg      240
   Gly Glu Gly Cys Leu Asp Val Gly Val Ala Gly Phe Val Phe Ala Ser
   65                  70                  75                  80
   ccc tct aca aag cag att gtt tca ggt ttg aag gca aag ccc tca gac      288
   Pro Ser Thr Lys Gln Ile Val Ser Gly Leu Lys Ala Lys Pro Ser Asp
                   85                  90                  95
   aaa ggt acg cta att gtg gtg aaa aat tac acc ggc gac att ctt cac      336
   Lys Gly Thr Leu Ile Val Val Lys Asn Tyr Thr Gly Asp Ile Leu His
               100                 105                 110
   ttt ggg ctt gct gca gag cgg gcc aag gcc gaa ggc gtc ccc gtg gag      384
   Phe Gly Leu Ala Ala Glu Arg Ala Lys Ala Glu Gly Val Pro Val Glu
           115                 120                 125
   ctg cta att gtc cag gac gac gtt tct gtg ggc aga acc aag aac gga      432
   Leu Leu Ile Val Gln Asp Asp Val Ser Val Gly Arg Thr Lys Asn Gly
       130                 135                 140
   atg gtg ggc aga cgc ggt ctg gcc ggt acg agt ctg gtg cac aag att      480
   Met Val Gly Arg Arg Gly Leu Ala Gly Thr Ser Leu Val His Lys Ile
   145                 150                 155                 160
   gtc ggt gcc aag gcc gcc aag gac tcg aac aaa gcc tcg ttg agc gag      528
   Val Gly Ala Lys Ala Ala Lys Asp Ser Asn Lys Ala Ser Leu Ser Glu
                   165                 170                 175
   gtg tac cag ctg ggc gag gcc gtg gtg gcc aat ctg gtg acc atc ggc      576
   Val Tyr Gln Leu Gly Glu Ala Val Val Ala Asn Leu Val Thr Ile Gly
               180                 185                 190
   gcg tcg ctc gac cac tgc aca att ccg ggt aac aga cac cac gag tcc      624
   Ala Ser Leu Asp His Cys Thr Ile Pro Gly Asn Arg His His Glu Ser
           195                 200                 205
   gag tcc gac gac gag gac gag cag aaa cat ctg ctc aag gag gac gag      672
   Glu Ser Asp Asp Glu Asp Glu Gln Lys His Leu Leu Lys Glu Asp Glu
       210                 215                 220
   atc gag gtg ggt atg ggg atc cac aac gag tcg ggc atc aag cgc gtt      720
   Ile Glu Val Gly Met Gly Ile His Asn Glu Ser Gly Ile Lys Arg Val
   225                 230                 235                 240
   tcg cca atc ccg acc atc gac acg ctt gtg gca gac ctg ctc aag tac      768
   Ser Pro Ile Pro Thr Ile Asp Thr Leu Val Ala Asp Leu Leu Lys Tyr
                   245                 250                 255
   ttg ctc gac aag agc gac gag gag aga cac tat gtg gac ttc gac tcg      816
   Leu Leu Asp Lys Ser Asp Glu Glu Arg His Tyr Val Asp Phe Asp Ser
               260                 265                 270
   tcg gac gag gtt gtg ctg atg atc aac aat ttg ggc ggc acg tcg aac      864
   Ser Asp Glu Val Val Leu Met Ile Asn Asn Leu Gly Gly Thr Ser Asn
           275                 280                 285
   ctc gag ctg tac gct atc cag aac act gtt gtt gag cag ctg gct acc      912
   Leu Glu Leu Tyr Ala Ile Gln Asn Thr Val Val Glu Gln Leu Ala Thr
       290                 295                 300
   gac tac aag atc aag ccc gca aga gtg tac acg ggc gcg tac acc acg      960
   Asp Tyr Lys Ile Lys Pro Ala Arg Val Tyr Thr Gly Ala Tyr Thr Thr
   305                 310                 315                 320
   tcg cta gac ggt cct ggt ttt tcc atc acg ttg ctg aac gtg act cgg      1008
   Ser Leu Asp Gly Pro Gly Phe Ser Ile Thr Leu Leu Asn Val Thr Arg
                   325                 330                 335
   gcg ggg ggc aag gag gtt ttc gat tgt ctg gac tac cca acc aag gtt      1056
   Ala Gly Gly Lys Glu Val Phe Asp Cys Leu Asp Tyr Pro Thr Lys Val
               340                 345                 350
   cct ggg tgg aac tcg tcg tac aca acg gca gaa tgg gcg gcg aaa tcc      1104
   Pro Gly Trp Asn Ser Ser Tyr Thr Thr Ala Glu Trp Ala Ala Lys Ser
           355                 360                 365
   gag tcg ttc gtc atc gac gct ccg cca gtg agc gac gcg tcg gcg acc      1152
   Glu Ser Phe Val Ile Asp Ala Pro Pro Val Ser Asp Ala Ser Ala Thr
       370                 375                 380
   tcg aaa gtg cgg ttc tca agc agc aca gtc aag gct gtg ttg gag agc      1200
   Ser Lys Val Arg Phe Ser Ser Ser Thr Val Lys Ala Val Leu Glu Ser
   385                 390                 395                 400
   gga tgc aag aag ttg ctg acc aag gag cca aag atc acg ctg tac gac      1248
   Gly Cys Lys Lys Leu Leu Thr Lys Glu Pro Lys Ile Thr Leu Tyr Asp
                   405                 410                 415
   acg gtt gcg ggc gac ggc gac tgc ggc gag acg ctg gcc aac ggc gcg      1296
   Thr Val Ala Gly Asp Gly Asp Cys Gly Glu Thr Leu Ala Asn Gly Ala
               420                 425                 430
   cac gcg atc ctg gac ctg ctg gct gcc gac aag ctg gag atc acc gac      1344
   His Ala Ile Leu Asp Leu Leu Ala Ala Asp Lys Leu Glu Ile Thr Asp
           435                 440                 445
   ggt gtc cgg agt ctg acg cag atc act gac gtc gtc gaa acg gct atg      1392
   Gly Val Arg Ser Leu Thr Gln Ile Thr Asp Val Val Glu Thr Ala Met
       450                 455                 460
   gga ggc acc tct ggt ggg ctt tac tcg atc ttt atc tct gca ctc gcc      1440
   Gly Gly Thr Ser Gly Gly Leu Tyr Ser Ile Phe Ile Ser Ala Leu Ala
   465                 470                 475                 480
   aag tcg ttg aag gac aga gag ctc cag cag ggc gga tac gag gtg acg      1488
   Lys Ser Leu Lys Asp Arg Glu Leu Gln Gln Gly Gly Tyr Glu Val Thr
                   485                 490                 495
   ccg cag atc ctg gct gca tcg ctc aag gac gcc ctg gag tcg ctg tac      1536
   Pro Gln Ile Leu Ala Ala Ser Leu Lys Asp Ala Leu Glu Ser Leu Tyr
               500                 505                 510
   aga tac aca cgg gcc cgt gct ggc gac cgg act ctg atc gac gcg ctt      1584
   Arg Tyr Thr Arg Ala Arg Ala Gly Asp Arg Thr Leu Ile Asp Ala Leu
           515                 520                 525
   gcg ccg ttt gtg gag cag ttt gcg gcc agc aag ggt gac ctc aac cag      1632
   Ala Pro Phe Val Glu Gln Phe Ala Ala Ser Lys Gly Asp Leu Asn Gln
       530                 535                 540
   gct aac aag gcg tgc cac gag gga gca gag tca acg cga aag ctc aag      1680
   Ala Asn Lys Ala Cys His Glu Gly Ala Glu Ser Thr Arg Lys Leu Lys
   545                 550                 555                 560
   gct aag ttt ggc cgc gcg tcc tac gtc agc gag gag gag ttc aag ccg      1728
   Ala Lys Phe Gly Arg Ala Ser Tyr Val Ser Glu Glu Glu Phe Lys Pro
                   565                 570                 575
   ttt gag gcc gag ggc ggg ctg ccg gat ccc ggc gcc atc ggg ctt gct      1776
   Phe Glu Ala Glu Gly Gly Leu Pro Asp Pro Gly Ala Ile Gly Leu Ala
               580                 585                 590
   gcg ctg gtc gac ggt ttt gcc gag gcg tac agc aaa ata ggc tcc aac      1824
   Ala Leu Val Asp Gly Phe Ala Glu Ala Tyr Ser Lys Ile Gly Ser Asn
           595                 600                 605
   ttg tag                                                              1830
   Leu
<210> 43
   <211> 609
   <212> PRT
   <213> Pichia angusta
<400> 43
   Met Ser Ser Lys His Trp Asn Tyr Lys Gln Asp Leu Val His Ala His
   1               5                   10                  15
   Leu Lys Gly Leu Cys His Ala Asn Pro Asp Leu Gln Phe Ile Glu Ser
               20                  25                  30
   Glu Arg Val Val Ile Asn Lys His Ser Lys Pro Asp Lys Val Met Ile
           35                  40                  45
   Leu Ser Gly Gly Gly Ser Gly His Glu Pro Leu His Ala Gly Phe Val
       50                  55                  60
   Gly Glu Gly Cys Leu Asp Val Gly Val Ala Gly Phe Val Phe Ala Ser
   65                  70                  75                  80
   Pro Ser Thr Lys Gln Ile Val Ser Gly Leu Lys Ala Lys Pro Ser Asp
                   85                  90                  95
   Lys Gly Thr Leu Ile Val Val Lys Asn Tyr Thr Gly Asp Ile Leu His
               100                 105                 110
   Phe Gly Leu Ala Ala Glu Arg Ala Lys Ala Glu Gly Val Pro Val Glu
           115                 120                 125
   Leu Leu Ile Val Gln Asp Asp Val Ser Val Gly Arg Thr Lys Asn Gly
       130                 135                 140
   Met Val Gly Arg Arg Gly Leu Ala Gly Thr Ser Leu Val His Lys Ile
   145                 150                 155                 160
   Val Gly Ala Lys Ala Ala Lys Asp Ser Asn Lys Ala Ser Leu Ser Glu
                   165                 170                 175
   Val Tyr Gln Leu Gly Glu Ala Val Val Ala Asn Leu Val Thr Ile Gly
               180                 185                 190
   Ala Ser Leu Asp His Cys Thr Ile Pro Gly Asn Arg His His Glu Ser
           195                 200                 205
   Glu Ser Asp Asp Glu Asp Glu Gln Lys His Leu Leu Lys Glu Asp Glu
       210                 215                 220
   Ile Glu Val Gly Met Gly Ile His Asn Glu Ser Gly Ile Lys Arg Val
   225                 230                 235                 240
   Ser Pro Ile Pro Thr Ile Asp Thr Leu Val Ala Asp Leu Leu Lys Tyr
                   245                 250                 255
   Leu Leu Asp Lys Ser Asp Glu Glu Arg His Tyr Val Asp Phe Asp Ser
               260                 265                 270
   Ser Asp Glu Val Val Leu Met Ile Asn Asn Leu Gly Gly Thr Ser Asn
           275                 280                 285
   Leu Glu Leu Tyr Ala Ile Gln Asn Thr Val Val Glu Gln Leu Ala Thr
       290                 295                 300
   Asp Tyr Lys Ile Lys Pro Ala Arg Val Tyr Thr Gly Ala Tyr Thr Thr
   305                 310                 315                 320
   Ser Leu Asp Gly Pro Gly Phe Ser Ile Thr Leu Leu Asn Val Thr Arg
                   325                 330                 335
   Ala Gly Gly Lys Glu Val Phe Asp Cys Leu Asp Tyr Pro Thr Lys Val
               340                 345                 350
   Pro Gly Trp Asn Ser Ser Tyr Thr Thr Ala Glu Trp Ala Ala Lys Ser
           355                 360                 365
   Glu Ser Phe Val Ile Asp Ala Pro Pro Val Ser Asp Ala Ser Ala Thr
       370                 375                 380
   Ser Lys Val Arg Phe Ser Ser Ser Thr Val Lys Ala Val Leu Glu Ser
   385                 390                 395                 400
   Gly Cys Lys Lys Leu Leu Thr Lys Glu Pro Lys Ile Thr Leu Tyr Asp
                   405                 410                 415
   Thr Val Ala Gly Asp Gly Asp Cys Gly Glu Thr Leu Ala Asn Gly Ala
               420                 425                 430
   His Ala Ile Leu Asp Leu Leu Ala Ala Asp Lys Leu Glu Ile Thr Asp
           435                 440                 445
   Gly Val Arg Ser Leu Thr Gln Ile Thr Asp Val Val Glu Thr Ala Met
       450                 455                 460
   Gly Gly Thr Ser Gly Gly Leu Tyr Ser Ile Phe Ile Ser Ala Leu Ala
   465                 470                 475                 480
   Lys Ser Leu Lys Asp Arg Glu Leu Gln Gln Gly Gly Tyr Glu Val Thr
                   485                 490                 495
   Pro Gln Ile Leu Ala Ala Ser Leu Lys Asp Ala Leu Glu Ser Leu Tyr
               500                 505                 510
   Arg Tyr Thr Arg Ala Arg Ala Gly Asp Arg Thr Leu Ile Asp Ala Leu
           515                 520                 525
   Ala Pro Phe Val Glu Gln Phe Ala Ala Ser Lys Gly Asp Leu Asn Gln
       530                 535                 540
   Ala Asn Lys Ala Cys His Glu Gly Ala Glu Ser Thr Arg Lys Leu Lys
   545                 550                 555                 560
   Ala Lys Phe Gly Arg Ala Ser Tyr Val Ser Glu Glu Glu Phe Lys Pro
                   565                 570                 575
   Phe Glu Ala Glu Gly Gly Leu Pro Asp Pro Gly Ala Ile Gly Leu Ala
               580                 585                 590
   Ala Leu Val Asp Gly Phe Ala Glu Ala Tyr Ser Lys Ile Gly Ser Asn
           595                 600                 605
   Leu
<210> 44
   <211> 1827
   <212> DNA
   <213> Pichia pastoris
<220>
   <221> CDS
   <222> (1).. (1827)
<400> 44
   atg tct agt aaa cat tgg gat tac aag aaa gat ctg gta ctc agc cat      48
   Met Ser Ser Lys His Trp Asp Tyr Lys Lys Asp Leu Val Leu Ser His
   1               5                   10                  15
   ctg gct gga tta tgt cag tcc aac ccc cat gta agg cta att gag tcc      96
   Leu Ala Gly Leu Cys Gln Ser Asn Pro His Val Arg Leu Ile Glu Ser
               20                  25                  30
   gaa aga gtc gtc atc tcc gcc gag aac cag gag gac aag att acc ttg     144
   Glu Arg Val Val Ile Ser Ala Glu Asn Gln Glu Asp Lys Ile Thr Leu
           35                  40                  45
   atc tct ggt gga ggt tca gga cac gag cct cta cat gct ggc ttt gtc     192
   Ile Ser Gly Gly Gly Ser Gly His Glu Pro Leu His Ala Gly Phe Val
       50                  55                  60
   acc aag gac ggt ctt ttg gat gcc gct gtg gcc ggg ttc atc ttt gcc      240
   Thr Lys Asp Gly Leu Leu Asp Ala Ala Val Ala Gly Phe Ile Phe Ala
   65                  70                  75                  80
   tcg cca tct act aaa cag ata ttt agc gca atc aaa gcc aaa cct tcg      288
   Ser Pro Ser Thr Lys Gln Ile Phe Ser Ala Ile Lys Ala Lys Pro Ser
                   85                  90                  95
   aag aag gga act ttg atc atc gtc aag aac tat aca gga gac att ctt      336
   Lys Lys Gly Thr Leu Ile Ile Val Lys Asn Tyr Thr Gly Asp Ile Leu
               100                 105                 110
   cac ttc ggt ctt gcc gcc gaa aag gcc aag gcc gaa ggt ctc aac gca      384
   His Phe Gly Leu Ala Ala Glu Lys Ala Lys Ala Glu Gly Leu Asn Ala
           115                 120                 125
   gaa ctg ctg att gtt cag gac gat gtc tct gtt ggt aaa gcc aag aat      432
   Glu Leu Leu Ile Val Gln Asp Asp Val Ser Val Gly Lys Ala Lys Asn
       130                 135                 140
   ggc ctg gta ggg cgt aga ggt ttg gct gga act tct tta gtg cac aag      480
   Gly Leu Val Gly Arg Arg Gly Leu Ala Gly Thr Ser Leu Val His Lys
   145                 150                 155                 160
   att ttg ggt gcc aaa gct tat tta caa aag gac aac ctg gaa ttg cat      528
   Ile Leu Gly Ala Lys Ala Tyr Leu Gln Lys Asp Asn Leu Glu Leu His
                   165                 170                 175
   cag ctg gtc act ttc ggt gag aag gtc gtt gcc aac ctg gtt aca att      576
   Gln Leu Val Thr Phe Gly Glu Lys Val Val Ala Asn Leu Val Thr Ile
               180                 185                 190
   ggt gct tcc ttg gac cat gtc acc att cca gcc aga gca aac aaa cag      624
   Gly Ala Ser Leu Asp His Val Thr Ile Pro Ala Arg Ala Asn Lys Gln
           195                 200                 205
   gag gaa gat gat tca gat gac gag cac ggc tac gaa gtg cta aag cac      672
   Glu Glu Asp Asp Ser Asp Asp Glu His Gly Tyr Glu Val Leu Lys His
       210                 215                 220
   gat gaa ttt gaa atc gga atg ggt att cac aac gaa cca ggt atc aag      720
   Asp Glu Phe Glu Ile Gly Met Gly Ile His Asn Glu Pro Gly Ile Lys
   225                 230                 235                 240
   aag tcg tcg cca att cca aca gta gac gaa ttg gtg gct gag cta ctg      768
   Lys Ser Ser Pro Ile Pro Thr Val Asp Glu Leu Val Ala Glu Leu Leu
                   245                 250                 255
   gag tac ctt ctt tcg act acc gat aag gac cga aat tac gtc caa ttt      816
   Glu Tyr Leu Leu Ser Thr Thr Asp Lys Asp Arg Asn Tyr Val Gln Phe
               260                 265                 270
   gac aag aat gat gaa gtt gtg cta ctg atc aat aac ttg gga gga acc      864
   Asp Lys Asn Asp Glu Val Val Leu Leu Ile Asn Asn Leu Gly Gly Thr
           275                 280                 285
   tca gtg ttg gaa ttg tac gct att cag aac att gtt gtc gac caa ctg      912
   Ser Val Leu Glu Leu Tyr Ala Ile Gln Asn Ile Val Val Asp Gln Leu
       290                 295                 300
   gcc tcc aaa tat tcc att aaa cca gtc cgc att ttc act ggc act ttc      960
   Ala Ser Lys Tyr Ser Ile Lys Pro Val Arg Ile Phe Thr Gly Thr Phe
   305                 310                 315                 320
   acc aca tca ctg gat ggg ccc ggt ttt tct atc act tta ttg aat gct     1008
   Thr Thr Ser Leu Asp Gly Pro Gly Phe Ser Ile Thr Leu Leu Asn Ala
                   325                 330                 335
   acc aag aca gga gat aaa gat atc cta aag ttt tta gac cac aag act     1056
   Thr Lys Thr Gly Asp Lys Asp Ile Leu Lys Phe Leu Asp His Lys Thr
               340                 345                 350
   agt gcc cct ggc tgg aac tcc aac ata tca gac tgg tct ggc agg gtt      1104
   Ser Ala Pro Gly Trp Asn Ser Asn Ile Ser Asp Trp Ser Gly Arg Val
           355                 360                 365
   gac aat ttt att gtg gct gcc cca gaa ata gac gaa gga gac agt tca      1152
   Asp Asn Phe Ile Val Ala Ala Pro Glu Ile Asp Glu Gly Asp Ser Ser
       370                 375                 380
   tct aag gta tct gtt gat gct aag ctg tat gct gac ctt ctg gag tct      1200
   Ser Lys Val Ser Val Asp Ala Lys Leu Tyr Ala Asp Leu Leu Glu Ser
   385                 390                 395                 400
   ggt gtg aag aag gtc att tca aaa gag cca aag atc aca ttg tat gac      1248
   Gly Val Lys Lys Val Ile Ser Lys Glu Pro Lys Ile Thr Leu Tyr Asp
                   405                 410                 415
   act gtt gca gga gat ggt gat tgt ggt gaa acg ttg gcg aat ggt tcc      1296
   Thr Val Ala Gly Asp Gly Asp Cys Gly Glu Thr Leu Ala Asn Gly Ser
               420                 425                 430
   aat gcc att tta aag gct ctg gct gaa gga aag ctt gat ctc aaa gac      1344
   Asn Ala Ile Leu Lys Ala Leu Ala Glu Gly Lys Leu Asp Leu Lys Asp
           435                 440                 445
   ggt gtt aaa tca ctg gtg caa att aca gac atc gtg gaa act gcc atg      1392
   Gly Val Lys Ser Leu Val Gln Ile Thr Asp Ile Val Glu Thr Ala Met
       450                 455                 460
   ggt gga aca tct gga ggt ctg tac tcc att ttc atc agc gct tta gca      1440
   Gly Gly Thr Ser Gly Gly Leu Tyr Ser Ile Phe Ile Ser Ala Leu Ala
   465                 470                 475                 480
   aag tct ttg aaa gag aag gaa ctt tcc gag ggt gcc tac aca ctg acc      1488
   Lys Ser Leu Lys Glu Lys Glu Leu Ser Glu Gly Ala Tyr Thr Leu Thr
                   485                 490                 495
   ctt gaa acg atc tct gga tcc tta cag gca gca ctt cag tct cta ttc      1536
   Leu Glu Thr Ile Ser Gly Ser Leu Gln Ala Ala Leu Gln Ser Leu Phe
               500                 505                 510
   aaa tac aca aga gca cgt act gga gac aga act ctt att gac gct ttg      1584
   Lys Tyr Thr Arg Ala Arg Thr Gly Asp Arg Thr Leu Ile Asp Ala Leu
           515                 520                 525
   gag cca ttt gta aag gaa ttc gcc aag tct aag gat ctg aaa ctt gca      1632
   Glu Pro Phe Val Lys Glu Phe Ala Lys Ser Lys Asp Leu Lys Leu Ala
       530                 535                 540
   aac aaa gct gcc cat gat ggt gct gaa gcc aca aga aag ctc gaa gcc      1680
   Asn Lys Ala Ala His Asp Gly Ala Glu Ala Thr Arg Lys Leu Glu Ala
   545                 550                 555                 560
   aag ttt gga aga gca tct tat gtt gcc gaa gaa gag ttc aaa caa ttc      1728
   Lys Phe Gly Arg Ala Ser Tyr Val Ala Glu Glu Glu Phe Lys Gln Phe
                   565                 570                 575
   gaa agt gaa ggt gga cta cct gat cct ggt gct att ggt ctt gcc gct      1776
   Glu Ser Glu Gly Gly Leu Pro Asp Pro Gly Ala Ile Gly Leu Ala Ala
               580                 585                 590
   ttg att tca gga att act gat gca tac ttc aaa tct gaa acc aag ttg      1824
   Leu Ile Ser Gly Ile Thr Asp Ala Tyr Phe Lys Ser Glu Thr Lys Leu
           595                 600                 605
   tag                                                                  1827
<210> 45
   <211> 608
   <212> PRT
   <213> Pichia pastoris
<400> 45
   Met Ser Ser Lys His Trp Asp Tyr Lys Lys Asp Leu Val Leu Ser His
   1               5                   10                  15
   Leu Ala Gly Leu Cys Gln Ser Asn Pro His Val Arg Leu Ile Glu Ser
               20                  25                  30
   Glu Arg Val Val Ile Ser Ala Glu Asn Gln Glu Asp Lys Ile Thr Leu
           35                  40                  45
   Ile Ser Gly Gly Gly Ser Gly His Glu Pro Leu His Ala Gly Phe Val
       50                  55                  60
   Thr Lys Asp Gly Leu Leu Asp Ala Ala Val Ala Gly Phe Ile Phe Ala
   65                  70                  75                  80
   Ser Pro Ser Thr Lys Gln Ile Phe Ser Ala Ile Lys Ala Lys Pro Ser
                   85                  90                  95
   Lys Lys Gly Thr Leu Ile Ile Val Lys Asn Tyr Thr Gly Asp Ile Leu
               100                 105                 110
   His Phe Gly Leu Ala Ala Glu Lys Ala Lys Ala Glu Gly Leu Asn Ala
           115                 120                 125
   Glu Leu Leu Ile Val Gln Asp Asp Val Ser Val Gly Lys Ala Lys Asn
       130                 135                 140
   Gly Leu Val Gly Arg Arg Gly Leu Ala Gly Thr Ser Leu Val His Lys
   145                 150                 155                 160
   Ile Leu Gly Ala Lys Ala Tyr Leu Gln Lys Asp Asn Leu Glu Leu His
                   165                 170                 175
   Gln Leu Val Thr Phe Gly Glu Lys Val Val Ala Asn Leu Val Thr Ile
               180                 185                 190
   Gly Ala Ser Leu Asp His Val Thr Ile Pro Ala Arg Ala Asn Lys Gln
           195                 200                 205
   Glu Glu Asp Asp Ser Asp Asp Glu His Gly Tyr Glu Val Leu Lys His
       210                 215                 220
   Asp Glu Phe Glu Ile Gly Met Gly Ile His Asn Glu Pro Gly Ile Lys
   225                 230                 235                 240
   Lys Ser Ser Pro Ile Pro Thr Val Asp Glu Leu Val Ala Glu Leu Leu
                   245                 250                 255
   Glu Tyr Leu Leu Ser Thr Thr Asp Lys Asp Arg Asn Tyr Val Gln Phe
               260                 265                 270
   Asp Lys Asn Asp Glu Val Val Leu Leu Ile Asn Asn Leu Gly Gly Thr
           275                 280                 285
   Ser Val Leu Glu Leu Tyr Ala Ile Gln Asn Ile Val Val Asp Gln Leu
       290                 295                 300
   Ala Ser Lys Tyr Ser Ile Lys Pro Val Arg Ile Phe Thr Gly Thr Phe
   305                 310                 315                 320
   Thr Thr Ser Leu Asp Gly Pro Gly Phe Ser Ile Thr Leu Leu Asn Ala
                   325                 330                 335
   Thr Lys Thr Gly Asp Lys Asp Ile Leu Lys Phe Leu Asp His Lys Thr
               340                 345                 350
   Ser Ala Pro Gly Trp Asn Ser Asn Ile Ser Asp Trp Ser Gly Arg Val
           355                 360                 365
   Asp Asn Phe Ile Val Ala Ala Pro Glu Ile Asp Glu Gly Asp Ser Ser
       370                 375                 380
   Ser Lys Val Ser Val Asp Ala Lys Leu Tyr Ala Asp Leu Leu Glu Ser
   385                 390                 395                 400
   Gly Val Lys Lys Val Ile Ser Lys Glu Pro Lys Ile Thr Leu Tyr Asp
                   405                 410                 415
   Thr Val Ala Gly Asp Gly Asp Cys Gly Glu Thr Leu Ala Asn Gly Ser
               420                 425                 430
   Asn Ala Ile Leu Lys Ala Leu Ala Glu Gly Lys Leu Asp Leu Lys Asp
           435                 440                 445
   Gly Val Lys Ser Leu Val Gln Ile Thr Asp Ile Val Glu Thr Ala Met
       450                 455                 460
   Gly Gly Thr Ser Gly Gly Leu Tyr Ser Ile Phe Ile Ser Ala Leu Ala
   465                 470                 475                 480
   Lys Ser Leu Lys Glu Lys Glu Leu Ser Glu Gly Ala Tyr Thr Leu Thr
                   485                 490                 495
   Leu Glu Thr Ile Ser Gly Ser Leu Gln Ala Ala Leu Gln Ser Leu Phe
               500                 505                 510
   Lys Tyr Thr Arg Ala Arg Thr Gly Asp Arg Thr Leu Ile Asp Ala Leu
           515                 520                 525
   Glu Pro Phe Val Lys Glu Phe Ala Lys Ser Lys Asp Leu Lys Leu Ala
       530                 535                 540
   Asn Lys Ala Ala His Asp Gly Ala Glu Ala Thr Arg Lys Leu Glu Ala
   545                 550                 555                 560
   Lys Phe Gly Arg Ala Ser Tyr Val Ala Glu Glu Glu Phe Lys Gln Phe
                   565                 570                 575
   Glu Ser Glu Gly Gly Leu Pro Asp Pro Gly Ala Ile Gly Leu Ala Ala
               580                 585                 590
   Leu Ile Ser Gly Ile Thr Asp Ala Tyr Phe Lys Ser Glu Thr Lys Leu
           595                 600                 605
<210> 46
   <211> 1824
   <212> DNA
   <213> Debaryomyces hansenii
<220>
   <221> CDS
   <222> (1).. (1824)
<400> 46
   atg tcg tta gct aaa cac tgg gga tac tca aag gat ttg gtt ttg gaa      48
   Met Ser Leu Ala Lys His Trp Gly Tyr Ser Lys Asp Leu Val Leu Glu
   1               5                   10                  15
   aat tta aag ggt ttg gct gct gca aac ccc aaa att tct ctt ata cca      96
   Asn Leu Lys Gly Leu Ala Ala Ala Asn Pro Lys Ile Ser Leu Ile Pro
               20                  25                  30
   gca gaa aaa aca gtc gtg tat aat gat tcc tta caa tct aaa gga aac     144
   Ala Glu Lys Thr Val Val Tyr Asn Asp Ser Leu Gln Ser Lys Gly Asn
           35                  40                  45
   aag aat att aat cgt ata atg gtt att tct gga gga ggt tca ggc cat     192
   Lys Asn Ile Asn Arg Ile Met Val Ile Ser Gly Gly Gly Ser Gly His
       50                  55                  60
   gaa ccg tta cat gcg gga ttt gtt ggt gta aat gca tta gat gct gct     240
   Glu Pro Leu His Ala Gly Phe Val Gly Val Asn Ala Leu Asp Ala Ala
   65                  70                  75                  80
   gta tct ggt tca ata ttt gcc tcc cct tca gcc aag cag att ttt gca     288
   Val Ser Gly Ser Ile Phe Ala Ser Pro Ser Ala Lys Gln Ile Phe Ala
                   85                  90                  95
   gcc att aag tca att agc tcc aaa caa aac aat tct aaa ggt act ttg     336
   Ala Ile Lys Ser Ile Ser Ser Lys Gln Asn Asn Ser Lys Gly Thr Leu
               100                 105                 110
   gta att gtc aaa aat tat aca gga gat gtt cta cat ttt gga ctt gct      384
   Val Ile Val Lys Asn Tyr Thr Gly Asp Val Leu His Phe Gly Leu Ala
           115                 120                 125
   gtc gaa aga gca aaa gca cat ggc tac aaa ata gac atg ata att gtt      432
   Val Glu Arg Ala Lys Ala His Gly Tyr Lys Ile Asp Met Ile Ile Val
       130                 135                 140
   ggt gat gac gca gct gta ggt agg tca aag ggt gga atg gtt gga aga      480
   Gly Asp Asp Ala Ala Val Gly Arg Ser Lys Gly Gly Met Val Gly Arg
   145                 150                 155                 160
   aga gca ttg gca gcc act gca ttg gtt cac aaa att gtt gga tct gct      528
   Arg Ala Leu Ala Ala Thr Ala Leu Val His Lys Ile Val Gly Ser Ala
                   165                 170                 175
   gct tct gaa att gaa gat cta agc aga ctt aaa ata ttg ggt gat tcc      576
   Ala Ser Glu Ile Glu Asp Leu Ser Arg Leu Lys Ile Leu Gly Asp Ser
               180                 185                 190
   gtt gcg aat aat acg gtg acc att ggt gcc acc tta gat cat tgc tct      624
   Val Ala Asn Asn Thr Val Thr Ile Gly Ala Thr Leu Asp His Cys Ser
           195                 200                 205
   gtt cct ggg cgt gat att gca aac ttt gag cct att ggc cag aat gat      672
   Val Pro Gly Arg Asp Ile Ala Asn Phe Glu Pro Ile Gly Gln Asn Asp
       210                 215                 220
   gct gaa ata ggt cta ggg ata cat aac gaa act tcg gtc aag aag gta      720
   Ala Glu Ile Gly Leu Gly Ile His Asn Glu Thr Ser Val Lys Lys Val
   225                 230                 235                 240
   aac cct gta cca atg ata gac tca tta gtt cag gac tta tta gaa ttc      768
   Asn Pro Val Pro Met Ile Asp Ser Leu Val Gln Asp Leu Leu Glu Phe
                   245                 250                 255
   cta tta aac gaa aat gac aaa gat cgt tac ttt gta cca ttt gat ctc      816
   Leu Leu Asn Glu Asn Asp Lys Asp Arg Tyr Phe Val Pro Phe Asp Leu
               260                 265                 270
   agt aat gat gaa acg gtc ttg ctt gtc aac aat ctc ggg ggt aca tct      864
   Ser Asn Asp Glu Thr Val Leu Leu Val Asn Asn Leu Gly Gly Thr Ser
           275                 280                 285
   act tta gaa atg tat gct att aca aat tgc gtt atc gaa aca tta tac      912
   Thr Leu Glu Met Tyr Ala Ile Thr Asn Cys Val Ile Glu Thr Leu Tyr
       290                 295                 300
   caa caa tac agt ttg aga cca aaa aaa gtg ata gtg gga gaa ttt gct      960
   Gln Gln Tyr Ser Leu Arg Pro Lys Lys Val Ile Val Gly Glu Phe Ala
   305                 310                 315                 320
   acg tct tta aat gcc cct ggg ttt tcg att acc tta ctt aac gtc tct     1008
   Thr Ser Leu Asn Ala Pro Gly Phe Ser Ile Thr Leu Leu Asn Val Ser
                   325                 330                 335
   tgt gca tct aaa caa tcc caa att tcc att tca cat ata atg agt tac     1056
   Cys Ala Ser Lys Gln Ser Gln Ile Ser Ile Ser His Ile Met Ser Tyr
               340                 345                 350
   ttg gat ttg cca aca gat gcc cct ggt tgg aag gca cat ccg tgt ggg     1104
   Leu Asp Leu Pro Thr Asp Ala Pro Gly Trp Lys Ala His Pro Cys Gly
           355                 360                 365
   ttt gga ctt gaa aga gac atc aat att gag aca tca atc aat ggt att     1152
   Phe Gly Leu Glu Arg Asp Ile Asn Ile Glu Thr Ser Ile Asn Gly Ile
       370                 375                 380
   gat tct ttt gtc aag tca caa tta aag ctt tcc aga gaa caa cag acg     1200
   Asp Ser Phe Val Lys Ser Gln Leu Lys Leu Ser Arg Glu Gln Gln Thr
   385                 390                 395                 400
   gac ttt aga agc agt cta gtt aat ggg ttg gaa aaa tta tta gac aaa      1248
   Asp Phe Arg Ser Ser Leu Val Asn Gly Leu Glu Lys Leu Leu Asp Lys
                   405                 410                 415
   gaa cca agc att aca ttt tat gat act gtt gct ggt gat ggt gac tgt      1296
   Glu Pro Ser Ile Thr Phe Tyr Asp Thr Val Ala Gly Asp Gly Asp Cys
               420                 425                 430
   ggt gaa acc tta gcg tct ggt gca aat gga ata ttg gaa tca tta agg      1344
   Gly Glu Thr Leu Ala Ser Gly Ala Asn Gly Ile Leu Glu Ser Leu Arg
           435                 440                 445
   aac aac gaa atc tgc ttt gaa gat cca gtt tat tcc ata tct caa ata      1392
   Asn Asn Glu Ile Cys Phe Glu Asp Pro Val Tyr Ser Ile Ser Gln Ile
       450                 455                 460
   gca aac att gta gag gat aaa atg ggc gga act tca gga ggc tta tat      1440
   Ala Asn Ile Val Glu Asp Lys Met Gly Gly Thr Ser Gly Gly Leu Tyr
   465                 470                 475                 480
   tca att ttc tta acc tcg ttg ata aaa cac ctt caa gat tgt act aca      1488
   Ser Ile Phe Leu Thr Ser Leu Ile Lys His Leu Gln Asp Cys Thr Thr
                   485                 490                 495
   ttg aac tta tgt gaa atg ttt gct agt tct ttg cat aat gcg cta tat      1536
   Leu Asn Leu Cys Glu Met Phe Ala Ser Ser Leu His Asn Ala Leu Tyr
               500                 505                 510
   cag ggc tta tat aaa tac act agg gca cga gtg ggt gga aga act ttg      1584
   Gln Gly Leu Tyr Lys Tyr Thr Arg Ala Arg Val Gly Gly Arg Thr Leu
           515                 520                 525
   att gat gct tta gag ccg ttt gtg aat acc ttc aac gac act cta aat      1632
   Ile Asp Ala Leu Glu Pro Phe Val Asn Thr Phe Asn Asp Thr Leu Asn
       530                 535                 540
   ttc tca aag gct gcc cag gct gct atc gat gga tct gaa tcg act agg      1680
   Phe Ser Lys Ala Ala Gln Ala Ala Ile Asp Gly Ser Glu Ser Thr Arg
   545                 550                 555                 560
   aaa tta gct gca aaa ttt ggg aga gcg tct tat gtc aat gaa caa gaa      1728
   Lys Leu Ala Ala Lys Phe Gly Arg Ala Ser Tyr Val Asn Glu Gln Glu
                   565                 570                 575
   ttt aaa cag ttc gac gaa gaa gga ggc tta ccg gat ccg gga gct att      1776
   Phe Lys Gln Phe Asp Glu Glu Gly Gly Leu Pro Asp Pro Gly Ala Ile
               580                 585                 590
   gga tta gcc acc tta att gct ggg ttt gcc ggt gtt gac tat aat taa      1824
   Gly Leu Ala Thr Leu Ile Ala Gly Phe Ala Gly Val Asp Tyr Asn
           595                 600                 605
<210> 47
   <211> 607
   <212> PRT
   <213> Debaryomyces hansenii
<400> 47
   Met Ser Leu Ala Lys His Trp Gly Tyr Ser Lys Asp Leu Val Leu Glu
   1               5                   10                  15
   Asn Leu Lys Gly Leu Ala Ala Ala Asn Pro Lys Ile Ser Leu Ile Pro
               20                  25                  30
   Ala Glu Lys Thr Val Val Tyr Asn Asp Ser Leu Gln Ser Lys Gly Asn
           35                  40                  45
   Lys Asn Ile Asn Arg Ile Met Val Ile Ser Gly Gly Gly Ser Gly His
   50 55 60
   Glu Pro Leu His Ala Gly Phe Val Gly Val Asn Ala Leu Asp Ala Ala
   65 70 75 80
   Val Ser Gly Ser Ile Phe Ala Ser Pro Ser Ala Lys Gln Ile Phe Ala
   85 90 95
   Ala Ile Lys Ser Ile Ser Ser Lys Gln Asn Asn Ser Lys Gly Thr Leu
   100 105 110
   Val Ile Val Lys Asn Tyr Thr Gly Asp Val Leu His Phe Gly Leu Ala
   115 120 125
   Val Glu Arg Ala Lys Ala His Gly Tyr Lys Ile Asp Met Ile Ile Val
   130 135 140
   Gly Asp Asp Ala Ala Val Gly Arg Ser Lys Gly Gly Met Val Gly Arg
   145 150 155 160
   Arg Ala Leu Ala Ala Thr Ala Leu Val His Lys Ile Val Gly Ser Ala
   165 170 175
   Ala Ser Glu Ile Glu Asp Leu Ser Arg Leu Lys Ile Leu Gly Asp Ser
   180 185 190
   Val Ala Asn Asn Thr Val Thr Ile Gly Ala Thr Leu Asp His Cys Ser
   195 200 205
   Val Pro Gly Arg Asp Ile Ala Asn Phe Glu Pro Ile Gly Gln Asn Asp
   210 215 220
   Ala Glu Ile Gly Leu Gly Ile His Asn Glu Thr Ser Val Lys Lys Val
   225 230 235 240
   Asn Pro Val Pro Met Ile Asp Ser Leu Val Gln Asp Leu Leu Glu Phe
   245 250 255
   Leu Leu Asn Glu Asn Asp Lys Asp Arg Tyr Phe Val Pro Phe Asp Leu
   260 265 270
   Ser Asn Asp Glu Thr Val Leu Leu Val Asn Asn Leu Gly Gly Thr Ser
   275 280 285
   Thr Leu Glu Met Tyr Ala Ile Thr Asn Cys Val Ile Glu Thr Leu Tyr
   290 295 300
   Gln Gln Tyr Ser Leu Arg Pro Lys Lys Val Ile Val Gly Glu Phe Ala
   305 310 315 320
   Thr Ser Leu Asn Ala Pro Gly Phe Ser Ile Thr Leu Leu Asn Val Ser
   325 330 335
   Cys Ala Ser Lys Gln Ser Gln Ile Ser Ile Ser His Ile Met Ser Tyr
   340 345 350
   Leu Asp Leu Pro Thr Asp Ala Pro Gly Trp Lys Ala His Pro Cys Gly
   355 360 365
   Phe Gly Leu Glu Arg Asp Ile Asn Ile Glu Thr Ser Ile Asn Gly Ile
   370 375 380
   Asp Ser Phe Val Lys Ser Gln Leu Lys Leu Ser Arg Glu Gln Gln Thr
   385 390 395 400
   Asp Phe Arg Ser Ser Leu Val Asn Gly Leu Glu Lys Leu Leu Asp Lys
   405 410 415
   Glu Pro Ser Ile Thr Phe Tyr Asp Thr Val Ala Gly Asp Gly Asp Cys
   420 425 430
   Gly Glu Thr Leu Ala Ser Gly Ala Asn Gly Ile Leu Glu Ser Leu Arg
   435 440 445
   Asn Asn Glu Ile Cys Phe Glu Asp Pro Val Tyr Ser Ile Ser Gln Ile
   450 455 460
   Ala Asn Ile Val Glu Asp Lys Met Gly Gly Thr Ser Gly Gly Leu Tyr
   465 470 475 480
   Ser Ile Phe Leu Thr Ser Leu Ile Lys His Leu Gln Asp Cys Thr Thr
   485 490 495
   Leu Asn Leu Cys Glu Met Phe Ala Ser Ser Leu His Asn Ala Leu Tyr
               500                 505                 510
   Gln Gly Leu Tyr Lys Tyr Thr Arg Ala Arg Val Gly Gly Arg Thr Leu
           515                 520                 525
   Ile Asp Ala Leu Glu Pro Phe Val Asn Thr Phe Asn Asp Thr Leu Asn
       530                 535                 540
   Phe Ser Lys Ala Ala Gln Ala Ala Ile Asp Gly Ser Glu Ser Thr Arg
   545                 550                 555                 560
   Lys Leu Ala Ala Lys Phe Gly Arg Ala Ser Tyr Val Asn Glu Gln Glu
                   565                 570                 575
   Phe Lys Gln Phe Asp Glu Glu Gly Gly Leu Pro Asp Pro Gly Ala Ile
               580                 585                 590
   Gly Leu Ala Thr Leu Ile Ala Gly Phe Ala Gly Val Asp Tyr Asn
           595                 600                 605
<210> 48
   <211> 1752
   <212> DNA
   <213> Escherichia blattae
<220>
   <221> CDS
   <222> (1).. (1752)
<400> 48
   atg gtc tgc ccg cca gac ctt aac tca caa cct ttt act tcc cga cac      48
   Met Val Cys Pro Pro Asp Leu Asn Ser Gln Pro Phe Thr Ser Arg His
   1               5                   10                  15
   acg cgc tac agc ccg ctg tac ggg tgt gtt gta cct aac gat cag gaa      96
   Thr Arg Tyr Ser Pro Leu Tyr Gly Cys Val Val Pro Asn Asp Gln Glu
               20                  25                  30
   acc gtt atg tcg caa ttt ttt tat aac caa cgc gaa aat ctt gtc agt     144
   Thr Val Met Ser Gln Phe Phe Tyr Asn Gln Arg Glu Asn Leu Val Ser
           35                  40                  45
   gac gcc atc gaa ggg gca atg att gcc agc ccg tgg aac aac ctg gcc     192
   Asp Ala Ile Glu Gly Ala Met Ile Ala Ser Pro Trp Asn Asn Leu Ala
       50                  55                  60
   cgt ctg gag agc gat ccc gcc atc cgc atc gtg gtg cgc cgg gat ctg     240
   Arg Leu Glu Ser Asp Pro Ala Ile Arg Ile Val Val Arg Arg Asp Leu
   65                  70                  75                  80
   gat aaa tcc cgg gtg gcg gtg atc tcc ggg ggc ggt gcc ggt cac gaa     288
   Asp Lys Ser Arg Val Ala Val Ile Ser Gly Gly Gly Ala Gly His Glu
                   85                  90                  95
   ccg gcc cat gtg ggc ttt gtg ggt aaa ggc atg ctg acc gcc gcc gtg     336
   Pro Ala His Val Gly Phe Val Gly Lys Gly Met Leu Thr Ala Ala Val
               100                 105                 110
   tgt ggt gat ctg ttt gcc tca ccg agc gtg gat gcg gtg ctt acc gcc     384
   Cys Gly Asp Leu Phe Ala Ser Pro Ser Val Asp Ala Val Leu Thr Ala
           115                 120                 125
   att cag gcg gtc acc ggc gag gcg ggc tgc ctg ctg att gtc aaa aac     432
   Ile Gln Ala Val Thr Gly Glu Ala Gly Cys Leu Leu Ile Val Lys Asn
       130                 135                 140
   tac acc ggt gac cgg ctg aac ttc ggg ctg gca gca gag aaa gcc cgg     480
   Tyr Thr Gly Asp Arg Leu Asn Phe Gly Leu Ala Ala Glu Lys Ala Arg
   145                 150                 155                 160
   cgc atg ggc tac aaa gtg gac atg gtg att gtg ggg gat gat atc tca      528
   Arg Met Gly Tyr Lys Val Asp Met Val Ile Val Gly Asp Asp Ile Ser
                   165                 170                 175
   ctg ccg gag aac aaa cac ccg cgc ggc att gcc ggg acg att atg atc      576
   Leu Pro Glu Asn Lys His Pro Arg Gly Ile Ala Gly Thr Ile Met Ile
               180                 185                 190
   cac aaa gtg gcg ggg tac ttc gcc gaa acc ggc tgc aac ctg gac acg      624
   His Lys Val Ala Gly Tyr Phe Ala Glu Thr Gly Cys Asn Leu Asp Thr
           195                 200                 205
   gtc gcc cgg gaa gcc cgg ctg gca atg gag cgg gta ttc agt att ggc      672
   Val Ala Arg Glu Ala Arg Leu Ala Met Glu Arg Val Phe Ser Ile Gly
       210                 215                 220
   gtg gcc ctt tcc agc tgc cac tta ccg gcc gat ccg cag gat ggc gtg      720
   Val Ala Leu Ser Ser Cys His Leu Pro Ala Asp Pro Gln Asp Gly Val
   225                 230                 235                 240
   cgc cat cac ccg ggc cag gct gag ctg ggc atg ggg atc cac ggg gag      768
   Arg His His Pro Gly Gln Ala Glu Leu Gly Met Gly Ile His Gly Glu
                   245                 250                 255
   ccc ggc gca agc gtc atc gac acc cag aac agc acc gac att gtg cgc      816
   Pro Gly Ala Ser Val Ile Asp Thr Gln Asn Ser Thr Asp Ile Val Arg
               260                 265                 270
   ctg atg gtg gca aaa atc cgc gct gcc ctg cct gaa acc ggc cgc ctg      864
   Leu Met Val Ala Lys Ile Arg Ala Ala Leu Pro Glu Thr Gly Arg Leu
           275                 280                 285
   ctg ctg atg ctg aat aac ctt ggc ggc gtc tca gtc acc gaa atg gcg      912
   Leu Leu Met Leu Asn Asn Leu Gly Gly Val Ser Val Thr Glu Met Ala
       290                 295                 300
   atc ctc acc cgg gag ctg gcc cac tgt gag ctt gcc acc cgc acc gac      960
   Ile Leu Thr Arg Glu Leu Ala His Cys Glu Leu Ala Thr Arg Thr Asp
   305                 310                 315                 320
   tgg ttg atg ggc ccg gca ccg ctg gtc agc gcc ctg gac atg aaa ggc     1008
   Trp Leu Met Gly Pro Ala Pro Leu Val Ser Ala Leu Asp Met Lys Gly
                   325                 330                 335
   ttt tcc atc acc gcc ctg gtc atg gaa gag agc att gaa aaa gcc ctg     1056
   Phe Ser Ile Thr Ala Leu Val Met Glu Glu Ser Ile Glu Lys Ala Leu
               340                 345                 350
   ctg gca gac gtg gaa acc gca ggc tgg ctg ccg cca gtg cgc ctg cgc     1104
   Leu Ala Asp Val Glu Thr Ala Gly Trp Leu Pro Pro Val Arg Leu Arg
           355                 360                 365
   gcc agc cag acc cag ccc tgc aat atc cgc agt gcc cgg gtg gca ttc     1152
   Ala Ser Gln Thr Gln Pro Cys Asn Ile Arg Ser Ala Arg Val Ala Phe
       370                 375                 380
   acc ccg tca gac aac ccg gta gtg ggc cag tat gtg gag acc gtc acc     1200
   Thr Pro Ser Asp Asn Pro Val Val Gly Gln Tyr Val Glu Thr Val Thr
   385                 390                 395                 400
   gcc aca ctc agc gcc cag gaa gcg gaa ctt aac gcc ctg gat gcc aaa     1248
   Ala Thr Leu Ser Ala Gln Glu Ala Glu Leu Asn Ala Leu Asp Ala Lys
                   405                 410                 415
   gtg ggc gac ggc gat acc ggc tcc acc ttt gcc gcc ggg gcc cgc gcc     1296
   Val Gly Asp Gly Asp Thr Gly Ser Thr Phe Ala Ala Gly Ala Arg Ala
               420                 425                 430
   att gca gaa ctg ctg cac cag cac cag ctg ccg ctc agc cag ctg gat     1344
   Ile Ala Glu Leu Leu His Gln His Gln Leu Pro Leu Ser Gln Leu Asp
           435                 440                 445
   acc ctg tgc gcc ctg atc ggc gaa cgc tta aca gta gtg atg ggg ggc      1392
   Thr Leu Cys Ala Leu Ile Gly Glu Arg Leu Thr Val Val Met Gly Gly
       450                 455                 460
   tcc agc ggt gtg ctg atg tcc atc ttc ttt acg gcc gca ggc cag gca      1440
   Ser Ser Gly Val Leu Met Ser Ile Phe Phe Thr Ala Ala Gly Gln Ala
   465                 470                 475                 480
   atc agc gaa ggg aaa ccg gtg gtg gcc gca ctc cag gcg ggc ctg gcg      1488
   Ile Ser Glu Gly Lys Pro Val Val Ala Ala Leu Gln Ala Gly Leu Ala
                   485                 490                 495
   cag atg aag tat tac ggc ggt gcc gat ctg ggt gac cgc acc ctg att      1536
   Gln Met Lys Tyr Tyr Gly Gly Ala Asp Leu Gly Asp Arg Thr Leu Ile
               500                 505                 510
   gat gcc ctg caa cct gcg ctg gca gct ctg gca cag cat ccg ggg gat      1584
   Asp Ala Leu Gln Pro Ala Leu Ala Ala Leu Ala Gln His Pro Gly Asp
           515                 520                 525
   ctg gcg gcg gca tac cag gca gcc cgg gac ggg gca gac gcc acc acc      1632
   Leu Ala Ala Ala Tyr Gln Ala Ala Arg Asp Gly Ala Asp Ala Thr Thr
       530                 535                 540
   cgc gcc acc aaa gcc aat gcc ggt cgc gcc tct tac ctg aac agc gac      1680
   Arg Ala Thr Lys Ala Asn Ala Gly Arg Ala Ser Tyr Leu Asn Ser Asp
   545                 550                 555                 560
   agc ctg gcg ggc aat atg gac ccg ggc gct cac gca gtc gca atg gtc      1728
   Ser Leu Ala Gly Asn Met Asp Pro Gly Ala His Ala Val Ala Met Val
                   565                 570                 575
   ttt aag gcg ctg gcg ggc gcc tga                                      1752
   Phe Lys Ala Leu Ala Gly Ala
               580
<210> 49
   <211> 583
   <212> PRT
   <213> Escherichia blattae
<400> 49
   Met Val Cys Pro Pro Asp Leu Asn Ser Gln Pro Phe Thr Ser Arg His
   1               5                   10                  15
   Thr Arg Tyr Ser Pro Leu Tyr Gly Cys Val Val Pro Asn Asp Gln Glu
               20                  25                  30
   Thr Val Met Ser Gln Phe Phe Tyr Asn Gln Arg Glu Asn Leu Val Ser
           35                  40                  45
   Asp Ala Ile Glu Gly Ala Met Ile Ala Ser Pro Trp Asn Asn Leu Ala
       50                  55                  60
   Arg Leu Glu Ser Asp Pro Ala Ile Arg Ile Val Val Arg Arg Asp Leu
   65                  70                  75                  80
   Asp Lys Ser Arg Val Ala Val Ile Ser Gly Gly Gly Ala Gly His Glu
                   85                  90                  95
   Pro Ala His Val Gly Phe Val Gly Lys Gly Met Leu Thr Ala Ala Val
               100                 105                 110
   Cys Gly Asp Leu Phe Ala Ser Pro Ser Val Asp Ala Val Leu Thr Ala
           115                 120                 125
   Ile Gln Ala Val Thr Gly Glu Ala Gly Cys Leu Leu Ile Val Lys Asn
       130                 135                 140
   Tyr Thr Gly Asp Arg Leu Asn Phe Gly Leu Ala Ala Glu Lys Ala Arg
   145                 150                 155                 160
   Arg Met Gly Tyr Lys Val Asp Met Val Ile Val Gly Asp Asp Ile Ser
   165 170 175
   Leu Pro Glu Asn Lys His Pro Arg Gly Ile Ala Gly Thr Ile Met Ile
   180 185 190
   His Lys Val Ala Gly Tyr Phe Ala Glu Thr Gly Cys Asn Leu Asp Thr
   195 200 205
   Val Ala Arg Glu Ala Arg Leu Ala Met Glu Arg Val Phe Ser Ile Gly
   210 215 220
   Val Ala Leu Ser Ser Cys His Leu Pro Ala Asp Pro Gln Asp Gly Val
   225 230 235 240
   Arg His His Pro Gly Gln Ala Glu Leu Gly Met Gly Ile His Gly Glu
   245 250 255
   Pro Gly Ala Ser Val Ile Asp Thr Gln Asn Ser Thr Asp Ile Val Arg
   260 265 270
   Leu Met Val Ala Lys Ile Arg Ala Ala Leu Pro Glu Thr Gly Arg Leu
   275 280 285
   Leu Leu Met Leu Asn Asn Leu Gly Gly Val Ser Val Thr Glu Met Ala
   290 295 300
   Ile Leu Thr Arg Glu Leu Ala His Cys Glu Leu Ala Thr Arg Thr Asp
   305 310 315 320
   Trp Leu Met Gly Pro Ala Pro Leu Val Ser Ala Leu Asp Met Lys Gly
   325 330 335
   Phe Ser Ile Thr Ala Leu Val Met Glu Glu Ser Ile Glu Lys Ala Leu
   340 345 350
   Leu Ala Asp Val Glu Thr Ala Gly Trp Leu Pro Pro Val Arg Leu Arg
   355 360 365
   Ala Ser Gln Thr Gln Pro Cys Asn Ile Arg Ser Ala Arg Val Ala Phe
   370 375 380
   Thr Pro Ser Asp Asn Pro Val Val Gly Gln Tyr Val Glu Thr Val Thr
   385 390 395 400
   Ala Thr Leu Ser Ala Gln Glu Ala Glu Leu Asn Ala Leu Asp Ala Lys
   405 410 415
   Val Gly Asp Gly Asp Thr Gly Ser Thr Phe Ala Ala Gly Ala Arg Ala
   420 425 430
   Ile Ala Glu Leu Leu His Gln His Gln Leu Pro Leu Ser Gln Leu Asp
   435 440 445
   Thr Leu Cys Ala Leu Ile Gly Glu Arg Leu Thr Val Val Met Gly Gly
   450 455 460
   Ser Ser Gly Val Leu Met Ser Ile Phe Phe Thr Ala Ala Gly Gln Ala
   465 470 475 480
   Ile Ser Glu Gly Lys Pro Val Val Ala Ala Leu Gln Ala Gly Leu Ala
   485 490 495
   Gln Met Lys Tyr Tyr Gly Gly Ala Asp Leu Gly Asp Arg Thr Leu Ile
   500 505 510
   Asp Ala Leu Gln Pro Ala Leu Ala Ala Leu Ala Gln His Pro Gly Asp
   515 520 525
   Leu Ala Ala Ala Tyr Gln Ala Ala Arg Asp Gly Ala Asp Ala Thr Thr
   530 535 540
   Arg Ala Thr Lys Ala Asn Ala Gly Arg Ala Ser Tyr Leu Asn Ser Asp
   545 550 555 560
   Ser Leu Ala Gly Asn Met Asp Pro Gly Ala His Ala Val Ala Met Val
   565 570 575
   Phe Lys Ala Leu Ala Gly Ala
   580
<210> 50
   <211> 1647
   <212> DNA
   <213> Enterobacter sp.638
<220>
   <221> CDS
   <222> (1).. (1647)
<400> 50
   atg tcc aga ttc ttt ttt aat gac cgc aaa cag ctg gtc aac gac gcc        48
   Met Ser Arg Phe Phe Phe Asn Asp Arg Lys Gln Leu Val Asn Asp Ala
   1               5                   10                  15
   att gaa ggc ata ctg att tcc gcg ccg cac ggg aat ctt gtc aaa ctt        96
   Ile Glu Gly Ile Leu Ile Ser Ala Pro His Gly Asn Leu Val Lys Leu
               20                  25                  30
   gat atc gat ccg gcc att cgg gtg gtt gcg cgt agc gac tgg gat aaa       144
   Asp Ile Asp Pro Ala Ile Arg Val Val Ala Arg Ser Asp Trp Asp Lys
           35                  40                  45
   agc cgc gta gcg gtg att tcc ggt ggt ggg tcg ggg cac gaa ccc gct       192
   Ser Arg Val Ala Val Ile Ser Gly Gly Gly Ser Gly His Glu Pro Ala
       50                  55                  60
   cat gcc gga ttt gtc ggc aaa ggg atg ttg acc gca gcc gtc tgt ggc       240
   His Ala Gly Phe Val Gly Lys Gly Met Leu Thr Ala Ala Val Cys Gly
   65                  70                  75                  80
   gat ctg ttt gcc tca ccg agc gta gat gcg gtg tta aac gcg att gtg       288
   Asp Leu Phe Ala Ser Pro Ser Val Asp Ala Val Leu Asn Ala Ile Val
                   85                  90                  95
   gcg gta acg ggc gat cgc ggt tgc ctg tta atc gtc aaa aat tat acc       336
   Ala Val Thr Gly Asp Arg Gly Cys Leu Leu Ile Val Lys Asn Tyr Thr
               100                 105                 110
   ggc gat cgg ctt aac ttt ggc ctc gcg gcg gaa aag gcc aaa cgc tat       384
   Gly Asp Arg Leu Asn Phe Gly Leu Ala Ala Glu Lys Ala Lys Arg Tyr
           115                 120                 125
   ggg ctg aag gtt gag atg gtg att gtt gct gat gac atc gcc ctg ccg       432
   Gly Leu Lys Val Glu Met Val Ile Val Ala Asp Asp Ile Ala Leu Pro
       130                 135                 140
   gat aac aaa cag ccg cgt ggc att gcg ggt acg gcg ctg gta cac aaa       480
   Asp Asn Lys Gln Pro Arg Gly Ile Ala Gly Thr Ala Leu Val His Lys
   145                 150                 155                 160
   att gcc gga tat gca gcc gaa cag ggg aaa tca ctg gct gac gtg cgg       528
   Ile Ala Gly Tyr Ala Ala Glu Gln Gly Lys Ser Leu Ala Asp Val Arg
                   165                 170                 175
   gat att gcg cag cag gcc tgt gac aat atc tgg agc ctg ggc gtg gcg       576
   Asp Ile Ala Gln Gln Ala Cys Asp Asn Ile Trp Ser Leu Gly Val Ala
               180                 185                 190
   atg caa acg tgc aac ctg ccg ggc agc gac gat gaa gaa ggg cgt atc       624
   Met Gln Thr Cys Asn Leu Pro Gly Ser Asp Asp Glu Glu Gly Arg Ile
           195                 200                 205
   aag gat gga cat gtc gaa ctg ggg ctg ggc att cac ggc gag ccg ggc       672
   Lys Asp Gly His Val Glu Leu Gly Leu Gly Ile His Gly Glu Pro Gly
       210                 215                 220
   gcg tcg gtg gtt gat acg cac aac agc aaa gag att atc gac acc ctg       720
   Ala Ser Val Val Asp Thr His Asn Ser Lys Glu Ile Ile Asp Thr Leu
   225                 230                 235                 240
   gtg aag ccg tta aaa gag acg gcc ggc gaa ggc aaa ttt gcg gtg ctg        768
   Val Lys Pro Leu Lys Glu Thr Ala Gly Glu Gly Lys Phe Ala Val Leu
                   245                 250                 255
   att aac aat ctc ggc ggt gta tcg gcg ctg gag atg gcg ctg ctc acg        816
   Ile Asn Asn Leu Gly Gly Val Ser Ala Leu Glu Met Ala Leu Leu Thr
               260                 265                 270
   aaa gaa ctg gcg gat tct gcg ctg aaa gaa aat att gcg tat ctg att        864
   Lys Glu Leu Ala Asp Ser Ala Leu Lys Glu Asn Ile Ala Tyr Leu Ile
           275                 280                 285
   ggc cct gcg ccg ctg gta agc tcg ctg gat atg aaa ggc ttt tcg ctg        912
   Gly Pro Ala Pro Leu Val Ser Ser Leu Asp Met Lys Gly Phe Ser Leu
       290                 295                 300
   tca ctg tta cag ctt aac gat acc ttt gag aaa gcc att aac gca ccc        960
   Ser Leu Leu Gln Leu Asn Asp Thr Phe Glu Lys Ala Ile Asn Ala Pro
   305                 310                 315                 320
   gtc gaa act atc ggc tgg caa aag ccg gta gca ttc gcg cca tta cgc       1008
   Val Glu Thr Ile Gly Trp Gln Lys Pro Val Ala Phe Ala Pro Leu Arg
                   325                 330                 335
   acg ctt tcg cat act gcg att cag gat cgt gtt gaa ttt acg cct tcc       1056
   Thr Leu Ser His Thr Ala Ile Gln Asp Arg Val Glu Phe Thr Pro Ser
               340                 345                 350
   ggg aac gac gag gtc gca gcg cga gtg gca gcg gcg acg caa acg ttg       1104
   Gly Asn Asp Glu Val Ala Ala Arg Val Ala Ala Ala Thr Gln Thr Leu
           355                 360                 365
   ctc gct ctg gag aac cgt tta aat gcg ctg gac gcc aaa gtg ggc gac       1152
   Leu Ala Leu Glu Asn Arg Leu Asn Ala Leu Asp Ala Lys Val Gly Asp
       370                 375                 380
   ggc gat acc ggg tcg act ttt gcg caa ggc gcg cgg gaa att gcg cag       1200
   Gly Asp Thr Gly Ser Thr Phe Ala Gln Gly Ala Arg Glu Ile Ala Gln
   385                 390                 395                 400
   ctt ctg gag caa aaa cag ctt ccg cta aac gat ctt tct aag ctg ctg       1248
   Leu Leu Glu Gln Lys Gln Leu Pro Leu Asn Asp Leu Ser Lys Leu Leu
                   405                 410                 415
   ttg ttg atc ggc gaa cgg ctg gcg acg gtc atg ggc ggg tcg agt ggc       1296
   Leu Leu Ile Gly Glu Arg Leu Ala Thr Val Met Gly Gly Ser Ser Gly
               420                 425                 430
   gtc ctg atg tcg atc ttc ttc aca gct gcc gga cag aaa atg cat gac       1344
   Val Leu Met Ser Ile Phe Phe Thr Ala Ala Gly Gln Lys Met His Asp
           435                 440                 445
   gga aaa tca ctg ccg gag gca ttg ctg agt ggg ctt gcg caa atg aag       1392
   Gly Lys Ser Leu Pro Glu Ala Leu Leu Ser Gly Leu Ala Gln Met Lys
       450                 455                 460
   cat tac ggc gga gcg gat ctt ggc gat cgt acc ttg atc gac gcg cta       1440
   His Tyr Gly Gly Ala Asp Leu Gly Asp Arg Thr Leu Ile Asp Ala Leu
   465                 470                 475                 480
   cag cct gca ctg gag acg ctg cat aac ggc gat att cag gcg gct gcc       1488
   Gln Pro Ala Leu Glu Thr Leu His Asn Gly Asp Ile Gln Ala Ala Ala
                   485                 490                 495
   cag gca gcg aaa aaa ggc gca gac gct acg gct ggc atg caa aaa gcg       1536
   Gln Ala Ala Lys Lys Gly Ala Asp Ala Thr Ala Gly Met Gln Lys Ala
               500                 505                 510
   gga gca ggg cgt tcg tcg tat gtg aat aaa gag aac ctg gaa ggt gta       1584
   Gly Ala Gly Arg Ser Ser Tyr Val Asn Lys Glu Asn Leu Glu Gly Val
           515                 520                 525
   ata gat cct ggg gca gtg gcc gtt gca gag gtg ttt gcg gca gtg gcc      1632
   Ile Asp Pro Gly Ala Val Ala Val Ala Glu Val Phe Ala Ala Val Ala
       530                 535                 540
   aaa gca aaa cag tag                                                  1647
   Lys Ala Lys Gln
   545
<210> 51
   <211> 548
   <212> PRT
   <213> Enterobacter sp.638
<400> 51
   Met Ser Arg Phe Phe Phe Asn Asp Arg Lys Gln Leu Val Asn Asp Ala
   1               5                   10                  15
   Ile Glu Gly Ile Leu Ile Ser Ala Pro His Gly Asn Leu Val Lys Leu
               20                  25                  30
   Asp Ile Asp Pro Ala Ile Arg Val Val Ala Arg Ser Asp Trp Asp Lys
           35                  40                  45
   Ser Arg Val Ala Val Ile Ser Gly Gly Gly Ser Gly His Glu Pro Ala
       50                  55                  60
   His Ala Gly Phe Val Gly Lys Gly Met Leu Thr Ala Ala Val Cys Gly
   65                  70                  75                  80
   Asp Leu Phe Ala Ser Pro Ser Val Asp Ala Val Leu Asn Ala Ile Val
                   85                  90                  95
   Ala Val Thr Gly Asp Arg Gly Cys Leu Leu Ile Val Lys Asn Tyr Thr
               100                 105                 110
   Gly Asp Arg Leu Asn Phe Gly Leu Ala Ala Glu Lys Ala Lys Arg Tyr
           115                 120                 125
   Gly Leu Lys Val Glu Met Val Ile Val Ala Asp Asp Ile Ala Leu Pro
       130                 135                 140
   Asp Asn Lys Gln Pro Arg Gly Ile Ala Gly Thr Ala Leu Val His Lys
   145                 150                 155                 160
   Ile Ala Gly Tyr Ala Ala Glu Gln Gly Lys Ser Leu Ala Asp Val Arg
                   165                 170                 175
   Asp Ile Ala Gln Gln Ala Cys Asp Asn Ile Trp Ser Leu Gly Val Ala
               180                 185                 190
   Met Gln Thr Cys Asn Leu Pro Gly Ser Asp Asp Glu Glu Gly Arg Ile
           195                 200                 205
   Lys Asp Gly His Val Glu Leu Gly Leu Gly Ile His Gly Glu Pro Gly
       210                 215                 220
   Ala Ser Val Val Asp Thr His Asn Ser Lys Glu Ile Ile Asp Thr Leu
   225                 230                 235                 240
   Val Lys Pro Leu Lys Glu Thr Ala Gly Glu Gly Lys Phe Ala Val Leu
                   245                 250                 255
   Ile Asn Asn Leu Gly Gly Val Ser Ala Leu Glu Met Ala Leu Leu Thr
               260                 265                 270
   Lys Glu Leu Ala Asp Ser Ala Leu Lys Glu Asn Ile Ala Tyr Leu Ile
           275                 280                 285
   Gly Pro Ala Pro Leu Val Ser Ser Leu Asp Met Lys Gly Phe Ser Leu
       290                 295                 300
   Ser Leu Leu Gln Leu Asn Asp Thr Phe Glu Lys Ala Ile Asn Ala Pro
   305                 310                 315                 320
   Val Glu Thr Ile Gly Trp Gln Lys Pro Val Ala Phe Ala Pro Leu Arg
   325 330 335
   Thr Leu Ser His Thr Ala Ile Gln Asp Arg Val Glu Phe Thr Pro Ser
   340 345 350
   Gly Asn Asp Glu Val Ala Ala Arg Val Ala Ala Ala Thr Gln Thr Leu
   355 360 365
   Leu Ala Leu Glu Asn Arg Leu Asn Ala Leu Asp Ala Lys Val Gly Asp
   370 375 380
   Gly Asp Thr Gly Ser Thr Phe Ala Gln Gly Ala Arg Glu Ile Ala Gln
   385 390 395 400
   Leu Leu Glu Gln Lys Gln Leu Pro Leu Asn Asp Leu Ser Lys Leu Leu
   405 410 415
   Leu Leu Ile Gly Glu Arg Leu Ala Thr Val Met Gly Gly Ser Ser Gly
   420 425 430
   Val Leu Met Ser Ile Phe Phe Thr Ala Ala Gly Gln Lys Met His Asp
   435 440 445
   Gly Lys Ser Leu Pro Glu Ala Leu Leu Ser Gly Leu Ala Gln Met Lys
   450 455 460
   His Tyr Gly Gly Ala Asp Leu Gly Asp Arg Thr Leu Ile Asp Ala Leu
   465 470 475 480
   Gln Pro Ala Leu Glu Thr Leu His Asn Gly Asp Ile Gln Ala Ala Ala
   485 490 495
   Gln Ala Ala Lys Lys Gly Ala Asp Ala Thr Ala Gly Met Gln Lys Ala
   500 505 510
   Gly Ala Gly Arg Ser Ser Tyr Val Asn Lys Glu Asn Leu Glu Gly Val
   515 520 525
   Ile Asp Pro Gly Ala Val Ala Val Ala Glu Val Phe Ala Ala Val Ala
   530 535 540
   Lys Ala Lys Gln
   545
<210> 52
   <211> 1695
   <212> DNA
   <213> Psychromonas sp. CNPT3
<220>
   <221> CDS
   <222> (1).. (1695)
<400> 52
   atg gtt ata tta ttt ttt aac cta ctt tat ttt aaa cta ctt ttt gga 48
   Met Val Ile Leu Phe Phe Asn Leu Leu Tyr Phe Lys Leu Leu Phe Gly
   1 5 10 15
   gta att atg agc cgt tta ttt att aat gat aaa gca acg tta gtc cat 96
   Val Ile Met Ser Arg Leu Phe Ile Asn Asp Lys Ala Thr Leu Val His
   20 25 30
   gat gcg att gat ggc atc tta tat agc aat aag cat aat aac ctt gtt 144
   Asp Ala Ile Asp Gly Ile Leu Tyr Ser Asn Lys His Asn Asn Leu Val
   35 40 45
   cgt ttg gat gtt gat ccg caa att aga att gtg acg cgt aat gat tgg 192
   Arg Leu Asp Val Asp Pro Gln Ile Arg Ile Val Thr Arg Asn Asp Trp
   50 55 60
   cat cac gat aaa gtt gcc att atc tct ggt ggt ggc tct ggg cat gag 240
   His His Asp Lys Val Ala Ile Ile Ser Gly Gly Gly Ser Gly His Glu
   65                  70                  75                  80
   cct gct cat gtt ggt ttt att ggt aaa ggt atg tta acg gct gct gtt      288
   Pro Ala His Val Gly Phe Ile Gly Lys Gly Met Leu Thr Ala Ala Val
                   85                  90                  95
   tgt ggc gat gtt ttt gcc tca cca agt gtg gat gcg gtg tta aat gcc      336
   Cys Gly Asp Val Phe Ala Ser Pro Ser Val Asp Ala Val Leu Asn Ala
               100                 105                 110
   ata gtc cat gtc aca gga gag aaa ggt tgt ttg gtc att gtt aaa aac      384
   Ile Val His Val Thr Gly Glu Lys Gly Cys Leu Val Ile Val Lys Asn
           115                 120                 125
   tat acc ggc gac cgt ttg aac ttt ggc ctt gct tgt gaa aaa gcc aaa      432
   Tyr Thr Gly Asp Arg Leu Asn Phe Gly Leu Ala Cys Glu Lys Ala Lys
       130                 135                 140
   aaa atg ggt tta aac gtt gaa atg gta ata gta gac gat gat atc tct      480
   Lys Met Gly Leu Asn Val Glu Met Val Ile Val Asp Asp Asp Ile Ser
   145                 150                 155                 160
   att cct gac aat ctt aaa cct cgt ggt att gct ggc aca ttg ttt gtc      528
   Ile Pro Asp Asn Leu Lys Pro Arg Gly Ile Ala Gly Thr Leu Phe Val
                   165                 170                 175
   cat aaa gtg gca ggt aat gca gca gag caa ggc gct tct tta aat gtc      576
   His Lys Val Ala Gly Asn Ala Ala Glu Gln Gly Ala Ser Leu Asn Val
               180                 185                 190
   gtt aaa aag gca gcc caa ggt gcg att gat gca acg gca agt att ggc      624
   Val Lys Lys Ala Ala Gln Gly Ala Ile Asp Ala Thr Ala Ser Ile Gly
           195                 200                 205
   ctc gca ctg aca agt tgc tct tta ccg gga gaa gaa tca acg cag cgt      672
   Leu Ala Leu Thr Ser Cys Ser Leu Pro Gly Glu Glu Ser Thr Gln Arg
       210                 215                 220
   att gca gaa ggc aag gct gaa tta ggg tta ggt att cat gga gag cct      720
   Ile Ala Glu Gly Lys Ala Glu Leu Gly Leu Gly Ile His Gly Glu Pro
   225                 230                 235                 240
   ggc att aaa aca att gat gta aca tgt tgt cgt gat ctt gtg atg atc      768
   Gly Ile Lys Thr Ile Asp Val Thr Cys Cys Arg Asp Leu Val Met Ile
                   245                 250                 255
   atg gtt gat aaa cta aaa caa tcg ttt tct gct cca gat att aaa att      816
   Met Val Asp Lys Leu Lys Gln Ser Phe Ser Ala Pro Asp Ile Lys Ile
               260                 265                 270
   gca gtg atg atc aac aat tta ggt ggc gtt tct cca tta gag atg agc      864
   Ala Val Met Ile Asn Asn Leu Gly Gly Val Ser Pro Leu Glu Met Ser
           275                 280                 285
   ctt ata tgt aaa gac atc gtg gaa tct gag tta aaa aat aat att gag      912
   Leu Ile Cys Lys Asp Ile Val Glu Ser Glu Leu Lys Asn Asn Ile Glu
       290                 295                 300
   tta gtg gtt ggg cct gct cca ttt atg acg gct att gat atg aaa gga      960
   Leu Val Val Gly Pro Ala Pro Phe Met Thr Ala Ile Asp Met Lys Gly
   305                 310                 315                 320
   ttt tca att tca gtg att gaa tta aca ggc gat cat gct caa gct ctg     1008
   Phe Ser Ile Ser Val Ile Glu Leu Thr Gly Asp His Ala Gln Ala Leu
                   325                 330                 335
   tgc gcc cct gtt gaa gtg gat gca tgg gtt gaa gcc att cca ttg cgc     1056
   Cys Ala Pro Val Glu Val Asp Ala Trp Val Glu Ala Ile Pro Leu Arg
               340                 345                 350
   cca tta aat gtg ata aaa aaa gat aaa gtg tcc att aac ttt gca ttt     1104
   Pro Leu Asn Val Ile Lys Lys Asp Lys Val Ser Ile Asn Phe Ala Phe
           355                 360                 365
   gaa gcg tct gaa aat gca cag gtt gca agt att gtt aaa aca gta acc      1152
   Glu Ala Ser Glu Asn Ala Gln Val Ala Ser Ile Val Lys Thr Val Thr
       370                 375                 380
   ttg gct ttg att aat gca gaa aaa gag tta aat cgt ctg gat acg tta      1200
   Leu Ala Leu Ile Asn Ala Glu Lys Glu Leu Asn Arg Leu Asp Thr Leu
   385                 390                 395                 400
   gtg ggc gat ggg gat acc ggt tca acg ttc tct gcg ggt gct cga caa      1248
   Val Gly Asp Gly Asp Thr Gly Ser Thr Phe Ser Ala Gly Ala Arg Gln
                   405                 410                 415
   gtt tta gct gag ctc aat gcg ggt aat tta cca ctt aat gat act ggc      1296
   Val Leu Ala Glu Leu Asn Ala Gly Asn Leu Pro Leu Asn Asp Thr Gly
               420                 425                 430
   gcg ttg ctt aat gtc att ggg gaa caa ctt gct acc gtt atg ggg gga      1344
   Ala Leu Leu Asn Val Ile Gly Glu Gln Leu Ala Thr Val Met Gly Gly
           435                 440                 445
   tcg tca ggc gta tta ttc tct atc ttc ttc aca gca gcg ggt cat cat      1392
   Ser Ser Gly Val Leu Phe Ser Ile Phe Phe Thr Ala Ala Gly His His
       450                 455                 460
   tac cag caa cat ggt gat aca gta caa gca tta caa gcc ggt tta caa      1440
   Tyr Gln Gln His Gly Asp Thr Val Gln Ala Leu Gln Ala Gly Leu Gln
   465                 470                 475                 480
   caa atg atg caa tac ggt gga gca aaa ccg ggt gat cgt acg atg att      1488
   Gln Met Met Gln Tyr Gly Gly Ala Lys Pro Gly Asp Arg Thr Met Ile
                   485                 490                 495
   gat gca atg tac cca gcc ttt atc gct tgg aaa aat gaa ggt ttt gaa      1536
   Asp Ala Met Tyr Pro Ala Phe Ile Ala Trp Lys Asn Glu Gly Phe Glu
               500                 505                 510
   gct gcc att gtt gcg gct aaa ata ggg gca gaa agc aca gcc act atg      1584
   Ala Ala Ile Val Ala Ala Lys Ile Gly Ala Glu Ser Thr Ala Thr Met
           515                 520                 525
   gtc gaa gcc aaa gca gga cgc tct tct tat tta aac agt gag agt tta      1632
   Val Glu Ala Lys Ala Gly Arg Ser Ser Tyr Leu Asn Ser Glu Ser Leu
       530                 535                 540
   aaa ggt gtt aaa gat ccg ggc tca gtc gct gtt gag ttg gtt ttt gac      1680
   Lys Gly Val Lys Asp Pro Gly Ser Val Ala Val Glu Leu Val Phe Asp
   545                 550                 555                 560
   gcg ttt aat gtg taa                                                  1695
   Ala Phe Asn Val
<210> 53
   <211> 564
   <212> PRT
   <213> Psychromonas sp. CNPT3
<400> 53
   Met Val Ile Leu Phe Phe Asn Leu Leu Tyr Phe Lys Leu Leu Phe Gly
   1               5                   10                  15
   Val Ile Met Ser Arg Leu Phe Ile Asn Asp Lys Ala Thr Leu Val His
               20                  25                  30
   Asp Ala Ile Asp Gly Ile Leu Tyr Ser Asn Lys His Asn Asn Leu Val
           35                  40                  45
   Arg Leu Asp Val Asp Pro Gln Ile Arg Ile Val Thr Arg Asn Asp Trp
       50                  55                  60
   His His Asp Lys Val Ala Ile Ile Ser Gly Gly Gly Ser Gly His Glu
   65 70 75 80
   Pro Ala His Val Gly Phe Ile Gly Lys Gly Met Leu Thr Ala Ala Val
   85 90 95
   Cys Gly Asp Val Phe Ala Ser Pro Ser Val Asp Ala Val Leu Asn Ala
   100 105 110
   Ile Val His Val Thr Gly Glu Lys Gly Cys Leu Val Ile Val Lys Asn
   115 120 125
   Tyr Thr Gly Asp Arg Leu Asn Phe Gly Leu Ala Cys Glu Lys Ala Lys
   130 135 140
   Lys Met Gly Leu Asn Val Glu Met Val Ile Val Asp Asp Asp Ile Ser
   145 150 155 160
   Ile Pro Asp Asn Leu Lys Pro Arg Gly Ile Ala Gly Thr Leu Phe Val
   165 170 175
   His Lys Val Ala Gly Asn Ala Ala Glu Gln Gly Ala Ser Leu Asn Val
   180 185 190
   Val Lys Lys Ala Ala Gln Gly Ala Ile Asp Ala Thr Ala Ser Ile Gly
   195 200 205
   Leu Ala Leu Thr Ser Cys Ser Leu Pro Gly Glu Glu Ser Thr Gln Arg
   210 215 220
   Ile Ala Glu Gly Lys Ala Glu Leu Gly Leu Gly Ile His Gly Glu Pro
   225 230 235 240
   Gly Ile Lys Thr Ile Asp Val Thr Cys Cys Arg Asp Leu Val Met Ile
   245 250 255
   Met Val Asp Lys Leu Lys Gln Ser Phe Ser Ala Pro Asp Ile Lys Ile
   260 265 270
   Ala Val Met Ile Asn Asn Leu Gly Gly Val Ser Pro Leu Glu Met Ser
   275 280 285
   Leu Ile Cys Lys Asp Ile Val Glu Ser Glu Leu Lys Asn Asn Ile Glu
   290 295 300
   Leu Val Val Gly Pro Ala Pro Phe Met Thr Ala Ile Asp Met Lys Gly
   305 310 315 320
   Phe Ser Ile Ser Val Ile Glu Leu Thr Gly Asp His Ala Gln Ala Leu
   325 330 335
   Cys Ala Pro Val Glu Val Asp Ala Trp Val Glu Ala Ile Pro Leu Arg
   340 345 350
   Pro Leu Asn Val Ile Lys Lys Asp Lys Val Ser Ile Asn Phe Ala Phe
   355 360 365
   Glu Ala Ser Glu Asn Ala Gln Val Ala Ser Ile Val Lys Thr Val Thr
   370 375 380
   Leu Ala Leu Ile Asn Ala Glu Lys Glu Leu Asn Arg Leu Asp Thr Leu
   385 390 395 400
   Val Gly Asp Gly Asp Thr Gly Ser Thr Phe Ser Ala Gly Ala Arg Gln
   405 410 415
   Val Leu Ala Glu Leu Asn Ala Gly Asn Leu Pro Leu Asn Asp Thr Gly
   420 425 430
   Ala Leu Leu Asn Val Ile Gly Glu Gln Leu Ala Thr Val Met Gly Gly
   435 440 445
   Ser Ser Gly Val Leu Phe Ser Ile Phe Phe Thr Ala Ala Gly His His
   450 455 460
   Tyr Gln Gln His Gly Asp Thr Val Gln Ala Leu Gln Ala Gly Leu Gln
   465 470 475 480
   Gln Met Met Gln Tyr Gly Gly Ala Lys Pro Gly Asp Arg Thr Met Ile
   485 490 495
   Asp Ala Met Tyr Pro Ala Phe Ile Ala Trp Lys Asn Glu Gly Phe Glu
                   500                 505                 510
   Ala Ala Ile Val Ala Ala Lys Ile Gly Ala Glu Ser Thr Ala Thr Met
               515                 520                 525
   Val Glu Ala Lys Ala Gly Arg Ser Ser Tyr Leu Asn Ser Glu Ser Leu
           530                 535                 540
   Lys Gly Val Lys Asp Pro Gly Ser Val Ala Val Glu Leu Val Phe Asp
   545                 550                 555                 560
   Ala Phe Asn Val
<210> 54
   <211> 1647
   <212> DNA
   <213> Stapia aggregata IAM12614
<220>
   <221> CDS
   <222> (1).. (1647)
<400> 54
   atg aag caa ttc atc aat acc aag gaa acg ctc gtc acc gaa gcg att      48
   Met Lys Gln Phe Ile Asn Thr Lys Glu Thr Leu Val Thr Glu Ala Ile
   1               5                   10                  15
   gac ggc atg ttg cgc acg gcc ggc ggg cgg ctt gcc cgg ctt gac ggc      96
   Asp Gly Met Leu Arg Thr Ala Gly Gly Arg Leu Ala Arg Leu Asp Gly
               20                  25                  30
   tat ccg cat atc aag gtg gtc gtg cgc acc gac tgg gac aaa tcg aag      144
   Tyr Pro His Ile Lys Val Val Val Arg Thr Asp Trp Asp Lys Ser Lys
           35                  40                  45
   gtg gct ctg gtg tcc ggc ggc ggt tcc ggc cac gag ccg agc cat gcc      192
   Val Ala Leu Val Ser Gly Gly Gly Ser Gly His Glu Pro Ser His Ala
       50                  55                  60
   ggt ttc gtc ggc cag ggc atg ctg acg gcg gcc gtc tgc ggc gaa gtg      240
   Gly Phe Val Gly Gln Gly Met Leu Thr Ala Ala Val Cys Gly Glu Val
   65                  70                  75                  80
   ttt gcc tcg cct tcc gtg gat gcg gtg ctg gcg ggc att ctg gcc gtc      288
   Phe Ala Ser Pro Ser Val Asp Ala Val Leu Ala Gly Ile Leu Ala Val
                   85                  90                  95
   acc ggc aag gct ggc tgc ctg ctg atc gtc aag aac tac acc ggc gac      336
   Thr Gly Lys Ala Gly Cys Leu Leu Ile Val Lys Asn Tyr Thr Gly Asp
               100                 105                 110
   cgg ctg aac ttc ggt ctg gcc gcc gag cgg gcc cgc tcc ttc gga ctg      384
   Arg Leu Asn Phe Gly Leu Ala Ala Glu Arg Ala Arg Ser Phe Gly Leu
           115                 120                 125
   aag gtc aac atg gtg atc gtc gac gac gac gtt gcc ctg ccg gac ctg      432
   Lys Val Asn Met Val Ile Val Asp Asp Asp Val Ala Leu Pro Asp Leu
       130                 135                 140
   ccg cag gcg cgc ggt gtc gcc ggc acg ctg ttc gtg cac aag atc gcc      480
   Pro Gln Ala Arg Gly Val Ala Gly Thr Leu Phe Val His Lys Ile Ala
   145                 150                 155                 160
   gga gcg ctt gcc gat cag ggt gcg gat ctg gaa acc atc acg gag gcc      528
   Gly Ala Leu Ala Asp Gln Gly Ala Asp Leu Glu Thr Ile Thr Glu Ala
                   165                 170                 175
   gcc agg aaa acc atc ggc ggt gcg att tcc atc ggc atg tcg ctg gac      576
   Ala Arg Lys Thr Ile Gly Gly Ala Ile Ser Ile Gly Met Ser Leu Asp
                   180                 185                 190
   acc tgc acg gtg ccg gga tcg ccc aag gaa gac cgc att gca cac ggc      624
   Thr Cys Thr Val Pro Gly Ser Pro Lys Glu Asp Arg Ile Ala His Gly
               195                 200                 205
   aag gcg gaa ctc gga ctt ggc att cac ggc gag gcg ggg atc gag cag      672
   Lys Ala Glu Leu Gly Leu Gly Ile His Gly Glu Ala Gly Ile Glu Gln
       210                 215                 220
   gtc gac tat tcc aac gcc cgc gcg gcc atg gcc atg gtg gtg gac cgg      720
   Val Asp Tyr Ser Asn Ala Arg Ala Ala Met Ala Met Val Val Asp Arg
   225                 230                 235                 240
   ctg gcg ccg aac ctc tcg ccc gga ccg cat gtg gcg atc ctc aac aat      768
   Leu Ala Pro Asn Leu Ser Pro Gly Pro His Val Ala Ile Leu Asn Asn
                   245                 250                 255
   ctg ggc agc acg acg ccg ctg gaa atg tcg gtg ctt ctg gaa gaa ctc      816
   Leu Gly Ser Thr Thr Pro Leu Glu Met Ser Val Leu Leu Glu Glu Leu
               260                 265                 270
   acg gct tcg cgc atc ggc agc cag atc cgc tgg gtc atc ggc ccg gcg      864
   Thr Ala Ser Arg Ile Gly Ser Gln Ile Arg Trp Val Ile Gly Pro Ala
           275                 280                 285
   gcg atg atg acc tcg ctc gac atg cat ggg ttc tcc gtg tcg ctg ctg      912
   Ala Met Met Thr Ser Leu Asp Met His Gly Phe Ser Val Ser Leu Leu
       290                 295                 300
   ccg gtc ggc aag acc gaa gaa gcc ttg ctg cag gcc ccg gtc gcg ccc      960
   Pro Val Gly Lys Thr Glu Glu Ala Leu Leu Gln Ala Pro Val Ala Pro
   305                 310                 315                 320
   tgg gca tgg ccc ggc tgc ctt gcg ctt ggc gca gtg tcc gtg ctg ccg     1008
   Trp Ala Trp Pro Gly Cys Leu Ala Leu Gly Ala Val Ser Val Leu Pro
                   325                 330                 335
   cta ccg gac ggc ctg acg ccg atc cag ccg ctg ccg tcc aag aac ccg     1056
   Leu Pro Asp Gly Leu Thr Pro Ile Gln Pro Leu Pro Ser Lys Asn Pro
               340                 345                 350
   gag acg cgg aag ttc atc gag cgc tgc tgc gat atc ctg atc gcc gcc     1104
   Glu Thr Arg Lys Phe Ile Glu Arg Cys Cys Asp Ile Leu Ile Ala Ala
           355                 360                 365
   gag gac gac ctc aat gcg ctc gac gcc aag tcg ggc gac ggc gac acc     1152
   Glu Asp Asp Leu Asn Ala Leu Asp Ala Lys Ser Gly Asp Gly Asp Thr
       370                 375                 380
   ggc agc acg ctt gcc acc gcc gcc cgg gcg ctg gtg aag gcg ctc gac     1200
   Gly Ser Thr Leu Ala Thr Ala Ala Arg Ala Leu Val Lys Ala Leu Asp
   385                 390                 395                 400
   cgg ctg ccg ctg gcg gat ctc acc cag ctc tac cgg gcc atc ggg ctg     1248
   Arg Leu Pro Leu Ala Asp Leu Thr Gln Leu Tyr Arg Ala Ile Gly Leu
                   405                 410                 415
   gaa ctc agc cag acc atg ggc ggg tct tcc ggg gtg ctg ctg gcg atc     1296
   Glu Leu Ser Gln Thr Met Gly Gly Ser Ser Gly Val Leu Leu Ala Ile
               420                 425                 430
   ttc ttt gcc gct gcg ggc gat gcg tcg tcc agc ggg cgc ggg gcc atc     1344
   Phe Phe Ala Ala Ala Gly Asp Ala Ser Ser Ser Gly Arg Gly Ala Ile
           435                 440                 445
   gga gcc ctg aag gcc ggt ctt gac cgg atc atg cag gtg ggc ggc gcc     1392
   Gly Ala Leu Lys Ala Gly Leu Asp Arg Ile Met Gln Val Gly Gly Ala
       450                 455                 460
   cag ccg ggc gac cgc acc atg atc gac gcg ctg ctg ccg gca ctg aat     1440
   Gln Pro Gly Asp Arg Thr Met Ile Asp Ala Leu Leu Pro Ala Leu Asn
   465                 470                 475                 480
   gcg ctg gaa aac ggt atc gag gct gcg gcg agc gag gcc cgt cag ggg      1488
   Ala Leu Glu Asn Gly Ile Glu Ala Ala Ala Ser Glu Ala Arg Gln Gly
                   485                 490                 495
   gcg gat gcg acg tcg cgg atc acg cgg gca cgc gcg ggc agg gcg tct      1536
   Ala Asp Ala Thr Ser Arg Ile Thr Arg Ala Arg Ala Gly Arg Ala Ser
               500                 505                 510
   tat gtc tcc gag gcc agc ctc tcc gga cac aac gat ccg ggc gcg gaa      1584
   Tyr Val Ser Glu Ala Ser Leu Ser Gly His Asn Asp Pro Gly Ala Glu
           515                 520                 525
   gcc gtt gcg cgg ctg ttc gag caa ttg acc ctt tct ccg gcc ctt acc      1632
   Ala Val Ala Arg Leu Phe Glu Gln Leu Thr Leu Ser Pro Ala Leu Thr
       530                 535                 540
   tcc aag tcc gca tag                                                  1647
   Ser Lys Ser Ala
   545
<210> 55
   <211> 548
   <212> PRT
   <213> Stapia aggregata IAM12614
<400> 55
   Met Lys Gln Phe Ile Asn Thr Lys Glu Thr Leu Val Thr Glu Ala Ile
   1               5                   10                  15
   Asp Gly Met Leu Arg Thr Ala Gly Gly Arg Leu Ala Arg Leu Asp Gly
               20                  25                  30
   Tyr Pro His Ile Lys Val Val Val Arg Thr Asp Trp Asp Lys Ser Lys
           35                  40                  45
   Val Ala Leu Val Ser Gly Gly Gly Ser Gly His Glu Pro Ser His Ala
       50                  55                  60
   Gly Phe Val Gly Gln Gly Met Leu Thr Ala Ala Val Cys Gly Glu Val
   65                  70                  75                  80
   Phe Ala Ser Pro Ser Val Asp Ala Val Leu Ala Gly Ile Leu Ala Val
                   85                  90                  95
   Thr Gly Lys Ala Gly Cys Leu Leu Ile Val Lys Asn Tyr Thr Gly Asp
               100                 105                 110
   Arg Leu Asn Phe Gly Leu Ala Ala Glu Arg Ala Arg Ser Phe Gly Leu
           115                 120                 125
   Lys Val Asn Met Val Ile Val Asp Asp Asp Val Ala Leu Pro Asp Leu
       130                 135                 140
   Pro Gln Ala Arg Gly Val Ala Gly Thr Leu Phe Val His Lys Ile Ala
   145                 150                 155                 160
   Gly Ala Leu Ala Asp Gln Gly Ala Asp Leu Glu Thr Ile Thr Glu Ala
                   165                 170                 175
   Ala Arg Lys Thr Ile Gly Gly Ala Ile Ser Ile Gly Met Ser Leu Asp
               180                 185                 190
   Thr Cys Thr Val Pro Gly Ser Pro Lys Glu Asp Arg Ile Ala His Gly
           195                 200                 205
   Lys Ala Glu Leu Gly Leu Gly Ile His Gly Glu Ala Gly Ile Glu Gln
       210                 215                 220
   Val Asp Tyr Ser Asn Ala Arg Ala Ala Met Ala Met Val Val Asp Arg
   225                 230                 235                 240
   Leu Ala Pro Asn Leu Ser Pro Gly Pro His Val Ala Ile Leu Asn Asn
   245 250 255
   Leu Gly Ser Thr Thr Pro Leu Glu Met Ser Val Leu Leu Glu Glu Leu
   260 265 270
   Thr Ala Ser Arg Ile Gly Ser Gln Ile Arg Trp Val Ile Gly Pro Ala
   275 280 285
   Ala Met Met Thr Ser Leu Asp Met His Gly Phe Ser Val Ser Leu Leu
   290 295 300
   Pro Val Gly Lys Thr Glu Glu Ala Leu Leu Gln Ala Pro Val Ala Pro
   305 310 315 320
   Trp Ala Trp Pro Gly Cys Leu Ala Leu Gly Ala Val Ser Val Leu Pro
   325 330 335
   Leu Pro Asp Gly Leu Thr Pro Ile Gln Pro Leu Pro Ser Lys Asn Pro
   340 345 350
   Glu Thr Arg Lys Phe Ile Glu Arg Cys Cys Asp Ile Leu Ile Ala Ala
   355 360 365
   Glu Asp Asp Leu Asn Ala Leu Asp Ala Lys Ser Gly Asp Gly Asp Thr
   370 375 380
   Gly Ser Thr Leu Ala Thr Ala Ala Arg Ala Leu Val Lys Ala Leu Asp
   385 390 395 400
   Arg Leu Pro Leu Ala Asp Leu Thr Gln Leu Tyr Arg Ala Ile Gly Leu
   405 410 415
   Glu Leu Ser Gln Thr Met Gly Gly Ser Ser Gly Val Leu Leu Ala Ile
   420 425 430
   Phe Phe Ala Ala Ala Gly Asp Ala Ser Ser Ser Gly Arg Gly Ala Ile
   435 440 445
   Gly Ala Leu Lys Ala Gly Leu Asp Arg Ile Met Gln Val Gly Gly Ala
   450 455 460
   Gln Pro Gly Asp Arg Thr Met Ile Asp Ala Leu Leu Pro Ala Leu Asn
   465 470 475 480
   Ala Leu Glu Asn Gly Ile Glu Ala Ala Ala Ser Glu Ala Arg Gln Gly
   485 490 495
   Ala Asp Ala Thr Ser Arg Ile Thr Arg Ala Arg Ala Gly Arg Ala Ser
   500 505 510
   Tyr Val Ser Glu Ala Ser Leu Ser Gly His Asn Asp Pro Gly Ala Glu
   515 520 525
   Ala Val Ala Arg Leu Phe Glu Gln Leu Thr Leu Ser Pro Ala Leu Thr
   530 535 540
   Ser Lys Ser Ala
   545
<210> 56
   <211> 1641
   <212> DNA
   <213> Rhizobium leguminosarum
<220>
   <221> CDS
   <222> (1).. (1641)
<400> 56
   atg aaa cac ttc ttc aac cgc agg gaa aac atc gtc acc gaa gcc ttg 48
   Met Lys His Phe Phe Asn Arg Arg Glu Asn Ile Val Thr Glu Ala Leu
   1 5 10 15
   gac ggt ctg ctt ctg acg agc agc aag ggt cgt ctt gcc cgc ctc gac 96
   Asp Gly Leu Leu Leu Thr Ser Ser Lys Gly Arg Leu Ala Arg Leu Asp
                   20                  25                  30
   agc ttt ccc gac atc aag gtg atc ctg cgc gct gac tgg gac aag tcg      144
   Ser Phe Pro Asp Ile Lys Val Ile Leu Arg Ala Asp Trp Asp Lys Ser
           35                  40                  45
   aag gtg gcg atc atc tca ggc ggc ggc gcc ggt cat gag ccc tcc cat      192
   Lys Val Ala Ile Ile Ser Gly Gly Gly Ala Gly His Glu Pro Ser His
       50                  55                  60
   gcc ggc ttc gtc ggt aag ggc atg ctg acg gct gcc gta tcc ggc gag      240
   Ala Gly Phe Val Gly Lys Gly Met Leu Thr Ala Ala Val Ser Gly Glu
   65                  70                  75                  80
   att ttc gcc tcg ccg agc gtc gat gcc gtg ctg aca gcg atc cgc gcc      288
   Ile Phe Ala Ser Pro Ser Val Asp Ala Val Leu Thr Ala Ile Arg Ala
                   85                  90                  95
   gtc gcc ggc gaa aag ggc gcc ttg ctg atc gtc aag aac tat acc ggc      336
   Val Ala Gly Glu Lys Gly Ala Leu Leu Ile Val Lys Asn Tyr Thr Gly
               100                 105                 110
   gac cgg ctg aat ttc ggc ctc gcc gcc gag aag gcg cgc gcc gaa ggt      384
   Asp Arg Leu Asn Phe Gly Leu Ala Ala Glu Lys Ala Arg Ala Glu Gly
           115                 120                 125
   ttc gac gtc gaa atg gtc atc gtc gcc gac gat atc gcc atc ccc gag      432
   Phe Asp Val Glu Met Val Ile Val Ala Asp Asp Ile Ala Ile Pro Glu
       130                 135                 140
   atc aac cag ccg cgc ggc gtc gcc ggg act ctg ttc gtc cac aag atc      480
   Ile Asn Gln Pro Arg Gly Val Ala Gly Thr Leu Phe Val His Lys Ile
   145                 150                 155                 160
   gct ggc tat cac gcc gaa agg ggc gag gac ctg aag acg gtc gca gcc      528
   Ala Gly Tyr His Ala Glu Arg Gly Glu Asp Leu Lys Thr Val Ala Ala
                   165                 170                 175
   cat gcc gcg gca gcg gcc ggc gac atc gtc tcg ctc ggc atg tct ctg      576
   His Ala Ala Ala Ala Ala Gly Asp Ile Val Ser Leu Gly Met Ser Leu
               180                 185                 190
   tcc acc tgc agc gtg ccc ggc cag gcg cat gag agc cgc ctc ggc gag      624
   Ser Thr Cys Ser Val Pro Gly Gln Ala His Glu Ser Arg Leu Gly Glu
           195                 200                 205
   aac gag ggc gaa ctc ggt ctc ggc atc cat ggc gag ccc ggc gtc gag      672
   Asn Glu Gly Glu Leu Gly Leu Gly Ile His Gly Glu Pro Gly Val Glu
       210                 215                 220
   cgc att gcg ctg cag ccg gtc gtc gat atc gtc gcc acc atg gtg gcg      720
   Arg Ile Ala Leu Gln Pro Val Val Asp Ile Val Ala Thr Met Val Ala
   225                 230                 235                 240
   cgc cta tcg cct gcg ctg cgc gaa ggg gga aac cac gcc ctt ctc atc      768
   Arg Leu Ser Pro Ala Leu Arg Glu Gly Gly Asn His Ala Leu Leu Ile
                   245                 250                 255
   aac aat ctc ggc gcc gta ccg ccg ctc gaa atg acc gtt att gcc aat      816
   Asn Asn Leu Gly Ala Val Pro Pro Leu Glu Met Thr Val Ile Ala Asn
               260                 265                 270
   gtg gtg ctg tcc tcg tcg ctt gcc gat cgc gtc agg ctg atc atc ggc      864
   Val Val Leu Ser Ser Ser Leu Ala Asp Arg Val Arg Leu Ile Ile Gly
           275                 280                 285
   ccg gcg ccg atg atg acc gcg ctc aac atg aac ggc ttc tcg ctg tcg      912
   Pro Ala Pro Met Met Thr Ala Leu Asn Met Asn Gly Phe Ser Leu Ser
       290                 295                 300
   ctg atc cga ctg gat gcc gct cgc gag gcg gcg ctg acg gca gcg gtc      960
   Leu Ile Arg Leu Asp Ala Ala Arg Glu Ala Ala Leu Thr Ala Ala Val
   305 310 315 320
   gaa ccg cat gcc tgg atg cca gcc gtc gaa cgc cac gag atc agg gtc 1008
   Glu Pro His Ala Trp Met Pro Ala Val Glu Arg His Glu Ile Arg Val
   325 330 335
   atc gcc gca ccg cga aca tca gcc gga ctg aac ggc gcg cca gtg gcc 1056
   Ile Ala Ala Pro Arg Thr Ser Ala Gly Leu Asn Gly Ala Pro Val Ala
   340 345 350
   ggg gat aat ctc cgc aac cgg cgt ctg atc aca gcg ctc tgc gag cat 1104
   Gly Asp Asn Leu Arg Asn Arg Arg Leu Ile Thr Ala Leu Cys Glu His
   355 360 365
   ctg atc tcg cag gaa agc gaa ctc aac cgg ctg gat ggc cgc gtc ggc 1152
   Leu Ile Ser Gln Glu Ser Glu Leu Asn Arg Leu Asp Gly Arg Val Gly
   370 375 380
   gac ggt gat acc ggc tcg acg gtg gcg aca ggc gcc cgc agc gtg ctt 1200
   Asp Gly Asp Thr Gly Ser Thr Val Ala Thr Gly Ala Arg Ser Val Leu
   385 390 395 400
   gcc cgc ctg gac acg ctg ccg ctt gat cgg ccg gct gca acg ctt gcc 1248
   Ala Arg Leu Asp Thr Leu Pro Leu Asp Arg Pro Ala Ala Thr Leu Ala
   405 410 415
   tcg ctc ggc gac atc ctc ggc acc agc atg ggc gga tcg agc ggc gtg 1296
   Ser Leu Gly Asp Ile Leu Gly Thr Ser Met Gly Gly Ser Ser Gly Val
   420 425 430
   ctg ctg tcg atc ttc ttc acc gca gcg gca aag gcg atg gcc gac aag 1344
   Leu Leu Ser Ile Phe Phe Thr Ala Ala Ala Lys Ala Met Ala Asp Lys
   435 440 445
   gcc gat ata tca gca gcc ctt att gcc ggg ctc gac agg atg acg ttc 1392
   Ala Asp Ile Ser Ala Ala Leu Ile Ala Gly Leu Asp Arg Met Thr Phe
   450 455 460
   tat ggc gga gcc gaa gtc ggc gac cgg acg atg gtc gat gcg ctg tcg 1440
   Tyr Gly Gly Ala Glu Val Gly Asp Arg Thr Met Val Asp Ala Leu Ser
   465 470 475 480
   cct gcc ctg cag gcg ctc gca tcc ggc gat gtc gcg gca gcg gcc agg 1488
   Pro Ala Leu Gln Ala Leu Ala Ser Gly Asp Val Ala Ala Ala Ala Arg
   485 490 495
   gct gct gcc gca ggt gcg gag tcg acg aag acg atg atg aaa gcg aga 1536
   Ala Ala Ala Ala Gly Ala Glu Ser Thr Lys Thr Met Met Lys Ala Arg
   500 505 510
   gcc ggc cgc gcc tcc tat gtc ggc gaa agg gat ctg gca ggt gtc gct 1584
   Ala Gly Arg Ala Ser Tyr Val Gly Glu Arg Asp Leu Ala Gly Val Ala
   515 520 525
   gat ccc ggc gcc gtc gcg gtt gcc ggc gcg ttc ggt gtg gcg gca agc 1632
   Asp Pro Gly Ala Val Ala Val Ala Gly Ala Phe Gly Val Ala Ala Ser
   530 535 540
   ctc gcc tga 1641
   Leu Ala
   545
<210> 57
   <211> 546
   <212> PRT
   <213> Rhizobium leguminosarum
<400> 57
   Met Lys His Phe Phe Asn Arg Arg Glu Asn Ile Val Thr Glu Ala Leu
   1 5 10 15
   Asp Gly Leu Leu Leu Thr Ser Ser Lys Gly Arg Leu Ala Arg Leu Asp
   20 25 30
   Ser Phe Pro Asp Ile Lys Val Ile Leu Arg Ala Asp Trp Asp Lys Ser
   35 40 45
   Lys Val Ala Ile Ile Ser Gly Gly Gly Ala Gly His Glu Pro Ser His
   50 55 60
   Ala Gly Phe Val Gly Lys Gly Met Leu Thr Ala Ala Val Ser Gly Glu
   65 70 75 80
   Ile Phe Ala Ser Pro Ser Val Asp Ala Val Leu Thr Ala Ile Arg Ala
   85 90 95
   Val Ala Gly Glu Lys Gly Ala Leu Leu Ile Val Lys Asn Tyr Thr Gly
   100 105 110
   Asp Arg Leu Asn Phe Gly Leu Ala Ala Glu Lys Ala Arg Ala Glu Gly
   115 120 125
   Phe Asp Val Glu Met Val Ile Val Ala Asp Asp Ile Ala Ile Pro Glu
   130 135 140
   Ile Asn Gln Pro Arg Gly Val Ala Gly Thr Leu Phe Val His Lys Ile
   145 150 155 160
   Ala Gly Tyr His Ala Glu Arg Gly Glu Asp Leu Lys Thr Val Ala Ala
   165 170 175
   His Ala Ala Ala Ala Ala Gly Asp Ile Val Ser Leu Gly Met Ser Leu
   180 185 190
   Ser Thr Cys Ser Val Pro Gly Gln Ala His Glu Ser Arg Leu Gly Glu
   195 200 205
   Asn Glu Gly Glu Leu Gly Leu Gly Ile His Gly Glu Pro Gly Val Glu
   210 215 220
   Arg Ile Ala Leu Gln Pro Val Val Asp Ile Val Ala Thr Met Val Ala
   225 230 235 240
   Arg Leu Ser Pro Ala Leu Arg Glu Gly Gly Asn His Ala Leu Leu Ile
   245 250 255
   Asn Asn Leu Gly Ala Val Pro Pro Leu Glu Met Thr Val Ile Ala Asn
   260 265 270
   Val Val Leu Ser Ser Ser Leu Ala Asp Arg Val Arg Leu Ile Ile Gly
   275 280 285
   Pro Ala Pro Met Met Thr Ala Leu Asn Met Asn Gly Phe Ser Leu Ser
   290 295 300
   Leu Ile Arg Leu Asp Ala Ala Arg Glu Ala Ala Leu Thr Ala Ala Val
   305 310 315 320
   Glu Pro His Ala Trp Met Pro Ala Val Glu Arg His Glu Ile Arg Val
   325 330 335
   Ile Ala Ala Pro Arg Thr Ser Ala Gly Leu Asn Gly Ala Pro Val Ala
   340 345 350
   Gly Asp Asn Leu Arg Asn Arg Arg Leu Ile Thr Ala Leu Cys Glu His
   355 360 365
   Leu Ile Ser Gln Glu Ser Glu Leu Asn Arg Leu Asp Gly Arg Val Gly
   370 375 380
   Asp Gly Asp Thr Gly Ser Thr Val Ala Thr Gly Ala Arg Ser Val Leu
   385 390 395 400
   Ala Arg Leu Asp Thr Leu Pro Leu Asp Arg Pro Ala Ala Thr Leu Ala
   405 410 415
   Ser Leu Gly Asp Ile Leu Gly Thr Ser Met Gly Gly Ser Ser Gly Val
   420 425 430
   Leu Leu Ser Ile Phe Phe Thr Ala Ala Ala Lys Ala Met Ala Asp Lys
   435 440 445
   Ala Asp Ile Ser Ala Ala Leu Ile Ala Gly Leu Asp Arg Met Thr Phe
       450                 455                 460
   Tyr Gly Gly Ala Glu Val Gly Asp Arg Thr Met Val Asp Ala Leu Ser
   465                 470                 475                 480
   Pro Ala Leu Gln Ala Leu Ala Ser Gly Asp Val Ala Ala Ala Ala Arg
                   485                 490                 495
   Ala Ala Ala Ala Gly Ala Glu Ser Thr Lys Thr Met Met Lys Ala Arg
               500                 505                 510
   Ala Gly Arg Ala Ser Tyr Val Gly Glu Arg Asp Leu Ala Gly Val Ala
           515                 520                 525
   Asp Pro Gly Ala Val Ala Val Ala Gly Ala Phe Gly Val Ala Ala Ser
       530                 535                 540
   Leu Ala
   545
<210> 58
   <211> 1701
   <212> DNA
   <213> Myxococcus xanthus
<220>
   <221> CDS
   <222> (1).. (1701)
<400> 58
   atg aag aag ctg gtc aac gcc cct cgc gcg gtg gtg cgg gag atg ctg      48
   Met Lys Lys Leu Val Asn Ala Pro Arg Ala Val Val Arg Glu Met Leu
   1               5                   10                  15
   gag ggg ttg gtc tcg ctc gcc ccc ggg cag gtg ctg ctg gac ggg gag      96
   Glu Gly Leu Val Ser Leu Ala Pro Gly Gln Val Leu Leu Asp Gly Glu
               20                  25                  30
   tcg gtg gtg ctc cgc gcc gac acg cct tcc gac gtc cgc gcg cgc aag     144
   Ser Val Val Leu Arg Ala Asp Thr Pro Ser Asp Val Arg Ala Arg Lys
           35                  40                  45
   gtg gct gtc atc tcc ggt ggc ggc agc ggc cat gag ccg gcg cac gcg     192
   Val Ala Val Ile Ser Gly Gly Gly Ser Gly His Glu Pro Ala His Ala
       50                  55                  60
   ggc tac gtg ggc gcg ggc atg ctg gac gcg gcg gtg gcc ggt gac gtc     240
   Gly Tyr Val Gly Ala Gly Met Leu Asp Ala Ala Val Ala Gly Asp Val
   65                  70                  75                  80
   ttc acc tcg ccc agc acc gat gcc gtg ctg gcc gcc atc cgc gcc gtc     288
   Phe Thr Ser Pro Ser Thr Asp Ala Val Leu Ala Ala Ile Arg Ala Val
                   85                  90                  95
   gcg ggg ccc gcg ggc gcg ctg ctc gtc gtg aag aac tac acc ggg gac     336
   Ala Gly Pro Ala Gly Ala Leu Leu Val Val Lys Asn Tyr Thr Gly Asp
               100                 105                 110
   cgg ctc aac ttc ggg ctc gcc gcc gag ctg gcg cgc gcc gag ggc atc     384
   Arg Leu Asn Phe Gly Leu Ala Ala Glu Leu Ala Arg Ala Glu Gly Ile
           115                 120                 125
   ccc gtg gag acg gtg gtg gtg gcg gac gac gtg tcc ctg cac gac acg     432
   Pro Val Glu Thr Val Val Val Ala Asp Asp Val Ser Leu His Asp Thr
       130                 135                 140
   gtg gag ccc gcg cgg cgc cgg ggc atc gct ggc acg gtg ctg gtc cac     480
   Val Glu Pro Ala Arg Arg Arg Gly Ile Ala Gly Thr Val Leu Val His
   145                 150                 155                 160
   aag gtc gcg ggc gcg gcg gcc gag gcg ggc gcg gcg ctc cag gac gtc      528
   Lys Val Ala Gly Ala Ala Ala Glu Ala Gly Ala Ala Leu Gln Asp Val
                   165                 170                 175
   ctc cgc gag gcc acc gcg gcg gcg gag gtg ctg ggc acc atg ggc gtg      576
   Leu Arg Glu Ala Thr Ala Ala Ala Glu Val Leu Gly Thr Met Gly Val
               180                 185                 190
   gcc ctg ggg ccc tgc acc gtg ccc gcg gcg ggc aag ccg ggc ttc acg      624
   Ala Leu Gly Pro Cys Thr Val Pro Ala Ala Gly Lys Pro Gly Phe Thr
           195                 200                 205
   ctg gag gag gac gaa atc gag ctg ggc ctg ggc atc cac ggc gag cag      672
   Leu Glu Glu Asp Glu Ile Glu Leu Gly Leu Gly Ile His Gly Glu Gln
       210                 215                 220
   ggc gtg cgg cgc gtg ccg atg cag acg gcg gac agc ctg gtg gac acg      720
   Gly Val Arg Arg Val Pro Met Gln Thr Ala Asp Ser Leu Val Asp Thr
   225                 230                 235                 240
   ctg ctc acc acc atc gtc gag gac cgg cgc atc acc tcg gga gac agg      768
   Leu Leu Thr Thr Ile Val Glu Asp Arg Arg Ile Thr Ser Gly Asp Arg
                   245                 250                 255
   gtg gtg ctg gtg gtc aac gga ttg ggc ggc acg ccg ccc atg gag ctg      816
   Val Val Leu Val Val Asn Gly Leu Gly Gly Thr Pro Pro Met Glu Leu
               260                 265                 270
   gcc atc gtc gcc cgg cgc gca ctg gcc gct ctg cgt cag ggc ggc atc      864
   Ala Ile Val Ala Arg Arg Ala Leu Ala Ala Leu Arg Gln Gly Gly Ile
           275                 280                 285
   cgc gtg gag cgc gcg tgg agc ggg acg ttc ctc tcc gcg ctg gag atg      912
   Arg Val Glu Arg Ala Trp Ser Gly Thr Phe Leu Ser Ala Leu Glu Met
       290                 295                 300
   ccc ggc tgc tcg ttg acg ctg ctg aag gtg gac gac gcg cgg ctg gcc      960
   Pro Gly Cys Ser Leu Thr Leu Leu Lys Val Asp Asp Ala Arg Leu Ala
   305                 310                 315                 320
   cgc ctg gat gcg gcg gtg gat gcg ccc gcg tgg ccc ggc gcg gga cgg     1008
   Arg Leu Asp Ala Ala Val Asp Ala Pro Ala Trp Pro Gly Ala Gly Arg
                   325                 330                 335
   ctg ccg aag gag ccg ggg gtg tac cgg cct tcg tcc acg gcg tct cca     1056
   Leu Pro Lys Glu Pro Gly Val Tyr Arg Pro Ser Ser Thr Ala Ser Pro
               340                 345                 350
   gca tcg ctt ccg gcg gag gcg ccg caa ccg ggg atg gac cgc ttc cgg     1104
   Ala Ser Leu Pro Ala Glu Ala Pro Gln Pro Gly Met Asp Arg Phe Arg
           355                 360                 365
   aag gcc gcc ttg cgg gtg gcg gac gca ttc gag cag tcg gag ccc cgg     1152
   Lys Ala Ala Leu Arg Val Ala Asp Ala Phe Glu Gln Ser Glu Pro Arg
       370                 375                 380
   ctg acc gcg ctc gat agc gcc gcg ggc gac ggt gac ctg ggc ctc agt     1200
   Leu Thr Ala Leu Asp Ser Ala Ala Gly Asp Gly Asp Leu Gly Leu Ser
   385                 390                 395                 400
   ctg gtg cgt ggc gcc gag gcg att cgc gct ctt ccg gag gac gcg tgg     1248
   Leu Val Arg Gly Ala Glu Ala Ile Arg Ala Leu Pro Glu Asp Ala Trp
                   405                 410                 415
   acg agc ccc gcg cgt gcg ctg acg gcc att ggc aat gcc ttg cgg cgc     1296
   Thr Ser Pro Ala Arg Ala Leu Thr Ala Ile Gly Asn Ala Leu Arg Arg
               420                 425                 430
   agc att ggc ggc agc tcg ggg ccc ttc tac gcg acg gcg ctg ctg cgc     1344
   Ser Ile Gly Gly Ser Ser Gly Pro Phe Tyr Ala Thr Ala Leu Leu Arg
           435                 440                 445
   gcc gcg cgc agg ctg gcg gaa ggg ccc gtg gat gcc gcc gca tgg gcc 1392
   Ala Ala Arg Arg Leu Ala Glu Gly Pro Val Asp Ala Ala Ala Trp Ala
   450 455 460
   gag gcc ttc gac ctc gcc gtc acc gcc gta tcg gag ctg ggc ggc gcg 1440
   Glu Ala Phe Asp Leu Ala Val Thr Ala Val Ser Glu Leu Gly Gly Ala
   465 470 475 480
   cgg cct ggg gac cgc acc atg ctc gat gca ctc cgg ccc gcc gcc gac 1488
   Arg Pro Gly Asp Arg Thr Met Leu Asp Ala Leu Arg Pro Ala Ala Asp
   485 490 495
   gcc ttc gcg aag gcg gtg cgt tgc ggg cag ggg gct cgc gag gcc tgg 1536
   Ala Phe Ala Lys Ala Val Arg Cys Gly Gln Gly Ala Arg Glu Ala Trp
   500 505 510
   gcc tcg gcg gtg cac gcg gcc gag gcg gga gag gag gcg acg tcc cgg 1584
   Ala Ser Ala Val His Ala Ala Glu Ala Gly Glu Glu Ala Thr Ser Arg
   515 520 525
   atg cag ccg cgc ctg gga cgc gcc agc tac ctg ggt gcg cgc gcc gtc 1632
   Met Gln Pro Arg Leu Gly Arg Ala Ser Tyr Leu Gly Ala Arg Ala Val
   530 535 540
   ggt gtg ccg gac gcg ggc gcc gcg gcc gtg gtg gtg tgg atg aag gcg 1680
   Gly Val Pro Asp Ala Gly Ala Ala Ala Val Val Val Trp Met Lys Ala
   545 550 555 560
   ctc acg cct ggc atc ggc tga 1701
   Leu Thr Pro Gly Ile Gly
   565
<210> 59
   <211> 566
   <212> PRT
   <213> Myxococcus xanthus
<400> 59
   Met Lys Lys Leu Val Asn Ala Pro Arg Ala Val Val Arg Glu Met Leu
   1 5 10 15
   Glu Gly Leu Val Ser Leu Ala Pro Gly Gln Val Leu Leu Asp Gly Glu
   20 25 30
   Ser Val Val Leu Arg Ala Asp Thr Pro Ser Asp Val Arg Ala Arg Lys
   35 40 45
   Val Ala Val Ile Ser Gly Gly Gly Ser Gly His Glu Pro Ala His Ala
   50 55 60
   Gly Tyr Val Gly Ala Gly Met Leu Asp Ala Ala Val Ala Gly Asp Val
   65 70 75 80
   Phe Thr Ser Pro Ser Thr Asp Ala Val Leu Ala Ala Ile Arg Ala Val
   85 90 95
   Ala Gly Pro Ala Gly Ala Leu Leu Val Val Lys Asn Tyr Thr Gly Asp
   100 105 110
   Arg Leu Asn Phe Gly Leu Ala Ala Glu Leu Ala Arg Ala Glu Gly Ile
   115 120 125
   Pro Val Glu Thr Val Val Val Ala Asp Asp Val Ser Leu His Asp Thr
   130 135 140
   Val Glu Pro Ala Arg Arg Arg Gly Ile Ala Gly Thr Val Leu Val His
   145 150 155 160
   Lys Val Ala Gly Ala Ala Ala Glu Ala Gly Ala Ala Leu Gln Asp Val
   165 170 175
   Leu Arg Glu Ala Thr Ala Ala Ala Glu Val Leu Gly Thr Met Gly Val
                   180                 185                 190
   Ala Leu Gly Pro Cys Thr Val Pro Ala Ala Gly Lys Pro Gly Phe Thr
               195                 200                 205
   Leu Glu Glu Asp Glu Ile Glu Leu Gly Leu Gly Ile His Gly Glu Gln
       210                 215                 220
   Gly Val Arg Arg Val Pro Met Gln Thr Ala Asp Ser Leu Val Asp Thr
   225                 230                 235                 240
   Leu Leu Thr Thr Ile Val Glu Asp Arg Arg Ile Thr Ser Gly Asp Arg
                   245                 250                 255
   Val Val Leu Val Val Asn Gly Leu Gly Gly Thr Pro Pro Met Glu Leu
               260                 265                 270
   Ala Ile Val Ala Arg Arg Ala Leu Ala Ala Leu Arg Gln Gly Gly Ile
           275                 280                 285
   Arg Val Glu Arg Ala Trp Ser Gly Thr Phe Leu Ser Ala Leu Glu Met
       290                 295                 300
   Pro Gly Cys Ser Leu Thr Leu Leu Lys Val Asp Asp Ala Arg Leu Ala
   305                 310                 315                 320
   Arg Leu Asp Ala Ala Val Asp Ala Pro Ala Trp Pro Gly Ala Gly Arg
                   325                 330                 335
   Leu Pro Lys Glu Pro Gly Val Tyr Arg Pro Ser Ser Thr Ala Ser Pro
               340                 345                 350
   Ala Ser Leu Pro Ala Glu Ala Pro Gln Pro Gly Met Asp Arg Phe Arg
           355                 360                 365
   Lys Ala Ala Leu Arg Val Ala Asp Ala Phe Glu Gln Ser Glu Pro Arg
       370                 375                 380
   Leu Thr Ala Leu Asp Ser Ala Ala Gly Asp Gly Asp Leu Gly Leu Ser
   385                 390                 395                 400
   Leu Val Arg Gly Ala Glu Ala Ile Arg Ala Leu Pro Glu Asp Ala Trp
                   405                 410                 415
   Thr Ser Pro Ala Arg Ala Leu Thr Ala Ile Gly Asn Ala Leu Arg Arg
               420                 425                 430
   Ser Ile Gly Gly Ser Ser Gly Pro Phe Tyr Ala Thr Ala Leu Leu Arg
           435                 440                 445
   Ala Ala Arg Arg Leu Ala Glu Gly Pro Val Asp Ala Ala Ala Trp Ala
       450                 455                 460
   Glu Ala Phe Asp Leu Ala Val Thr Ala Val Ser Glu Leu Gly Gly Ala
   465                 470                 475                 480
   Arg Pro Gly Asp Arg Thr Met Leu Asp Ala Leu Arg Pro Ala Ala Asp
                   485                 490                 495
   Ala Phe Ala Lys Ala Val Arg Cys Gly Gln Gly Ala Arg Glu Ala Trp
               500                 505                 510
   Ala Ser Ala Val His Ala Ala Glu Ala Gly Glu Glu Ala Thr Ser Arg
           515                 520                 525
   Met Gln Pro Arg Leu Gly Arg Ala Ser Tyr Leu Gly Ala Arg Ala Val
       530                 535                 540
   Gly Val Pro Asp Ala Gly Ala Ala Ala Val Val Val Trp Met Lys Ala
   545                 550                 555                 560
   Leu Thr Pro Gly Ile Gly
                   565
<210> 60
   <211> 1701
   <212> DNA
   <213> Burkholderia sp. 383
<220>
   <221> CDS
   <222> (1).. (1701)
<400> 60
   atg aaa aag ctt gtc aac cgc ccg tcc gat gtc gtg cga gaa atg ctg       48
   Met Lys Lys Leu Val Asn Arg Pro Ser Asp Val Val Arg Glu Met Leu
   1               5                   10                  15
   gaa ggc atc gcg cgg cag tcg ccg cat gtc gcg atc ctc ggc gac gag       96
   Glu Gly Ile Ala Arg Gln Ser Pro His Val Ala Ile Leu Gly Asp Glu
               20                  25                  30
   cac gtg ctc gtc cgc cag ccg ctg ccc gag ccc gcg caa cgc ccc gtc      144
   His Val Leu Val Arg Gln Pro Leu Pro Glu Pro Ala Gln Arg Pro Val
           35                  40                  45
   gcc atc ctg tcc ggt ggc ggc agc ggc cac gag ccc gcg cac ggc ggc      192
   Ala Ile Leu Ser Gly Gly Gly Ser Gly His Glu Pro Ala His Gly Gly
       50                  55                  60
   tat gtc ggc gaa gga atg ctg agc gcg gcc gtc tgc ggc gaa gtg ttc      240
   Tyr Val Gly Glu Gly Met Leu Ser Ala Ala Val Cys Gly Glu Val Phe
   65                  70                  75                  80
   acg tcg ccg tcc aca gac gcc gtg ctc gcc gcg atc cgc gcg agc gcc      288
   Thr Ser Pro Ser Thr Asp Ala Val Leu Ala Ala Ile Arg Ala Ser Ala
                   85                  90                  95
   ggc ccg aac ggc gcc ttg ctg atc gtg aag aac tac acg ggc gac cgg      336
   Gly Pro Asn Gly Ala Leu Leu Ile Val Lys Asn Tyr Thr Gly Asp Arg
               100                 105                 110
   ctc aat ttc ggg ctc gcc gcc gaa ctc gca cgc gcc gaa ggc att ccg      384
   Leu Asn Phe Gly Leu Ala Ala Glu Leu Ala Arg Ala Glu Gly Ile Pro
           115                 120                 125
   gtc gag acg gtc atc gtc gcc gac gac gta tcg ctg cgc ggc cgc gtc      432
   Val Glu Thr Val Ile Val Ala Asp Asp Val Ser Leu Arg Gly Arg Val
       130                 135                 140
   gag cgc ggc cag cgg cgc ggg atc gcc ggt acc gtg ctg atc cac aag      480
   Glu Arg Gly Gln Arg Arg Gly Ile Ala Gly Thr Val Leu Ile His Lys
   145                 150                 155                 160
   ctc gcc ggc gcg gca gcc gcg cgc ggg ctg ccg ctc gcc cgc gtc gcg      528
   Leu Ala Gly Ala Ala Ala Ala Arg Gly Leu Pro Leu Ala Arg Val Ala
                   165                 170                 175
   gcc atc gcg cgc gac gcg gcg gcc gaa ctc ggc acg atg ggt gtc gca      576
   Ala Ile Ala Arg Asp Ala Ala Ala Glu Leu Gly Thr Met Gly Val Ala
               180                 185                 190
   ctc gac ggc tgc acg atc ccg ggc gcc gac aag tcg ggc ttc agc ctc      624
   Leu Asp Gly Cys Thr Ile Pro Gly Ala Asp Lys Ser Gly Phe Ser Leu
           195                 200                 205
   ggc gat cac gag atc gaa ctc ggc ctc ggc atc cat ggc gag aaa ggc      672
   Gly Asp His Glu Ile Glu Leu Gly Leu Gly Ile His Gly Glu Lys Gly
       210                 215                 220
   gtc gag cgc cgc gcg ccg ctg ccg gcc gat gcg ctt gtc gac acg ctg      720
   Val Glu Arg Arg Ala Pro Leu Pro Ala Asp Ala Leu Val Asp Thr Leu
   225                 230                 235                 240
   ctg tcg agc atc gcc gcc gat ctc gtg ctc gac cgc ggc gaa cgc gtt      768
   Leu Ser Ser Ile Ala Ala Asp Leu Val Leu Asp Arg Gly Glu Arg Val
                   245                 250                 255
   gcg ctg ttc gtc aac ggc ctc ggc gcg acg ccg gac atg gaa ctc gcg      816
   Ala Leu Phe Val Asn Gly Leu Gly Ala Thr Pro Asp Met Glu Leu Ala
                   260                 265                 270
   atc gtg ctg cgc gcc gcg cac gac aac ctg cac cgg cgc ggc atc gtc        864
   Ile Val Leu Arg Ala Ala His Asp Asn Leu His Arg Arg Gly Ile Val
               275                 280                 285
   gtc gcg cgt gcg tgg gcc ggc acg ttc ctg tcg gcg ctg aac atg ccc        912
   Val Ala Arg Ala Trp Ala Gly Thr Phe Leu Ser Ala Leu Asn Met Pro
       290                 295                 300
   ggc tgc tcg atc tcg gtg ctg cgg ctg aac gac gaa cgc gcg gtg ctg        960
   Gly Cys Ser Ile Ser Val Leu Arg Leu Asn Asp Glu Arg Ala Val Leu
   305                 310                 315                 320
   ctc gac gca ccg acg cag gcg cgt gca tgg cca ggc ggc ggc gcg gtg       1008
   Leu Asp Ala Pro Thr Gln Ala Arg Ala Trp Pro Gly Gly Gly Ala Val
                   325                 330                 335
   aat acg cag atc cgt gtg gcc tcg gcc gcc gtg cag gaa gcg ccg ttg       1056
   Asn Thr Gln Ile Arg Val Ala Ser Ala Ala Val Gln Glu Ala Pro Leu
                   340                 345                 350
   ccg ccg ctc gat gcg gcc ggc cgc gcg tgg gcc gcg cgc ctg caa ccg       1104
   Pro Pro Leu Asp Ala Ala Gly Arg Ala Trp Ala Ala Arg Leu Gln Pro
               355                 360                 365
   gca ttg cac gcg gtc gcg caa acg ctg atc gat cac gag cag acg ctg       1152
   Ala Leu His Ala Val Ala Gln Thr Leu Ile Asp His Glu Gln Thr Leu
       370                 375                 380
   acc gac ctc gat gcg gcg gcc ggc gac ggc gat ctc ggc gcg agc atg       1200
   Thr Asp Leu Asp Ala Ala Ala Gly Asp Gly Asp Leu Gly Ala Ser Met
   385                 390                 395                 400
   ctg cgc gcc gcg cag gcg atc ctc gca ctg ccg gaa agc gca tac ggc       1248
   Leu Arg Ala Ala Gln Ala Ile Leu Ala Leu Pro Glu Ser Ala Tyr Gly
                   405                 410                 415
   acg ccg gcc ggc gcg ctc tcg gcg ctc ggc gcc gcg ttg cgc cgc gcg       1296
   Thr Pro Ala Gly Ala Leu Ser Ala Leu Gly Ala Ala Leu Arg Arg Ala
                   420                 425                 430
   atc gcc ggc agc tcg ggg ccg ttc tat gcg acc gcg ctg ctg cgc gcg       1344
   Ile Ala Gly Ser Ser Gly Pro Phe Tyr Ala Thr Ala Leu Leu Arg Ala
               435                 440                 445
   tcg cgc cgg ctg gcc gat atc gcc gag ccg tcc gca cgc gac tgg gcc       1392
   Ser Arg Arg Leu Ala Asp Ile Ala Glu Pro Ser Ala Arg Asp Trp Ala
       450                 455                 460
   gcg gcg ttc cgc ggc gcg gtg gat tcg atc agc gaa ctg ggc ggc gcg       1440
   Ala Ala Phe Arg Gly Ala Val Asp Ser Ile Ser Glu Leu Gly Gly Ala
   465                 470                 475                 480
   cac gcc ggc gac cgg acc atg ctc gat gcg ctg gtc ccg gcc gtc gcg       1488
   His Ala Gly Asp Arg Thr Met Leu Asp Ala Leu Val Pro Ala Val Ala
                   485                 490                 495
   gca ttc gag cgg gcg ctc gac aac gat cgc gat ccc gcc ggc gca tgg       1536
   Ala Phe Glu Arg Ala Leu Asp Asn Asp Arg Asp Pro Ala Gly Ala Trp
                   500                 505                 510
   acg gcc gcg gtc gaa gcc gcc gag cac ggt gcg cag gaa acc gca cgc       1584
   Thr Ala Ala Val Glu Ala Ala Glu His Gly Ala Gln Glu Thr Ala Arg
               515                 520                 525
   atg acg cca cgc gcc ggg cgc gcg agc tat ctc ggc gaa cgt gcg atc       1632
   Met Thr Pro Arg Ala Gly Arg Ala Ser Tyr Leu Gly Glu Arg Ala Ile
       530                 535                 540
   ggc acg ccg gac ggc ggc gcg gtc gcg gtg tcg tat tgg ctg cgt gcg       1680
   Gly Thr Pro Asp Gly Gly Ala Val Ala Val Ser Tyr Trp Leu Arg Ala
   545 550 555 560
   ttg cag gca cac atc ggg tga 1701
   Leu Gln Ala His Ile Gly
   565
<210> 61
   <211> 566
   <212> PRT
   <213> Burkholderia sp. 383
<400> 61
   Met Lys Lys Leu Val Asn Arg Pro Ser Asp Val Val Arg Glu Met Leu
   1 5 10 15
   Glu Gly Ile Ala Arg Gln Ser Pro His Val Ala Ile Leu Gly Asp Glu
   20 25 30
   His Val Leu Val Arg Gln Pro Leu Pro Glu Pro Ala Gln Arg Pro Val
   35 40 45
   Ala Ile Leu Ser Gly Gly Gly Ser Gly His Glu Pro Ala His Gly Gly
   50 55 60
   Tyr Val Gly Glu Gly Met Leu Ser Ala Ala Val Cys Gly Glu Val Phe
   65 70 75 80
   Thr Ser Pro Ser Thr Asp Ala Val Leu Ala Ala Ile Arg Ala Ser Ala
   85 90 95
   Gly Pro Asn Gly Ala Leu Leu Ile Val Lys Asn Tyr Thr Gly Asp Arg
   100 105 110
   Leu Asn Phe Gly Leu Ala Ala Glu Leu Ala Arg Ala Glu Gly Ile Pro
   115 120 125
   Val Glu Thr Val Ile Val Ala Asp Asp Val Ser Leu Arg Gly Arg Val
   130 135 140
   Glu Arg Gly Gln Arg Arg Gly Ile Ala Gly Thr Val Leu Ile His Lys
   145 150 155 160
   Leu Ala Gly Ala Ala Ala Ala Arg Gly Leu Pro Leu Ala Arg Val Ala
   165 170 175
   Ala Ile Ala Arg Asp Ala Ala Ala Glu Leu Gly Thr Met Gly Val Ala
   180 185 190
   Leu Asp Gly Cys Thr Ile Pro Gly Ala Asp Lys Ser Gly Phe Ser Leu
   195 200 205
   Gly Asp His Glu Ile Glu Leu Gly Leu Gly Ile His Gly Glu Lys Gly
   210 215 220
   Val Glu Arg Arg Ala Pro Leu Pro Ala Asp Ala Leu Val Asp Thr Leu
   225 230 235 240
   Leu Ser Ser Ile Ala Ala Asp Leu Val Leu Asp Arg Gly Glu Arg Val
   245 250 255
   Ala Leu Phe Val Asn Gly Leu Gly Ala Thr Pro Asp Met Glu Leu Ala
   260 265 270
   Ile Val Leu Arg Ala Ala His Asp Asn Leu His Arg Arg Gly Ile Val
   275 280 285
   Val Ala Arg Ala Trp Ala Gly Thr Phe Leu Ser Ala Leu Asn Met Pro
   290 295 300
   Gly Cys Ser Ile Ser Val Leu Arg Leu Asn Asp Glu Arg Ala Val Leu
   305 310 315 320
   Leu Asp Ala Pro Thr Gln Ala Arg Ala Trp Pro Gly Gly Gly Ala Val
   325 330 335
   Asn Thr Gln Ile Arg Val Ala Ser Ala Ala Val Gln Glu Ala Pro Leu
                   340                 345                 350
   Pro Pro Leu Asp Ala Ala Gly Arg Ala Trp Ala Ala Arg Leu Gln Pro
           355                 360                 365
   Ala Leu His Ala Val Ala Gln Thr Leu Ile Asp His Glu Gln Thr Leu
       370                 375                 380
   Thr Asp Leu Asp Ala Ala Ala Gly Asp Gly Asp Leu Gly Ala Ser Met
   385                 390                 395                 400
   Leu Arg Ala Ala Gln Ala Ile Leu Ala Leu Pro Glu Ser Ala Tyr Gly
                   405                 410                 415
   Thr Pro Ala Gly Ala Leu Ser Ala Leu Gly Ala Ala Leu Arg Arg Ala
               420                 425                 430
   Ile Ala Gly Ser Ser Gly Pro Phe Tyr Ala Thr Ala Leu Leu Arg Ala
           435                 440                 445
   Ser Arg Arg Leu Ala Asp Ile Ala Glu Pro Ser Ala Arg Asp Trp Ala
       450                 455                 460
   Ala Ala Phe Arg Gly Ala Val Asp Ser Ile Ser Glu Leu Gly Gly Ala
   465                 470                 475                 480
   His Ala Gly Asp Arg Thr Met Leu Asp Ala Leu Val Pro Ala Val Ala
                   485                 490                 495
   Ala Phe Glu Arg Ala Leu Asp Asn Asp Arg Asp Pro Ala Gly Ala Trp
               500                 505                 510
   Thr Ala Ala Val Glu Ala Ala Glu His Gly Ala Gln Glu Thr Ala Arg
           515                 520                 525
   Met Thr Pro Arg Ala Gly Arg Ala Ser Tyr Leu Gly Glu Arg Ala Ile
       530                 535                 540
   Gly Thr Pro Asp Gly Gly Ala Val Ala Val Ser Tyr Trp Leu Arg Ala
   545                 550                 555                 560
   Leu Gln Ala His Ile Gly
                   565
<210> 62
   <211> 1704
   <212> DNA
   <213> Burkholderia thailandensis E264
<220>
   <221> CDS
   <222> (1).. (1704)
<400> 62
   atg aag aag ctc gtc aac cac ccg tcc gac gtc gtg cgc gaa atg ctg      48
   Met Lys Lys Leu Val Asn His Pro Ser Asp Val Val Arg Glu Met Leu
   1               5                   10                  15
   gag ggc atc gcg cgg cag tcg ccg cat gtc gcg atg ctc ggc gac gaa      96
   Glu Gly Ile Ala Arg Gln Ser Pro His Val Ala Met Leu Gly Asp Glu
               20                  25                  30
   cac gtg ctg atc cgg cgc ccc ttg ccg gag ccg gcg cgg cgt gcg gtc     144
   His Val Leu Ile Arg Arg Pro Leu Pro Glu Pro Ala Arg Arg Ala Val
           35                  40                  45
   gcg atc atc tcc ggc ggc ggc agc ggc cac gag ccg gcg cac ggc ggc     192
   Ala Ile Ile Ser Gly Gly Gly Ser Gly His Glu Pro Ala His Gly Gly
       50                  55                  60
   tac gtc ggc gcg ggg atg ctg agc gcg gcc gtg tgc ggc gag gtc ttc     240
   Tyr Val Gly Ala Gly Met Leu Ser Ala Ala Val Cys Gly Glu Val Phe
   65                  70                  75                  80
   acg tcg ccg ccc gcc gat gcg gtg ctc gcc gcg att cgc gcg acc gcg      288
   Thr Ser Pro Pro Ala Asp Ala Val Leu Ala Ala Ile Arg Ala Thr Ala
                   85                  90                  95
   ggc cag aac ggc gcg ctc ctc atc gtg aag aac tac acg ggc gat cgc      336
   Gly Gln Asn Gly Ala Leu Leu Ile Val Lys Asn Tyr Thr Gly Asp Arg
               100                 105                 110
   ctc aat ttc ggg ctc gcg gcc gag ctc gcg cgc gcg cag ggc att ccg      384
   Leu Asn Phe Gly Leu Ala Ala Glu Leu Ala Arg Ala Gln Gly Ile Pro
           115                 120                 125
   gtc gag atc gtc gtc gtc gcg gac gac gtg tcg ctg cgc gaa ctc acc      432
   Val Glu Ile Val Val Val Ala Asp Asp Val Ser Leu Arg Glu Leu Thr
       130                 135                 140
   gag cgc ggg cgc cgc cgc ggc atc gcc ggc acc gtg ctc gtg cac aag      480
   Glu Arg Gly Arg Arg Arg Gly Ile Ala Gly Thr Val Leu Val His Lys
   145                 150                 155                 160
   ctc gcc ggc gcg gcc gcc gag cgc ggc ctc gcg ctg cgg gag gtg gcc      528
   Leu Ala Gly Ala Ala Ala Glu Arg Gly Leu Ala Leu Arg Glu Val Ala
                   165                 170                 175
   gcc gtc gcg agc gag gcg gcg gcg aat ctc ggc acg atg ggc gtc gca      576
   Ala Val Ala Ser Glu Ala Ala Ala Asn Leu Gly Thr Met Gly Val Ala
               180                 185                 190
   ctc gac ggc tgc acg att ccg ggc gcc ggg caa tcg ggc ttc cgc ctc      624
   Leu Asp Gly Cys Thr Ile Pro Gly Ala Gly Gln Ser Gly Phe Arg Leu
           195                 200                 205
   gcc gat cac gag atc gag ctc gga ttg ggc att cac ggc gaa aag ggc      672
   Ala Asp His Glu Ile Glu Leu Gly Leu Gly Ile His Gly Glu Lys Gly
       210                 215                 220
   gtg cag cgc acg gcg ccg atg ccg gcc gac gcg ctg tcg gaa acg ctc      720
   Val Gln Arg Thr Ala Pro Met Pro Ala Asp Ala Leu Ser Glu Thr Leu
   225                 230                 235                 240
   gtg gcg acg atc gtc gac gat cag gcg atc gcg cgc ggc gat cgg gtc      768
   Val Ala Thr Ile Val Asp Asp Gln Ala Ile Ala Arg Gly Asp Arg Val
                   245                 250                 255
   gcg ctt ctg gtg aac ggg ctc ggc gcg acg ccg gac atg gag ctc ggc      816
   Ala Leu Leu Val Asn Gly Leu Gly Ala Thr Pro Asp Met Glu Leu Gly
               260                 265                 270
   atc gtg ctg cgc gcg gcg tac gac agc ctg agc cgg cgt ggc gtc gag      864
   Ile Val Leu Arg Ala Ala Tyr Asp Ser Leu Ser Arg Arg Gly Val Glu
           275                 280                 285
   gtg gcg cgc gcg tgg gcg ggc acg ttc ctg tcc gcg ctc gac atg ccc      912
   Val Ala Arg Ala Trp Ala Gly Thr Phe Leu Ser Ala Leu Asp Met Pro
       290                 295                 300
   ggc tgc tcg att tcg ctg ctc aag ctg aac gat cgc atg ctc gaa ctg      960
   Gly Cys Ser Ile Ser Leu Leu Lys Leu Asn Asp Arg Met Leu Glu Leu
   305                 310                 315                 320
   ctc gac gcg ccg acg caa gcg cga gcg tgg ccg ggc ggc ggc gcg gtg     1008
   Leu Asp Ala Pro Thr Gln Ala Arg Ala Trp Pro Gly Gly Gly Ala Val
                   325                 330                 335
   aac cgg gac att cgc gtg gcc gcc gcc ggg acc ggc gca gga gac gga     1056
   Asn Arg Asp Ile Arg Val Ala Ala Ala Gly Thr Gly Ala Gly Asp Gly
               340                 345                 350
   cag ccg gaa tgg gcg acg gcc ggc gcg gcg ggt tcc gac gga ctg cgg     1104
   Gln Pro Glu Trp Ala Thr Ala Gly Ala Ala Gly Ser Asp Gly Leu Arg
           355                 360                 365
   ccc gcg ctg cat gcg gtt gcc gcc gcg ctg atc gac agc gag ccc gtg      1152
   Pro Ala Leu His Ala Val Ala Ala Ala Leu Ile Asp Ser Glu Pro Val
       370                 375                 380
   ttg acc gag ctc gat tcc gtc gcc ggc gac ggc gat ctg ggc gcg agc      1200
   Leu Thr Glu Leu Asp Ser Val Ala Gly Asp Gly Asp Leu Gly Ala Ser
   385                 390                 395                 400
   atg cgc cgc gcg gcg aac gcg atg ctc gcg ctg ccc gcc gat gcg tat      1248
   Met Arg Arg Ala Ala Asn Ala Met Leu Ala Leu Pro Ala Asp Ala Tyr
                   405                 410                 415
   cga ggg ccg gcg aac ctg ctc gcc gcg ctg ggc atg gcg ctg cgc cgg      1296
   Arg Gly Pro Ala Asn Leu Leu Ala Ala Leu Gly Met Ala Leu Arg Arg
               420                 425                 430
   gcg atc gcg ggc agc tcc ggg ccg ttt tac gca acg gcg ctg gtg cgc      1344
   Ala Ile Ala Gly Ser Ser Gly Pro Phe Tyr Ala Thr Ala Leu Val Arg
           435                 440                 445
   gcg ggc cgc cgg ctc gcc gat gca tcg gcg ccg acc gcg cgc gac tgg      1392
   Ala Gly Arg Arg Leu Ala Asp Ala Ser Ala Pro Thr Ala Arg Asp Trp
       450                 455                 460
   gcg agc gcg ttc cgg agc ggt gtc gac gcg atc ggc gat ctg ggc ggc      1440
   Ala Ser Ala Phe Arg Ser Gly Val Asp Ala Ile Gly Asp Leu Gly Gly
   465                 470                 475                 480
   gcg aag ccc gga gac cgc acg atg ctc gac gcc ttg gtg ccc gcc gtc      1488
   Ala Lys Pro Gly Asp Arg Thr Met Leu Asp Ala Leu Val Pro Ala Val
                   485                 490                 495
   gat gcg ttc gag cac gcg ctg tcg gcg ggc ggg agc gcg agc gat gcg      1536
   Asp Ala Phe Glu His Ala Leu Ser Ala Gly Gly Ser Ala Ser Asp Ala
               500                 505                 510
   tgg gcg gcg gcg gtg cgc gcc gcg gag gca ggc gcg gcg aaa acc gcg      1584
   Trp Ala Ala Ala Val Arg Ala Ala Glu Ala Gly Ala Ala Lys Thr Ala
           515                 520                 525
   ggc atg acg ccg cgc gcg ggg cgc gcg agc tat ctg ggc gag cgc gcc      1632
   Gly Met Thr Pro Arg Ala Gly Arg Ala Ser Tyr Leu Gly Glu Arg Ala
       530                 535                 540
   gtg ggc tcg ccc gac ggc ggc gcg gtg gcc gtg gcg tgc tgg atg cgc      1680
   Val Gly Ser Pro Asp Gly Gly Ala Val Ala Val Ala Cys Trp Met Arg
   545                 550                 555                 560
   gcg ttg cag ccg cac gtc gcg tag                                      1704
   Ala Leu Gln Pro His Val Ala
                   565
<210> 63
   <211> 567
   <212> PRT
   <213> Burkholderia thailandensis E264
<400> 63
   Met Lys Lys Leu Val Asn His Pro Ser Asp Val Val Arg Glu Met Leu
   1               5                   10                  15
   Glu Gly Ile Ala Arg Gln Ser Pro His Val Ala Met Leu Gly Asp Glu
               20                  25                  30
   His Val Leu Ile Arg Arg Pro Leu Pro Glu Pro Ala Arg Arg Ala Val
           35                  40                  45
   Ala Ile Ile Ser Gly Gly Gly Ser Gly His Glu Pro Ala His Gly Gly
       50                  55                  60
   Tyr Val Gly Ala Gly Met Leu Ser Ala Ala Val Cys Gly Glu Val Phe
   65 70 75 80
   Thr Ser Pro Pro Ala Asp Ala Val Leu Ala Ala Ile Arg Ala Thr Ala
   85 90 95
   Gly Gln Asn Gly Ala Leu Leu Ile Val Lys Asn Tyr Thr Gly Asp Arg
   100 105 110
   Leu Asn Phe Gly Leu Ala Ala Glu Leu Ala Arg Ala Gln Gly Ile Pro
   115 120 125
   Val Glu Ile Val Val Val Ala Asp Asp Val Ser Leu Arg Glu Leu Thr
   130 135 140
   Glu Arg Gly Arg Arg Arg Gly Ile Ala Gly Thr Val Leu Val His Lys
   145 150 155 160
   Leu Ala Gly Ala Ala Ala Glu Arg Gly Leu Ala Leu Arg Glu Val Ala
   165 170 175
   Ala Val Ala Ser Glu Ala Ala Ala Asn Leu Gly Thr Met Gly Val Ala
   180 185 190
   Leu Asp Gly Cys Thr Ile Pro Gly Ala Gly Gln Ser Gly Phe Arg Leu
   195 200 205
   Ala Asp His Glu Ile Glu Leu Gly Leu Gly Ile His Gly Glu Lys Gly
   210 215 220
   Val Gln Arg Thr Ala Pro Met Pro Ala Asp Ala Leu Ser Glu Thr Leu
   225 230 235 240
   Val Ala Thr Ile Val Asp Asp Gln Ala Ile Ala Arg Gly Asp Arg Val
   245 250 255
   Ala Leu Leu Val Asn Gly Leu Gly Ala Thr Pro Asp Met Glu Leu Gly
   260 265 270
   Ile Val Leu Arg Ala Ala Tyr Asp Ser Leu Ser Arg Arg Gly Val Glu
   275 280 285
   Val Ala Arg Ala Trp Ala Gly Thr Phe Leu Ser Ala Leu Asp Met Pro
   290 295 300
   Gly Cys Ser Ile Ser Leu Leu Lys Leu Asn Asp Arg Met Leu Glu Leu
   305 310 315 320
   Leu Asp Ala Pro Thr Gln Ala Arg Ala Trp Pro Gly Gly Gly Ala Val
   325 330 335
   Asn Arg Asp Ile Arg Val Ala Ala Ala Gly Thr Gly Ala Gly Asp Gly
   340 345 350
   Gln Pro Glu Trp Ala Thr Ala Gly Ala Ala Gly Ser Asp Gly Leu Arg
   355 360 365
   Pro Ala Leu His Ala Val Ala Ala Ala Leu Ile Asp Ser Glu Pro Val
   370 375 380
   Leu Thr Glu Leu Asp Ser Val Ala Gly Asp Gly Asp Leu Gly Ala Ser
   385 390 395 400
   Met Arg Arg Ala Ala Asn Ala Met Leu Ala Leu Pro Ala Asp Ala Tyr
   405 410 415
   Arg Gly Pro Ala Asn Leu Leu Ala Ala Leu Gly Met Ala Leu Arg Arg
   420 425 430
   Ala Ile Ala Gly Ser Ser Gly Pro Phe Tyr Ala Thr Ala Leu Val Arg
   435 440 445
   Ala Gly Arg Arg Leu Ala Asp Ala Ser Ala Pro Thr Ala Arg Asp Trp
   450 455 460
   Ala Ser Ala Phe Arg Ser Gly Val Asp Ala Ile Gly Asp Leu Gly Gly
   465 470 475 480
   Ala Lys Pro Gly Asp Arg Thr Met Leu Asp Ala Leu Val Pro Ala Val
   485 490 495
   Asp Ala Phe Glu His Ala Leu Ser Ala Gly Gly Ser Ala Ser Asp Ala
                       500                 505                 510
   Trp Ala Ala Ala Val Arg Ala Ala Glu Ala Gly Ala Ala Lys Thr Ala
               515                 520                 525
   Gly Met Thr Pro Arg Ala Gly Arg Ala Ser Tyr Leu Gly Glu Arg Ala
           530                 535                 540
   Val Gly Ser Pro Asp Gly Gly Ala Val Ala Val Ala Cys Trp Met Arg
   545                 550                 555                 560
   Ala Leu Gln Pro His Val Ala
                   565
<210> 64
   <211> 1851
   <212> DNA
   <213> Burkholderia multivorans
<220>
   <221> CDS
   <222> (1).. (1851)
<400> 64
   atg tat cgt ggc acc gaa gcg cgc acc ggc gag atc cat ggc act gct      48
   Met Tyr Arg Gly Thr Glu Ala Arg Thr Gly Glu Ile His Gly Thr Ala
   1               5                   10                  15
   cca cgc cac cgc cgc cac ggc gcg cgc att ccc gca acc cga cag ccc      96
   Pro Arg His Arg Arg His Gly Ala Arg Ile Pro Ala Thr Arg Gln Pro
               20                  25                  30
   gag gcg ccg gat cgg ttc ggc ccg cgg gct ttc atc tgc ccg gaa acc     144
   Glu Ala Pro Asp Arg Phe Gly Pro Arg Ala Phe Ile Cys Pro Glu Thr
           35                  40                  45
   gtc gcc atg aaa aaa ctc gtg aac cgt ccg tcc gac gtc gtg cgt gaa     192
   Val Ala Met Lys Lys Leu Val Asn Arg Pro Ser Asp Val Val Arg Glu
       50                  55                  60
   atg ctc gaa ggc att gcg cga cag tcg ccg cat ctc gcg atg ctc ggc     240
   Met Leu Glu Gly Ile Ala Arg Gln Ser Pro His Leu Ala Met Leu Gly
   65                  70                  75                  80
   gac gag cac gtg ctc gtc cgc cgc ccg ctg ccc gaa ccg tcg cag cgc     288
   Asp Glu His Val Leu Val Arg Arg Pro Leu Pro Glu Pro Ser Gln Arg
                   85                  90                  95
   acg gtt gcg gtg ctg tcg ggc ggc ggc agc ggg cac gag cct gcg cac     336
   Thr Val Ala Val Leu Ser Gly Gly Gly Ser Gly His Glu Pro Ala His
               100                 105                 110
   ggc ggc tat gtc ggc gac gga atg ctc agt gcg gcc gtg tgc ggc gaa     384
   Gly Gly Tyr Val Gly Asp Gly Met Leu Ser Ala Ala Val Cys Gly Glu
           115                 120                 125
   gtg ttc acg tcg ccg tcc acc gac gcg gtg ctc gcc gcg atc cgc gcg     432
   Val Phe Thr Ser Pro Ser Thr Asp Ala Val Leu Ala Ala Ile Arg Ala
       130                 135                 140
   acg gcc ggc ccg aac ggc gcg ctg ctc gtc gtg aag aac tac acc ggc     480
   Thr Ala Gly Pro Asn Gly Ala Leu Leu Val Val Lys Asn Tyr Thr Gly
   145                 150                 155                 160
   gac cgg ctc aac ttc ggt ctg gca gcc gaa ctc gcg cgc gca gaa ggc     528
   Asp Arg Leu Asn Phe Gly Leu Ala Ala Glu Leu Ala Arg Ala Glu Gly
                   165                 170                 175
   att ccg gtc gag acc gtg atc gtc gcg gac gac gta tcg ctg cgc ggc     576
   Ile Pro Val Glu Thr Val Ile Val Ala Asp Asp Val Ser Leu Arg Gly
                   180                 185                 190
   cgc gtc gag cgc gga cag cgg cgc ggc atc gcg ggc acc gtg ctg atc       624
   Arg Val Glu Arg Gly Gln Arg Arg Gly Ile Ala Gly Thr Val Leu Ile
           195                 200                 205
   cac aag ctc gcg ggc gcg gcg gcc gcg cgc ggg ctg tcg ctg ccg cgc       672
   His Lys Leu Ala Gly Ala Ala Ala Ala Arg Gly Leu Ser Leu Pro Arg
       210                 215                 220
   gtc gcg gcg atc gcg cgc gat gcg gcc gcc gat ctc ggc acg atg ggc       720
   Val Ala Ala Ile Ala Arg Asp Ala Ala Ala Asp Leu Gly Thr Met Gly
   225                 230                 235                 240
   gtc gcg ctc gac ggc tgt acg ctg ccg ggc gcc gac cag tcc gga ttc       768
   Val Ala Leu Asp Gly Cys Thr Leu Pro Gly Ala Asp Gln Ser Gly Phe
                   245                 250                 255
   agc ctc gcc gac gac gaa atc gag ctc ggt ctc ggc att cat ggc gaa       816
   Ser Leu Ala Asp Asp Glu Ile Glu Leu Gly Leu Gly Ile His Gly Glu
               260                 265                 270
   aaa ggc gtc gaa cgc acg gcg ccg ctg ccg gcc gac gcg ctc gcc gat       864
   Lys Gly Val Glu Arg Thr Ala Pro Leu Pro Ala Asp Ala Leu Ala Asp
           275                 280                 285
   acg ctg ctg tcc ggg atc gtc gcc gac ctc gtg ctc gat cgc ggc gaa       912
   Thr Leu Leu Ser Gly Ile Val Ala Asp Leu Val Leu Asp Arg Gly Glu
       290                 295                 300
   cgc gtc gcg ctg ctc gtc aac ggt ctc ggc gcg acg ccc gac atg gag       960
   Arg Val Ala Leu Leu Val Asn Gly Leu Gly Ala Thr Pro Asp Met Glu
   305                 310                 315                 320
   ctt gcg atc gtg ctg cgc gcc gcc tac gag aac ctg agc cgt cgc ggc      1008
   Leu Ala Ile Val Leu Arg Ala Ala Tyr Glu Asn Leu Ser Arg Arg Gly
                   325                 330                 335
   atc gcg gtc gag cgc gcg tgg gcc ggt acg ttc ctg tcg gcg ctg aac      1056
   Ile Ala Val Glu Arg Ala Trp Ala Gly Thr Phe Leu Ser Ala Leu Asn
               340                 345                 350
   atg ccc ggc tgt tcg atc tcg gtg ctg cgg ctc gac gac gag cgg gcg      1104
   Met Pro Gly Cys Ser Ile Ser Val Leu Arg Leu Asp Asp Glu Arg Ala
           355                 360                 365
   gcg ctg ctc gac gca ccg acg caa gcg cgc gcg tgg ccc ggc ggc ggc      1152
   Ala Leu Leu Asp Ala Pro Thr Gln Ala Arg Ala Trp Pro Gly Gly Gly
       370                 375                 380
   gcc gtc aac gca cag atc cgc atc gcc gcc gcg gcg ccg cag gaa ccg      1200
   Ala Val Asn Ala Gln Ile Arg Ile Ala Ala Ala Ala Pro Gln Glu Pro
   385                 390                 395                 400
   tcg ccg ccg ccg ctc gac gcg gcg ggc cgc gtg tgg gcc gag cgg ctg      1248
   Ser Pro Pro Pro Leu Asp Ala Ala Gly Arg Val Trp Ala Glu Arg Leu
                   405                 410                 415
   cgc ccg gcg ctg cac gcg gtc gcg cac acg ctg atc gac cac gaa gca      1296
   Arg Pro Ala Leu His Ala Val Ala His Thr Leu Ile Asp His Glu Ala
               420                 425                 430
   acg ctg acc gag ctc gat gcc gcc gcc ggc gac ggc gac ctc ggc gcg      1344
   Thr Leu Thr Glu Leu Asp Ala Ala Ala Gly Asp Gly Asp Leu Gly Ala
           435                 440                 445
   agc atg cgt cgt gcc gcg gac gcg atg ctc gcg ttg ccg gaa acc gcg      1392
   Ser Met Arg Arg Ala Ala Asp Ala Met Leu Ala Leu Pro Glu Thr Ala
       450                 455                 460
   tat gcg acg ccg gcc ggt gcg ctc gcc gcg ctc ggc gcc gcg ctg cgc      1440
   Tyr Ala Thr Pro Ala Gly Ala Leu Ala Ala Leu Gly Ala Ala Leu Arg
   465                 470                 475                 480
   cgt gcg atc gcg ggc agc tcg ggc ccc ttc tac gcg acc gcg ctg ctg      1488
   Arg Ala Ile Ala Gly Ser Ser Gly Pro Phe Tyr Ala Thr Ala Leu Leu
                   485                 490                 495
   cgc gca tcg cgg cgc ctg gcc ggc ctc gcg cag ccg tcg gca cgc gac      1536
   Arg Ala Ser Arg Arg Leu Ala Gly Leu Ala Gln Pro Ser Ala Arg Asp
               500                 505                 510
   tgg gcc gcg gca ttg cgc agc gca gcc gat gcg atc gcc gaa ctc ggc      1584
   Trp Ala Ala Ala Leu Arg Ser Ala Ala Asp Ala Ile Ala Glu Leu Gly
           515                 520                 525
   ggc gcc cgt gcc ggt gac aga acg atg ctc gac gcg ctg gtg ccg gcc      1632
   Gly Ala Arg Ala Gly Asp Arg Thr Met Leu Asp Ala Leu Val Pro Ala
       530                 535                 540
   gcc gcc gcg ttc gac cgt gca ctc gac gac ggt cgc gac agc gcc ggc      1680
   Ala Ala Ala Phe Asp Arg Ala Leu Asp Asp Gly Arg Asp Ser Ala Gly
   545                 550                 555                 560
   gcg tgg gcg gcc gcc gtc gac gcc gcc gag cgc ggt gcg cag gac acc      1728
   Ala Trp Ala Ala Ala Val Asp Ala Ala Glu Arg Gly Ala Gln Asp Thr
                   565                 570                 575
   gcg cgc atg acg ccg cgc gcg ggc cgt gcg agc tat ctc ggc gaa cgc      1776
   Ala Arg Met Thr Pro Arg Ala Gly Arg Ala Ser Tyr Leu Gly Glu Arg
               580                 585                 590
   gcg atc ggc acg ccc gac ggc ggc gcc atc gcc gtc gcg tac tgg ctg      1824
   Ala Ile Gly Thr Pro Asp Gly Gly Ala Ile Ala Val Ala Tyr Trp Leu
           595                 600                 605
   cgc gca ctg ctg ccg cac gtc cga taa                                  1851
   Arg Ala Leu Leu Pro His Val Arg
       610                 615
<210> 65
   <211> 616
   <212> PRT
   <213> Burkholderia multivorans
<400> 65
   Met Tyr Arg Gly Thr Glu Ala Arg Thr Gly Glu Ile His Gly Thr Ala
   1               5                   10                  15
   Pro Arg His Arg Arg His Gly Ala Arg Ile Pro Ala Thr Arg Gln Pro
               20                  25                  30
   Glu Ala Pro Asp Arg Phe Gly Pro Arg Ala Phe Ile Cys Pro Glu Thr
           35                  40                  45
   Val Ala Met Lys Lys Leu Val Asn Arg Pro Ser Asp Val Val Arg Glu
       50                  55                  60
   Met Leu Glu Gly Ile Ala Arg Gln Ser Pro His Leu Ala Met Leu Gly
   65                  70                  75                  80
   Asp Glu His Val Leu Val Arg Arg Pro Leu Pro Glu Pro Ser Gln Arg
                   85                  90                  95
   Thr Val Ala Val Leu Ser Gly Gly Gly Ser Gly His Glu Pro Ala His
               100                 105                 110
   Gly Gly Tyr Val Gly Asp Gly Met Leu Ser Ala Ala Val Cys Gly Glu
           115                 120                 125
   Val Phe Thr Ser Pro Ser Thr Asp Ala Val Leu Ala Ala Ile Arg Ala
       130                 135                 140
   Thr Ala Gly Pro Asn Gly Ala Leu Leu Val Val Lys Asn Tyr Thr Gly
   145                 150                 155                 160
   Asp Arg Leu Asn Phe Gly Leu Ala Ala Glu Leu Ala Arg Ala Glu Gly
                   165                 170                 175
   Ile Pro Val Glu Thr Val Ile Val Ala Asp Asp Val Ser Leu Arg Gly
               180                 185                 190
   Arg Val Glu Arg Gly Gln Arg Arg Gly Ile Ala Gly Thr Val Leu Ile
           195                 200                 205
   His Lys Leu Ala Gly Ala Ala Ala Ala Arg Gly Leu Ser Leu Pro Arg
       210                 215                 220
   Val Ala Ala Ile Ala Arg Asp Ala Ala Ala Asp Leu Gly Thr Met Gly
   225                 230                 235                 240
   Val Ala Leu Asp Gly Cys Thr Leu Pro Gly Ala Asp Gln Ser Gly Phe
                   245                 250                 255
   Ser Leu Ala Asp Asp Glu Ile Glu Leu Gly Leu Gly Ile His Gly Glu
               260                 265                 270
   Lys Gly Val Glu Arg Thr Ala Pro Leu Pro Ala Asp Ala Leu Ala Asp
           275                 280                 285
   Thr Leu Leu Ser Gly Ile Val Ala Asp Leu Val Leu Asp Arg Gly Glu
       290                 295                 300
   Arg Val Ala Leu Leu Val Asn Gly Leu Gly Ala Thr Pro Asp Met Glu
   305                 310                 315                 320
   Leu Ala Ile Val Leu Arg Ala Ala Tyr Glu Asn Leu Ser Arg Arg Gly
                   325                 330                 335
   Ile Ala Val Glu Arg Ala Trp Ala Gly Thr Phe Leu Ser Ala Leu Asn
               340                 345                 350
   Met Pro Gly Cys Ser Ile Ser Val Leu Arg Leu Asp Asp Glu Arg Ala
           355                 360                 365
   Ala Leu Leu Asp Ala Pro Thr Gln Ala Arg Ala Trp Pro Gly Gly Gly
       370                 375                 380
   Ala Val Asn Ala Gln Ile Arg Ile Ala Ala Ala Ala Pro Gln Glu Pro
   385                 390                 395                 400
   Ser Pro Pro Pro Leu Asp Ala Ala Gly Arg Val Trp Ala Glu Arg Leu
                   405                 410                 415
   Arg Pro Ala Leu His Ala Val Ala His Thr Leu Ile Asp His Glu Ala
               420                 425                 430
   Thr Leu Thr Glu Leu Asp Ala Ala Ala Gly Asp Gly Asp Leu Gly Ala
           435                 440                 445
   Ser Met Arg Arg Ala Ala Asp Ala Met Leu Ala Leu Pro Glu Thr Ala
       450                 455                 460
   Tyr Ala Thr Pro Ala Gly Ala Leu Ala Ala Leu Gly Ala Ala Leu Arg
   465                 470                 475                 480
   Arg Ala Ile Ala Gly Ser Ser Gly Pro Phe Tyr Ala Thr Ala Leu Leu
                   485                 490                 495
   Arg Ala Ser Arg Arg Leu Ala Gly Leu Ala Gln Pro Ser Ala Arg Asp
               500                 505                 510
   Trp Ala Ala Ala Leu Arg Ser Ala Ala Asp Ala Ile Ala Glu Leu Gly
           515                 520                 525
   Gly Ala Arg Ala Gly Asp Arg Thr Met Leu Asp Ala Leu Val Pro Ala
       530                 535                 540
   Ala Ala Ala Phe Asp Arg Ala Leu Asp Asp Gly Arg Asp Ser Ala Gly
   545                 550                 555                 560
   Ala Trp Ala Ala Ala Val Asp Ala Ala Glu Arg Gly Ala Gln Asp Thr
                   565                 570                 575
   Ala Arg Met Thr Pro Arg Ala Gly Arg Ala Ser Tyr Leu Gly Glu Arg
               580                 585                 590
   Ala Ile Gly Thr Pro Asp Gly Gly Ala Ile Ala Val Ala Tyr Trp Leu
   595 600 605
   Arg Ala Leu Leu Pro His Val Arg
   610 615
<210> 66
   <211> 1920
   <212> DNA
   <213> Escherichia coli
<220>
   <221> CDS
   <222> (1).. (1920)
<400> 66
   atg agt ggc gct ttt aac aac gat ggt cgg ggc ata tct ccc tta att 48
   Met Ser Gly Ala Phe Asn Asn Asp Gly Arg Gly Ile Ser Pro Leu Ile
   1 5 10 15
   gca acc tcc tgg gag cga tgc aat aag ctg atg aaa cgg gag aca tgg 96
   Ala Thr Ser Trp Glu Arg Cys Asn Lys Leu Met Lys Arg Glu Thr Trp
   20 25 30
   aac gta cca cat cag gcc cag ggc gtg aca ttt gct tct att tat cgg 144
   Asn Val Pro His Gln Ala Gln Gly Val Thr Phe Ala Ser Ile Tyr Arg
   35 40 45
   cgt aag aaa gcg atg ctg acg ctc ggg cag gct gcg ctg gaa gat gcc 192
   Arg Lys Lys Ala Met Leu Thr Leu Gly Gln Ala Ala Leu Glu Asp Ala
   50 55 60
   tgg gaa tat atg gca ccg cga gag tgt gcg ctg ttt atc ctc gat gaa 240
   Trp Glu Tyr Met Ala Pro Arg Glu Cys Ala Leu Phe Ile Leu Asp Glu
   65 70 75 80
   acc gcc tgc att ctc agc cgt aat ggc gat ccg caa acc ttg cag cag 288
   Thr Ala Cys Ile Leu Ser Arg Asn Gly Asp Pro Gln Thr Leu Gln Gln
   85 90 95
   cta agt gca ctg gga ttc aat gac ggc acg tat tgc gcc gag gga att 336
   Leu Ser Ala Leu Gly Phe Asn Asp Gly Thr Tyr Cys Ala Glu Gly Ile
   100 105 110
   att ggt act tgt gcg cta tcg tta gcg gct atc tct ggt cag gcc gtg 384
   Ile Gly Thr Cys Ala Leu Ser Leu Ala Ala Ile Ser Gly Gln Ala Val
   115 120 125
   aaa acg atg gcc gat caa cat ttc aaa cag gta ctc tgg aac tgg gcc 432
   Lys Thr Met Ala Asp Gln His Phe Lys Gln Val Leu Trp Asn Trp Ala
   130 135 140
   ttt tgt gca acg ccg ttg ttt gac agc aag ggc cga ttg acg gga aca 480
   Phe Cys Ala Thr Pro Leu Phe Asp Ser Lys Gly Arg Leu Thr Gly Thr
   145 150 155 160
   ata gcg ctg gcg tgt ccg gtt gag caa act acc gca gct gat ttg ccg 528
   Ile Ala Leu Ala Cys Pro Val Glu Gln Thr Thr Ala Ala Asp Leu Pro
   165 170 175
   ttg acg ttg gca atc gcc cgc gag gtc gga aat tta ctg ctg acg gac 576
   Leu Thr Leu Ala Ile Ala Arg Glu Val Gly Asn Leu Leu Leu Thr Asp
   180 185 190
   agt ttg ctc gct gaa act aac cgt cat tta aat caa ctt aat gcc ctg 624
   Ser Leu Leu Ala Glu Thr Asn Arg His Leu Asn Gln Leu Asn Ala Leu
   195 200 205
   tta gaa agt atg gat gat ggc gtg att agc tgg gac gag cag ggt aat      672
   Leu Glu Ser Met Asp Asp Gly Val Ile Ser Trp Asp Glu Gln Gly Asn
       210                 215                 220
   ttg caa ttt att aat gcc cag gcg gcg cgg gtc ttg cgc ctt gac gcg      720
   Leu Gln Phe Ile Asn Ala Gln Ala Ala Arg Val Leu Arg Leu Asp Ala
   225                 230                 235                 240
   acg gca agt cag gga cgg gca atc act gaa ctc tta acg tta ccc gcc      768
   Thr Ala Ser Gln Gly Arg Ala Ile Thr Glu Leu Leu Thr Leu Pro Ala
                   245                 250                 255
   gta ttg caa caa gca ata aaa cag gca cat ccg ctc aaa cac gta gaa      816
   Val Leu Gln Gln Ala Ile Lys Gln Ala His Pro Leu Lys His Val Glu
               260                 265                 270
   gca acc ttt gaa agc cag cac cag ttt att gat gcg gtg ata acc ctt      864
   Ala Thr Phe Glu Ser Gln His Gln Phe Ile Asp Ala Val Ile Thr Leu
           275                 280                 285
   aaa ccg ata ata gaa acg cag gga acc agc ttt att ttg ttg ctc cat      912
   Lys Pro Ile Ile Glu Thr Gln Gly Thr Ser Phe Ile Leu Leu Leu His
       290                 295                 300
   cct gtg gaa cag atg cgg cag ttg atg acc agt caa tta gga aaa gtc      960
   Pro Val Glu Gln Met Arg Gln Leu Met Thr Ser Gln Leu Gly Lys Val
   305                 310                 315                 320
   agc cat acc ttc gct cat atg cca cag gac gat ccg caa acc cgc cgc     1008
   Ser His Thr Phe Ala His Met Pro Gln Asp Asp Pro Gln Thr Arg Arg
                   325                 330                 335
   ttg att cat ttt ggt cgc cag gcg gcg cgc agt agc ttt cct gtc ctg     1056
   Leu Ile His Phe Gly Arg Gln Ala Ala Arg Ser Ser Phe Pro Val Leu
               340                 345                 350
   ctt tgt gga gaa gag ggc gtg ggc aag gca ctg cta agt cag gca att     1104
   Leu Cys Gly Glu Glu Gly Val Gly Lys Ala Leu Leu Ser Gln Ala Ile
           355                 360                 365
   cat aat gaa agc gag cgt gct gca ggt cct tat atc gcc gtc aat tgt     1152
   His Asn Glu Ser Glu Arg Ala Ala Gly Pro Tyr Ile Ala Val Asn Cys
       370                 375                 380
   gag tta tat ggt gat gct gcg ctg gcg gaa gaa ttt att ggt ggc gat     1200
   Glu Leu Tyr Gly Asp Ala Ala Leu Ala Glu Glu Phe Ile Gly Gly Asp
   385                 390                 395                 400
   cgc acg gac aat gaa aat ggc cgt ctg agt cgg ctg gaa ctg gca cac     1248
   Arg Thr Asp Asn Glu Asn Gly Arg Leu Ser Arg Leu Glu Leu Ala His
                   405                 410                 415
   ggc ggc acg ctg ttt ctt gaa aag att gaa tat ctg gcg gtg gag tta     1296
   Gly Gly Thr Leu Phe Leu Glu Lys Ile Glu Tyr Leu Ala Val Glu Leu
               420                 425                 430
   cag tct gct ttg ctt cag gtt atc aag cag ggg gtt atc acg cga ctg     1344
   Gln Ser Ala Leu Leu Gln Val Ile Lys Gln Gly Val Ile Thr Arg Leu
           435                 440                 445
   gat gcg cgg cgt tta ata cca att gat gtc aaa gtg att gca aca acg     1392
   Asp Ala Arg Arg Leu Ile Pro Ile Asp Val Lys Val Ile Ala Thr Thr
       450                 455                 460
   acc gcg gac ctc gca atg ctg gtg gaa caa aat cgt ttt agt cgc cag     1440
   Thr Ala Asp Leu Ala Met Leu Val Glu Gln Asn Arg Phe Ser Arg Gln
   465                 470                 475                 480
   ctg tat tac gcg ctg cat gca ttt gaa att acc atc ccg cct ctg cgt     1488
   Leu Tyr Tyr Ala Leu His Ala Phe Glu Ile Thr Ile Pro Pro Leu Arg
                   485                 490                 495
   atg cgg cgt ggc agc att ccg gcg ctg gtg aat aac aaa tta cgc agt     1536
   Met Arg Arg Gly Ser Ile Pro Ala Leu Val Asn Asn Lys Leu Arg Ser
   500 505 510
   ctt gaa aaa cgc ttc tct acg cgg ctg aaa att gat gac gat gcc ctc 1584
   Leu Glu Lys Arg Phe Ser Thr Arg Leu Lys Ile Asp Asp Asp Ala Leu
   515 520 525
   gct cgc ctg gtt tct tgt gca tgg cca ggc aac gat ttt gaa ctt tac 1632
   Ala Arg Leu Val Ser Cys Ala Trp Pro Gly Asn Asp Phe Glu Leu Tyr
   530 535 540
   agc gtc atc gag aat ctt gct ctg agt agt gat aac ggg cgc att cgc 1680
   Ser Val Ile Glu Asn Leu Ala Leu Ser Ser Asp Asn Gly Arg Ile Arg
   545 550 555 560
   gtc agt gat ttg ccg gaa cat ctg ttt acc gag cag gcg aca gat gat 1728
   Val Ser Asp Leu Pro Glu His Leu Phe Thr Glu Gln Ala Thr Asp Asp
   565 570 575
   gtc agc gcc acc cgc ctt tcc acc agt ctg tca ttt gcg gaa gtt gaa 1776
   Val Ser Ala Thr Arg Leu Ser Thr Ser Leu Ser Phe Ala Glu Val Glu
   580 585 590
   aaa gag gca att att aac gca gcc cag gtc aca ggc ggt cgc att cag 1824
   Lys Glu Ala Ile Ile Asn Ala Ala Gln Val Thr Gly Gly Arg Ile Gln
   595 600 605
   gaa atg tcg gct tta ctt ggg atc ggc cgc act acg ctg tgg cgg aaa 1872
   Glu Met Ser Ala Leu Leu Gly Ile Gly Arg Thr Thr Leu Trp Arg Lys
   610 615 620
   atg aag caa cat ggc att gat gca ggg cag ttt aag cgc cgg gta tga 1920
   Met Lys Gln His Gly Ile Asp Ala Gly Gln Phe Lys Arg Arg Val
   625 630 635
<210> 67
   <211> 639
   <212> PRT
   <213> Escherichia coli
<400> 67
   Met Ser Gly Ala Phe Asn Asn Asp Gly Arg Gly Ile Ser Pro Leu Ile
   1 5 10 15
   Ala Thr Ser Trp Glu Arg Cys Asn Lys Leu Met Lys Arg Glu Thr Trp
   20 25 30
   Asn Val Pro His Gln Ala Gln Gly Val Thr Phe Ala Ser Ile Tyr Arg
   35 40 45
   Arg Lys Lys Ala Met Leu Thr Leu Gly Gln Ala Ala Leu Glu Asp Ala
   50 55 60
   Trp Glu Tyr Met Ala Pro Arg Glu Cys Ala Leu Phe Ile Leu Asp Glu
   65 70 75 80
   Thr Ala Cys Ile Leu Ser Arg Asn Gly Asp Pro Gln Thr Leu Gln Gln
   85 90 95
   Leu Ser Ala Leu Gly Phe Asn Asp Gly Thr Tyr Cys Ala Glu Gly Ile
   100 105 110
   Ile Gly Thr Cys Ala Leu Ser Leu Ala Ala Ile Ser Gly Gln Ala Val
   115 120 125
   Lys Thr Met Ala Asp Gln His Phe Lys Gln Val Leu Trp Asn Trp Ala
   130 135 140
   Phe Cys Ala Thr Pro Leu Phe Asp Ser Lys Gly Arg Leu Thr Gly Thr
   145 150 155 160
   Ile Ala Leu Ala Cys Pro Val Glu Gln Thr Thr Ala Ala Asp Leu Pro
   165 170 175
   Leu Thr Leu Ala Ile Ala Arg Glu Val Gly Asn Leu Leu Leu Thr Asp
   180 185 190
   Ser Leu Leu Ala Glu Thr Asn Arg His Leu Asn Gln Leu Asn Ala Leu
   195 200 205
   Leu Glu Ser Met Asp Asp Gly Val Ile Ser Trp Asp Glu Gln Gly Asn
   210 215 220
   Leu Gln Phe Ile Asn Ala Gln Ala Ala Arg Val Leu Arg Leu Asp Ala
   225 230 235 240
   Thr Ala Ser Gln Gly Arg Ala Ile Thr Glu Leu Leu Thr Leu Pro Ala
   245 250 255
   Val Leu Gln Gln Ala Ile Lys Gln Ala His Pro Leu Lys His Val Glu
   260 265 270
   Ala Thr Phe Glu Ser Gln His Gln Phe Ile Asp Ala Val Ile Thr Leu
   275 280 285
   Lys Pro Ile Ile Glu Thr Gln Gly Thr Ser Phe Ile Leu Leu Leu His
   290 295 300
   Pro Val Glu Gln Met Arg Gln Leu Met Thr Ser Gln Leu Gly Lys Val
   305 310 315 320
   Ser His Thr Phe Ala His Met Pro Gln Asp Asp Pro Gln Thr Arg Arg
   325 330 335
   Leu Ile His Phe Gly Arg Gln Ala Ala Arg Ser Ser Phe Pro Val Leu
   340 345 350
   Leu Cys Gly Glu Glu Gly Val Gly Lys Ala Leu Leu Ser Gln Ala Ile
   355 360 365
   His Asn Glu Ser Glu Arg Ala Ala Gly Pro Tyr Ile Ala Val Asn Cys
   370 375 380
   Glu Leu Tyr Gly Asp Ala Ala Leu Ala Glu Glu Phe Ile Gly Gly Asp
   385 390 395 400
   Arg Thr Asp Asn Glu Asn Gly Arg Leu Ser Arg Leu Glu Leu Ala His
   405 410 415
   Gly Gly Thr Leu Phe Leu Glu Lys Ile Glu Tyr Leu Ala Val Glu Leu
   420 425 430
   Gln Ser Ala Leu Leu Gln Val Ile Lys Gln Gly Val Ile Thr Arg Leu
   435 440 445
   Asp Ala Arg Arg Leu Ile Pro Ile Asp Val Lys Val Ile Ala Thr Thr
   450 455 460
   Thr Ala Asp Leu Ala Met Leu Val Glu Gln Asn Arg Phe Ser Arg Gln
   465 470 475 480
   Leu Tyr Tyr Ala Leu His Ala Phe Glu Ile Thr Ile Pro Pro Leu Arg
   485 490 495
   Met Arg Arg Gly Ser Ile Pro Ala Leu Val Asn Asn Lys Leu Arg Ser
   500 505 510
   Leu Glu Lys Arg Phe Ser Thr Arg Leu Lys Ile Asp Asp Asp Ala Leu
   515 520 525
   Ala Arg Leu Val Ser Cys Ala Trp Pro Gly Asn Asp Phe Glu Leu Tyr
   530 535 540
   Ser Val Ile Glu Asn Leu Ala Leu Ser Ser Asp Asn Gly Arg Ile Arg
   545 550 555 560
   Val Ser Asp Leu Pro Glu His Leu Phe Thr Glu Gln Ala Thr Asp Asp
   565 570 575
   Val Ser Ala Thr Arg Leu Ser Thr Ser Leu Ser Phe Ala Glu Val Glu
   580 585 590
   Lys Glu Ala Ile Ile Asn Ala Ala Gln Val Thr Gly Gly Arg Ile Gln
   595 600 605
   Glu Met Ser Ala Leu Leu Gly Ile Gly Arg Thr Thr Leu Trp Arg Lys
       610                 615                 620
   Met Lys Gln His Gly Ile Asp Ala Gly Gln Phe Lys Arg Arg Val
   625                 630                 635
<210> 68
   <211> 663
   <212> DNA
   <213> Escherichia coli
<220>
   <221> CDS
   <222> (1).. (663)
<400> 68
   atg gaa ctg tat ctg gat act tca gac gtt gtt gcg gtg aag gcg ctg       48
   Met Glu Leu Tyr Leu Asp Thr Ser Asp Val Val Ala Val Lys Ala Leu
   1               5                   10                  15
   tca cgt att ttt ccg ctg gcg ggt gtg acc act aac cca agc att atc       96
   Ser Arg Ile Phe Pro Leu Ala Gly Val Thr Thr Asn Pro Ser Ile Ile
               20                  25                  30
   gcc gcg ggt aaa aaa ccg ctg gat gtt gtg ctt ccg caa ctt cat gaa      144
   Ala Ala Gly Lys Lys Pro Leu Asp Val Val Leu Pro Gln Leu His Glu
           35                  40                  45
   gcg atg ggc ggt cag ggg cgt ctg ttt gcc cag gta atg gct acc act      192
   Ala Met Gly Gly Gln Gly Arg Leu Phe Ala Gln Val Met Ala Thr Thr
       50                  55                  60
   gcc gaa ggg atg gtt aat gac gcg ctt aag ctg cgt tct att att gcg      240
   Ala Glu Gly Met Val Asn Asp Ala Leu Lys Leu Arg Ser Ile Ile Ala
   65                  70                  75                  80
   gat atc gtg gtg aaa gtt ccg gtg acc gcc gag ggg ctg gca gct att      288
   Asp Ile Val Val Lys Val Pro Val Thr Ala Glu Gly Leu Ala Ala Ile
                   85                  90                  95
   aag atg tta aaa gcg gaa ggg att ccg acg ctg gga acc gcg gta tat      336
   Lys Met Leu Lys Ala Glu Gly Ile Pro Thr Leu Gly Thr Ala Val Tyr
               100                 105                 110
   ggc gca gca caa ggg ctg ctg tcg gcg ctg gca ggt gcg gaa tat gtt      384
   Gly Ala Ala Gln Gly Leu Leu Ser Ala Leu Ala Gly Ala Glu Tyr Val
           115                 120                 125
   gcg cct tac gtt aat cgt att gat gct cag ggc ggt agc ggc att cag      432
   Ala Pro Tyr Val Asn Arg Ile Asp Ala Gln Gly Gly Ser Gly Ile Gln
       130                 135                 140
   act gtg acc gac tta cac cag tta ttg aaa atg cat gcg ccg cag gcg      480
   Thr Val Thr Asp Leu His Gln Leu Leu Lys Met His Ala Pro Gln Ala
   145                 150                 155                 160
   aaa gtg ctg gca gcg agt ttc aaa acc ccg cgt cag gcg ctg gac tgc      528
   Lys Val Leu Ala Ala Ser Phe Lys Thr Pro Arg Gln Ala Leu Asp Cys
                   165                 170                 175
   tta ctg gca gga tgt gaa tca att act ctg cca ctg gat gtg gca caa      576
   Leu Leu Ala Gly Cys Glu Ser Ile Thr Leu Pro Leu Asp Val Ala Gln
               180                 185                 190
   cag atg att agc tat ccg gcg gtt gat gcc gct gtg gcg aag ttt gag      624
   Gln Met Ile Ser Tyr Pro Ala Val Asp Ala Ala Val Ala Lys Phe Glu
           195                 200                 205
   cag gac tgg cag gga gcg ttt ggc aga acg tcg att taa                    663
   Gln Asp Trp Gln Gly Ala Phe Gly Arg Thr Ser Ile
       210                 215                 220
<210> 69
   <211> 220
   <212> PRT
   <213> Escherichia coli
<400> 69
   Met Glu Leu Tyr Leu Asp Thr Ser Asp Val Val Ala Val Lys Ala Leu
   1               5                   10                  15
   Ser Arg Ile Phe Pro Leu Ala Gly Val Thr Thr Asn Pro Ser Ile Ile
               20                  25                  30
   Ala Ala Gly Lys Lys Pro Leu Asp Val Val Leu Pro Gln Leu His Glu
           35                  40                  45
   Ala Met Gly Gly Gln Gly Arg Leu Phe Ala Gln Val Met Ala Thr Thr
       50                  55                  60
   Ala Glu Gly Met Val Asn Asp Ala Leu Lys Leu Arg Ser Ile Ile Ala
   65                  70                  75                  80
   Asp Ile Val Val Lys Val Pro Val Thr Ala Glu Gly Leu Ala Ala Ile
                   85                  90                  95
   Lys Met Leu Lys Ala Glu Gly Ile Pro Thr Leu Gly Thr Ala Val Tyr
               100                 105                 110
   Gly Ala Ala Gln Gly Leu Leu Ser Ala Leu Ala Gly Ala Glu Tyr Val
           115                 120                 125
   Ala Pro Tyr Val Asn Arg Ile Asp Ala Gln Gly Gly Ser Gly Ile Gln
       130                 135                 140
   Thr Val Thr Asp Leu His Gln Leu Leu Lys Met His Ala Pro Gln Ala
   145                 150                 155                 160
   Lys Val Leu Ala Ala Ser Phe Lys Thr Pro Arg Gln Ala Leu Asp Cys
                   165                 170                 175
   Leu Leu Ala Gly Cys Glu Ser Ile Thr Leu Pro Leu Asp Val Ala Gln
               180                 185                 190
   Gln Met Ile Ser Tyr Pro Ala Val Asp Ala Ala Val Ala Lys Phe Glu
           195                 200                 205
   Gln Asp Trp Gln Gly Ala Phe Gly Arg Thr Ser Ile
       210                 215                 220
<210> 70
   <211> 663
   <212> DNA
   <213> Escherichia coli
<220>
   <221> CDS
   <222> (1).. (663)
<400> 70
   atg gaa ctg tat ctg gac acc gct aac gtc gca gaa gtc gaa cgt ctg      48
   Met Glu Leu Tyr Leu Asp Thr Ala Asn Val Ala Glu Val Glu Arg Leu
   1               5                   10                  15
   gca cgc ata ttc ccc att gcc ggg gtg aca act aac ccg agc att atc      96
   Ala Arg Ile Phe Pro Ile Ala Gly Val Thr Thr Asn Pro Ser Ile Ile
               20                  25                  30
   gct gcc agc aag gag tcc ata tgg gaa gtg ctg ccg cgt ctg caa aaa      144
   Ala Ala Ser Lys Glu Ser Ile Trp Glu Val Leu Pro Arg Leu Gln Lys
           35                  40                  45
   gcg att ggt gat gag ggc att ctg ttt gct cag acc atg agc cgc gac      192
   Ala Ile Gly Asp Glu Gly Ile Leu Phe Ala Gln Thr Met Ser Arg Asp
       50                  55                  60
   gcg cag ggg atg gtg gaa gaa gcg aag cgc ctg cgc gac gct att ccg      240
   Ala Gln Gly Met Val Glu Glu Ala Lys Arg Leu Arg Asp Ala Ile Pro
   65                  70                  75                  80
   ggt att gtg gtg aaa atc ccg gtg act tcc gaa ggt ctg gca gca att      288
   Gly Ile Val Val Lys Ile Pro Val Thr Ser Glu Gly Leu Ala Ala Ile
                   85                  90                  95
   aaa ata ctg aaa aaa gag ggt att act aca ctt ggc act gct gta tat      336
   Lys Ile Leu Lys Lys Glu Gly Ile Thr Thr Leu Gly Thr Ala Val Tyr
               100                 105                 110
   agc gcc gca caa ggg tta tta gcc gca ctg gca ggg gca aaa tac gtt      384
   Ser Ala Ala Gln Gly Leu Leu Ala Ala Leu Ala Gly Ala Lys Tyr Val
           115                 120                 125
   gct ccg tat gtt aac cgc gta gat gcc cag ggc gga gac ggc att cgt      432
   Ala Pro Tyr Val Asn Arg Val Asp Ala Gln Gly Gly Asp Gly Ile Arg
       130                 135                 140
   acg gtt cag gag ctg caa acg ctg tta gaa atg cac gcg cca gaa agc      480
   Thr Val Gln Glu Leu Gln Thr Leu Leu Glu Met His Ala Pro Glu Ser
   145                 150                 155                 160
   atg gtg ctg gca gcc agc ttt aaa acg ccg cgt cag gcg ctg gac tgt      528
   Met Val Leu Ala Ala Ser Phe Lys Thr Pro Arg Gln Ala Leu Asp Cys
                   165                 170                 175
   tta ctg gca gga tgt gaa tcc atc acc ctg ccc tta gat gta gcg caa      576
   Leu Leu Ala Gly Cys Glu Ser Ile Thr Leu Pro Leu Asp Val Ala Gln
               180                 185                 190
   caa atg ctc aac acc cct gcg gta gag tca gct ata gag aag ttc gaa      624
   Gln Met Leu Asn Thr Pro Ala Val Glu Ser Ala Ile Glu Lys Phe Glu
           195                 200                 205
   cac gac tgg aat gcc gca ttt ggc act act cat ctc taa                  663
   His Asp Trp Asn Ala Ala Phe Gly Thr Thr His Leu
       210                 215                 220
<210> 71
   <211> 220
   <212> PRT
   <213> Escherichia coli
<400> 71
   Met Glu Leu Tyr Leu Asp Thr Ala Asn Val Ala Glu Val Glu Arg Leu
   1               5                   10                  15
   Ala Arg Ile Phe Pro Ile Ala Gly Val Thr Thr Asn Pro Ser Ile Ile
               20                  25                  30
   Ala Ala Ser Lys Glu Ser Ile Trp Glu Val Leu Pro Arg Leu Gln Lys
           35                  40                  45
   Ala Ile Gly Asp Glu Gly Ile Leu Phe Ala Gln Thr Met Ser Arg Asp
       50                  55                  60
   Ala Gln Gly Met Val Glu Glu Ala Lys Arg Leu Arg Asp Ala Ile Pro
   65                  70                  75                  80
   Gly Ile Val Val Lys Ile Pro Val Thr Ser Glu Gly Leu Ala Ala Ile
                   85                  90                  95
   Lys Ile Leu Lys Lys Glu Gly Ile Thr Thr Leu Gly Thr Ala Val Tyr
               100                 105                 110
   Ser Ala Ala Gln Gly Leu Leu Ala Ala Leu Ala Gly Ala Lys Tyr Val
           115                 120                 125
   Ala Pro Tyr Val Asn Arg Val Asp Ala Gln Gly Gly Asp Gly Ile Arg
       130                 135                 140
   Thr Val Gln Glu Leu Gln Thr Leu Leu Glu Met His Ala Pro Glu Ser
   145                 150                 155                 160
   Met Val Leu Ala Ala Ser Phe Lys Thr Pro Arg Gln Ala Leu Asp Cys
                   165                 170                 175
   Leu Leu Ala Gly Cys Glu Ser Ile Thr Leu Pro Leu Asp Val Ala Gln
               180                 185                 190
   Gln Met Leu Asn Thr Pro Ala Val Glu Ser Ala Ile Glu Lys Phe Glu
           195                 200                 205
   His Asp Trp Asn Ala Ala Phe Gly Thr Thr His Leu
       210                 215                 220
<210> 72
   <211> 1053
   <212> DNA
   <213> Escherichia coli
<220>
   <221> CDS
   <222> (1).. (1053)
<400> 72
   atg aca gat att gcg cag ttg ctt ggc aaa gac gcc gac aac ctt tta      48
   cag cac cgt tgt atg aca att cct tct gac cag ctt tat ctc ccc gga      96
   cat gac tac gta gac cgc gta atg att gac aat aat cgc ccg cca gcg      144
   gtg tta cgt aat atg cag acg ttg tac aac acc ggg cgt ctg gct ggc      192
   aca gga tat ctt tct att ctg ccg gtt gac cag ggc gtt gag cac tct      240
   gcc gga gct tca ttt gct gct aac ccg ctc tac ttt gac ccg aaa aac      288
   att gtt gaa ctg gcg atc gaa gcg ggc tgt aac tgt gtg gcg tca act      336
   tac ggc gtg ctg gcg tcg gta tcg cgg cgt tat gcg cat cgc att cca      384
   ttc ctc gtc aaa ctt aat cac aac gag acg cta agt tac ccg aat acc        432
   tac gat caa acg ctg tat gcc agc gtg gag cag gcg ttc aac atg ggc        480
   gcg gtt gcg gtt ggt gcg act atc tat ttt ggc tcg gaa gag tca cgt        528
   cgc cag att gaa gaa att tct gcg gct ttt gaa cgt gcg cac gag ctg        576
   ggt atg gtg aca gtg ctg tgg gcc tat ttg cgt aac tcc gcc ttt aag        624
   aaa gat ggc gtt gat tac cat gtt tcc gcc gac ctg acc ggt cag gca        672
   aac cat ctg gcg gca acc atc ggt gca gat atc gtc aaa caa aaa atg        720
   gcg gaa aat aac ggc ggc tat aaa gca att aat tac ggt tac acc gac        768
   gat cgt gtt tac agc aaa ttg acc agc gaa aac ccg att gat ctg gtg        816
   cgt tat cag tta gct aac tgc tat atg ggt cgg gct ggg ttg ata aac        864
   tcc ggc ggt gct gcg ggc ggt gaa act gac ctc agc gat gca gtg cgt        912
   act gcg gtt atc aac aaa cgc gca ggc gga atg ggg ctg att ctt gga        960
   cgt aaa gcg ttc aag aaa tcg atg gct gac ggc gtg aaa ctg att aac       1008
   gcc gtg cag gac gtt tat ctc gat agc aaa att act atc gcc tga           1053
<210> 73
   <211> 350
   <212> PRT
   <213> Escherichia coli
<400> 73
<210> 74
   <211> 1143
   <212> DNA
   <213> Shigella dysenteriae
<220>
   <221> CDS
   <222> (1).. (1143)
<400> 74
   atg ccg cat ttg gca cta ctc atc tct aaa gga gca att atg gac cgc 48
   Met Pro His Leu Ala Leu Leu Ile Ser Lys Gly Ala Ile Met Asp Arg
   1 5 10 15
   att att caa tca ccg ggt aaa tac atc cag ggc gct gat gtg att aat 96
   Ile Ile Gln Ser Pro Gly Lys Tyr Ile Gln Gly Ala Asp Val Ile Asn
               20                  25                  30
   cgt ctg ggc gaa tac ctg aag ccg ctg gca gaa ctc tgg tta gtg gtg      144
   Arg Leu Gly Glu Tyr Leu Lys Pro Leu Ala Glu Leu Trp Leu Val Val
           35                  40                  45
   ggt gac aaa ttt gtt tta ggt ttt gct caa tcc act gtc gag aaa agc      192
   Gly Asp Lys Phe Val Leu Gly Phe Ala Gln Ser Thr Val Glu Lys Ser
       50                  55                  60
   ttt aaa gat gct gga ctg gta gta gaa att gcg ccg ttt ggc ggt gaa      240
   Phe Lys Asp Ala Gly Leu Val Val Glu Ile Ala Pro Phe Gly Gly Glu
   65                  70                  75                  80
   tgt tcg caa aat gag atc gac cgt ctg cgt ggc atc gcg gag act gcg      288
   Cys Ser Gln Asn Glu Ile Asp Arg Leu Arg Gly Ile Ala Glu Thr Ala
                   85                  90                  95
   cag tgt ggc gca att ctc ggt atc ggt ggc gga aaa act ttc gat act      336
   Gln Cys Gly Ala Ile Leu Gly Ile Gly Gly Gly Lys Thr Phe Asp Thr
               100                 105                 110
   gcc aaa gca ctg gca cat ttc atg ggt gtt ccg gta gcg atc gca ccg      384
   Ala Lys Ala Leu Ala His Phe Met Gly Val Pro Val Ala Ile Ala Pro
           115                 120                 125
   acg atc gcc tct acc gac gca ccg tgc agc gca ttg tct gtt atc tac      432
   Thr Ile Ala Ser Thr Asp Ala Pro Cys Ser Ala Leu Ser Val Ile Tyr
       130                 135                 140
   acc gat gag ggt gag ttt gac cgc tat ctg ctg ttg cca aat aac cct      480
   Thr Asp Glu Gly Glu Phe Asp Arg Tyr Leu Leu Leu Pro Asn Asn Pro
   145                 150                 155                 160
   aat atg gtc att gtc gac acc aaa atc gtc gct ggc gca cct gca cgt      528
   Asn Met Val Ile Val Asp Thr Lys Ile Val Ala Gly Ala Pro Ala Arg
                   165                 170                 175
   ctg tta gcg gcg ggt atc ggc gat gcg ctg gca acc tgg ttt gaa gcg      576
   Leu Leu Ala Ala Gly Ile Gly Asp Ala Leu Ala Thr Trp Phe Glu Ala
               180                 185                 190
   cgt gcc tgc tct cgt agc ggc gcg acc acc atg gcg ggc ggc aag tgc      624
   Arg Ala Cys Ser Arg Ser Gly Ala Thr Thr Met Ala Gly Gly Lys Cys
           195                 200                 205
   acc cag gct gcg ctg gca ctg gct gaa ctg tgc tac aac acc ctg ctg      672
   Thr Gln Ala Ala Leu Ala Leu Ala Glu Leu Cys Tyr Asn Thr Leu Leu
       210                 215                 220
   gaa gaa ggc gaa aaa gcg atg ctt gct gcc gaa cag cat gta gtg act      720
   Glu Glu Gly Glu Lys Ala Met Leu Ala Ala Glu Gln His Val Val Thr
   225                 230                 235                 240
   ccg gcg ctg gag cgc gtg att gaa gcg aac acc tat ttg agc ggt gtt      768
   Pro Ala Leu Glu Arg Val Ile Glu Ala Asn Thr Tyr Leu Ser Gly Val
                   245                 250                 255
   ggt ttt gaa agt ggt ggt ctg gct gcg gcg cac gca gtg cat aac ggc      816
   Gly Phe Glu Ser Gly Gly Leu Ala Ala Ala His Ala Val His Asn Gly
               260                 265                 270
   ctg acc gct atc ccg gac gcg cat cac tat tat cac ggt gaa aaa gtg      864
   Leu Thr Ala Ile Pro Asp Ala His His Tyr Tyr His Gly Glu Lys Val
           275                 280                 285
   gca ttc ggt acg ctg acg cag ctg gtt ctg gaa aat gcg ccg gtg gag      912
   Ala Phe Gly Thr Leu Thr Gln Leu Val Leu Glu Asn Ala Pro Val Glu
       290                 295                 300
   gaa atc gaa acc gta gct gcc ctt agc cat gcg gta ggt ttg cca ata      960
   Glu Ile Glu Thr Val Ala Ala Leu Ser His Ala Val Gly Leu Pro Ile
   305                 310                 315                 320
   act ctc gct caa ctg gat att aaa gaa gat gtc ccg gcg aaa atg cga      1008
   Thr Leu Ala Gln Leu Asp Ile Lys Glu Asp Val Pro Ala Lys Met Arg
                   325                 330                 335
   att gtg gca gaa gcg gca tgt gca gaa ggt gaa acc att cac aac atg      1056
   Ile Val Ala Glu Ala Ala Cys Ala Glu Gly Glu Thr Ile His Asn Met
               340                 345                 350
   cct ggc ggc gcg acg cca gat cag gtt tac gcc gca ctg ctg gta gct      1104
   Pro Gly Gly Ala Thr Pro Asp Gln Val Tyr Ala Ala Leu Leu Val Ala
           355                 360                 365
   gac cag tat gga caa cgt ttc ctg caa gag tgg gaa taa                  1143
   Asp Gln Tyr Gly Gln Arg Phe Leu Gln Glu Trp Glu
       370                 375                 380
<210> 75
   <211> 380
   <212> PRT
   <213> Shigella dysenteriae
<400> 75
   Met Pro His Leu Ala Leu Leu Ile Ser Lys Gly Ala Ile Met Asp Arg
   1               5                   10                  15
   Ile Ile Gln Ser Pro Gly Lys Tyr Ile Gln Gly Ala Asp Val Ile Asn
               20                  25                  30
   Arg Leu Gly Glu Tyr Leu Lys Pro Leu Ala Glu Leu Trp Leu Val Val
           35                  40                  45
   Gly Asp Lys Phe Val Leu Gly Phe Ala Gln Ser Thr Val Glu Lys Ser
       50                  55                  60
   Phe Lys Asp Ala Gly Leu Val Val Glu Ile Ala Pro Phe Gly Gly Glu
   65                  70                  75                  80
   Cys Ser Gln Asn Glu Ile Asp Arg Leu Arg Gly Ile Ala Glu Thr Ala
                   85                  90                  95
   Gln Cys Gly Ala Ile Leu Gly Ile Gly Gly Gly Lys Thr Phe Asp Thr
               100                 105                 110
   Ala Lys Ala Leu Ala His Phe Met Gly Val Pro Val Ala Ile Ala Pro
           115                 120                 125
   Thr Ile Ala Ser Thr Asp Ala Pro Cys Ser Ala Leu Ser Val Ile Tyr
       130                 135                 140
   Thr Asp Glu Gly Glu Phe Asp Arg Tyr Leu Leu Leu Pro Asn Asn Pro
   145                 150                 155                 160
   Asn Met Val Ile Val Asp Thr Lys Ile Val Ala Gly Ala Pro Ala Arg
                   165                 170                 175
   Leu Leu Ala Ala Gly Ile Gly Asp Ala Leu Ala Thr Trp Phe Glu Ala
               180                 185                 190
   Arg Ala Cys Ser Arg Ser Gly Ala Thr Thr Met Ala Gly Gly Lys Cys
           195                 200                 205
   Thr Gln Ala Ala Leu Ala Leu Ala Glu Leu Cys Tyr Asn Thr Leu Leu
       210                 215                 220
   Glu Glu Gly Glu Lys Ala Met Leu Ala Ala Glu Gln His Val Val Thr
   225                 230                 235                 240
   Pro Ala Leu Glu Arg Val Ile Glu Ala Asn Thr Tyr Leu Ser Gly Val
                   245                 250                 255
   Gly Phe Glu Ser Gly Gly Leu Ala Ala Ala His Ala Val His Asn Gly
               260                 265                 270
   Leu Thr Ala Ile Pro Asp Ala His His Tyr Tyr His Gly Glu Lys Val
           275                 280                 285
   Ala Phe Gly Thr Leu Thr Gln Leu Val Leu Glu Asn Ala Pro Val Glu
       290                 295                 300
   Glu Ile Glu Thr Val Ala Ala Leu Ser His Ala Val Gly Leu Pro Ile
   305                 310                 315                 320
   Thr Leu Ala Gln Leu Asp Ile Lys Glu Asp Val Pro Ala Lys Met Arg
                   325                 330                 335
   Ile Val Ala Glu Ala Ala Cys Ala Glu Gly Glu Thr Ile His Asn Met
               340                 345                 350
   Pro Gly Gly Ala Thr Pro Asp Gln Val Tyr Ala Ala Leu Leu Val Ala
           355                 360                 365
   Asp Gln Tyr Gly Gln Arg Phe Leu Gln Glu Trp Glu
       370                 375                 380
<210> 76
   <211> 1104
   <212> DNA
   <213> Salmonella typhimurium
<220>
   <221> CDS
   <222> (1).. (1104)
<400> 76
   atg gat cgc att att cag tca cca ggt aag tat att cag ggt gca aac      48
   Met Asp Arg Ile Ile Gln Ser Pro Gly Lys Tyr Ile Gln Gly Ala Asn
   1               5                   10                  15
   gtc atc gcg cgt ctt ggc gat tat tta aaa cca atg gcg aac aac tgg      96
   Val Ile Ala Arg Leu Gly Asp Tyr Leu Lys Pro Met Ala Asn Asn Trp
               20                  25                  30
   ctg gtt gtg ggc gat aaa ttc gtg ctg gga ttt gcc gaa gag acg ctg     144
   Leu Val Val Gly Asp Lys Phe Val Leu Gly Phe Ala Glu Glu Thr Leu
           35                  40                  45
   cgc aaa agc ctg acg gat gcc ggt ttg tca gta gaa atc gcc ccg ttt     192
   Arg Lys Ser Leu Thr Asp Ala Gly Leu Ser Val Glu Ile Ala Pro Phe
       50                  55                  60
   ggc ggc gaa tgt tcg caa aat gag atc gac agg ctg cgc gcc gtc gcc     240
   Gly Gly Glu Cys Ser Gln Asn Glu Ile Asp Arg Leu Arg Ala Val Ala
   65                  70                  75                  80
   gaa aaa agt cag tgt ggc gcc gta ctg ggt atc ggc ggc ggt aaa acg     288
   Glu Lys Ser Gln Cys Gly Ala Val Leu Gly Ile Gly Gly Gly Lys Thr
                   85                  90                  95
   ctg gat acc gcc aaa gcg ctg gcg cac ttt atg aac gtc ccg gtc gct     336
   Leu Asp Thr Ala Lys Ala Leu Ala His Phe Met Asn Val Pro Val Ala
               100                 105                 110
   atc gcg ccg acc atc gcc tct acc gac gca ccg tgc agc gca ctc tcg     384
   Ile Ala Pro Thr Ile Ala Ser Thr Asp Ala Pro Cys Ser Ala Leu Ser
           115                 120                 125
   gtt att tat acc gat gcc ggt gag ttt gac cgt tat ctg ctg ctg ccg     432
   Val Ile Tyr Thr Asp Ala Gly Glu Phe Asp Arg Tyr Leu Leu Leu Pro
       130                 135                 140
   cat aac ccg aat atg gtt att gtc gat acg cag ata gtg gcg ggc gcg     480
   His Asn Pro Asn Met Val Ile Val Asp Thr Gln Ile Val Ala Gly Ala
   145                 150                 155                 160
   ccg gcg cgt ctg ctg gca gcc ggt atc ggc gat gca ctg gcg acc tgg      528
   Pro Ala Arg Leu Leu Ala Ala Gly Ile Gly Asp Ala Leu Ala Thr Trp
                   165                 170                 175
   ttt gaa gcg cgc gcc tgc tca cgc agc ggc gcc acc aca atg gcg ggc      576
   Phe Glu Ala Arg Ala Cys Ser Arg Ser Gly Ala Thr Thr Met Ala Gly
               180                 185                 190
   ggc aag tgt aca cag gcc gcg ctg gcg ctg gcg gag cta tgc tat aac      624
   Gly Lys Cys Thr Gln Ala Ala Leu Ala Leu Ala Glu Leu Cys Tyr Asn
           195                 200                 205
   acg ctg atc gaa gaa ggc gaa aaa gcc atg ttg gcc gcc gaa cag cac      672
   Thr Leu Ile Glu Glu Gly Glu Lys Ala Met Leu Ala Ala Glu Gln His
       210                 215                 220
   gtc gtc acg cca gcg ctg gaa cgc gtc atc gaa gcc aac acc tac ctg      720
   Val Val Thr Pro Ala Leu Glu Arg Val Ile Glu Ala Asn Thr Tyr Leu
   225                 230                 235                 240
   agc ggg gtc ggt ttt gaa agc ggc ggt ctg gcc gca gcg cac gcg att      768
   Ser Gly Val Gly Phe Glu Ser Gly Gly Leu Ala Ala Ala His Ala Ile
                   245                 250                 255
   cat aac ggt tta acg gcg att ccg gat gcg cac cac tat tat cac ggt      816
   His Asn Gly Leu Thr Ala Ile Pro Asp Ala His His Tyr Tyr His Gly
               260                 265                 270
   gag aag gtc gct ttc ggt acg ctg acg caa ctg gtg ctg gaa aac gcg      864
   Glu Lys Val Ala Phe Gly Thr Leu Thr Gln Leu Val Leu Glu Asn Ala
           275                 280                 285
   ccg gtc gaa gaa atc gaa acc gtt gcg gcg ctg tgc cat tcc gtt ggc      912
   Pro Val Glu Glu Ile Glu Thr Val Ala Ala Leu Cys His Ser Val Gly
       290                 295                 300
   ctg ccg att acg ctg gcg caa ctg gat att aaa cag gat att ccg gcc      960
   Leu Pro Ile Thr Leu Ala Gln Leu Asp Ile Lys Gln Asp Ile Pro Ala
   305                 310                 315                 320
   aag atg cgc acc gtc gcg gaa gcc tcc tgc gca gaa ggt gaa act att     1008
   Lys Met Arg Thr Val Ala Glu Ala Ser Cys Ala Glu Gly Glu Thr Ile
                   325                 330                 335
   cat aac atg cct ggc ggc gca acg ccg gat gaa gtg tac gcc gcg ctg     1056
   His Asn Met Pro Gly Gly Ala Thr Pro Asp Glu Val Tyr Ala Ala Leu
               340                 345                 350
   ctg gtc gcc gac cag tac ggt caa cgc ttc ttg cag gaa tgg gaa taa     1104
   Leu Val Ala Asp Gln Tyr Gly Gln Arg Phe Leu Gln Glu Trp Glu
           355                 360                 365
<210> 77
   <211> 367
   <212> PRT
   <213> Salmonella typhimurium
<400> 77
   Met Asp Arg Ile Ile Gln Ser Pro Gly Lys Tyr Ile Gln Gly Ala Asn
   1               5                   10                  15
   Val Ile Ala Arg Leu Gly Asp Tyr Leu Lys Pro Met Ala Asn Asn Trp
               20                  25                  30
   Leu Val Val Gly Asp Lys Phe Val Leu Gly Phe Ala Glu Glu Thr Leu
           35                  40                  45
   Arg Lys Ser Leu Thr Asp Ala Gly Leu Ser Val Glu Ile Ala Pro Phe
           50                  55                  60
   Gly Gly Glu Cys Ser Gln Asn Glu Ile Asp Arg Leu Arg Ala Val Ala
   65                  70                  75                  80
   Glu Lys Ser Gln Cys Gly Ala Val Leu Gly Ile Gly Gly Gly Lys Thr
                   85                  90                  95
   Leu Asp Thr Ala Lys Ala Leu Ala His Phe Met Asn Val Pro Val Ala
               100                 105                 110
   Ile Ala Pro Thr Ile Ala Ser Thr Asp Ala Pro Cys Ser Ala Leu Ser
           115                 120                 125
   Val Ile Tyr Thr Asp Ala Gly Glu Phe Asp Arg Tyr Leu Leu Leu Pro
       130                 135                 140
   His Asn Pro Asn Met Val Ile Val Asp Thr Gln Ile Val Ala Gly Ala
   145                 150                 155                 160
   Pro Ala Arg Leu Leu Ala Ala Gly Ile Gly Asp Ala Leu Ala Thr Trp
                   165                 170                 175
   Phe Glu Ala Arg Ala Cys Ser Arg Ser Gly Ala Thr Thr Met Ala Gly
               180                 185                 190
   Gly Lys Cys Thr Gln Ala Ala Leu Ala Leu Ala Glu Leu Cys Tyr Asn
           195                 200                 205
   Thr Leu Ile Glu Glu Gly Glu Lys Ala Met Leu Ala Ala Glu Gln His
       210                 215                 220
   Val Val Thr Pro Ala Leu Glu Arg Val Ile Glu Ala Asn Thr Tyr Leu
   225                 230                 235                 240
   Ser Gly Val Gly Phe Glu Ser Gly Gly Leu Ala Ala Ala His Ala Ile
                   245                 250                 255
   His Asn Gly Leu Thr Ala Ile Pro Asp Ala His His Tyr Tyr His Gly
               260                 265                 270
   Glu Lys Val Ala Phe Gly Thr Leu Thr Gln Leu Val Leu Glu Asn Ala
           275                 280                 285
   Pro Val Glu Glu Ile Glu Thr Val Ala Ala Leu Cys His Ser Val Gly
       290                 295                 300
   Leu Pro Ile Thr Leu Ala Gln Leu Asp Ile Lys Gln Asp Ile Pro Ala
   305                 310                 315                 320
   Lys Met Arg Thr Val Ala Glu Ala Ser Cys Ala Glu Gly Glu Thr Ile
                   325                 330                 335
   His Asn Met Pro Gly Gly Ala Thr Pro Asp Glu Val Tyr Ala Ala Leu
               340                 345                 350
   Leu Val Ala Asp Gln Tyr Gly Gln Arg Phe Leu Gln Glu Trp Glu
           355                 360                 365
<210> 78
   <211> 1098
   <212> DNA
   <213> Pseudomonas putida
<220>
   <221> CDS
   <222> (1).. (1098)
<400> 78
   atg gac cgc gcc att caa tca ccc ggc aaa tat gta caa ggg gcc gat      48
   Met Asp Arg Ala Ile Gln Ser Pro Gly Lys Tyr Val Gln Gly Ala Asp
   1               5                   10                  15
   gcg ctg caa cgg ctg ggg gac tac ctc aag ccg ctg gcg gac agc tgg      96
   Ala Leu Gln Arg Leu Gly Asp Tyr Leu Lys Pro Leu Ala Asp Ser Trp
   20 25 30
   ctg gtg att gcc gac aag ttc gtg ctg ggc ttt gcc gaa gac acc atc 144
   Leu Val Ile Ala Asp Lys Phe Val Leu Gly Phe Ala Glu Asp Thr Ile
   35 40 45
   cgc caa agc ctc agc aag gcc ggg ctg gcc atg gac atc gtc gcc ttc 192
   Arg Gln Ser Leu Ser Lys Ala Gly Leu Ala Met Asp Ile Val Ala Phe
   50 55 60
   aac ggc gaa tgc tcg cag ggc gag gtc gat cgc ctg tgc caa ctg gcc 240
   Asn Gly Glu Cys Ser Gln Gly Glu Val Asp Arg Leu Cys Gln Leu Ala
   65 70 75 80
   acg caa aac ggg cgc agc gcc atc gtc ggc att ggt ggc ggc aag acg 288
   Thr Gln Asn Gly Arg Ser Ala Ile Val Gly Ile Gly Gly Gly Lys Thr
   85 90 95
   ctg gac acc gcc aag gcc gtg gcc ttt ttc cag aaa gtg ccc gtg gcc 336
   Leu Asp Thr Ala Lys Ala Val Ala Phe Phe Gln Lys Val Pro Val Ala
   100 105 110
   gtg gcc ccc acc atc gcc tcc acc gac gcg ccc tgc agc gcg ctg tcg 384
   Val Ala Pro Thr Ile Ala Ser Thr Asp Ala Pro Cys Ser Ala Leu Ser
   115 120 125
   gtg ctc tat acc gat gaa ggt gag ttc gac cgc tat ctg atg ctg ccc 432
   Val Leu Tyr Thr Asp Glu Gly Glu Phe Asp Arg Tyr Leu Met Leu Pro
   130 135 140
   acc aac ccc gcc ctg gtg gtg gtg gac acc gcc atc gtc gcc cgt gca 480
   Thr Asn Pro Ala Leu Val Val Val Asp Thr Ala Ile Val Ala Arg Ala
   145 150 155 160
   ccg gcg cgg ctg ttg gcg gcc ggc att ggt gat gcc ctg gcc acc tgg 528
   Pro Ala Arg Leu Leu Ala Ala Gly Ile Gly Asp Ala Leu Ala Thr Trp
   165 170 175
   ttc gag gcg cgt gcc gca tcg cgc agc agc gct gcc acc atg gcc ggc 576
   Phe Glu Ala Arg Ala Ala Ser Arg Ser Ser Ala Ala Thr Met Ala Gly
   180 185 190
   ggc ccg gcc acg cag acc gca ctg aac ctg gcc agg ttc tgc tac gac 624
   Gly Pro Ala Thr Gln Thr Ala Leu Asn Leu Ala Arg Phe Cys Tyr Asp
   195 200 205
   acc ctg ctg gaa gag ggt gaa aaa gcc atg ttg gcc gtg cag gcc cag 672
   Thr Leu Leu Glu Glu Gly Glu Lys Ala Met Leu Ala Val Gln Ala Gln
   210 215 220
   gtg gtg acg ccg gcg ctg gag cgc atc gtc gag gcc aac acc tat ctg 720
   Val Val Thr Pro Ala Leu Glu Arg Ile Val Glu Ala Asn Thr Tyr Leu
   225 230 235 240
   agc ggg gtc ggg ttt gaa agc ggt ggc gtg gcc gcc gcc cac gcg gtg 768
   Ser Gly Val Gly Phe Glu Ser Gly Gly Val Ala Ala Ala His Ala Val
   245 250 255
   cac aac ggc ctg acc gcc gtg gcc gaa acc cac cac ttc tac cac ggc 816
   His Asn Gly Leu Thr Ala Val Ala Glu Thr His His Phe Tyr His Gly
   260 265 270
   gaa aaa gtg gcg ttt ggc gtg ctg gtg caa ctg gcg ctg gaa aac gcc 864
   Glu Lys Val Ala Phe Gly Val Leu Val Gln Leu Ala Leu Glu Asn Ala
   275 280 285
   tcc aac gcg gaa atg cag gaa gtg atg tcg ctg tgc cac gcc gtg ggc 912
   Ser Asn Ala Glu Met Gln Glu Val Met Ser Leu Cys His Ala Val Gly
   290 295 300
   ctg ccc atc acg ctg gcg cag ctg gac att acc gaa gac atc ccc acc 960
   Leu Pro Ile Thr Leu Ala Gln Leu Asp Ile Thr Glu Asp Ile Pro Thr
   305 310 315 320
   aag atg cgc gcc gtg gcc gag ctg gcc tgc gcc cca ggc gag acc atc 1008
   Lys Met Arg Ala Val Ala Glu Leu Ala Cys Ala Pro Gly Glu Thr Ile
   325 330 335
   cac aac atg ccc ggc ggc gtg acg gtg gag cag gtc tat ggc gcg ctg 1056
   His Asn Met Pro Gly Gly Val Thr Val Glu Gln Val Tyr Gly Ala Leu
   340 345 350
   ctg gtg gcg gac cag ctg ggg cag cat ttt ctg gag ttt tga 1098
   Leu Val Ala Asp Gln Leu Gly Gln His Phe Leu Glu Phe
   355 360 365
<210> 79
   <211> 365
   <212> PRT
   <213> Pseudomonas putida
<400> 79
   Met Asp Arg Ala Ile Gln Ser Pro Gly Lys Tyr Val Gln Gly Ala Asp
   1 5 10 15
   Ala Leu Gln Arg Leu Gly Asp Tyr Leu Lys Pro Leu Ala Asp Ser Trp
   20 25 30
   Leu Val Ile Ala Asp Lys Phe Val Leu Gly Phe Ala Glu Asp Thr Ile
   35 40 45
   Arg Gln Ser Leu Ser Lys Ala Gly Leu Ala Met Asp Ile Val Ala Phe
   50 55 60
   Asn Gly Glu Cys Ser Gln Gly Glu Val Asp Arg Leu Cys Gln Leu Ala
   65 70 75 80
   Thr Gln Asn Gly Arg Ser Ala Ile Val Gly Ile Gly Gly Gly Lys Thr
   85 90 95
   Leu Asp Thr Ala Lys Ala Val Ala Phe Phe Gln Lys Val Pro Val Ala
   100 105 110
   Val Ala Pro Thr Ile Ala Ser Thr Asp Ala Pro Cys Ser Ala Leu Ser
   115 120 125
   Val Leu Tyr Thr Asp Glu Gly Glu Phe Asp Arg Tyr Leu Met Leu Pro
   130 135 140
   Thr Asn Pro Ala Leu Val Val Val Asp Thr Ala Ile Val Ala Arg Ala
   145 150 155 160
   Pro Ala Arg Leu Leu Ala Ala Gly Ile Gly Asp Ala Leu Ala Thr Trp
   165 170 175
   Phe Glu Ala Arg Ala Ala Ser Arg Ser Ser Ala Ala Thr Met Ala Gly
   180 185 190
   Gly Pro Ala Thr Gln Thr Ala Leu Asn Leu Ala Arg Phe Cys Tyr Asp
   195 200 205
   Thr Leu Leu Glu Glu Gly Glu Lys Ala Met Leu Ala Val Gln Ala Gln
   210 215 220
   Val Val Thr Pro Ala Leu Glu Arg Ile Val Glu Ala Asn Thr Tyr Leu
   225 230 235 240
   Ser Gly Val Gly Phe Glu Ser Gly Gly Val Ala Ala Ala His Ala Val
   245 250 255
   His Asn Gly Leu Thr Ala Val Ala Glu Thr His His Phe Tyr His Gly
   260 265 270
   Glu Lys Val Ala Phe Gly Val Leu Val Gln Leu Ala Leu Glu Asn Ala
   275 280 285
   Ser Asn Ala Glu Met Gln Glu Val Met Ser Leu Cys His Ala Val Gly
           290                 295                 300
   Leu Pro Ile Thr Leu Ala Gln Leu Asp Ile Thr Glu Asp Ile Pro Thr
   305                 310                 315                 320
   Lys Met Arg Ala Val Ala Glu Leu Ala Cys Ala Pro Gly Glu Thr Ile
                       325                 330                 335
   His Asn Met Pro Gly Gly Val Thr Val Glu Gln Val Tyr Gly Ala Leu
               340                 345                 350
   Leu Val Ala Asp Gln Leu Gly Gln His Phe Leu Glu Phe
           355                 360                 365
<210> 80
   <211> 1104
   <212> DNA
   <213> Bacillus coagulans
<220>
   <221> CDS
   <222> (1).. (1104)
<400> 80
   atg acg aaa atc att acc tct cca agc aag ttt ata caa ggc ccc gat      48
   Met Thr Lys Ile Ile Thr Ser Pro Ser Lys Phe Ile Gln Gly Pro Asp
   1               5                   10                  15
   gaa ttg tcc agg ctt tcg gcg tat acg gaa agg ctt ggc aaa aaa gca      96
   Glu Leu Ser Arg Leu Ser Ala Tyr Thr Glu Arg Leu Gly Lys Lys Ala
               20                  25                  30
   ttt att att gcg gat gat ttt gtc acc ggc ctt gtc ggc aaa acg gtt     144
   Phe Ile Ile Ala Asp Asp Phe Val Thr Gly Leu Val Gly Lys Thr Val
           35                  40                  45
   gaa gaa agc tat gcc ggc aaa gaa acg ggg tat caa atg gca tta ttc     192
   Glu Glu Ser Tyr Ala Gly Lys Glu Thr Gly Tyr Gln Met Ala Leu Phe
       50                  55                  60
   ggt ggt gag tgt tct aaa ccg gaa atc gaa cgg ctt tgt gaa atg agc     240
   Gly Gly Glu Cys Ser Lys Pro Glu Ile Glu Arg Leu Cys Glu Met Ser
   65                  70                  75                  80
   aaa tcc gag gaa gcc gat gtc gtt gtc gga atc ggc ggc gga aaa aca     288
   Lys Ser Glu Glu Ala Asp Val Val Val Gly Ile Gly Gly Gly Lys Thr
                   85                  90                  95
   ttg gat acc gca aaa gca gtc ggg tat tac aat aac att ccg gtg att     336
   Leu Asp Thr Ala Lys Ala Val Gly Tyr Tyr Asn Asn Ile Pro Val Ile
               100                 105                 110
   gtc gcg ccg acc atc gct tcc acc gat gcc ccg aca agc gcc ctg tct     384
   Val Ala Pro Thr Ile Ala Ser Thr Asp Ala Pro Thr Ser Ala Leu Ser
           115                 120                 125
   gtt att tac aaa gag aac ggc gag ttt gaa gaa tac ttg atg ctg ccg     432
   Val Ile Tyr Lys Glu Asn Gly Glu Phe Glu Glu Tyr Leu Met Leu Pro
       130                 135                 140
   ctg aac ccg act ttt gtc att atg gat acg aaa gtg att gcc tct gcc     480
   Leu Asn Pro Thr Phe Val Ile Met Asp Thr Lys Val Ile Ala Ser Ala
   145                 150                 155                 160
   cct gcc cgc ctg ctc gtt tcc ggc atg gga gat gcg ctt gca acg tat     528
   Pro Ala Arg Leu Leu Val Ser Gly Met Gly Asp Ala Leu Ala Thr Tyr
                   165                 170                 175
   ttt gaa gcg cgc gcc act aag cgg gca aat aaa acg acg atg gca ggc     576
   Phe Glu Ala Arg Ala Thr Lys Arg Ala Asn Lys Thr Thr Met Ala Gly
               180                 185                 190
   ggg cgt gtt acg gaa gcg gcg atc gcg ctt gca aaa ctt tgt tat gac      624
   Gly Arg Val Thr Glu Ala Ala Ile Ala Leu Ala Lys Leu Cys Tyr Asp
           195                 200                 205
   acg caa att tcg gaa ggt tta aaa gca aaa ctg gca gcg gaa aaa cat      672
   Thr Gln Ile Ser Glu Gly Leu Lys Ala Lys Leu Ala Ala Glu Lys His
       210                 215                 220
   ctt gtt acg gaa gca gtg gaa aaa atc att gaa gcg aat acg tat ctg      720
   Leu Val Thr Glu Ala Val Glu Lys Ile Ile Glu Ala Asn Thr Tyr Leu
   225                 230                 235                 240
   agc gga atc ggt ttt gaa agc ggc ggc ctt gct gcg gca cat gcg atc      768
   Ser Gly Ile Gly Phe Glu Ser Gly Gly Leu Ala Ala Ala His Ala Ile
                   245                 250                 255
   cat aat ggg ctt acc gtg ctc gaa gaa acc cat cat atg tac cac ggc      816
   His Asn Gly Leu Thr Val Leu Glu Glu Thr His His Met Tyr His Gly
               260                 265                 270
   gaa aaa gtg gca ttc ggt acc ctc gcc cag ctg att ttg gaa gat gcg      864
   Glu Lys Val Ala Phe Gly Thr Leu Ala Gln Leu Ile Leu Glu Asp Ala
           275                 280                 285
   ccg aaa gcg gaa att gaa gag gtg gtc tcc ttc tgc ctg agt gtc gga      912
   Pro Lys Ala Glu Ile Glu Glu Val Val Ser Phe Cys Leu Ser Val Gly
       290                 295                 300
   ctt ccc gtc acg ctc ggg gat ttg ggc gtg aaa gaa ctg aat gag gaa      960
   Leu Pro Val Thr Leu Gly Asp Leu Gly Val Lys Glu Leu Asn Glu Glu
   305                 310                 315                 320
   aag ctc cga aaa gtg gct gaa ctt tcc tgt gcg gaa ggc gaa acg att     1008
   Lys Leu Arg Lys Val Ala Glu Leu Ser Cys Ala Glu Gly Glu Thr Ile
                   325                 330                 335
   tat aac atg ccg ttt gaa gtc acg cct gac ctt gtg tac gca gca atc     1056
   Tyr Asn Met Pro Phe Glu Val Thr Pro Asp Leu Val Tyr Ala Ala Ile
               340                 345                 350
   gtt acc gct gat tcc gtc ggg cgg tat tat aag gaa aaa tgg gca tga     1104
   Val Thr Ala Asp Ser Val Gly Arg Tyr Tyr Lys Glu Lys Trp Ala
           355                 360                 365
<210> 81
   <211> 367
   <212> PRT
   <213> Bacillus coagulans
<400> 81
   Met Thr Lys Ile Ile Thr Ser Pro Ser Lys Phe Ile Gln Gly Pro Asp
   1               5                   10                  15
   Glu Leu Ser Arg Leu Ser Ala Tyr Thr Glu Arg Leu Gly Lys Lys Ala
               20                  25                  30
   Phe Ile Ile Ala Asp Asp Phe Val Thr Gly Leu Val Gly Lys Thr Val
           35                  40                  45
   Glu Glu Ser Tyr Ala Gly Lys Glu Thr Gly Tyr Gln Met Ala Leu Phe
       50                  55                  60
   Gly Gly Glu Cys Ser Lys Pro Glu Ile Glu Arg Leu Cys Glu Met Ser
   65                  70                  75                  80
   Lys Ser Glu Glu Ala Asp Val Val Val Gly Ile Gly Gly Gly Lys Thr
                   85                  90                  95
   Leu Asp Thr Ala Lys Ala Val Gly Tyr Tyr Asn Asn Ile Pro Val Ile
   100 105 110
   Val Ala Pro Thr Ile Ala Ser Thr Asp Ala Pro Thr Ser Ala Leu Ser
   115 120 125
   Val Ile Tyr Lys Glu Asn Gly Glu Phe Glu Glu Tyr Leu Met Leu Pro
   130 135 140
   Leu Asn Pro Thr Phe Val Ile Met Asp Thr Lys Val Ile Ala Ser Ala
   145 150 155 160
   Pro Ala Arg Leu Leu Val Ser Gly Met Gly Asp Ala Leu Ala Thr Tyr
   165 170 175
   Phe Glu Ala Arg Ala Thr Lys Arg Ala Asn Lys Thr Thr Met Ala Gly
   180 185 190
   Gly Arg Val Thr Glu Ala Ala Ile Ala Leu Ala Lys Leu Cys Tyr Asp
   195 200 205
   Thr Gln Ile Ser Glu Gly Leu Lys Ala Lys Leu Ala Ala Glu Lys His
   210 215 220
   Leu Val Thr Glu Ala Val Glu Lys Ile Ile Glu Ala Asn Thr Tyr Leu
   225 230 235 240
   Ser Gly Ile Gly Phe Glu Ser Gly Gly Leu Ala Ala Ala His Ala Ile
   245 250 255
   His Asn Gly Leu Thr Val Leu Glu Glu Thr His His Met Tyr His Gly
   260 265 270
   Glu Lys Val Ala Phe Gly Thr Leu Ala Gln Leu Ile Leu Glu Asp Ala
   275 280 285
   Pro Lys Ala Glu Ile Glu Glu Val Val Ser Phe Cys Leu Ser Val Gly
   290 295 300
   Leu Pro Val Thr Leu Gly Asp Leu Gly Val Lys Glu Leu Asn Glu Glu
   305 310 315 320
   Lys Leu Arg Lys Val Ala Glu Leu Ser Cys Ala Glu Gly Glu Thr Ile
   325 330 335
   Tyr Asn Met Pro Phe Glu Val Thr Pro Asp Leu Val Tyr Ala Ala Ile
   340 345 350
   Val Thr Ala Asp Ser Val Gly Arg Tyr Tyr Lys Glu Lys Trp Ala
   355 360 365
<210> 82
   <211> 999
   <212> DNA
   <213> Escherichia coli
<220>
   <221> CDS
   <222> (1).. (999)
<400> 82
   atg aaa acg tta ggt gaa ttt att gtc gaa aag cag cac gag ttt tct 48
   Met Lys Thr Leu Gly Glu Phe Ile Val Glu Lys Gln His Glu Phe Ser
   1 5 10 15
   cat gct acc ggt gag ctc act gct ttg ctg tcg gca ata aaa ctg ggc 96
   His Ala Thr Gly Glu Leu Thr Ala Leu Leu Ser Ala Ile Lys Leu Gly
   20 25 30
   gcc aag att atc cat cgc gat atc aac aaa gca gga ctg gtt gat atc 144
   Ala Lys Ile Ile His Arg Asp Ile Asn Lys Ala Gly Leu Val Asp Ile
   35 40 45
   ctg ggt gcc agc ggt gct gag aac gtg cag ggc gag gtt cag cag aaa      192
   Leu Gly Ala Ser Gly Ala Glu Asn Val Gln Gly Glu Val Gln Gln Lys
       50                  55                  60
   ctc gac ttg ttc gct aat gaa aaa ctg aaa gcc gca ctg aaa gca cgc      240
   Leu Asp Leu Phe Ala Asn Glu Lys Leu Lys Ala Ala Leu Lys Ala Arg
   65                  70                  75                  80
   gat atc gtt gcg ggc att gcc tct gaa gaa gaa gat gag att gtc gtc      288
   Asp Ile Val Ala Gly Ile Ala Ser Glu Glu Glu Asp Glu Ile Val Val
                   85                  90                  95
   ttt gaa ggc tgt gaa cac gca aaa tac gtg gtg ctg atg gac ccc ctg      336
   Phe Glu Gly Cys Glu His Ala Lys Tyr Val Val Leu Met Asp Pro Leu
               100                 105                 110
   gat ggc tcg tcc aac atc gat gtt aac gtc tct gtc ggt acc att ttc      384
   Asp Gly Ser Ser Asn Ile Asp Val Asn Val Ser Val Gly Thr Ile Phe
           115                 120                 125
   tcc atc tac cgc cgc gtt acg cct gtt ggc acg ccg gta acg gaa gaa      432
   Ser Ile Tyr Arg Arg Val Thr Pro Val Gly Thr Pro Val Thr Glu Glu
       130                 135                 140
   gat ttc ctc cag cct ggt aac aaa cag gtt gcg gca ggt tac gtg gta      480
   Asp Phe Leu Gln Pro Gly Asn Lys Gln Val Ala Ala Gly Tyr Val Val
   145                 150                 155                 160
   tac ggc tcc tct acc atg ctg gtt tac acc acc gga tgc ggt gtt cac      528
   Tyr Gly Ser Ser Thr Met Leu Val Tyr Thr Thr Gly Cys Gly Val His
                   165                 170                 175
   gcc ttt act tac gat cct tcg ctc ggc gtt ttc tgc ctg tgc cag gaa      576
   Ala Phe Thr Tyr Asp Pro Ser Leu Gly Val Phe Cys Leu Cys Gln Glu
               180                 185                 190
   cgg atg cgc ttc ccg gag aaa ggc aaa acc tac tcc atc aac gaa gga      624
   Arg Met Arg Phe Pro Glu Lys Gly Lys Thr Tyr Ser Ile Asn Glu Gly
           195                 200                 205
   aac tac att aag ttt ccg aac ggg gtg aag aag tac att aaa ttc tgc      672
   Asn Tyr Ile Lys Phe Pro Asn Gly Val Lys Lys Tyr Ile Lys Phe Cys
       210                 215                 220
   cag gaa gaa gat aaa tcc acc aac cgc cct tat acc tca cgt tat atc      720
   Gln Glu Glu Asp Lys Ser Thr Asn Arg Pro Tyr Thr Ser Arg Tyr Ile
   225                 230                 235                 240
   ggt tca ctg gtc gcg gat ttc cac cgt aac ctg ctg aaa ggc ggt att      768
   Gly Ser Leu Val Ala Asp Phe His Arg Asn Leu Leu Lys Gly Gly Ile
                   245                 250                 255
   tat ctc tac cca agc acc gcc agc cac ccg gac ggc aaa ctg cgt ttg      816
   Tyr Leu Tyr Pro Ser Thr Ala Ser His Pro Asp Gly Lys Leu Arg Leu
               260                 265                 270
   ctg tat gag tgc aac ccg atg gca ttc ctg gcg gaa caa gcg ggc ggt      864
   Leu Tyr Glu Cys Asn Pro Met Ala Phe Leu Ala Glu Gln Ala Gly Gly
           275                 280                 285
   aaa gcg agc gat ggc aaa gag cgt att ctg gat atc atc ccg gaa acc      912
   Lys Ala Ser Asp Gly Lys Glu Arg Ile Leu Asp Ile Ile Pro Glu Thr
       290                 295                 300
   ctg cac cag cgc cgt tca ttc ttt gtc ggc aac gac cat atg gtt gaa      960
   Leu His Gln Arg Arg Ser Phe Phe Val Gly Asn Asp His Met Val Glu
   305                 310                 315                 320
   gat gtc gaa cgc ttt atc cgt gag ttc ccg gac gcg taa                  999
   Asp Val Glu Arg Phe Ile Arg Glu Phe Pro Asp Ala
                   325                 330
<210> 83
   <211> 332
   <212> PRT
   <213> Escherichia coli
<400> 83
   Met Lys Thr Leu Gly Glu Phe Ile Val Glu Lys Gln His Glu Phe Ser
   1 5 10 15
   His Ala Thr Gly Glu Leu Thr Ala Leu Leu Ser Ala Ile Lys Leu Gly
   20 25 30
   Ala Lys Ile Ile His Arg Asp Ile Asn Lys Ala Gly Leu Val Asp Ile
   35 40 45
   Leu Gly Ala Ser Gly Ala Glu Asn Val Gln Gly Glu Val Gln Gln Lys
   50 55 60
   Leu Asp Leu Phe Ala Asn Glu Lys Leu Lys Ala Ala Leu Lys Ala Arg
   65 70 75 80
   Asp Ile Val Ala Gly Ile Ala Ser Glu Glu Glu Asp Glu Ile Val Val
   85 90 95
   Phe Glu Gly Cys Glu His Ala Lys Tyr Val Val Leu Met Asp Pro Leu
   100 105 110
   Asp Gly Ser Ser Asn Ile Asp Val Asn Val Ser Val Gly Thr Ile Phe
   115 120 125
   Ser Ile Tyr Arg Arg Val Thr Pro Val Gly Thr Pro Val Thr Glu Glu
   130 135 140
   Asp Phe Leu Gln Pro Gly Asn Lys Gln Val Ala Ala Gly Tyr Val Val
   145 150 155 160
   Tyr Gly Ser Ser Thr Met Leu Val Tyr Thr Thr Gly Cys Gly Val His
   165 170 175
   Ala Phe Thr Tyr Asp Pro Ser Leu Gly Val Phe Cys Leu Cys Gln Glu
   180 185 190
   Arg Met Arg Phe Pro Glu Lys Gly Lys Thr Tyr Ser Ile Asn Glu Gly
   195 200 205
   Asn Tyr Ile Lys Phe Pro Asn Gly Val Lys Lys Tyr Ile Lys Phe Cys
   210 215 220
   Gln Glu Glu Asp Lys Ser Thr Asn Arg Pro Tyr Thr Ser Arg Tyr Ile
   225 230 235 240
   Gly Ser Leu Val Ala Asp Phe His Arg Asn Leu Leu Lys Gly Gly Ile
   245 250 255
   Tyr Leu Tyr Pro Ser Thr Ala Ser His Pro Asp Gly Lys Leu Arg Leu
   260 265 270
   Leu Tyr Glu Cys Asn Pro Met Ala Phe Leu Ala Glu Gln Ala Gly Gly
   275 280 285
   Lys Ala Ser Asp Gly Lys Glu Arg Ile Leu Asp Ile Ile Pro Glu Thr
   290 295 300
   Leu His Gln Arg Arg Ser Phe Phe Val Gly Asn Asp His Met Val Glu
   305 310 315 320
   Asp Val Glu Arg Phe Ile Arg Glu Phe Pro Asp Ala
   325 330
<210> 84
   <211> 819
   <212> DNA
   <213> Escherichia coli
<220>
   <221> CDS
   <222> (1).. (819)
<400> 84
   atg acc aca cgc gtg att gct ctc gac tta gac ggc acc tta ttg acc      48
   Met Thr Thr Arg Val Ile Ala Leu Asp Leu Asp Gly Thr Leu Leu Thr
   1               5                   10                  15
   ccg aaa aag acc ctg ctt cct tca tcg ata gaa gcc ctg gcc cgc gct      96
   Pro Lys Lys Thr Leu Leu Pro Ser Ser Ile Glu Ala Leu Ala Arg Ala
               20                  25                  30
   cgc gaa gca ggc tat caa tta atc atc gtc aca ggt cgc cat cac gtc     144
   Arg Glu Ala Gly Tyr Gln Leu Ile Ile Val Thr Gly Arg His His Val
           35                  40                  45
   gct att cat cct ttt tat cag gcg ctg gcg ctg gat aca cct gct att     192
   Ala Ile His Pro Phe Tyr Gln Ala Leu Ala Leu Asp Thr Pro Ala Ile
       50                  55                  60
   tgc tgt aat ggc acc tat ttg tat gat tat cat gca aaa acc gtg ctg     240
   Cys Cys Asn Gly Thr Tyr Leu Tyr Asp Tyr His Ala Lys Thr Val Leu
   65                  70                  75                  80
   gaa gcg gac cca atg ccc gtt att aaa gcc ctg caa ctc att gag atg     288
   Glu Ala Asp Pro Met Pro Val Ile Lys Ala Leu Gln Leu Ile Glu Met
                   85                  90                  95
   ctg aat gaa cac cac att cac ggt ctg atg tat gtc gat gat gca atg     336
   Leu Asn Glu His His Ile His Gly Leu Met Tyr Val Asp Asp Ala Met
               100                 105                 110
   gtc tat gag cac ccg acc ggg cat gtc att cgc aca tct aac tgg gcg     384
   Val Tyr Glu His Pro Thr Gly His Val Ile Arg Thr Ser Asn Trp Ala
           115                 120                 125
   caa acc ctg ccg ccg gaa cag cgt ccg act ttc aca caa gtc gct tct     432
   Gln Thr Leu Pro Pro Glu Gln Arg Pro Thr Phe Thr Gln Val Ala Ser
       130                 135                 140
   ctg gct gaa acg gcg caa caa gtt aac gcc gta tgg aag ttc gcc ctg     480
   Leu Ala Glu Thr Ala Gln Gln Val Asn Ala Val Trp Lys Phe Ala Leu
   145                 150                 155                 160
   acg cac gat gac ctg ccg caa ttg cag cat ttt ggt aag cat gtc gaa     528
   Thr His Asp Asp Leu Pro Gln Leu Gln His Phe Gly Lys His Val Glu
                   165                 170                 175
   cat gaa ctg gga ctg gag tgt gaa tgg tcc tgg cac gat cag gtt gat     576
   His Glu Leu Gly Leu Glu Cys Glu Trp Ser Trp His Asp Gln Val Asp
               180                 185                 190
   att gca cgc ggc ggc aac agc aaa ggt aaa cgt ttg acg aaa tgg gtt     624
   Ile Ala Arg Gly Gly Asn Ser Lys Gly Lys Arg Leu Thr Lys Trp Val
           195                 200                 205
   gag gcg caa ggt tgg tcg atg gaa aac gtc gtg gca ttc ggc gat aac     672
   Glu Ala Gln Gly Trp Ser Met Glu Asn Val Val Ala Phe Gly Asp Asn
       210                 215                 220
   ttt aat gat atc agt atg ctg gaa gcc gct ggt aca ggc gtg gcg atg     720
   Phe Asn Asp Ile Ser Met Leu Glu Ala Ala Gly Thr Gly Val Ala Met
   225                 230                 235                 240
   ggc aac gcc gat gac gcg gta aaa gcg cgc gcc aac att gtg att ggt     768
   Gly Asn Ala Asp Asp Ala Val Lys Ala Arg Ala Asn Ile Val Ile Gly
                   245                 250                 255
   gat aac acc acc gac agc att gcc cag ttc att tat agc cac ctg att     816
   Asp Asn Thr Thr Asp Ser Ile Ala Gln Phe Ile Tyr Ser His Leu Ile
               260                 265                 270
   taa                                                                 819
<210> 85
   <211> 272
   <212> PRT
   <213> Escherichia coli
<400> 85
   Met Thr Thr Arg Val Ile Ala Leu Asp Leu Asp Gly Thr Leu Leu Thr
   1               5                   10                  15
   Pro Lys Lys Thr Leu Leu Pro Ser Ser Ile Glu Ala Leu Ala Arg Ala
               20                  25                  30
   Arg Glu Ala Gly Tyr Gln Leu Ile Ile Val Thr Gly Arg His His Val
           35                  40                  45
   Ala Ile His Pro Phe Tyr Gln Ala Leu Ala Leu Asp Thr Pro Ala Ile
       50                  55                  60
   Cys Cys Asn Gly Thr Tyr Leu Tyr Asp Tyr His Ala Lys Thr Val Leu
   65                  70                  75                  80
   Glu Ala Asp Pro Met Pro Val Ile Lys Ala Leu Gln Leu Ile Glu Met
                   85                  90                  95
   Leu Asn Glu His His Ile His Gly Leu Met Tyr Val Asp Asp Ala Met
               100                 105                 110
   Val Tyr Glu His Pro Thr Gly His Val Ile Arg Thr Ser Asn Trp Ala
           115                 120                 125
   Gln Thr Leu Pro Pro Glu Gln Arg Pro Thr Phe Thr Gln Val Ala Ser
       130                 135                 140
   Leu Ala Glu Thr Ala Gln Gln Val Asn Ala Val Trp Lys Phe Ala Leu
   145                 150                 155                 160
   Thr His Asp Asp Leu Pro Gln Leu Gln His Phe Gly Lys His Val Glu
                   165                 170                 175
   His Glu Leu Gly Leu Glu Cys Glu Trp Ser Trp His Asp Gln Val Asp
               180                 185                 190
   Ile Ala Arg Gly Gly Asn Ser Lys Gly Lys Arg Leu Thr Lys Trp Val
           195                 200                 205
   Glu Ala Gln Gly Trp Ser Met Glu Asn Val Val Ala Phe Gly Asp Asn
       210                 215                 220
   Phe Asn Asp Ile Ser Met Leu Glu Ala Ala Gly Thr Gly Val Ala Met
   225                 230                 235                 240
   Gly Asn Ala Asp Asp Ala Val Lys Ala Arg Ala Asn Ile Val Ile Gly
                   245                 250                 255
   Asp Asn Thr Thr Asp Ser Ile Ala Gln Phe Ile Tyr Ser His Leu Ile
               260                 265                 270
<210> 86
   <211> 1728
   <212> DNA
   <213> Escherichia coli
<220>
   <221> CDS
   <222> (1).. (1728)
<400> 86
   atg att tca ggc att tta gca tcc ccg ggt atc gct ttc ggt aaa gct       48
   Met Ile Ser Gly Ile Leu Ala Ser Pro Gly Ile Ala Phe Gly Lys Ala
   1               5                   10                  15
   ctg ctt ctg aaa gaa gac gaa att gtc att gac cgg aaa aaa att tct       96
   Leu Leu Leu Lys Glu Asp Glu Ile Val Ile Asp Arg Lys Lys Ile Ser
               20                  25                  30
   gcc gac cag gtt gat cag gaa gtt gaa cgt ttt ctg agc ggt cgt gcc      144
   Ala Asp Gln Val Asp Gln Glu Val Glu Arg Phe Leu Ser Gly Arg Ala
           35                  40                  45
   aag gca tca gcc cag ctg gaa acg atc aaa acg aaa gct ggt gaa acg      192
   Lys Ala Ser Ala Gln Leu Glu Thr Ile Lys Thr Lys Ala Gly Glu Thr
       50                  55                  60
   ttc ggt gaa gaa aaa gaa gcc atc ttt gaa ggg cat att atg ctg ctc      240
   Phe Gly Glu Glu Lys Glu Ala Ile Phe Glu Gly His Ile Met Leu Leu
   65                  70                  75                  80
   gaa gat gag gag ctg gag cag gaa atc ata gcc ctg att aaa gat aag      288
   Glu Asp Glu Glu Leu Glu Gln Glu Ile Ile Ala Leu Ile Lys Asp Lys
                   85                  90                  95
   cac atg aca gct gac gca gct gct cat gaa gtt atc gaa ggt cag gct      336
   His Met Thr Ala Asp Ala Ala Ala His Glu Val Ile Glu Gly Gln Ala
               100                 105                 110
   tct gcc ctg gaa gag ctg gat gat gaa tac ctg aaa gaa cgt gcg gct      384
   Ser Ala Leu Glu Glu Leu Asp Asp Glu Tyr Leu Lys Glu Arg Ala Ala
           115                 120                 125
   gac gta cgt gat atc ggt aag cgc ctg ctg cgc aac atc ctg ggc ctg      432
   Asp Val Arg Asp Ile Gly Lys Arg Leu Leu Arg Asn Ile Leu Gly Leu
       130                 135                 140
   aag att atc gac ctg agc gcc att cag gat gaa gtc att ctg gtt gcc      480
   Lys Ile Ile Asp Leu Ser Ala Ile Gln Asp Glu Val Ile Leu Val Ala
   145                 150                 155                 160
   gct gac ctg acg ccg tcc gaa acc gca cag ctg aac ctg aag aag gtg      528
   Ala Asp Leu Thr Pro Ser Glu Thr Ala Gln Leu Asn Leu Lys Lys Val
                   165                 170                 175
   ctg ggt ttc atc acc gac gcg ggt ggc cgt act tcc cac acc tct atc      576
   Leu Gly Phe Ile Thr Asp Ala Gly Gly Arg Thr Ser His Thr Ser Ile
               180                 185                 190
   atg gcg cgt tct ctg gaa cta cct gct atc gtg ggt acc ggt agc gtc      624
   Met Ala Arg Ser Leu Glu Leu Pro Ala Ile Val Gly Thr Gly Ser Val
           195                 200                 205
   acc tct cag gtg aaa aat gac gac tat ctg att ctg gat gcc gta aat      672
   Thr Ser Gln Val Lys Asn Asp Asp Tyr Leu Ile Leu Asp Ala Val Asn
       210                 215                 220
   aat cag gtt tac gtc aat cca acc aac gaa gtt att gat aaa atg cgc      720
   Asn Gln Val Tyr Val Asn Pro Thr Asn Glu Val Ile Asp Lys Met Arg
   225                 230                 235                 240
   gct gtt cag gag caa gtg gct tct gaa aaa gca gag ctt gct aaa ctg      768
   Ala Val Gln Glu Gln Val Ala Ser Glu Lys Ala Glu Leu Ala Lys Leu
                   245                 250                 255
   aaa gat ctg cca gct att acg ctg gac ggt cac cag gta gaa gta tgc      816
   Lys Asp Leu Pro Ala Ile Thr Leu Asp Gly His Gln Val Glu Val Cys
               260                 265                 270
   gct aac att ggt acg gtt cgt gac gtt gaa ggt gca gag cgt aac ggc      864
   Ala Asn Ile Gly Thr Val Arg Asp Val Glu Gly Ala Glu Arg Asn Gly
               275                 280                 285
   gct gaa ggc gtt ggt ctg tat cgt act gag ttc ctg ttc atg gac cgc      912
   Ala Glu Gly Val Gly Leu Tyr Arg Thr Glu Phe Leu Phe Met Asp Arg
       290                 295                 300
   gac gca ctg ccc act gaa gaa gaa cag ttt gct gct tac aaa gca gtg      960
   Asp Ala Leu Pro Thr Glu Glu Glu Gln Phe Ala Ala Tyr Lys Ala Val
   305                 310                 315                 320
   gct gaa gcg tgt ggc tcg caa gcg gtt atc gtt cgt acc atg gac atc     1008
   Ala Glu Ala Cys Gly Ser Gln Ala Val Ile Val Arg Thr Met Asp Ile
                   325                 330                 335
   ggc ggc gac aaa gag ctg cca tac atg aac ttc ccg aaa gaa gag aac     1056
   Gly Gly Asp Lys Glu Leu Pro Tyr Met Asn Phe Pro Lys Glu Glu Asn
               340                 345                 350
   ccg ttc ctc ggc tgg cgc gct atc cgt atc gcg atg gat cgt aga gag     1104
   Pro Phe Leu Gly Trp Arg Ala Ile Arg Ile Ala Met Asp Arg Arg Glu
           355                 360                 365
   atc ctg cgc gat cag ctc cgc gct atc ctg cgt gcc tcg gct ttc ggt     1152
   Ile Leu Arg Asp Gln Leu Arg Ala Ile Leu Arg Ala Ser Ala Phe Gly
       370                 375                 380
   aaa ttg cgc att atg ttc ccg atg atc atc tct gtt gaa gaa gtg cgt     1200
   Lys Leu Arg Ile Met Phe Pro Met Ile Ile Ser Val Glu Glu Val Arg
   385                 390                 395                 400
   gca ctg cgc aaa gag atc gaa atc tac aaa cag gaa ctg cgc gac gaa     1248
   Ala Leu Arg Lys Glu Ile Glu Ile Tyr Lys Gln Glu Leu Arg Asp Glu
                   405                 410                 415
   ggt aaa gcg ttt gac gag tca att gaa atc ggc gta atg gtg gaa aca     1296
   Gly Lys Ala Phe Asp Glu Ser Ile Glu Ile Gly Val Met Val Glu Thr
               420                 425                 430
   ccg gct gcc gca aca att gca cgt cat tta gcc aaa gaa gtt gat ttc     1344
   Pro Ala Ala Ala Thr Ile Ala Arg His Leu Ala Lys Glu Val Asp Phe
           435                 440                 445
   ttt agt atc ggc acc aat gat tta acg cag tac act ctg gca gtt gac     1392
   Phe Ser Ile Gly Thr Asn Asp Leu Thr Gln Tyr Thr Leu Ala Val Asp
       450                 455                 460
   cgt ggt aat gat atg att tca cac ctt tac cag cca atg tca ccg tcc     1440
   Arg Gly Asn Asp Met Ile Ser His Leu Tyr Gln Pro Met Ser Pro Ser
   465                 470                 475                 480
   gtg ctg aac ttg atc aag caa gtt att gat gct tct cat gct gaa ggc     1488
   Val Leu Asn Leu Ile Lys Gln Val Ile Asp Ala Ser His Ala Glu Gly
                   485                 490                 495
   aaa tgg act ggc atg tgt ggt gag ctt gct ggc gat gaa cgt gct aca     1536
   Lys Trp Thr Gly Met Cys Gly Glu Leu Ala Gly Asp Glu Arg Ala Thr
               500                 505                 510
   ctt ctg ttg ctg ggg atg ggt ctg gac gaa ttc tct atg agc gcc att     1584
   Leu Leu Leu Leu Gly Met Gly Leu Asp Glu Phe Ser Met Ser Ala Ile
           515                 520                 525
   tct atc ccg cgc att aag aag att atc cgt aac acg aac ttc gaa gat     1632
   Ser Ile Pro Arg Ile Lys Lys Ile Ile Arg Asn Thr Asn Phe Glu Asp
       530                 535                 540
   gcg aag gtg tta gca gag cag gct ctt gct caa ccg aca acg gac gag     1680
   Ala Lys Val Leu Ala Glu Gln Ala Leu Ala Gln Pro Thr Thr Asp Glu
   545                 550                 555                 560
   tta atg acg ctg gtt aac aag ttc att gaa gaa aaa aca atc tgc taa     1728
   Leu Met Thr Leu Val Asn Lys Phe Ile Glu Glu Lys Thr Ile Cys
                   565                 570                 575
<210> 87
   <211> 575
   <212> PRT
   <213> Escherichia coli
<400> 87
   Met Ile Ser Gly Ile Leu Ala Ser Pro Gly Ile Ala Phe Gly Lys Ala
   1               5                   10                  15
   Leu Leu Leu Lys Glu Asp Glu Ile Val Ile Asp Arg Lys Lys Ile Ser
               20                  25                  30
   Ala Asp Gln Val Asp Gln Glu Val Glu Arg Phe Leu Ser Gly Arg Ala
           35                  40                  45
   Lys Ala Ser Ala Gln Leu Glu Thr Ile Lys Thr Lys Ala Gly Glu Thr
       50                  55                  60
   Phe Gly Glu Glu Lys Glu Ala Ile Phe Glu Gly His Ile Met Leu Leu
   65                  70                  75                  80
   Glu Asp Glu Glu Leu Glu Gln Glu Ile Ile Ala Leu Ile Lys Asp Lys
                   85                  90                  95
   His Met Thr Ala Asp Ala Ala Ala His Glu Val Ile Glu Gly Gln Ala
               100                 105                 110
   Ser Ala Leu Glu Glu Leu Asp Asp Glu Tyr Leu Lys Glu Arg Ala Ala
           115                 120                 125
   Asp Val Arg Asp Ile Gly Lys Arg Leu Leu Arg Asn Ile Leu Gly Leu
       130                 135                 140
   Lys Ile Ile Asp Leu Ser Ala Ile Gln Asp Glu Val Ile Leu Val Ala
   145                 150                 155                 160
   Ala Asp Leu Thr Pro Ser Glu Thr Ala Gln Leu Asn Leu Lys Lys Val
                   165                 170                 175
   Leu Gly Phe Ile Thr Asp Ala Gly Gly Arg Thr Ser His Thr Ser Ile
               180                 185                 190
   Met Ala Arg Ser Leu Glu Leu Pro Ala Ile Val Gly Thr Gly Ser Val
           195                 200                 205
   Thr Ser Gln Val Lys Asn Asp Asp Tyr Leu Ile Leu Asp Ala Val Asn
       210                 215                 220
   Asn Gln Val Tyr Val Asn Pro Thr Asn Glu Val Ile Asp Lys Met Arg
   225                 230                 235                 240
   Ala Val Gln Glu Gln Val Ala Ser Glu Lys Ala Glu Leu Ala Lys Leu
                   245                 250                 255
   Lys Asp Leu Pro Ala Ile Thr Leu Asp Gly His Gln Val Glu Val Cys
               260                 265                 270
   Ala Asn Ile Gly Thr Val Arg Asp Val Glu Gly Ala Glu Arg Asn Gly
           275                 280                 285
   Ala Glu Gly Val Gly Leu Tyr Arg Thr Glu Phe Leu Phe Met Asp Arg
       290                 295                 300
   Asp Ala Leu Pro Thr Glu Glu Glu Gln Phe Ala Ala Tyr Lys Ala Val
   305                 310                 315                 320
   Ala Glu Ala Cys Gly Ser Gln Ala Val Ile Val Arg Thr Met Asp Ile
                   325                 330                 335
   Gly Gly Asp Lys Glu Leu Pro Tyr Met Asn Phe Pro Lys Glu Glu Asn
               340                 345                 350
   Pro Phe Leu Gly Trp Arg Ala Ile Arg Ile Ala Met Asp Arg Arg Glu
           355                 360                 365
   Ile Leu Arg Asp Gln Leu Arg Ala Ile Leu Arg Ala Ser Ala Phe Gly
       370                 375                 380
   Lys Leu Arg Ile Met Phe Pro Met Ile Ile Ser Val Glu Glu Val Arg
   385                 390                 395                 400
   Ala Leu Arg Lys Glu Ile Glu Ile Tyr Lys Gln Glu Leu Arg Asp Glu
                   405                 410                 415
   Gly Lys Ala Phe Asp Glu Ser Ile Glu Ile Gly Val Met Val Glu Thr
               420                 425                 430
   Pro Ala Ala Ala Thr Ile Ala Arg His Leu Ala Lys Glu Val Asp Phe
           435                 440                 445
   Phe Ser Ile Gly Thr Asn Asp Leu Thr Gln Tyr Thr Leu Ala Val Asp
       450                 455                 460
   Arg Gly Asn Asp Met Ile Ser His Leu Tyr Gln Pro Met Ser Pro Ser
   465                 470                 475                 480
   Val Leu Asn Leu Ile Lys Gln Val Ile Asp Ala Ser His Ala Glu Gly
                   485                 490                 495
   Lys Trp Thr Gly Met Cys Gly Glu Leu Ala Gly Asp Glu Arg Ala Thr
               500                 505                 510
   Leu Leu Leu Leu Gly Met Gly Leu Asp Glu Phe Ser Met Ser Ala Ile
           515                 520                 525
   Ser Ile Pro Arg Ile Lys Lys Ile Ile Arg Asn Thr Asn Phe Glu Asp
       530                 535                 540
   Ala Lys Val Leu Ala Glu Gln Ala Leu Ala Gln Pro Thr Thr Asp Glu
   545                 550                 555                 560
   Leu Met Thr Leu Val Asn Lys Phe Ile Glu Glu Lys Thr Ile Cys
                   565                 570                 575
<210> 88
   <211> 2903
   <212> DNA
   <213> Artificial sequence
<220>
   <223> PtacM fsaB-gldA::Cm
<400> 88
   tctagacgct caagttagta taaaaaagct gaacgagaaa cgtaaaatga tataaatatc      60
   aatatattaa attagatttt gcataaaaaa cagactacat aatactgtaa aacacaacat     120
   atgcagtcac tatgaatcaa ctacttagat ggtattagtg acctgtaaca gactgcagtg     180
   gtcgaaaaaa aaagcccgca ctgtcaggtg cgggcttttt tctgtgttaa gcttcgacga     240
   atttctgcca ttcatccgct tattatcact tattcaggcg tagcaccagg cgtttaaggg     300
   caccaataac tgccttaaaa aaattacgcc ccgccctgcc actcatcgca gtactgttgt     360
   aattcattaa gcattctgcc gacatggaag ccatcacaga cggcatgatg aacctgaatc     420
   gccagcggca tcagcacctt gtcgccttgc gtataatatt tgcccatggt gaaaacgggg     480
   gcgaagaagt tgtccatatt ggccacgttt aaatcaaaac tggtgaaact cacccaggga     540
   ttggctgaga cgaaaaacat attctcaata aaccctttag ggaaataggc caggttttca     600
   ccgtaacacg ccacatcttg cgaatatatg tgtagaaact gccggaaatc gtcgtggtat     660
   tcactccaga gcgatgaaaa cgtttcagtt tgctcatgga aaacggtgta acaagggtga     720
   acactatccc atatcaccag ctcaccgtct ttcattgcca tacggaattc cggatgagca     780
   ttcatcaggc gggcaagaat gtgaataaag gccggataaa acttgtgctt atttttcttt     840
   acggtcttta aaaaggccgt aatatccagc tgaacggtct ggttataggt acattgagca     900
   actgactgaa atgcctcaaa atgttcttta cgatgccatt gggatatatc aacggtggta     960
   tatccagtga tttttttctc cattttagct tccttagctc ctgaaaatct cggatccggc   1020
   caagctagct tggctctagc tagagcgccc ggttgacgct gctagtgtta cctagcgatt   1080
   tgtatcttac tgcatgttac ttcatgttgt caatacctgt ttttcgtgcg acttatcagg   1140
   ctgtctactt atccggagat ccacaggacg ggtgtggtcg ccatgatcgc gtagtcgata   1200
   gtggctccaa gtagcgaagc gagcaggact gggcggcggc caaagcggtc ggacagtgct   1260
   ccgagaacgg gtgcgcatag aaattgcatc aacgcatata gcgctagcag cacgccatag   1320
   tgactggcga tgctgtcgga atggacgata tcccgcaaga ggcccggcag taccggcata   1380
   accaagccta tgcctacagc atccagggtg acggtgccga ggatgacgat gagcgcattg   1440
   ttagatttca tacacggtgc ctgactgcgt tagcaattta actgtgataa actaccgcat   1500
   taaagcttat cgatgataag ctgtcaaaca tgagaattcg aaatcaaata atgattttat   1560
   tttgactgat agtgacctgt tcgttgcaac aaattgataa gcaatgcttt tttataatgc 1620
   caacttagta taaaaaagca ggcttcaaga tcttctctcc ccatccccct gttcacaatt 1680
   aatcatcggc tcgtataatg tgtggaattg tgagcggata acaatttcac acaggacatc   1740
   atggaactgt atctggacac cgctaacgtc gcagaagtcg aacgtctggc acgcatattc   1800
   cccattgccg gggtgacaac taacccgagc attatcgctg ccagcaagga gtccatatgg   1860
   gaagtgctgc cgcgtctgca aaaagcgatt ggtgatgagg gcattctgtt tgctcagacc   1920
   atgagccgcg acgcgcaggg gatggtggaa gaagcgaagc gcctgcgcga cgctattccg   1980
   ggtattgtgg tgaaaatccc ggtgacttcc gaaggtctgg cagcaattaa aatactgaaa   2040
   aaagagggta ttactacact tggcactgct gtatatagcg ccgcacaagg gttattagcc   2100
   gcactggcag gggcaaaata cgttgctccg tatgttaacc gcgtagatgc ccagggcgga   2160
   gacggcattc gtacggttca ggagctgcaa acgctgttag aaatgcacgc gccagaaagc   2220
   atggtgctgg cagccagctt taaaacgccg cgtcaggcgc tggactgttt actggcagga   2280
   tgtgaatcca tcaccctgcc cttagatgta gcgcaacaaa tgctcaacac ccctgcggta   2340
   gagtcagcta tagagaagtt cgaacacgac tggaatgccg catttggcac tactcatctc   2400
   taaaggagca attatggacc gcattattca atcaccgggt aaatacatcc agggcgctga   2460
   tgtgattaat cgtctgggcg aatacctgaa gccgctggca gaacgctggt tagtggtggg   2520
   tgacaaattt gttttaggtt ttgctcaatc cactgtcgag aaaagcttta aagatgctgg   2580
   actggtagta gaaattgcgc cgtttggcgg tgaatgttcg caaaatgaga tcgaccgtct   2640
   gcgtggcatc gcggagactg cgcagtgtgg cgcaattctc ggtatcggtg gcggaaaaac   2700
   cctcgatact gccaaagcac tggcacattt catgggtgtt ccggtagcga tcgcaccgac   2760
   tatcgcctct accgatgcac cgtgcagcgc attgtctgtt atctacaccg atgagggtga   2820
   gtttgaccgc tatctgctgt tgccaaataa cccgaatatg gtcattgtcg acaccaaaat   2880
   cgtcgctggc gcacctgcac gtc                                           2903
<210> 89
   <211> 79
   <212> DNA
   <213> Artificial sequence
<220>
   <223> atL-Ptac-fsaB
<400> 89
   tgcgtgccag acgttcgact tctgcgacgt tagcggtgtc cagatacagt tccatgatgt     60
   cctgtgtgaa attgttatc                                                  79
<210> 90
   <211> 77
   <212> DNA
   <213> Artificial sequence
<220>
   <223> atR-Ptac-fsaB
<400> 90
   aacgccgcct ctgccgacgc tatcgccagc ctgctgcaac atgaactgga actgtaaatc     60
   tagacgctca agttagt                                                    77
<210> 91
   <211> 2847
   <212> DNA
   <213> Artificial sequence
<220>
   <223> PtacM2gldA::Cm
<400> 91
   tctagacgct caagttagta taaaaaagct gaacgagaaa cgtaaaatga tataaatatc     60
   aatatattaa attagatttt gcataaaaaa cagactacat aatactgtaa aacacaacat    120
   atgcagtcac tatgaatcaa ctacttagat ggtattagtg acctgtaaca gactgcagtg    180
   gtcgaaaaaa aaagcccgca ctgtcaggtg cgggcttttt tctgtgttaa gcttcgacga    240
   atttctgcca ttcatccgct tattatcact tattcaggcg tagcaccagg cgtttaaggg    300
   caccaataac tgccttaaaa aaattacgcc ccgccctgcc actcatcgca gtactgttgt    360
   aattcattaa gcattctgcc gacatggaag ccatcacaga cggcatgatg aacctgaatc    420
   gccagcggca tcagcacctt gtcgccttgc gtataatatt tgcccatggt gaaaacgggg    480
   gcgaagaagt tgtccatatt ggccacgttt aaatcaaaac tggtgaaact cacccaggga    540
   ttggctgaga cgaaaaacat attctcaata aaccctttag ggaaataggc caggttttca    600
   ccgtaacacg ccacatcttg cgaatatatg tgtagaaact gccggaaatc gtcgtggtat    660
   tcactccaga gcgatgaaaa cgtttcagtt tgctcatgga aaacggtgta acaagggtga    720
   acactatccc atatcaccag ctcaccgtct ttcattgcca tacggaattc cggatgagca    780
   ttcatcaggc gggcaagaat gtgaataaag gccggataaa acttgtgctt atttttcttt    840
   acggtcttta aaaaggccgt aatatccagc tgaacggtct ggttataggt acattgagca    900
   actgactgaa atgcctcaaa atgttcttta cgatgccatt gggatatatc aacggtggta    960
   tatccagtga tttttttctc cattttagct tccttagctc ctgaaaatct cggatccggc   1020
   caagctagct tggctctagc tagagcgccc ggttgacgct gctagtgtta cctagcgatt   1080
   tgtatcttac tgcatgttac ttcatgttgt caatacctgt ttttcgtgcg acttatcagg   1140
   ctgtctactt atccggagat ccacaggacg ggtgtggtcg ccatgatcgc gtagtcgata   1200
   gtggctccaa gtagcgaagc gagcaggact gggcggcggc caaagcggtc ggacagtgct   1260
   ccgagaacgg gtgcgcatag aaattgcatc aacgcatata gcgctagcag cacgccatag   1320
   tgactggcga tgctgtcgga atggacgata tcccgcaaga ggcccggcag taccggcata   1380
   accaagccta tgcctacagc atccagggtg acggtgccga ggatgacgat gagcgcattg   1440
   ttagatttca tacacggtgc ctgactgcgt tagcaattta actgtgataa actaccgcat   1500
   taaagcttat cgatgataag ctgtcaaaca tgagaattcg aaatcaaata atgattttat   1560
   tttgactgat agtgacctgt tcgttgcaac aaattgataa gcaatgcttt tttataatgc   1620
   caacttagta taaaaaagca ggcttcaaga tcttctctcc ccatccccct gtgtacaatt   1680
   aatcatcggc tcgtataatg tgtggaattg tgagcggata acaatttcac acaggagact   1740
   gccatggacc gcattattca atcaccgggt aaatacatcc agggcgctga tgtgattaat   1800
   cgtctgggcg aatacctgaa gccgctggca gaacgctggt tagtggtggg tgacaaattt   1860
   gttttaggtt ttgctcaatc cactgtcgag aaaagcttta aagatgctgg actggtagta   1920
   gaaattgcgc cgtttggcgg tgaatgttcg caaaatgaga tcgaccgtct gcgtggcatc   1980
   gcggagactg cgcagtgtgg cgcaattctc ggtatcggtg gcggaaaaac cctcgatact   2040
   gccaaagcac tggcacattt catgggtgtt ccggtagcga tcgcaccgac tatcgcctct   2100
   accgatgcac cgtgcagcgc attgtctgtt atctacaccg atgagggtga gtttgaccgc   2160
   tatctgctgt tgccaaataa cccgaatatg gtcattgtcg acaccaaaat cgtcgctggc   2220
   gcacctgcac gtctgttagc ggcgggtatc ggcgatgcgc tggcaacctg gtttgaagcg   2280
   cgtgcctgct ctcgtagcgg cgcgaccacc atggcgggcg gcaagtgcac ccaggctgcg   2340
   ctggcactgg ctgaactgtg ctacaacacc ctgctggaag aaggcgaaaa agcgatgctt   2400
   gctgccgaac agcatgtagt gactccggcg ctggagcgcg tgattgaagc gaacacctat   2460
   ttgagcggtg ttggttttga aagtggtggt ctggctgcgg cgcacgcagt gcataacggc   2520
   ctgaccgcta tcccggacgc gcatcactat tatcacggtg aaaaagtggc attcggtacg   2580
   ctgacgcagc tggttctgga aaatgcgccg gtggaggaaa tcgaaaccgt agctgccctt 2640
   agccatgcgg taggtttgcc aataactctc gctcaactgg atattaaaga agatgtcccg 2700
   gcgaaaatgc gaattgtggc agaagcggca tgtgcagaag gtgaaaccat tcacaacatg 2760
   cctggcggcg cgacgccaga tcaggtttac gccgctctgc tggtagccga ccagtacggt 2820
   cagcgtttcc tgcaagagtg ggaataa 2847
<210> 92
   <211> 1740
   <212> DNA
   <213> Artificial sequence
<220>
   <223> attR-cat-attL-PtacM2-SD-spacer
<400> 92
   tctagacgct caagttagta taaaaaagct gaacgagaaa cgtaaaatga tataaatatc 60
   aatatattaa attagatttt gcataaaaaa cagactacat aatactgtaa aacacaacat 120
   atgcagtcac tatgaatcaa ctacttagat ggtattagtg acctgtaaca gactgcagtg 180
   gtcgaaaaaa aaagcccgca ctgtcaggtg cgggcttttt tctgtgttaa gcttcgacga 240
   atttctgcca ttcatccgct tattatcact tattcaggcg tagcaccagg cgtttaaggg 300
   caccaataac tgccttaaaa aaattacgcc ccgccctgcc actcatcgca gtactgttgt 360
   aattcattaa gcattctgcc gacatggaag ccatcacaga cggcatgatg aacctgaatc 420
   gccagcggca tcagcacctt gtcgccttgc gtataatatt tgcccatggt gaaaacgggg 480
   gcgaagaagt tgtccatatt ggccacgttt aaatcaaaac tggtgaaact cacccaggga 540
   ttggctgaga cgaaaaacat attctcaata aaccctttag ggaaataggc caggttttca 600
   ccgtaacacg ccacatcttg cgaatatatg tgtagaaact gccggaaatc gtcgtggtat 660
   tcactccaga gcgatgaaaa cgtttcagtt tgctcatgga aaacggtgta acaagggtga 720
   acactatccc atatcaccag ctcaccgtct ttcattgcca tacggaattc cggatgagca 780
   ttcatcaggc gggcaagaat gtgaataaag gccggataaa acttgtgctt atttttcttt 840
   acggtcttta aaaaggccgt aatatccagc tgaacggtct ggttataggt acattgagca 900
   actgactgaa atgcctcaaa atgttcttta cgatgccatt gggatatatc aacggtggta 960
   tatccagtga tttttttctc cattttagct tccttagctc ctgaaaatct cggatccggc 1020
   caagctagct tggctctagc tagagcgccc ggttgacgct gctagtgtta cctagcgatt 1080
   tgtatcttac tgcatgttac ttcatgttgt caatacctgt ttttcgtgcg acttatcagg 1140
   ctgtctactt atccggagat ccacaggacg ggtgtggtcg ccatgatcgc gtagtcgata 1200
   gtggctccaa gtagcgaagc gagcaggact gggcggcggc caaagcggtc ggacagtgct 1260
   ccgagaacgg gtgcgcatag aaattgcatc aacgcatata gcgctagcag cacgccatag 1320
   tgactggcga tgctgtcgga atggacgata tcccgcaaga ggcccggcag taccggcata 1380
   accaagccta tgcctacagc atccagggtg acggtgccga ggatgacgat gagcgcattg 1440
   ttagatttca tacacggtgc ctgactgcgt tagcaattta actgtgataa actaccgcat 1500
   taaagcttat cgatgataag ctgtcaaaca tgagaattcg aaatcaaata atgattttat 1560
   tttgactgat agtgacctgt tcgttgcaac aaattgataa gcaatgcttt tttataatgc 1620
   caacttagta taaaaaagca ggcttcaaga tctctcccca tccccctgtg tacaattaat 1680
   catcggctcg tataatgtgt ggaattgtga gcggataaca atttcacaca ggagactgcc 1740
<210> 93
   <211> 77
   <212> DNA
   <213> Artificial sequence
<220>
   <223> atL-ptac-gldA
<400> 93
   acatcagcgc cctggatgta tttacccggt gattgaataa tgcggtccat ggcagtctcc     60
   tgtgtgaaat tgttatc                                                    77
<210> 94
   <211> 77
   <212> DNA
   <213> Artificial sequence
<220>
   <223> atL-Ptac-fsaB1
<400> 94
   aacgccgcct ctgccgacgc tatcgccagc ctgctgcaac atgaactgga actgtaaatc     60
   tagacgctca agttagt                                                    77

## Description

### Technical field

The present invention relates to a method for producing an L-amino acid using a bacterium. More precisely, this method for producing an L-amino acid uses glycerol as a carbon source. L-Amino acids are industrially useful as additives for animal feed, components of health food, amino acid infusions, and so forth.

### Background Art

L-Amino acids are industrially produced by fermentation using microorganisms belonging to the genus *Brevibacterium, Corynebacterium, Escherichia,* and the like. In such production methods, strains can be used which are isolated from nature, or artificial variants of such strains can be used. Furthermore, microorganism strains can be used which are modified by a recombinant DNA technique so that the activity of a basic L-amino acid biosynthesis enzyme is increased, and so forth (EP 0643135 B, EP 0733712 B, EP 1477565 A, EP 0796912 A, EP 0837134 A, WO01/53459, EP 1170376 A, WO2005/010175, WO96/17930).

When L-amino acids are produced using microorganisms, sugars are generally used as a main component of the substrate, but glycerol can also be used as a substrate, similar to sugars (EP 1715055 A, EP 1715056 A).

It is known that *Escherichia coli* has a plurality of pathways which participate in the glycerol metabolism. One of these pathways starts with glycerol entering into the glycolysis system via glycerol-3-phosphate, and glycerol kinase and glycerol-3-phosphate dehydrogenase are responsible for the conversion. Furthermore, another pathway is known which startswith glycerol entering into the glycolysis system via dihydroxyacetone, and its first reaction is catalyzed by glycerol dehydrogenase.

It has been revealed that the major glycerol assimilation pathway of *E. coli* is the former pathway, since a mutant strain deficient in *glpK* (the gene coding for glycerol kinase) or *glpD* (the gene coding for glycerol-3-phosphate dehydrogenase), cannot grow in a medium containing glycerol as the sole carbon source (J. Bacteriol., 23, 8259-8271 (2006)).

Furthermore, it is known that if the glycerol dehydrogenase activity is increased when screening using a mutant strain deficient in *glpK, glpD* and *glpR,* which is a repressor gene of the *glp* regulon, the strain recovers from lethality in a medium containing glycerol as a sole carbon source (J. Bacteriol., 131, 1026-1028 (1977)).

To date, the pathway via glycerol-3-phosphate has been thought to be the main glycerol assimilation pathway of microorganisms belonging to the family *Enterobacteriaceae* as described above, and the glycerol assimilation pathway via dihydroxyacetone has been thought to be an unnecessary pathway for glycerol assimilation of microorganisms belonging to the family *Enterobacteriaceae.*

It has been considered that, in a conventional L-amino acid production culture using a sugar as the carbon source, it is desirable to keep the concentration of the sugar low (WO05/14841, WO05/14842, WO05/014843). Therefore, sugar concentration controlling apparatuses for performing culture at a low concentration of sugar and so forth have been developed (US 5912113 B). Furthermore, although it is known that the Km value of glycerol dehydrogenase for glycerol is high (J. Biol. Chem., 259 (4), 2124-2129), the influence of glycerol concentration in the culture medium on substance production using glycerol as a carbon source has not been examined.

The patent document WO 2008/102861 A1 discloses a method for producing an L-amino acid comprising:
A) culturing a bacterium, which belongs to the family Enterobacteriaceae and has an L-amino acid-producing ability, in a medium containing glycerol to cause accumulation of the L-amino acid in the medium, and
B) collecting the L-amino acid from the medium,
wherein the culturing may be performed as a batch culture, a fed-batch culture or a continuous culture, and
wherein glycerol contained in the medium can be contained in the starting medium, the feed medium, or both, wherein the feed medium used for the fed-batch culture is preferably a medium containing glycerol, and the glycerol concentration in the fermentation medium can be controlled to be at predetermined concentrations or lower.

However, the document is silent about the level of such a predetermined concentration.

### DISCLOSURE OF THE INVENTION

An aspect of the present invention is to provide a method for producing an L-amino acid by fermentation using a substrate containing glycerol, which is improved as compared to conventional techniques.

The inventors of the present invention found that if a culture was performed with controlling glycerol concentration in the medium to be 5 g/L or higher, the productivity of L-amino acids improved.

It is an aspect of the present invention to provide a method for producing an L-amino acid comprising culturing a bacterium which belongs to the family *Enterobacteriaceae* and has an L-amino acid-producing ability, in a fermentation medium containing glycerol to cause accumulation of the L-amino acid in the medium, and collecting the L-amino acid from the medium, wherein the culturing is performed as a fed-batch culture or a continuous culture, and a feed medium containing glycerol is added to the fermentation medium so that the glycerol concentration of the fermentation medium is 5 g/L or higher.

It is a further aspect of the present invention to provide the method as described above, wherein the glycerol concentration is 5 g/L or higher for a period which is at least 50% or more of the entire culture period.

It is a further aspect of the present invention to provide he method as described above, wherein the culturing comprises a proliferation phase during which the bacterium proliferates, and a production phase during which L-amino acid is produced, wherein the production phase follows the proliferation phase, and wherein the production phase is at least 70% or more of the entire culture period, and the glycerol concentration is 5 g/L or higher for a period which is at least 70% or more of the production phase.

It is a further aspect of the present invention to provide the method as described above, wherein an activity of an enzyme of the dihydroxyacetone pathway has been enhanced in the bacterium.

It is a further aspect of the present invention to provide he method as described above, wherein the enzyme is selected from the group consisting of glycerol dehydrogenase, dihydroxyacetone kinase, fructose-6-phosphate aldolase, and combinations thereof.

It is a further aspect of the present invention to provide the method as described above, wherein the enzyme comprises glycerol dehydrogenase, dihydroxyacetone kinase, and fructose-6-phosphate aldolase.

It is a further aspect of the present invention to provide the method as described above, wherein the activity of the enzyme has been enhanced by increasing copy number of a gene coding for the enzyme, or by modifying an expression control sequence of the gene.

It is a further aspect of the present invention to provide the method as described above, wherein the bacterium is an *Escherichia* bacterium.

It is a further aspect of the present invention to provide the method as described above, wherein the bacterium is *Escherichia coli.*

It is a further aspect of the present invention to provide the method as described above, wherein the L-amino acid is L-lysine or L-threonine.

### BRIEF DESCRIPTION OF THE DRAWINGS

Fig. 1 shows the change in glycerol concentration over time.
   ● indicates a glycerol concentration of 0 to 5 g/L, ▲ indicates a glycerol concentration of 10 to 15 g/L, and
   ■ indicates a glycerol concentration of 15 to 20 g/L (the same shall apply to Figs. 2 and 3).
Fig. 2 shows the change in L-lysine accumulation over time.
Fig. 3 shows the change in cell growth over time.
Fig. 4 shows the change in glycerol concentration over time.
   ● indicates a glycerol concentration of 0 to 5 g/L,
   ▲ indicates a glycerol concentration of 5 to 10 g/L, and
   ■ indicates a glycerol concentration of 10 to 15 g/L (the same shall apply to Fig. 5).
Fig. 5 shows the change in cell growth over time.

### DETAILED DESCRIPTION OF EXEMPLARY EMBODIMENTS

Hereafter, the present invention will be explained in detail.

### <1> Method of the present invention

The method in accordance with the presently disclosed subject matter is a method for producing an L-amino acid which includes the steps of culturing a microorganism which belongs to the family *Enterobacteriaceae* and has an L-amino acid-producing ability in a fermentation medium containing glycerol as a carbon source to cause accumulation of an L-amino acid in the medium, and collecting the L-amino acid from the medium, wherein the culturing is performed as a fed-batch culture or a continuous culture, and a feed medium containing glycerol is added to the fermentation medium so that the glycerol concentration in the fermentation medium is at least 5 g/L or higher.

The ability to produce an L-amino acid (L-amino acid-producing ability) can mean the ability of microorganisms used in the present invention to produce and accumulate an L-amino acid in a medium or cells when cultured in the medium. An exemplary microorganism can have the ability to produce two or more kinds of L-amino acids. Although the microorganism having L-amino acid-producing ability may inherently have an L-amino acid-producing ability, such a microorganism can be obtained by modifying such microorganisms as mentioned below using a mutagenesis method or a recombinant DNA technique so that they have L-amino acid-producing ability.

Although the type of the L-amino acid is not particularly limited, examples include basic amino acids such as L-lysine, L-ornithine, L-arginine, L-histidine and L-citrulline; aliphatic amino acids such as L-isoleucine, L-alanine, L-valine, L-leucine and L-glycine;, amino acids which are hydroxy-monoaminocarboxylic acids such as L-threonine and L-serine; cyclic amino acids such as L-proline; aromatic amino acids such as L-phenylalanine, L-tyrosine and L-tryptophan; sulfur-containing amino acids such as L-cysteine, L-cystine, and L-methionine; acidic amino acids such as L-glutamic acid and L-aspartic acid; and amino acids with an amide group at the side chain such as L-glutamine and L-asparagine. An exemplary microorganism can have the ability to produce two or more kinds of L-amino acids.

In the present invention, the term "L-amino acid" includes L-amino acids in a free form and salts thereof, such as sulfates, hydrochlorides, and carbonates.

The medium or the culture can contain glycerol as the carbon source, and in addition, can contain counter ions that are required for L-amino acid fermentation, a nitrogen source, sulfur, phosphorus, etc. required for growth of the chosen microorganism. A substance serving as a supply source of the counter ions can be a substance containing ammonium ions that serve as a nitrogen source; sulfate ions, chloride ions, or carbonate ions that serve as counter ions of carboxylic acid, organic acids, amino acids, and so forth.

The concentration of glycerol in the medium is controlled by adding a feed medium containing glycerol. That is, the culture is performed as a fed-batch culture or a continuous culture. A fed-batch culture is a culture method in which the medium is continuously or intermittently added into the culture vessel during the culture, and the medium is not extracted until the end of the culture. A continuous culture is a method in which the medium is continuously or intermittently added into the culture vessel during culture, and the medium is extracted from the vessel (usually in a volume equal to the volume of the added medium) at the same time. However, glycerol may be present in the starting medium.

The "starting medium" can mean a medium used in a batch culture before adding the feed medium in the fed-batch culture or continuous culture, that is the medium used at the start of the culture.. The "feed medium" can mean a medium which is supplied to the fermentation tank in the fed-batch culture or continuous culture. The feed medium is not particularly limited so long as it contains glycerol, and it may contain all or a part of the components required for growth of the microorganism. It may also contain only glycerol.

The term "fermentation medium" means the medium present in a fermentation tank, and an L-amino acid is collected from this fermentation medium. Furthermore, the term "fermentation tank" means a vessel in which the L-amino acid fermentation is performed. For example, a typical fermentation tank or a jar fermenter may be used. Furthermore, the volume of the fermentation tank is not limited so long as an L-amino acid can be produced and collected.

The culture is performed by controlling the glycerol concentration in the medium in the fermentation tank to be 5 g/L or higher during the culture. The glycerol concentration in the medium is controlled by the adding glycerol so that the concentration is 5 g/L or higher, or 7 g/L or higher, or even 10 g/L or higher. Although the upper limit of the glycerol concentration in the medium is not defined so long as the L-amino acid is efficiently produced, the glycerol concentration can be, for example, 25 g/L or lower, or 20 g/L or lower, or even 15 g/L or lower.

The glycerol concentration can be controlled so that it is constant for the entire culture period. However, it can also be controlled for only a portion of the culture period.

The glycerol concentration can be controlled for a period corresponding to 50% or more, or 70% or more, or even 80% or more, of the entire culture period.

When the method includes a period of proliferation of the bacterium having an L-amino acid-producing ability (proliferation phase), and a period of productionof the L-amino acid (production phase), the glycerol concentration can be controlled so that it is not lower than a certain level during the production phase. The "proliferation phase" can mean a period when the carbon source is primarily used for cell growth, and is usually within a period of usually 5 hours, or 10 hours, or even 15 hours, after the start of the culture, that is, the period when the microorganism is logarithmically proliferating. The "production phase" can mean the period of the culture following the proliferation phase, when the carbon source is mainly used for production of the L-amino acid, and usually occurs after a period of, for example, usually 5 hours, or 10 hours, or even 15 hours, from the start of the culture. The production phase can encompass 60% or more, or 70% or more, or even 80% or more, of the entire culture period (from the start to the end of the culture).

The glycerol concentration does not need to be within the aforementioned range over the entire period of the production phase, and when the concentration is below the aforementioned range for a certain period of time, it may be increased by timely addition of glycerol. Moreover, glycerol may be intermittently supplementedwhen the concentration is insufficient. The glycerol concentration of the medium in the fermentation tank can be controlled for 50% or more, or 70% or more, or even 80% or more, of the production phase.

In the proliferation phase, the glycerol concentration may be controlled to be within the aforementioned range, or may be at a concentration lower than the aforementioned range.

So long as the glycerol concentration of the medium in a fermentation tank is maintained to be within the aforementioned range, the feed medium may contain glycerol in a necessary amount, or glycerol may temporarily run short in the feed medium. The term "temporarily" can mean, for example, 15% or less, or 10% or less, or even 5% or less, of the production phase. When glycerol runs short, the glycerol concentration may temporarily be zero. During L-amino acid production, in particular, the concentration of the carbon source can be 0 at the end of culture in view of the subsequent processing step. The end of culture can mean a time point when the L-amino acid concentration reaches an intended concentration, or the culture for a predetermined period is completed. After the end of culture, the method usually advances to the step of collecting the amino acid from fermentation medium.

The glycerol concentration can be controlled by, for example, measuring the glycerol concentration in the medium, and when it becomes lower than a certain concentration, adding a feed medium containing glycerol to the fermentation tank. The glycerol concentration can be measured with, for example, BF-5 produced by Oji Scientific Instruments. If the conditions to maintain the glycerol concentration within a predetermined range, including time and volume of the additional feed medium, are determined or known for the chosen strain, the culture can be performed using those same conditions, and measuring the glycerol concentration and controlling it on the basis of the measurement becomes unnecessary.

The glycerol in the medium can be the sole carbon source, or a mixed medium can be used which contains other carbon sources in addition to glycerol. Examples of the other carbon sources can include saccharides such as glucose, fructose, sucrose, lactose, galactose, blackstrap molasses, and sugar solution obtained by hydrolysis of starch hydrolysate or biomass, alcohols such as ethanol, and organic acids such as fumaric acid, citric acid, and succinic acid. When two or more types of carbon sources are used, the amount of glycerol relative to the amount of all the carbon sources can be 50% or more, 60% or more, 70% or more, 80% or more, or even 90% or more.

The glycerol may be reagent grade, or may be crude glycerol. Examples of the crude glycerol can include glycerol produced as a by-product of biodiesel fuel production (Mu, Y., et al, Biotechnol Lett., 28, 1755-91759 (2006); Haas M.J., et al., Bioresour. Technol., 97, 4, 671-8678 (2006); WO2008/102861).

When crude glycerol is used, the glycerol concentration can mean the concentration of the glycerol itself.

As for other components which can be added to the medium, besides the carbon source, conventional medium components such as a nitrogen source, inorganic ions, and other organic components as required can be used.

As the nitrogen source, ammonia, ammonium salts such as ammonium sulfate, ammonium carbonate, ammonium chloride, ammonium phosphate, ammonium acetate and urea, nitrates, and so forth can be used. Ammonia gas and aqueous ammonia that are used to adjust the pH can also be utilized as the nitrogen source. Furthermore, peptone, yeast extract, meat extract, malt extract, corn steep liquor, soybean hydrolysate, and so forth can also be utilized. The medium can contain one or more of these nitrogen sources. These nitrogen sources can also be used for both the starting medium and the feed medium. Furthermore, the same nitrogen source can be used for both the starting medium and the feed medium, or the nitrogen source of the feed medium may be different from that of the starting medium.

The medium can contain a phosphoric acid source and a sulfur source in addition to the carbon source and the nitrogen source. As the phosphoric acid source, potassium dihydrogenphosphate, dipotassium hydrogenphosphate, phosphate polymers such as pyrophosphoric acid, and so forth can be utilized. The sulfur source may be any sulfur source so long as it has a sulfur atom, and can include salts of sulfuric acid such as sulfates, thiosulfates and sulfites and sulfur-containing amino acids such as cysteine, cystine and glutathione. Among these, ammonium sulfate is one example.

Furthermore, the medium can contain a growth-promoting factor (nutrient having a growth-promoting effect) in addition to the carbon source, the nitrogen source and sulfur. As the growth-promoting factor, trace metals, amino acids, vitamins, nucleic acids as well as peptone, casamino acid, yeast extract, soybean protein degradation product, and so forth containing the foregoing substances can be used. Examples of the trace metals include iron, manganese, magnesium, calcium, and so forth. Examples of the vitamins include vitamin B₁, vitamin B₂, vitamin B₆, nicotinic acid, nicotinic acid amide, vitamin B₁₂, and so forth.

Furthermore, when an auxotrophic mutant that requires an amino acid or the like for growth is used, the required nutrients can be added to the medium. In particular, since the L-lysine biosynthetic pathway is enhanced and the L-lysine degrading ability is attenuated in many of L-lysine-producing bacteria as described below, one or more types of substances including L-threonine, L-homoserine, L-isoleucine and L-methionine can be added.

The aforementioned components may be present in the starting medium or the feed medium, or both.

The starting medium and the feed medium can have the same or different compositions. Furthermore, the starting medium and the feed medium may have the same or different concentrations of each component. Furthermore, when the feed medium is added at multiple stages, the compositions of the feed media added at the various times may be the same or different.

The culture can be performed as an aeration culture at a fermentation temperature of 20 to 45°C, or at 33 to 42°C. The oxygen concentration is adjusted to 5 to 50%, or about 10%. Furthermore, the aeration culture can be performed with the pH adjusted to 5 to 9. If the pH drops during the culture, for example, calcium carbonate or an alkali such as an ammonia gas and aqueous ammonia can be added to neutralize the culture. When the culture is performed under such conditions for about 10 to 120 hours, a marked amount of L-amino acid is produced in the culture medium. Although the concentration of L-amino acid which accumulates is not limited so long as it is such a concentration that the L-amino acid can be isolated and collected from the medium, it can be 50 g/L or higher, 75 g/L or higher, or even 100 g/L or higher.

The L-amino acid can be collected by a known collection method from the culture medium after the culture. For example, the cells can be removed from the culture medium by centrifugation or the like, and then the L-amino acid can be crystallized by concentration, and the L-amino acid can be collected.

The culture of the microorganism can be performed as seed culture and main culture in order to ensure accumulation of the L-amino acid higher than a certain level. The seed culture can be performed as a shaking culture using a flask or the like, or a batch culture, and the main culture can be performed as a fed-batch culture or a continuous culture.

When the continuous culture method is used, the medium may be extracted and added simultaneously, or a part of the medium may be extracted, and then the medium may be added. Furthermore, the method may also be a continuous culture method which includes extracting the culture medium containing the L-amino acid and bacterial cells, and returning only the cells to the fermentation tank to reuse the cells (French Patent No. 2669935). As the method of continuously or intermittently feeding a nutrient source, the same method as used in the fed-batch culture is used.

The continuous culture method of reusing bacterial cells includes intermittently or continuously extracting the fermentation medium when the amino acid concentration reaches a predetermined level, extracting only the L-amino acid and re-circulating filtration residues containing bacterial cells into the fermentation tank, and it can be performed by referring to, for example, French Patent No. 2669935.

When the culture medium is intermittently extracted, a portion of the amount of L-amino acid can be extracted when the L-amino acid concentration reaches a predetermined level, and fresh medium is added to continue the culture. Furthermore, as for the volume of the medium to be added, the culture can be performed so that the final volume after the addition of the medium is equal to the volume of the culture medium before the extraction. The term "equal" can mean that the volume corresponds to about 93 to 107% of the volume of the culture medium before the extraction.

When the culture medium is continuously extracted, the extraction can be started at the same time as, or after the addition of the nutrient medium. For example, the extraction can be started within 5 hours, 3 hours, or even 1 hour, after the start of the feeding. Furthermore, the extraction volume of the culture medium can be equal to the volume of the medium fed.

When a basic amino acid such as L-lysine is produced, the fermentation can be performed by controlling the pH of the medium during culture to be 6.5 to 9.0, and the pH of the medium at the end of the culture to be 7.2 to 9.0, and the culture period can be when the medium contains 20 mM or more of bicarbonate ions and/or carbonate ions, so that these bicarbonate ions and/or carbonate ions serve as counter ions for the basic amino acid, and the objective basic amino acid is then collected (Japanese Patent Laid-open No. 2002-65287, U.S. Patent Published Application No. 2002/0025564, EP 1813677 A).

When a microorganism having a basic amino acid-producing ability is cultured in a medium under aerobic conditions, carbonate ions, bicarbonate ions, or both can be used as major counter ions of the basic amino acid. To provide carbonate ions and/or bicarbonate ions in an amount required to serve as counter ions of the basic amino acid, it is known that the pH of the medium can be controlled to be 6.5 to 9.0, or 6.5 to 8.0, during the culture, and can be 7.2 to 9.0 at the end of the culture. The pressure in the fermentation tank can be controlled so that it is positive during fermentation, or carbon dioxide or a mixed gas containing carbon dioxide can be supplied into the medium (Japanese Patent Laid-open No. 2002-65287, U.S. Patent Published Application No. 2002/0025564, EP 1813677 A).

The pressure in the fermentation tank may be controlled to be positive during the fermentation, and at the same time, carbon dioxide gas or a mixed gas containing carbon dioxide gas may be supplied to the medium. Both the above operations are performed so that there is a culture period when 20 mM or more, 30 mM or more, or even 40 mM or more, of bicarbonate ions and/or carbonate ions are present in the medium. The internal pressure of the fermentation tank, supply amount of carbon dioxide or mixed gas containing carbon dioxide, or the limited gas supply volume can be determined by, for example, measuring bicarbonate ions or carbonate ions in the medium, or the pH or ammonia concentration of the medium.

In the above embodiment, the pH of the medium is controlled to be 6.0 to 9.0, or 6.5 to 8.0, during the culture, and 7.2 to 9.0 at the end of the culture. According to the above embodiment, it is possible to suppress the pH of the medium so that the amount of bicarbonate ions and/or carbonate ions present in the medium to serve as counter ions is lower as compared to the conventional methods. When the pH is controlled with ammonia, ammonia is supplied in order to increase the pH, and it can serve as a nitrogen source for the basic amino acid. Examples of cations other than the basic amino acid in the medium include K, Na, Mg, Ca etc. originating in medium components. These can exist in an amount of 50% or less of the total cations.

Furthermore, the internal pressure of the fermentation tank during fermentation can be made positive by, for example, making the gas supply pressure higher than the exhaust pressure. By making the internal pressure of the fermentation tank positive, the carbon dioxide generated by fermentation dissolves in the culture medium to generate bicarbonate ions or carbonate ions, and these can serve as counter ions of the basic amino acid. The internal pressure of the fermentation tank is, specifically, 0.03 to 0.2 MPa, 0.05 to 0.15 MPa, or 0.1 to 0.3 MPa, in terms of the gage pressure (pressure difference with respect to the atmospheric pressure). Moreover, by supplying carbon dioxide or a mixed gas containing carbon dioxide to the culture medium, carbon dioxide may be dissolved in the medium. Furthermore, when supplying carbon dioxide or a mixed gas containing carbon dioxide to the medium, the internal pressure of the fermentation tank may be adjusted to be positive.

The internal pressure of the fermentation tank may be adjusted to be positive by, for example, making the gas supply pressure higher than the exhaust pressure. Furthermore, when carbon dioxide is supplied to the medium, for example, pure carbon dioxide or a mixed gas containing 5 volume % or more of carbon dioxide may be bubbled in the medium.

The aforementioned methods for dissolving bicarbonate ions and/or carbonate ions in the medium may be used independently, or two or more of them may be used in combination.

In the conventional methods, a sufficient amount of ammonium sulfate or ammonium chloride is usually added to the medium to serve as counter anions of the basic amino acid to be produced. Sulfuric acid or hydrochloric acid decomposition products of proteins etc. can be added to the medium as a nutrient component, and thus the generated sulfate ions and chloride ions are present in the medium. Therefore, the concentration of the weakly acidic carbonate ions is extremely low during the culture, such as a ppm order. The above embodiment is characterized in that these sulfate ions and chloride ions are reduced, and the carbon dioxide released by the microorganism during fermentation is dissolved in the medium in the aforementioned fermentation environment and can be used as counter ions. Therefore, in the above embodiment, it is not required to add sulfate ions or chloride ions to the medium in an amount more than the amount required for the growth. An appropriate amount of ammonium sulfate or the like can be added to the medium at an early stage of the culture, and the addition is terminated in the middle of the culture. Alternatively, ammonium sulfate or the like may be added while maintaining the balance with the dissolved carbonate ions or bicarbonate ions in the medium. Moreover, as a nitrogen source of the basic amino acid, ammonia may be added to the medium. Ammonia may be supplied to the medium independently, or together with other gases.

Lower concentrations of anions other than bicarbonate ions and/or carbonate ions can be in the medium so long as they are present in amounts that are required for the growth of the microorganism. Examples of such anions include chloride ions, sulfate ions, phosphate ions, ionized organic acids, hydroxide ions, and so forth. The total molar concentration of these other ions is usually 900 mM or lower, 700 mM or lower, 500 mM or lower, 300 mM or lower, or even 200 mM or lower.

To reduce the necessary amounts of sulfate ions and/or chloride ions is one of the objects of the above embodiment, and the total amount of sulfate ions or chloride ions, or both contained in the medium is usually 700 mM or lower, 500 mM or lower, 300 mM or lower, 200 mM or lower, or even 100 mM or lower.

If ammonium sulfate is added to a medium as a counter ion source of a basic amino acid, carbon dioxide in the culture medium is usually eliminated by sulfate ions. However, in the above embodiment, it is not necessary to add an excess amount of ammonium sulfate to the medium, and therefore carbon dioxide can be easily dissolved in the fermentation medium.

Furthermore, in the above embodiment, it is preferable to control the total ammonia concentration in the medium to such an extent that "production of the basic amino acid is not inhibited". Examples of the the conditions to attain such a purpose include, for example, when the yield and/or productivity is 50% or more, 70% or more, or even 90% or more, of the yield and/or productivity which is obtained under optimal conditions. Specifically, the total ammonia concentration in the medium is preferably 300 mM or lower, 250 mM or lower, or 200 mM or lower. The dissociation degree of the ammonia decreases as the pH becomes higher. Non-dissociating ammonia is more toxic to bacteria as compared to ammonium ions. Therefore, the upper limit of the total ammonia concentration should be determined depending on the pH of the culture medium. That is, as the pH of the culture medium increases, the acceptable total ammonia concentration decreases. Therefore, the aforementioned total ammonia concentration "which does not inhibit the basic amino acid production" can be determined for each specific pH value. However, the total ammonia concentration range that is acceptable at the highest pH level during the culture may be used as the upper limit of the total ammonia concentration throughout the entire culture period.

On the other hand, the total ammonia concentration which functions as a source of nitrogen required for growth of the microorganism and production of the basic substance is not particularly limited, and can be appropriately determined, so long as an a reduced level of the nitrogen source which can result in continuous depletion of ammonia during the culture does not reduce productivity of the objective substance by the microorganism. For example, the ammonia concentration can be measured over time during the culture, and if ammonia in the medium is depleted, a small amount of ammonia may be added to the medium. Although the total ammonia concentration after the addition of ammonia is not particularly limited, the total ammonia concentration may be, for example, 1 mM or higher, 10 mM or higher, or even 20 mM or higher.

The L-amino acid can be usually collected from the fermentation medium by a combination of known methods including the ion-exchange resin method (Nagai, H. et al., Separation Science and Technology, 39(16), 3691-3710), precipitation method, membrane separation method (Japanese Patent Laid-open Nos. 9-164323 and 9-173792), crystallization method (WO2008/078448, WO2008/078646), and so forth. When the L-amino acid accumulates in the cells, for example, the cells can be disrupted by ultrasonication or the like, and the supernatant can be obtained by removing the cells by centrifugation. The L-amino acid can then be collected from the supernatant by the ion-exchange resin method or the like.

The collected L-amino acid may contain bacterial cells, medium components, moisture, and by-product metabolites of the bacterium in addition to the L-amino acid. Purity of the collected L-amino acid can be 50% or higher, 85% or higher, or even 95% or higher (Japanese Patent No. 1214636, U.S. Patent Nos. 5,431,933, 4,956,471, 4,777,051, 4,946,654, 5,840,358, 6,238,714, U.S. Patent Published Application No. 2005/0025878).

When the L-amino acid is precipitated in the medium, it can be collected by centrifugation or filtration. When the L-amino acid is precipitated in the medium, the L-amino acid dissolved in the medium can be crystallized, and then they can be collected together.

### <2> Bacteria belonging to the family Enterobacteriaceae

The microorganism is one which belongs to the family *Enterobacteriaceae.* It has the ability to produce an L-amino acid, and is capable of producing the L-amino acid by using glycerol as the carbon source.

Typical examples of microorganisms belonging to the family *Enterobacteriaceae* include *Escherichia* bacteria and *Pantoea* bacteria. Other examples of microorganisms belonging to the family *Enterobacteriaceae* include γ-proteobacteria such as bacteria of the genus *Enterobacter, Klebsiella, Serratia, Erwinia, Salmonella, Morganella,* or the like.

A strain in which the activity of an enzyme of the dihydroxyacetone pathway (DHA pathway) is enhanced can be used. In the dihydroxyacetone pathway, glycerol is incorporated into cells, then converted into dihydroxyacetone, and enters the glycolysis system. In particular, glycerol dehydrogenase, dihydroxyacetone kinase, and fructose-6-phosphate aldolase are enzymes of the DHA pathway. The DHA pathway separates into two pathways at the point when the dihydroxyacetone is generated from glycerol by the glycerol dehydrogenase reaction. One pathway enters into the glycolysis system via dihydroxyacetone phosphate, which is generated from dihydroxyacetone by dihydroxyacetone kinase, and the other enters into the glycolysis system via fructose-6-phosphate, which is generated from dihydroxyacetone by fructose-6-phosphate aldolase. Although the activity of any of glycerol dehydrogenase, dihydroxyacetone kinase, and fructose-6-phosphate aldolase can be enhanced, the combination of glycerol dehydrogenase and dihydroxyacetone kinase, the combination of glycerol dehydrogenase, and fructose-6-phosphate aldolase, and the combination of three of these enzymes are examples. All three enzyme activities can also be enhanced.

"Glycerol dehydrogenase" can mean an enzyme which reversibly catalyzes the following oxidation reaction that converts glycerol into dihydroxyacetone by using NAD as a coenzyme (EC:1.1.1.6).

Glycerol + NAD -> Dihydroxyacetone + NADH + H⁺

The phrase "modified to increase the glycerol dehydrogenase activity" can mean that the number of the glycerol dehydrogenase molecules per cell can be increased as compared to that of a wild-type or non-modified strain, or that the activity of the glycerol dehydrogenase per molecule can be improved as compared to that of a wild-type or non-modified strain. Moreover, when the enzyme activity is undetectable in a wild-type strain, and it is improved to a detectable level, the activity is also considered to be "increased". The glycerol dehydrogenase activity can be at any level so long as it is detectable, but glycerol dehydrogenase can be modified so that the activity is 0.05 U/mg or higher, 0.25 U/mg or higher, or 0.5 U/mg or higher. Examples of wild-type strains of microorganisms belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth. The glycerol dehydrogenase activity can be measured by referring to the method of Ansis, R.E. et al. (J. Biol. Chem., 2-3, 153-159 (1953))

"Dihydroxyacetone kinase" is an enzyme which reversibly catalyzes the following reaction that converts dihydroxyacetone into dihydroxyacetone phosphate, and either ATP (EC 2.7.1.29) or PEP (phosphoenolpyruvate) (EC 2.7.1.29) is used as a phosphate donor (Cell. Mol. Life Sci., 63 (2006) 890-900; Biochemistry, 43 (2004) 13037-13045)

ATP + Dihydroxyacetone -> ADP + Dihydroxyacetone phosphate (EC 2.7.1.29)

Phosphoenolpyruvate + Dihydroxyacetone -> Pyruvate + Dihydroxyacetone phosphate (EC2.7.1.29)

It is particularly preferable that dihydroxyacetone kinase uses ATP as a phosphate donor.

The phrase "modified to increase the dihydroxyacetone kinase activity" can mean that the number of the dihydroxyacetone kinase molecules per cell can be increased as compared to that of a wild-type or non-modified strain, or that the activity of the dihydroxyacetone kinase per molecule can be improved as compared to that of a wild-type or non-modified strain. Moreover, when the enzyme activity is undetectable in a wild-type strain, and it is improved to a detectable level, the activity is also considered to be "increased". Dihydroxyacetone kinase activity per cell can be modified so that it is improved to 150% or more, 200% or more, or even 300% or more, of the activity of a wild-type strain or non-modified strain. Examples of wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea ananatis* AJ13335 strain (FERM BP-6615), and so forth. The dihydroxyacetone kinase activity can be measured by referring to the method of Johnson E.A. (J. Bacteriol., 1984 Oct; 160(1):55-60).

Examples of the gene coding for glycerol dehydrogenase include the *gldA* gene, including the *gldA* gene from a microorganism belonging the family *Enterobacteriaceae.* Examples of the microorganism belonging the family *Enterobacteriaceae* include *Escherichia coli.* Examples of the gene of *Escherichia coli* include, for example, the *gldA* gene of SEQ ID NO: 1 (complementary strand of the nucleotide numbers 4135955..4137058 of GenBank Accession No. NC_000913).

Furthermore, the gene coding for glycerol dehydrogenase may be a homologue of the *gldA* gene. The homologue of the *gldA* gene can be cloned on the basis of homology to the gene exemplified above from a bacterium of the genus *Escherichia, Enterobacter, Klebsiella, Serratia, Erwinia, Yersinia, Shigella, Salmonella, Vibrio, Aeromonas, Bacillus, Staphylococcus, Lactobacillus, Enterococcus, Clostridium, Pseudomonas, Agrobacterium, Citrobacter, Corynebacterium,* or the like. Examples of the gene thath have high homology to the *gldA* gene of *Escherichia coli* and can be used as the gene coding for glycerol dehydrogenase are mentioned in Table 1.

**Table 1: Genes showing high homology to gldA gene of Escherichia coli and coding for glycerol dehydrogenase**

| Gene | Microorganism | Description | Genbank Accession No. | SEQ ID NO |
|---|---|---|---|---|
| *gldA* | *Shigella dysenteriae* Sdl97 | Glycerol dehydrogenase, (NAD) | YP_405216.1 | 74, 75 |
| | | | GI:82778867 | |
| *gldA* | *Salmonella typhimurium* LT2 | Similar to *E. coli* glycerol dehydrogenasem, (NAD) | AE008892.1 | 76, 77 |
| | | | GI:16422675 | |
| *gldA* | *Pseudomonas putida* | Glycerol dehydrogenase | AF148496.1 | 78, 79 |
| | | | GI:6552505 | |
| *gldA* | *Bacillus coagulans* | Glycerol dehydrogenase and related enzymes | ZP_01697292.1 | 80, 81 |
| | | | GI:124522908 | |

Homology or identity of amino acid sequences and nucleotide sequences can be determined by using, for example, the algorithm BLAST of Karlin and Altschul (Pro. Natl. Acad. Sci. USA, 90, 5873 (1993)) or FASTA of Pearson (Methods Enzymol., 183, 63 (1990)). Programs called BLASTN and BLASTX have been developed on the basis of this algorithm BLAST (refer to www.ncbi.nlm.nih.gov).

As the gene coding for dihydroxyacetone kinase, the *dhaKLM* genes, *dak1, dhaK* and *dhbK* genes can be used. Examples of the gene coding for the enzyme using PEP as a phosphate donor include genes from *Escherichia coli,* such as the *dhaK* gene of SEQ ID NO: 34 (complementary strand of the nucleotide numbers 1248991..1250061 of GenBank Accession No. NC_000913), the *dhaL* gene of SEQ ID NO: 36 (complementary strand of the nucleotide numbers 1248348..1248980 of GenBank Accession No. NC_000913), and the *dhaM* gene of SEQ ID NO: 38 (complementary strand of the nucleotide numbers 1246919..1248337 of GenBank Accession No. NC_000913).

The gene coding for dihydroxyacetone kinase that uses ATP as a phosphate donor can be used, and it includes the *dak1* gene from yeast, the *dhbK* gene from *Agrobacterium* bacteria, and the *dhaK* gene from *Citrobacter* bacteria. Examples of the *dakl* gene from yeast include the *dakl* gene of SEQ ID NO: 3 from *Saccharomyces cerevisiae* (GenBank Accession No NP_013641.1 GI: 6323570), examples of the *dhbK* gene from *Agrobacterium* bacteria include the *dhbK* gene of SEQ ID NO: 5 from *Agrobacterium tumefaciens* (GenBank Accession No. NP_357070.1 GI: 15891398), and examples of the *dhaK* gene from *Citrobacter* bacteria include the *dhaK* gene of SEQ ID NO: 7 from *Citrobacter freundii* (GenBank Accession No. U09771).

Furthermore, the gene coding for dihydroxyacetone kinase may be a homologue of the aforementioned genes. Such homologues can be cloned on the basis of homology to the genes exemplified above from a bacterium such as bacteria of the genus *Escherichia, Enterobacter, Klebsiella, Serratia, Erwinia, Yersinia, Shigella, Salmonella, Vibrio, Aeromonas, Bacillus, Staphylococcus, Lactobacillus, Enterococcus, Clostridium, Agrobacterium, Citrobacter,* and *Mycobacterium,* yeast such as those of the genus *Saccharomyces, Schizosaccharomyces* or *Pichia,* or the like.

In particular, as the gene coding for dihydroxyacetone kinase that uses ATP as a phosphate donor, the following sequences can be used. Genes coding for dihydroxyacetone kinase and showing high homology to the *dakl* gene from *Saccharomyces cerevisiae* are shown in Table 2, dihydroxyacetone kinase genes showing high homology to the *dhbK* gene from *Agrobacterium tumefaciens* are shown in Table 3, and dihydroxyacetone kinase genes showing high homology to the *dhaK* gene from *Citrobacter freundii* are shown in Table 4.

**Table 2: Genes coding for dihydroxyacetone kinase and showing high homology to the dakl gene from Saccharomyces cerevisiae**

| Gene | Microorganism | Description | Genbank Accession No. | SEQ ID NO |
|---|---|---|---|---|
| T43702 | *Schizosaccharomyces pombe* | Dihydroxyacetone kinase | gi\|3493578\|gb\| AAC78808.1\| | 40, 41 |
| AAC27705 | *Pichia angusta* | Dihydroxyacetone kinase | gi\|3171001\|gb\| AAC27705.1\| | 42, 43 |
| AAC39490.1 | *Pichia pastoris* | Dihydroxyacetone kinase | gi\|3287486\|gb\| AAC39490.1\| | 44, 45 |
| CAG88710.1 | *Debaryomyces hansenii* CBS767 | Dihydroxyacetone kinase | gi\|49656075\|emb\| CAG88710.1\| | 46, 47 |

**Table 3: Genes coding for dihydroxyacetone kinase and showing high homology to the dhbK gene from Agrobacterium tumefaciens**

| Gene | Microorganism | Description | Genbank Accession No. | SEQ ID NO |
|---|---|---|---|---|
| ABF89849.1 | *Myxoccoccus xanthus* DK 1622 | Dihydroxyacetone kinase family protein | gi\|108464664\| gb\|ABF89849.1\| | 58, 59 |
| ABB06761.1 | *Burkholderia* sp. 383 | Glycerone kinase | gi\|77965380\| gb\|ABB06761.1\| Glycerone kinase [*Burkholderia* sp. 383] | 60, 61 |
| ABC38950.1 | *Burkholderia thailandensis* E264 | Dihydroxyacetone kinase | gi\|83654887\| gb\|ABC38950.1\| | 62, 63 |
| EAV65448.1 | *Burkholderia multivorans* ATCC 17616 | Glycerone kinase | gi\|118658702\| gb\|EAV65448.1\| | 64, 65 |

**Table 4: Genes coding for dihydroxyacetone kinase and showing high homology to the dhaK gene from Citrobacter freundii**

| Gene | Microorganism | Description | Genbank Accession No. | SEQ ID NO |
|---|---|---|---|---|
| AAX12907.1 | *Escherichia blattae* | Dihydroxyacetone kinase | gi\|60099603\| gb\|AAX12907.1\| | 48, 49 |
| EAV82971.1 | *Enterobacter* sp. 638 | Dihydroxyacetone kinase | Gi\|118676428\| gb\|EAV82971.1\| | 50, 51 |
| EAS39398.1 | *Psychromonas* sp. CNPT3 | Dihydroxyacetone kinase | gi\|90311294\| gb\|EAS39398.1\| | 52, 53 |
| EAV42339.1 | *Stappia aggregata* IAM 12614 | Dihydroxyacetone kinase protein | gi\|118435694\| gb\|EVA42339.1\| | 54, 55 |
| CAK08390.1 | *Rhizobium leguminosarum* bv. *viciae* 3841 | Putative dihydroxyacetone kinase | gi\|115257295\| emb\|CAK08390.1\| | 56, 57 |

"Fructose-6-phosphate aldolase" is an enzyme which reversibly catalyzes the following reaction that converts dihydroxyacetone into fructose-6-phosphate.

D-Fructose-6-phosphate -> Dihydroxyacetone + D-Glyceraldehyde-3-phosphate

The phrase "modified to increase the fructose-6-phosphate aldolase activity" can mean that the number of the fructose-6-phosphate aldolase molecules per cell can be increased as compared to that of a wild-type strain or non-modified strain, or that the activity of the fructose-6-phosphate aldolase per molecule can be improved as compared to that of a wild-type strain or non-modified strain. Fructose-6-phosphate aldolase can be modified so that the activity per cell is improved to 150% or more, 200% or more, or even 300% or more, of the activity observed in a wild-type strain or non-modified strain. Examples of the wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth.

Examples of the gene coding for fructose-6-phosphate aldolase include the *fsaA* gene coding for type I aldolase (SEQ ID NO: 68, the nucleotide numbers 862865..863527 of GenBank Accession No. NC_000913), and the *fsaB* gene *(talC* gene) (SEQ ID NO: 70, complementary strand of the nucleotide numbers 4137069..4137731 of GenBank Accession No. NC_000913) coding for type II aldolase, which are originated from *Escherichia coli.*

Homologues of the gene coding for fructose-6-phosphate aldolase may be a homologue of the aforementioned genes. Such homologues may be cloned on the basis of homology to the *fsaA* gene or the *fsaB* gene mentioned above from a bacterium such as bacteria of *Escherichia, Enterobacter, Klebsiella, Serratia, Erwinia, Yersinia, Shigella, Salmonella, Vibrio, Aeromonas, Bacillus, Staphylococcus, Lactobacillus, Enterococcus, Clostridium, Pseudomonas, Agrobacterium, Citrobacter,* and *Corynebacterium.*

The fructose-6-phosphate aldolase activity can be measured by using the method of Schurmann M., Sprenger G.A. et al. (J. Biol. Chem., 2001 Apr 6, 276 (14):11055-61).

Homologues of the aforementioned genes be mutant genes derived from other microorganisms, or natural or artificial mutant genes, which show high structural similarity to the aforementioned genes and have a function of improving the glycerol dehydrogenase, dihydroxyacetone kinase or fructose-6-phosphate aldolase activity when they are introduced into a host or amplified. Homologues of glycerol dehydrogenase, dihydroxyacetone kinase, or fructose-6-phosphate aldolase genes mean genes coding for a protein having a homology of 80% or more, 90% or more, 95% or more, 98% or more, or even 99% or more, to the total amino acid sequence of SEQ ID NOs: 2, 4, 6, 8, 69 or 71, or any of the amino acid sequences encoded by the sequences listed in Tables 1 to 4, and having a function of glycerol dehydrogenase, dihydroxyacetone kinase, or fructose-6-phosphate aldolase. The term "homology" may also refer to "identity". Whether a gene codes for a protein having glycerol dehydrogenase, dihydroxyacetone kinase, or fructose-6-phosphate aldolase activity can be confirmed by expressing the gene in a host cell and examining whether the enzymatic activity is increased as compared to a non-modified strain according to the aforementioned enzymatic activity measurement method. Moreover, whether a gene is a homologue or not can be confirmed by preparing a gene-disrupted strain in which the corresponding wild-type gene is disrupted, introducing the gene into the disrupted strain, and examining whether the gene complements the function of the wild-type gene, for example, whether the enzymatic activity reduced by the gene disruption is restored.

Furthermore, the genes coding for glycerol dehydrogenase, dihydroxyacetone kinase, or fructose-6-phosphate aldolase are not limited to wild-type genes, and they may be conservative variants, i.e., mutant or artificially modified genes coding for a protein having an amino acid sequence of SEQ ID NO: 2, 4, 6, 8, 69 or 71, or any of the amino acid sequences encoded by the sequences listed in Table 1 to 4, which can include substitution, deletion, insertion, addition or the like of one or more amino acid residues at one or more positions so long as the function of encoded glycerol dehydrogenase, dihydroxyacetone kinase, or fructose-6-phosphate aldolase is not reduced. Although the number of the "one or several" amino acid residues may differ depending on positions in the three-dimensional structure or types of amino acid residues of the protein, it may be specifically 1 to 20, 1 to 10, 1 to 5, or even 1 to 3. These mutations can be conservative substitutions. The conservative substitution is a mutation wherein substitution takes place mutually among Phe, Trp and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group. Examples of the conservative substitution include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned amino acid substitution, deletion, insertion, addition, inversion or the like may be the result of a naturally-occurring mutation due to an individual difference or difference of species (mutant or variant) of a microorganism having the genes coding for glycerol dehydrogenase, dihydroxyacetone kinase, or fructose-6-phosphate aldolase.

The genes coding for glycerol dehydrogenase, dihydroxyacetone kinase, or fructose-6-phosphate aldolase and conservative variants thereof may also be a DNA which is able to hybridize with a sequence complementary to the nucleotide sequence of SEQ ID NO: 1, 3, 5, 7, 68 or 70, or any of the nucleotide sequences mentioned in Table 1 to 4, or a probe that can be prepared from the nucleotide sequences, under stringent conditions, and codes for a protein having a function of glycerol dehydrogenase, dihydroxyacetone kinase, or fructose-6-phosphate aldolase. The "stringent conditions" are conditions under which a so-called specific hybrid is formed, and a non-specific hybrid is not formed. Although it is difficult to clearly define the condition with numerical values, examples of the stringent condition include those under which highly homologous DNAs hybridize to each other, for example, DNAs not less than 80% homologous, not less than 90% homologous, not less than 95% homologous, not less than 98% homologous, or even not less than 99% homologous, hybridize to each other, and DNAs less homologous than the above do not hybridize to each other, or conditions of washing for typical Southern hybridization, that is, washing once, or 2 or 3 times, at a salt concentration and temperature corresponding to 1 x SSC, 0.1% SDS at 60°C, 0.1 x SSC, 0.1% SDS at 60°C, or 0.1 x SSC, 0.1% SDS at 68°C.

The aforementioned methods can be similarly applied to enhancing the activities of triosephosphate isomerase, fructose bisphosphate aldolase, and fructose-1,6-bisphosphatase described below.

The phrase "intracellular activity of an enzyme increases" means when the intracellular activity of the enzyme is increased as compared to a wild-type strain (for example, *Escherichia coli* W3110 and MG1655 strains), or a parent strain (strain in which intracellular activities of all the enzymes specified are not enhanced), and also include when the cells have the activity that a wild-type or the parent strain does not have.

Examples of methods for increasing the intracellular activity include the following and combinations thereof; however, the methods are not limited to the following. To increase the activities of glycerol dehydrogenase, dihydroxyacetone kinase, and fructose-6-phosphate aldolase, any of the following (1) to (5) can be used in any combination or alone.
(1) Increase in the copy number of a gene coding for each protein by transformation with a vector containing the gene.
(2) Increase in the copy number of a gene coding for each protein by integration of the gene into a chromosome.
(3) Increase in the expression of a gene coding for each protein by modification of an expression control region of the gene.
(4) Increase in expression by modification of a factor which has an effect on expression or the control thereof.
(5) Increase in the enzymatic activity by introduction of a mutation into a coding region of a gene coding for each protein.
(6) Increase in the amount of protein by improvement of the translation efficiency.

Henceforth, the genes coding for enzymes of the DHA pathway may be each referred to as the objective gene.

### (1) Increase in the copy number of a gene coding for each protein by transformation with a vector containing the gene

For example, a DNA fragment containing an objective gene can be ligated to a vector, such as a multi-copy vector, which is able to function in the chosen host microorganism to prepare a recombinant DNA. This recombinant DNA can then be introduced into a microorganism to transform it. The objective gene can be obtained by PCR (polymerase chain reaction, refer to White, T.J. et al., Trends Genet., 5, 185 (1989)) using chromosomal DNA of *Escherichia coli,* yeast, *Citrobacter* bacterium, *Agrobacterium* bacterium or the like as a template. The objective genes from other microorganisms can also be obtained from the chromosomal DNA or a chromosomal DNA library of each microorganism by PCR using, as primers, oligonucleotides prepared based on the known objective gene of the microorganism, or sequence information of the objective gene or the protein of a microorganism of another species, or hybridization using an oligonucleotide prepared based on such sequence information as mentioned above as a probe. A chromosomal DNA can be prepared from a microorganism that serves as a DNA donor by the method of Saito and Miura (refer to Saito H. and Miura K., Biochem. Biophys. Acta, 72, 619 (1963); Experimental Manual for Biotechnology, edited by The Society for Biotechnology, Japan, pp.97-98, Baifukan Co., Ltd., 1992) or the like.

Then, the objective gene which has been amplified by PCR can be ligated to a vector DNA which is able to function in the cell of a host microorganism to prepare a recombinant DNA. Examples of the vectors which can function in the host microorganism include vectors which are autonomously replicable in cells of the host microorganism.

Examples of vectors which are autonomously replicable in microorganisms belonging to the family *Enterobacteriaceae* include pUC19, pUC18, pHSG299, pHSG399, pHSG398, pACYC184, (pHSG and pACYC series vectors are available from Takara Bio), RSF1010 (Gene, vol. 75(2), p271-288, 1989), pBR322, pMW219, pMW119 (pMW series vectors are available form Nippon Gene), pSTV28, pSTV29 (Takara Bio) and so forth. A phage DNA vector can also be used.

To prepare recombinant DNA by ligating any of the genes to the above-mentioned vector, the vector is digested with a restriction enzyme corresponding to termini of a DNA fragment containing the objective gene. Ligation is generally performed by using a ligase such as T4 DNA ligase. As methods for digesting and ligating DNA, preparation of chromosomal DNA, PCR, preparation of plasmid DNA, transformation, design of oligonucleotides to be used as primers and so forth, methods well known to a person skilled in the art can be employed. These methods are described in Sambrook, J., Fritsch, E.F., and Maniatis, T., "Molecular Cloning A Laboratory Manual, Second Edition", Cold Sprig Harbor Laboratory Press, (1989), and so forth.

The recombinant DNA prepared as described above may be introduced into a bacterium in accordance with a conventional known transformation method. Examples include electroporation (Canadian Journal of Microbiology, 43, 197 (1997)). It is also possible to use a method of increasing the DNA permeability by treating recipient cells with calcium chloride, which is reported for *Escherichia coli* K-12 (Mandel, M. and Higa, A., J. Mol. Biol., 53, 159 (1970), or a method of introducing a DNA into a competent cell prepared from a cell at proliferation phase, which is reported for *Bacillus subtilis* (Duncan, C.H., Wilson, G.A and Young, F.E, Gene, 1, 153 (1977)).

### (2) Increase in the copy number of DNA coding for each protein by integration of the gene into a chromosome

Increase of the intracellular activity of each enzyme can be achieved by increasing the copy number of the objective gene by introducing the objective gene into chromosomal DNA of the microorganism. Introduction of the objective gene into the chromosomal DNA of the microorganism can be attained by homologous recombination using a target sequence present on the chromosomal DNA in multiple copies. As sequences present on a chromosomal DNA in multiple copies, a repetitive DNA or an inverted repeat present on the termini of a transposing element can be used. Alternatively, as disclosed in Japanese Patent Laid-open (Kokai) No. 2-109985, the objective gene can be introduced into the chromosomal DNA by inserting the gene into a transposon, and transferring it so that the gene is integrated into the chromosomal DNA. Moreover, it is also possible to introduce an objective gene into a chromosome by using the Red driven integration method (WO2005/010175). An objective gene can also be introduced into a chromosome by transduction using a phage such as P1 phage, or by using a vector for conjugative transfer. Transfer of a gene to a chromosome can be confirmed by Southern hybridization using a part of the gene as a probe. Amplification of the copy number can be confirmed by Southern hybridization using a probe complementary to the objective gene. The gene coding for glycerol dehydrogenase can be amplified by two or more copies, three or more copies, or even five or more copies. The gene coding for dihydroxyacetone kinase can be amplified by two or more copies, three or more copies, or even five or more copies. The gene coding for fructose-6-phosphate aldolase can be amplified by two or more copies, three or more copies, or even five or more copies. When the gene is not native to the chosen host microorganism, any number of copies can be introduced, so long as at least one or more copies are introduced.

### (3) Increase in theexpression of a gene coding for each protein by modification of an expression control region of the gene

Furthermore, besides increasing the copy number of objective gene mentioned above, increasing the intracellular activity of each enzyme can be achieved by replacing an expression regulatory sequence, such as a promoter, of the gene on a chromosomal DNA or on a plasmid with a stronger promoter by the method described in WO00/18935. Examples of strong promoters include the lac promoter, trp promoter, trc promoter, lambda phage PR promoter, PL promoter, lpp promoter, T7 promoter, tet promoter, and so forth. In particular, to amplify glycerol dehydrogenase, the tacM promoter (SEQ ID NO: 10) can be used. *dhaK, dhaL,* and *dhaM* coding for dihydroxyacetone kinase of *Escherichia coli* exist as an operon structure, and expression of all the three genes can be improved by enhancing the promoter which is located upstream of *dhaK.*

Moreover, it is also possible to introduce a nucleotide substitution or the like into the promoter region of an objective gene to modify it into a stronger promoter. Methods for evaluating potency of promoters and examples of potent promoters are described in the paper of Goldstein et al. (Prokaryotic promoters in biotechnology, Biotechnol. Annu. Rev., 1995, 1, 105-128), and so forth. Furthermore, it is known that the substitution of several nucleotides in the spacer region between the ribosome binding site (RBS) and the start codon, in particular, in the region immediately upstream of the start codon, significantly affects the translation efficiency of mRNA, and such a region can also be modified. Expression of the objective gene is enhanced by such substitution or modification of promoter.

A promoter located upstream of the objective gene on a genome can be replaced with a stronger promoter by transforming a microorganism belonging to the family *Enterobacteriaceae* with a DNA containing the stronger promoter amplified by PCR or the like to cause recombination of the stronger promoter and the wild-type promoter on the genome. For such gene substitution utilizing homologous recombination, there can be utilized the method called Red-driven integration (Datsenko, K.A, and Wanner, B.L., Proc. Natl. Acad. Sci. USA, 97:6640-6645 (2000)), a method of using a linear DNA such as a method utilizing the Red driven integration in combination with an excisive system derived from λ phage (Cho, E.H., Gumport, R.I., Gardner, J.F., J. Bacteriol., 184:5200-5203 (2002)) (refer to WO2005/010175), a method of using a plasmid containing a temperature sensitive replication origin (Datsenko, K.A, and Wanner, B.L., Proc. Natl. Acad. Sci. USA, 97:6640-6645 (2000), U.S. Patent No. 6,303,383, Japanese Patent Laid-open No. 05-007491), and so forth.

### (4) Increase in the expression by modification of factor which has an effect on expression or the control thereof

Increase in expression by modification of a factor which effects expression or expression control can be attained by amplifying a gene coding for an activator which increases expression of the genes coding for glycerol dehydrogenase, dihydroxyacetone kinase, or fructose-6-phosphate aldolase, or by deleting or attenuating a gene coding for a regulator which reduces expression of the genes. Examples of an activator of *dhaKLM* coding for dihydroxyacetone kinase include, for example, *dhaR* (SEQ ID NO: 66, the nucleotide numbers 1250289..1252208 of GenBank Accession No. NC_000913), and expression of *dhaKLM* coding for dihydroxyacetone kinase is increased by a mutation of the *dhaR* gene (1: EMBO J., 2005 Jan 26, 24(2):283-93). The expression of *dhaKLM* coding for dihydroxyacetone kinase is also increased by disruption of the *ptsI* gene (SEQ ID NO: 86, the nucleotide numbers 2532088..2533815 of GenBank Accession No. NC_000913) (Microbiology, 147 (2001) 247-253).

### (5) Increase in enzymatic activity by introduction of mutation into a coding region of gene coding for each protein

Furthermore, the activities of glycerol dehydrogenase, dihydroxyacetone kinase, and fructose-6-phosphate aldolase can also be increased by introducing a mutation which increases the specific activities of the proteins or improves the substrate specificities of the enzymes into the coding regions of the objective genes.

Such a gene coding for each enzyme having a mutation can be obtained by, for example, modifying the nucleotide sequence of the SEQ ID NO: 1, 3, 5, 7, 68 or 70, or a coding region in any of the nucleotide sequences mentioned in Tables 1 to 4, so that amino acid residues of a specific part of the encoded protein include substitution, deletion, insertion, addition or the like of amino acid residues. Furthermore, conventionally known mutagenizing treatments, such as those described below can be used. For example, by treating the nucleotide sequence of the SEQ ID NO: 1, 3, 5, 7, 68 or 70, any of the nucleotide sequences mentioned in Tables 1 to 4, or a coding region sequence encompassed by any of these, with hydroxylamine or the like *in vitro,* or by treating a *Enterobacteriaceae* microorganism containing the gene with ultraviolet radiation or a typical mutagenizing agent, such as N-methyl-N'-nitro-N-nitrosoguanidine (NTG) or ethyl methanesulfonate (EMS), error-prone PCR (Cadwell, R.C., PCR Meth. Appl., 2, 28 (1992)), DNA shuffling (Stemmer, W.P., Nature, 370, 389 (1994)), or StEP-PCR (Zhao, H., Nature Biotechnol., 16, 258 (1998)), a mutation can be artificially introduced into the genes coding for glycerol dehydrogenase, dihydroxyacetone kinase, or fructose-6-phosphate aldolase by gene recombination, resulting in genes coding for highly active glycerol dehydrogenase, dihydroxyacetone kinase, or fructose-6-phosphate aldolase. Whether such mutant genes code for glycerol dehydrogenase, dihydroxyacetone kinase, or fructose-6-phosphate aldolase can be confirmed by, for example, introducing the genes into a microorganism belonging to the family *Enterobacteriaceae* and having an L-amino acid-producing ability, culturing it in a medium containing glycerol as a carbon source, and confirming whether the L-amino acid-producing ability is improved, or measuring the enzyme activities by the aforementioned methods.

### (6) Increase in the amount of protein by improvement of the translation efficiency

The amount of protein can be increased by improvement of translation efficiency by increasing the tRNA corresponding to codons less frequently used in the host, or by modifying the objective gene so that it has optimal codons according to frequency of use of codons in the host (Gene 85, 109-114 (1989), Biochemistry, 31, 2598-2608 (1992), J. Bacteriol., 175, 716-722 (1993), Protein Expression and Purification, 50, 49-57 (2006)). Increase in the amount of the objective protein as compared to a non-modified strain or wild-type strain can be confirmed by, for example, detection by Western blotting using antibodies (Molecular Cloning (Cold Spring Harbor Laboratory Press, Cold spring Harbor (USA), 2001)).

The microorganism can be modified to increase glycerol uptake activity, in addition to enhancing glycerol dehydrogenase, dihydroxyacetone kinase, and fructose-6-phosphate aldolase activities. The glycerol uptake activity can mean the activity for incorporating glycerol into the cytoplasm, and a glycerol facilitator, which is a membrane protein, is involved. Examples of the gene coding for the glycerol facilitator include, for example, the *glpF* gene of *Escherichia coli* (SEQ ID NO: 16, complementary strand of the nucleotide numbers 4115268..4116113 of GenBank Accession No. NC_000913).

The gene coding for the glycerol facilitator may be a DNA which hybridizes with a complementary sequence of the nucleotide sequence of SEQ ID NO: 16, or a probe which can be prepared from the complementary sequence under stringent conditions, and codes for a protein having glycerol uptake activity. Examples also include a DNA coding for the protein of SEQ ID NO: 17. The protein can have homology of 80% or more, 90% or more, 95% or more, 98% or more, or even 99% or more, to the total amino acid sequence of SEQ ID NO: 17, so long as it increases the glycerol uptake ability in a microorganism belonging to the family *Enterobacteriaceae,* when it is introduced into the microorganism.

Moreover, the gene may be a DNA coding for a protein having an amino acid sequence of SEQ ID NO: 17, but which can include substitution, deletion, insertion, addition or the like of one or several amino acid residues, so long as the glycerol uptake activity is not reduced. The activity can be increased by a method similar to the aforementioned methods for enhancing glycerol dehydrogenase, dihydroxyacetone kinase, or fructose-6-phosphate aldolase activity.

The glycerol uptake activity can be measured by using the transport assay method using a membrane protein (Voegele, R.T., Sweet, G.D., and Boos, W.J., Bacteriol., 175:1087-1094 (1993)).

The microorganism can be modified to increase the activities of one or more enzymes such as triosephosphate isomerase, fructose bisphosphate aldolase, and fructose-1,6-bisphosphatase, in addition to enhancing glycerol dehydrogenase, dihydroxyacetone kinase, and fructose-6-phosphate aldolase activities, and the enhancement of glycerol uptake activity.

Triosephosphate isomerase catalyzes the reaction which reversibly converts dihydroxyacetone phosphate into glyceraldehyde-3-phosphate (EC:5.3.1.1).

Dihydroxyacetone phosphate -> D-Glyceraldehyde-3-phosphate

The phrase "modified to increase the triosephosphate isomerase activity" can mean that the number of the triosephosphate isomerase molecules per cell can be increased as compared to that of a wild-type strain or non-modified strain, or that the activity of the triosephosphate isomerase per molecule can be improved as compared to that of a wild-type strain or non-modified strain. The triosephosphate isomerase can be modified so that the activity per cell is improved to 150% or more, 200% or moreor even 300% or more, of the activity of a wild-type strain or non-modified strain. Examples of wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth.

Examples of the gene coding for triosephosphate isomerase include the *tpiA* gene derived from *Escherichia coli* (SEQ ID NO: 18, complementary strand of the nucleotide numbers 4108763..4109530 of GenBank Accession No. NC_000913).

The gene coding for triosephosphate isomerase may be a DNA which hybridizes with a complementary sequence of the nucleotide sequence of SEQ ID NO: 18, or a probe which can be prepared from the complementary sequence, under stringent conditions, and codes for a protein having the triosephosphate isomerase activity. Examples also include a DNA coding for the protein of SEQ ID NO: 19. The protein can have homology of 80% or more, 90% or more, 95% or more, 98% or more, or even 99% or more, to the total amino acid sequence of SEQ ID NO: 19, so long as it shows increased triosephosphate isomerase activity in a microorganism belonging to the family *Enterobacteriaceae,* when it is introduced into the microorganism.

Moreover, the gene may be a DNA coding for a protein having an amino acid sequence of SEQ ID NO: 19, but which can include substitution, deletion, insertion, addition or the like of one or several amino acid residues, so long as the triosephosphate isomerase activity is not reduced.

The triosephosphate isomerase activity can be measured by using the method of Andersen and Cooper (FEBS Lett., 4, 19-20 (1969)). The activity can be increased by methods similar to the aforementioned methods for enhancing glycerol dehydrogenase, dihydroxyacetone kinase, and fructose-6-phosphate aldolase activities.

"Fructose bisphosphate aldolase" reversibly catalyzes the following reaction which converts dihydroxyacetone phosphate and glyceroaldehyde-3-phosphate into D-fructose-1,6-bisphosphate (EC:4.1.2.13).

Dihydroxyacetone phosphate (Glycerone phosphate) + D-Glyceraldehyde-3-phosphate -> D-Fructose-1,6-bisphosphate

The phrase "modified to increase the fructose bisphosphate aldolase activity" can mean that the number of the fructose bisphosphate aldolase molecules per cell can be increased as compared to that of a wild-type strain or non-modified strain, or that the activity of the fructose bisphosphate aldolase per molecule can be improved as compared to that of a wild-type strain or non-modified strain. The fructose bisphosphate aldolase can be modified so that the activity per cell is improved to 150% or more, 200% or more, or even 300% or more, of the activity of a wild-type strain or non-modified strain. Examples of wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth.

• Examples of the gene coding for fructose bisphosphate aldolase include the *fbaA* gene derived from *Escherichia coli* (SEQ ID NO: 20, complementary strand of the nucleotide numbers 3068187..3069266 of GenBank Accession No. NC_000913) and the *fbaB* gene derived from *Escherichia coli* (SEQ ID NO: 72, complementary strand of the nucleotide numbers 2175534..2176586 of GenBank Accession No. NC_000913).

The gene coding for fructose bisphosphate aldolase can be a DNA which hybridizes with a complementary sequence of the nucleotide sequence of SEQ ID NO: 20 or 72, or a probe which can be prepared from the complementary sequence, under stringent conditions, and codes for a protein having the fructose bisphosphate aldolase activity. Examples also include a DNA coding for the protein of SEQ ID NO: 21 or 73. The protein can have homology of 80% or more, 90% or more, 95% or more, 98% or more, or even 99% or more, to the total amino acid sequence of SEQ ID NO: 21, so long as it shows increased fructose bisphosphate aldolase activity in a microorganism belonging to the family *Enterobacteriaceae,* when it is introduced into the microorganism.

Moreover, the gene may be a DNA coding for a protein having an amino acid sequence of SEQ ID NO: 21 or 73, but which can include substitution, deletion, insertion, addition or the like of one or several amino acid residues, so long as the fructose bisphosphate aldolase activity is not reduced.

The fructose bisphosphate aldolase activity can be measured by using the method of Richard & Rutter (J. Biol. Chem., 236, 3177-3184). The activity can be increased by methods similar to the aforementioned methods for enhancing glycerol dehydrogenase, dihydroxyacetone kinase, and fructose-6-phosphate aldolase activities.

"Fructose-1,6-bisphosphatase" reversibly catalyzes the following reaction that converts D-fructose-1,6-bisphosphate into D-fructose-6-phosphate (EC:3.1.3.11).

D-Fructose-1,6-bisphosphate + H₂O -> D-Fructose-6-phosphate + Phosphate

The phrase "being modified to increase the fructose-1,6-bisphosphatase activity" can mean that the number of the fructose-1,6-bisphosphatase molecules per cell can be increased as compared to that of a wild-type strain or non-modified strain, or that the activity of the fructose-1,6-bisphosphatase per molecule is improved as compared to that of a wild-type strain or non-modified strain. The fructose-1,6-bisphosphatase can be modified so that the activity per cell is improved to 150% or more, 200% or more, or even 300% or more, of the activity of a wild-type strain or non-modified strain. Examples of wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth.

Examples of the gene coding for fructose-1,6-bisphosphatase include the *glpX* gene (SEQ ID NO: 22, complementary strand of the nucleotide numbers 4112592..4113602 of GenBank Accession No. NC_000913), the *fbp* gene (SEQ ID NO: 82, the nucleotide numbers 4452634..4453632 of GenBank Accession No. NC_000913), and the *ybhA* gene (SEQ ID NO: 84, the nucleotide numbers 796836..7976554 of GenBank Accession No. NC_000913), which are derived from *Escherichia coli.*

The gene coding for the fructose-1,6-bisphosphatase may be a DNA which hybridizes with a complementary sequence of the nucleotide sequence of SEQ ID NO: 22, 82 or 84, or a probe which can be prepared from the complementary sequence, under stringent conditions, and codes for a protein having the fructose-1,6-bisphosphatase activity. Examples also include a DNA coding for the protein of SEQ ID NO: 23, 83 or 85. The protein can be a protein showing a homology of 80% or more, preferably 90% or more, more preferably 95% or more, still more preferably 98% or more, particularly preferably 99% or more, to the total amino acid sequence of SEQ ID NO: 23, 83 or 85, so long as it shows increased fructose-1,6-bisphosphatase activity in a microorganism belonging to the family *Enterobacteriaceae,* when it is introduced into the microorganism.

Moreover, the gene may be a DNA coding for a protein having an amino acid sequence of SEQ ID NO: 23, 83 or 85, but which can include substitution, deletion, insertion, addition or the like of one or several amino acid residues, so long as the fructose-1,6-bisphosphatase activity is not reduced.

The fructose-1,6-bisphosphatase activity can be measured by using the method of Nakajima et al. (Protein Nucleic Enzyme, 22, 1585-1589). The activity can be increased by methods similar to the aforementioned methods for enhancing glycerol dehydrogenase and dihydroxyacetone kinase.

The microorganism can be modified to reduce glycerol kinase and/or membrane-binding type glycerol-3-phosphate dehydrogenase activity, in addition to the enhancement of glycerol dehydrogenase, dihydroxyacetone kinase, and fructose-6-phosphate aldolase, the enhancement of the glycerol uptake activity, and the enhancement of activities of one or more kinds of of triosephosphate isomerase, fructose bisphosphate aldolase, and fructose-1,6-bisphosphatase.

"Glycerol kinase" reversibly catalyzes the following reaction that generates glycerol-3-phosphate and ADP from glycerol and ATP (EC2.7.1.30)

ATP + Glycerol -> ADP + sn-Glycerol-3-phosphate

The phrase "modified to reduce the glycerol kinase activity" can mean that the number of the glycerol kinase molecules per cell is decreased as compared to that of a wild-type strain or non-modified strain, or that the activity of the glycerol kinase per molecule is reduced as compared to that of a wild-type strain or non-modified strain. The glycerol kinase is modified so that the activity per cell is reduced to 70% or less, 50% or less, 30% or less, or even 20% or less, of the activity of a wild-type strain or non-modified strain, and the enzymatic activity may be deleted. The enzymatic activity can be decreased by reducing the expression of the gene coding for the enzyme. Reduction of the expression of the gene includes reduction of the transcription amount of the mRNA transcribed from the gene and reduction of the translation amount of the mRNA.

The production of the enzyme protein molecule can be completely eliminated, or the activity per enzyme protein molecule can be reduced or deleted by disrupting the gene coding for the enzyme. Examples of wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth.

Examples of the gene coding for glycerol kinase include the *glpK* gene (SEQ ID NO: 24, complementary strand of the nucleotide numbers 4113737..4115245 of GenBank Accession No. NC_000913) derived from *Escherichia coli.* The enzymatic activity of glycerol kinase can be measured by the method of Thorner & Paulus (The Enzymes, 3rd ed., 8, 487-508).

"Membrane-binding type glycerol-3-phosphate dehydrogenase" catalyzes the oxidation reaction converting glycerol-3-phosphate to dihydroxyacetone phosphate, and reversibly catalyzes the following reaction.

sn-Glycerol-3P + Ubiquinone -> Dihydroxyacetone-P + Ubiquinol (EC: 1.1.99.5)

The phrase "modified to reduce the membrane-binding type glycerol-3-phosphate dehydrogenase activity" can mean that the number of the membrane-binding type glycerol-3-phosphate dehydrogenase molecules per cell is decreased as compared to that of a wild-type strain or non-modified strain, or that the activity of the membrane-binding type glycerol-3-phosphate dehydrogenase per molecule is reduced as compared to that of a wild-type strain or non-modified strain. The membrane-binding type glycerol-3-phosphate dehydrogenase can be modified so that the activity per cell is reduced to 70% or less, 50% or less, or even 30% or less, of the activity of a wild-type strain or non-modified strain, and the enzymatic activity may be deleted. The enzymatic activity can be decreased by reducing the expression of the gene coding for the enzyme. Examples of wild-type strains of the microorganism belonging to the family *Enterobacteriaceae* which can serve as a reference for comparison include the *Escherichia coli* MG1655 strain (ATCC No. 47076) and W3110 strain (ATCC No. 27325), *Pantoea* ananatis AJ13335 strain (FERM BP-6615), and so forth.

The membrane-binding type glycerol-3-phosphate dehydrogenase is encoded by the *glpABC* operon and the *glpD* gene, and examples of the *glpA* gene of *Escherichia coli* include the sequence of SEQ ID NO: 26 (the nucleotide numbers 2350669..2352297 of GenBank Accession No. NC_000913), examples of the *glpB* gene of *Escherichia coli* include the sequence of SEQ ID NO: 28 (the nucleotide numbers 2352287..2353546 of GenBank Accession No. NC_000913), examples of the *glpC* gene of *Escherichia coli* include the sequence of SEQ ID NO: 30 (the nucleotide numbers 2353543..2354733 of GenBank Accession No. NC_000913), and examples of the *glpD* gene of *Escherichia coli* include the sequence of SEQ ID NO: 32 (the nucleotide numbers 3560036..3561541 of GenBank Accession No. NC_000913).

Activity of an objective enzyme such as glycerol kinase and glycerol-3-phosphate dehydrogenase mentioned above can be reduced by
(1) reduction or deletion of the enzymatic activity by introduction of a mutation into the coding region of the gene coding for the objective enzyme, or
(2) reduction or deletion of the enzymatic activity by modification of an expression control sequence of the gene coding for the objective enzyme.

### (1) Reduction or deletion of the enzymatic activity by introduction of a mutation into the coding region of gene coding for the objective enzyme

A mutation can be introduced into the coding region of the gene coding for an objective enzyme by introducing a mutation for an amino acid substitution (missense mutation), a stop codon (nonsense mutation), or a frame shift mutation which adds or deletes one or two nucleotides into a region of the objective gene coding for the enzyme on a chromosome by genetic recombination (Journal of Biological Chemistry, 272:8611-8617 (1997); Proceedings of the National Academy of Sciences, USA, 95 5511-5515 (1998); Journal of Biological Chemistry, 266, 20833-20839 (1991)). Apart or all of the gene in the coding region can be deleted. Specifically, a mutation can be introduced into a part of the DNA of SEQ ID NO: 24, 26, 28, 30 or 32, or deleting a part or all of such DNA.

The enzymatic activity can also be reduced or deleted by constructing a gene coding for a mutant enzyme in which the coding region is deleted or into which a mutation is introduced, and substituting the mutated gene for the normal gene on the chromosome by homologous recombination or the like, or by introducing a transposon or IS factor into the gene.

For introduction of such mutations for reducing or deleting activity of an enzyme as described above into a gene by genetic recombination, for example, the following methods can be used. A partial sequence of an objective gene can be modified to prepare a mutant gene designed so that it does not produce an enzyme that functions normally, and transforming a microorganism belonging to the family *Enterobacteriaceae* with a DNA containing the gene to cause recombination of the mutant gene and the corresponding gene on the chromosome. In this way, the objective gene on the chromosome can be replaced with the mutant gene. For such gene substitution utilizing homologous recombination, there can be utilized a method called Red-driven integration (Datsenko, K.A, and Wanner, B.L., Proc. Natl. Acad. Sci. USA, 97:6640-6645 (2000)), a method of using a linear DNA such as a method utilizing the Red driven integration in combination with an excisive system derived from λ phage (Cho, E.H., Gumport, R.I., Gardner, J.F., J. Bacteriol., 184:5200-5203 (2002)) (refer to WO2005/010175), a method of using a plasmid containing a temperature sensitive replication origin (Datsenko, K.A, and Wanner, B.L., Proc. Natl. Acad. Sci. USA, 97:6640-6645 (2000), U.S. Patent No. 6,303,383, Japanese Patent Laid-open No. 05-007491), and so forth. Moreover, such site-specific mutagenesis based on gene substitution utilizing homologous recombination as described above can also be performed by using a plasmid which is not able to replicate in a host. Moreover, the enzymatic activity can also be reduced or deleted by introducing a mutation into the coding region of an objective gene using a usual mutation treatment, such as X-ray or ultraviolet irradiation, or by using a mutation agent such as N-methyl-N'-nitro-N-nitrosoguanidine.

### (2) Reduction or deletion of enzymatic activity by modification of expression control sequence of the gene coding for objective enzyme

Enzymatic activity by modification of an expression control sequence of the gene coding for an objective enzyme can also be reduced or deleted by reducing the expression by introducing a mutation into an expression control sequence, such as a promoter or a SD sequence, on a chromosomal DNA, by amplifying the gene coding for a regulator which reduces expression of the gene, or by deleting or attenuating the gene coding for an activator which improves expression of the gene. Methods for evaluating the potency of promoters and examples of potent promoters are described in the paper of Goldstein et al. (Prokaryotic promoters in biotechnology, Biotechnol. Annu. Rev., 1995, 1, 105-128), and so forth. Furthermore, it is known that by replacing several nucleotides in the spacer region between the ribosome binding site (RBS) and the start codon, in particular, in the region immediately upstream from the start codon, the translation efficiency of mRNA can be significantly affected, and such a region can also be modified. In particular, the *glpA, B,* and *C* genes make up an operon structure, and therefore the expression of the entire operon can be reduced by introducing a mutation into an expression control region such as the promoter region located upstream of *glpA.*

In the bacterium, in order to further enhance the glycerol assimilation ability, expression of the *glpR* gene (EP 1715056) may be attenuated, or expression of the glycerol metabolism genes such as *glpE, glpG, glpQ, glpT,* and *dhK* genes (EP 1715055 A, International Patent Publication WO2007/013695) may be enhanced.

### <3> Microorganisms and methods for imparting L-amino acid-producing ability

A bacterium belonging to the family *Enterobacteriaceae* that is able to produce L-amino acids, which can metabolize glycerol as a carbon source, can be used as a parent strain. A bacterium obtained by imparting the aforementioned properties suitable for glycerol metabolism to such a parent strain can also be used. Alternatively, the L-amino acid-producing ability may be imparted by the method described below to a bacterium already imparted with the aforementioned properties.

The family *Enterobacteriaceae* encompasses bacteria belonging to the genera of *Escherichia, Enterobacter, Erwinia, Klebsiella, Pantoea, Photorhabdus, Providencia, Salmonella, Serratia, Shigella, Morganella, Yersinia,* and so forth. In particular, bacteria classified into the family *Enterobacteriaceae* according to the taxonomy used by the NCBI (National Center for Biotechnology Information) database (http://www.ncbi.nlm.nih.gov/Taxonomy/Browser/wwwtax.cgi?id=91347) are preferred.

The expression "a bacterium belonging to the genus *Escherichia*" can mean that the bacterium is classified into the genus *Escherichia* according to classification known to a person skilled in the art of microbiology, although the bacterium is not particularly limited. Examples of the bacterium belonging to the genus *Escherichia* include, but are not limited to, *Escherichia coli (E. coli).*

The bacterium belonging to the genus *Escherichia* is not particularly limited. However, examples include, for example, the bacteria of the phyletic groups described in the work of Bachmann et al., Table 1 (Bachmann, B.J., 1996, pp.2460-2488, In F.D. Neidhardt (ed.), Escherichia coli and Salmonella: Cellular and Molecular Biology/Second Edition, American Society for Microbiology Press, Washington, D.C.). Specific examples include *Escherichia coli* W3110 (ATCC 27325), *Escherichia coli* MG1655 (ATCC 47076), and so forth, which are derived from the prototype wild-type strain, K12 strain.

These strains are available from, for example, American Type Culture Collection (Address: 12301 Parklawn Drive, Rockville, Maryland 20852, United States of America). That is, accession numbers are given to each of the strains, and the strains can be ordered by using these numbers. The accession numbers of the strains are listed in the catalogue of the American Type Culture Collection.

The expression of a bacterium belonging to the genus *Pantoea* means that the bacterium is classified into the genus *Pantoea* according to classification known to a person skilled in the art of microbiology. Some strains of *Enterobacter agglomerans* have been recently re-classified into *Pantoea agglomerans, Pantoea ananatis, Pantoea stewartii* or the like based on the nucleotide sequence analysis of 16S rRNA etc. (Int. J. Syst. Bacteriol., 43, 162-173 (1993)). Bacteria belonging to the genus *Pantoea* can encompass such bacteria which have been re-classified into the genus *Pantoea* as described above.

A bacterium having an L-amino acid-producing ability (the ability to produce an L-amino acid) means a bacterium that can produce and secret an L-amino acid in a medium when it is cultured in the medium. It can also mean a bacterium which can accumulate an objective L-amino acid in the medium in an amount not less than 50 g/L, not less than 75 g/L, or even not less than 100 g/L. The "L-amino acid" can encompass L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. L-Threonine and L-lysine are particular examples.

Any of the L-amino acid-producing bacteria reported so far can be used as a parent strain, so long as the chosen strain that can assimilate glycerol or to which glycerol assimilation ability can be imparted. Hereafter, L-amino acid-producing bacteria are described for each L-amino acid.

### L-Threonine-producing bacteria

Examples of L-threonine-producing bacteria and parent strains which can be used to derive such bacteria include, but are not limited to, strains belonging to the genus *Escherichia,* such as *E. coli* TDH-6/pVIC40 (VKPM B-3996) (U.S. Patent No. 5,175,107, U.S. Patent No. 5,705,371), *E. coli* 472T23/pYN7 (ATCC 98081) (U.S. Patent No. 5,631,157), *E*. *coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E. coli* MG442 (Gusyatiner et al., Genetika (in Russian), 14, 947-956 (1978)), *E. coli* VL643 and VL2055 (EP 1149911 A) and so forth.

The strain TDH-6 is deficient in the *thrC* gene, as well as being sucrose-assimilative, and the *ilvA* gene has a leaky mutation. This strain also has a mutation in the *rhtA* gene, which imparts resistance to high concentration of threonine or homoserine. The B-3996 strain harbors the plasmid pVIC40 obtained by inserting a *thrA *BC* operon containing a mutant *thrA* gene into a RSF1010-derived vector. This mutant *thrA* gene encodes aspartokinase homoserine dehydrogenase I which is substantially desensitized to feedback inhibition by threonine. The B-3996 strain was deposited on November 19, 1987 at the All-Union Scientific Center of Antibiotics (Nagatinskaya Street 3-A, 117105 Moscow, Russia) under the accession number RIA 1867. The strain was also deposited at the Russian National Collection of Industrial Microorganisms (VKPM) on April 7, 1987 under the accession number VKPM B-3996.

*E. coli* VKPM B-5318 (EP 0593792 B) can also be used as an L-threonine-producing bacterium or a parent strain for deriving it. The B-5318 strain is prototrophic with regard to isoleucine, and in this strain, a temperature-sensitive lambda-phage Cl repressor and PR promoter replace the regulatory region of the threonine operon in the plasmid pVIC40. The strain VKPM B-5318 was deposited at the Russian National Collection of Industrial Microorganisms (VKPM) (1 Dorozhny proezd., 1 Moscow 117545, Russia) on May 3, 1990 under the accession number of VKPM B-5318.

The bacterium can be additionally modified to increase expression of one or more of the following genes:
- the mutant *thrA* gene which codes for aspartokinase-homoserine dehydrogenase I resistant to feed back inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase).

The *thrA* gene which encodes aspartokinase-homoserine dehydrogenase I of *Escherichia coli* has been elucidated (nucleotide numbers 337 to 2799, GenBank accession NC_000913.2, gi: 49175990). The *thrA* gene is located between the *thrL* and *thrB* genes on the chromosome *of E. coli* K-12. The *thrB* gene which encodes homoserine kinase of *Escherichia coli* has been elucidated (nucleotide numbers 2801 to 3733, GenBank accession NC_000913.2, gi: 49175990). The *thrB* gene is located between the *thrA* and *thrC* genes on the chromosome *of E. coli* K-12. The *thrC* gene which encodes threonine synthase of *Escherichia coli* has been elucidated (nucleotide numbers 3734 to 5020, GenBank accession NC_000913.2, gi: 49175990). The *thrC* gene is located between the *thrB* gene and the *yaaX* open reading frame on the chromosome of *E. coli* K-12. All three of these genes function as a single threonine operon. To increase expression of the threonine operon, the attenuator region which affects the transcription can be removed from the operon (WO2005/049808, WO2003/097839).

The mutant *thrA* gene which codes for aspartokinase-homoserine dehydrogenase I resistant to feed back inhibition by threonine as well as the *thrB* and *thrC* genes can be obtained as one operon from the well-known plasmid pVIC40 which is present in the threonine-producing *E. coli* strain VKPM B-3996. The plasmid pVIC40 is described in detail in U.S. Patent No. 5,705,371.

The *rhtA* gene is present at 18 min on the *E. coli* chromosome close to the *glnHPQ* operon, which encodes components of the glutamine transport system. The *rhtA* gene is identical to ORF1 (*ybiF* gene, nucleotide numbers 764 to 1651, GenBank accession number AAA218541, gi:440181) and is located between the *pexB* and *ompX* genes. The unit expressing a protein encoded by the ORF1 has been designated *rhtA* gene (rht: resistance to homoserine and threonine). It has also been revealed that the *rhtA23* mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457; EP 1013765 A).

The *asd* gene *of E. coli* has already been elucidated (nucleotide numbers 3572511 to 3571408, GenBank Accession NC_000913.1, gi:16131307), and can be obtained by PCR (refer to White, T.J., Arnheim, N., and Erlich, H.A., Trends Genet., 5, 185-189 (1989)) utilizing primers prepared based on the nucleotide sequence of the gene. The *asd* genes of other microorganisms can be obtained in a similar manner.

The *aspC* gene *of E. coli* has also already been elucidated (nucleotide numbers 983742 to 984932, GenBank Accession NC_000913.1, gi:16128895), and can be obtained by PCR. The *aspC* genes of other microorganisms can be obtained in a similar manner.

### L-Lysine-producing bacteria

Examples of L-lysine-producing bacteria belonging to the genus *Escherichia* include mutants having resistance to an L-lysine analogue. L-Lysine analogues inhibit growth of bacteria belonging to the genus *Escherichia,* but this inhibition is fully or partially desensitized when L-lysine is present in a medium. Examples of the L-lysine analogue include, but are not limited to, oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The WC196 strain can be used as an L-lysine-producing bacterium of *Escherichia coli.* This bacterial strain was bred from the W3110 strain, which was derived from *Escherichia coli* K-12, by replacing the *lysC* gene on the chromosome of the W3110 strain with a mutant *lysC* gene encoding a mutant aspartokinase III in which threonine at position 352 had been replaced with isoleucine, resulting in desensitization of the feedback inhibition thereof by L-lysine (U.S. patent No. 5,661,012), and then conferring AEC resistance to the resulting strain. This strain was designated *Escherichia coli* AJ13069 and was deposited at the National Institute of Bioscience and Human-Technology, Agency of Industrial Science and Technology (currently National Institute of Advanced Industrial Science and Technology, International Patent Organism Depositary, Central 6, 1-1, Higashi 1-Chome, Tsukuba-shi, Ibaraki-ken, 305-8566, Japan) on December 6, 1994 and assigned an accession number of FERM P-14690. Then, it was converted to an international deposit under the provisions of the Budapest Treaty on September 29, 1995, and assigned an accession number of FERM BP-5252 (U.S. Patent No. 5,827,698).

Examples of L-lysine-producing bacteria and parent strains which can be used to derive such bacteria also include strains in which expression of one or more genes encoding an L-lysine biosynthetic enzyme is increased. Examples of such genes include, but are not limited to, dihydrodipicolinate synthase gene (*dapA*)*,* aspartokinase gene (*lysC*)*,* dihydrodipicolinate reductase gene (*dapB*), diaminopimelate decarboxylase gene (*lysA*), diaminopimelate dehydrogenase gene (*ddh*) (U.S. Patent No. 6,040,160), phosphoenolpyrvate carboxylase gene (*ppc*), aspartate semialdehyde dehydrogenease gene (*asd*)*,* and aspartase gene (*aspA*) (EP 1253195 A). In addition, the parent strains can have an increased level of expression of the gene involved in energy efficiency (*cyo*) (EP 1170376 A), the gene encoding nicotinamide nucleotide transhydrogenase (*pntAB*) (U.S. Patent No. 5,830,716), *the ybjE* gene (WO2005/073390), the gene coding for glutamate dehydrogenase (*gdhA,* Gene, 23:199-209 (1983)), or combinations thereof. Abbreviations of the genes are indicated in the parentheses.

It is known that wild-type dihydrodipicolinate synthetase derived from *Escherichia coli* suffers from feedback inhibition by L-lysine, while wild-type aspartokinase derived from *Escherichia coli* suffers from suppression and feedback inhibition by L-lysine. Therefore, when the *dapA* and *lysC* genes are used, these genes can be mutant genes coding the enzymes that do not suffer from the feedback inhibition by L-lysine.

Examples of DNA encoding a mutant dihydrodipicolinate synthetase desensitized to feedback inhibition by L-lysine include a DNA encoding a protein that has the amino acid sequence of the enzyme in which the histidine at position 118 is replaced by tyrosine. Examples of DNA encoding a mutant aspartokinase desensitized to feedback inhibition by L-lysine include a DNA encoding an AKIII having the amino acid sequence in which the threonine at position 352, the glycine at position 323, and the methionine at position 318 are replaced by isoleucine, asparagine and isoleucine, respectively (for these mutants, see U.S. Patent No. 5,661,012 and U.S. Patent No. 6,040,160). Such mutant DNAs can be obtained by site-specific mutagenesis using PCR or the like.

Wide host-range plasmids RSFD80, pCAB1, and pCABD2 are known as plasmids containing a mutant *dapA* gene encoding a mutant dihydrodipicolinate synthetase and a mutant *lysC* gene encoding a mutant aspartokinase (U.S. Patent No. 6,040,160). *Escherichia coli* JM109 strain transformed with RSFD80 was named AJ12396 (U.S. Patent No. 6,040,160), the strain was deposited at the National Institute of Bioscience and Human-Technology, Agency of Industrial Science and Technology, Ministry of International Trade and Industry (currently, International Patent Organism Depositary, National Institute of Advanced Industrial Science and Technology) on October 28, 1993 and assigned an accession number of FERM P-13936, and the deposit was then converted to an international deposit under the provisions of Budapest Treaty on November 1, 1994 and assigned an accession number of FERM BP-4859. RSFD80 can be obtained from the AJ12396 strain by a known method.

Examples of L-lysine-producing bacteria and parent strains which can be used to derive such bacteria also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase, lysine decarboxylase (U.S. Patent No. 5,827,698), and the malic enzyme (WO2005/010175). In order to reduce or delete the lysine decarboxylase activity, it is preferable to reduce expression of both the *cadA* gene and *ldcC* gene coding for lysine decarboxylase (International Patent Publication WO2006/038695).

Examples of *cadA* gene- and *ldcC* gene-disrupted strain include the *Escherichia coli* WC196LC (WC196ΔcadAΔldc) strain. The WC196LC strain, which was designated AJ110692, was deposited at National Institute of Advanced Industrial Science and Technology, International Patent Organism Depositary (Central 6, 1-1, Higashi 1-Chome, Tsukuba-shi, Ibaraki-ken, 305-8566, Japan) on October 7, 2008 as an international deposit, and assigned an accession number of FERM BP-11027.

### L-Cysteine-producing bacteria

Examples of L-cysteine-producing bacteria and parent strains which can be used to derive such bacteria include, but are not limited to, strains belonging to the genus *Escherichia,* such as *E. coli* JM15 which is transformed with different *cysE* alleles coding for feedback-resistant serine acetyltransferases (U.S. Patent No. 6,218,168, Russian Patent Application No. 2003121601); *E. coli* W3110 having over-expressed genes which encode proteins suitable for secreting substances toxic for cells (U.S. Patent No. 5,972,663); *E. coli* strains having lowered cysteine desulfohydrase activity (Japanese Patent Laid-open No. 11-155571); and *E. coli* W3110 with increased activity of a positive transcriptional regulator for cysteine regulon encoded by the *cysB* gene (WO01/27307).

### L-Leucine-producing bacteria

Examples of L-leucine-producing bacteria and parent strains which can be used to derive such bacteria include, but are not limited to, strains belonging to the genus *Escherichia,* such as *E. coli* strains resistant to leucine (for example, the strain 57 (VKPM B-7386, U.S. Patent No. 6,124,121)) or leucine analogues including β-2-thienylalanine, 3-hydroxyleucine, 4-azaleucine and 5,5,5-trifluoroleucine (Japanese Patent Publication (Kokoku) No. 62-34397 and Japanese Patent Laid-open No. 8-70879); *E. coli* strains obtained by a gene engineering method described in WO96/06926; and *E. coli* H-9068 (Japanese Patent Laid-open No. 8-70879).

The bacterium can be improved by enhancing expression of one or more genes involved in L-leucine biosynthesis. Such genes include the genes of the *leuABCD* operon, of which a typical example is a mutant *leuA* gene coding for isopropyl malate synthase desensitized to feedback inhibition by L-leucine (U.S. Patent No. 6,403,342). In addition, the bacterium can be improved by increasing expression of one or more genes coding for proteins which excrete L-amino acid from bacterial cells. Examples of such genes include the *b2682* and *b2683* genes *(ygaZH* genes) (EP 1239041 A2).

### L-Histidine-producing bacteria

Examples of L-histidine-producing bacteria and parent strains which can be used to derive such bacteria include, but are not limited to, strains belonging to the genus *Escherichia,* such as *E. coli* strain 24 (VKPM B-5945, RU 2003677); *E. coli* strain 80 (VKPM B-7270, RU 2119536); *E. coli* NRRL B-12116 to B12121 (U.S. Patent No. 4,388,405); *E. coli* H-9342 (FERM BP-6675) and H-9343 (FERM BP-6676) (U.S. Patent No. 6,344,347); *E. coli* H-9341 (FERM BP-6674) (EP 1085087); and *E. coli* AI80/pFM201 (U.S. Patent No. 6,258,554).

Examples of L-histidine-producing bacteria and parent strains which can be used to derive such bacteria also include strains in which expression of one or more genes encoding an L-histidine biosynthetic enzyme is increased. Examples of such genes include ATP phosphoribosyl transferase gene (*hisG*)*,* phosphoribosyl AMP cyclohydrolase gene (*hisI*)*,* phosphoribosyl-ATP pyrophosphohydrolase gene (*hisI*)*,* phosphoribosylformimino-5-aminoimidazole carboxamide ribotide isomerase gene (*hisA*), amidotransferase gene (*hisH*)*,* histidinol phosphate aminotransferase gene (*hisC*)*,* histidinol phosphatase gene (*hisB*), histidinol dehydrogenase gene (*hisD*), and so forth.

It is known that the L-histidine biosynthetic enzymes encoded by *hisG* and *hisBHAFI* are inhibited by L-histidine, and therefore L-histidine-producing ability can also be efficiently enhanced by introducing a mutation which confers resistance to the feedback inhibition into the ATP phosphoribosyl transferase gene (*hisG*) (Russian Patent Nos. 2003677 and 2119536).

Specific examples of strains having L-histidine-producing ability include *E. coli* FERM-P 5038 and 5048 which are introduced with a vector carrying a DNA encoding an L-histidine biosynthetic enzyme (Japanese Patent Laid-open No. 56-005099), *E. coli* strains introduced with a gene for amino acid-export (EP 1016710 A), *E. coli* 80 strain imparted with sulfaguanidine, DL-1,2,4-triazole-3-alanine, and streptomycin resistance (VKPM B-7270, Russian Patent No. 2119536), and so forth.

### L-Glutamic acid-producing bacteria

Examples of L-glutamic acid-producing bacteria and parent strains which can be used to derive such bacteria include, but are not limited to, strains belonging to the genus *Escherichia,* such as *E. coli* VL334thrC⁺ (EP 1172433). *E. coli* VL334 (VKPM B-1641) is an L-isoleucine and L-threonine auxotrophic strain having mutations in *thrC* and *ilvA* genes (U.S. Patent No. 4,278,765). A wild-type allele of the *thrC* gene was transferred by the method of general transduction using a bacteriophage PI grown on the wild-type *E. coli* K12 strain (VKPM B-7) cells. As a result, an L-isoleucine auxotrophic L-glutamic acid-producing strain VL334thrC⁺ (VKPM B-8961) was obtained.

Examples of L-glutamic acid-producing bacteria and parent strains which can be used to derive such bacteria include, but are not limited to, strains in which expression of one or more genes encoding an L-glutamic acid biosynthetic enzyme is increased. Examples of such genes include genes encoding glutamate dehydrogenase (*gdhA*), glutamine synthetase (*glnA*)*,* glutamate synthetase (*gltAB*), isocitrate dehydrogenase (*icdA*)*,* aconitate hydratase (*acnA, acnB*)*,* citrate synthase (*gltA*), phosphoenolpyruvate carboxylase (*ppc*), pyruvate dehydrogenase (*aceEF, lpdA*)*,* pyruvate kinase (*pykA,pykF*)*,* phosphoenolpyruvate synthase (*ppsA*)*,* enolase (*eno*), phosphoglyceromutase (*pgmA, pgmI*)*,* phosphoglycerate kinase (*pgk*), glyceraldehyde-3-phophate dehydrogenase (*gapA*)*,* triose phosphate isomerase (*tpiA*)*,* fructose bisphosphate aldolase (*fbp*)*,* phosphofructokinase (*pjkA, pjkB*)*,* glucose phosphate isomerase (*pgi*), and so forth.

Examples of strains modified to increase expression of the citrate synthetase gene, the phosphoenolpyruvate carboxylase gene, and/or the glutamate dehydrogenase gene include those disclosed in EP 1078989 A, EP 955368 A and EP 952221 A.

Examples of L-glutamic acid-producing bacteria and parent strains which can be used to derive such bacteria also include strains having decreased or eliminated activity of an enzyme that catalyzes synthesis of a compound other than L-glutamic acid by branching off from an L-glutamic acid biosynthesis pathway. Examples of such enzymes include isocitrate lyase (*aceA*)*,* α-ketoglutarate dehydrogenase (*sucA*)*,* phosphotransacetylase (*pta*)*,* acetate kinase (*ack*)*,* acetohydroxy acid synthase (*ilvG*), acetolactate synthase (*ilvI*)*,* formate acetyltransferase (*pfl*)*,* lactate dehydrogenase (*ldh*)*,* glutamate decarboxylase (*gadAB*), γ-glutamyl transferase (*ggt*), γ-glutamylcysteine synthetase (*gshA*)*,* γ-glutamylputrescine synthetase (*ycjK*)*,* and so forth. Bacteria belonging to the genus *Escherichia* deficient in α-ketoglutarate dehydrogenase activity or having reduced α-ketoglutarate dehydrogenase activity and methods for obtaining them are described in U.S. Patent Nos. 5,378,616 and 5,573,945.

Specific examples of such strains include the followings:
*E. coli* W3110sucA::Km^{r}
*E. coli* AJ12624 (FERM BP-3853)
*E. coli* AJ12628 (FERM BP-3854)
*E. coli* AJ12949 (FERM BP-4881)
*E. coli* W3110sucA::Km^{r} is a strain obtained by disrupting the α-ketoglutarate dehydrogenase gene (hereinafter also referred to as "*sucA* gene") *of E. coli* W3110. This strain is completely deficient in α-ketoglutarate dehydrogenase.

Other examples of L-glutamic acid-producing bacteria include those which belong to the genus *Escherichia* and have resistance to an aspartic acid antimetabolite. These strains may also be deficient in α-ketoglutarate dehydrogenase, and examples include, for example, *E. coli* AJ13199 (FERM BP-5807) (U.S. Patent No. 5,908,768), FFRM P-12379) which additionally has a lowered L-glutamic acid decomposing ability (U.S. Patent No. 5,393,671); AJ13138 (FERM BP-5565) (U.S. Patent No. 6,110,714), and so forth.

Examples of L-glutamic acid-producing bacteria include bacteria belonging to the genus *Pantoea* which are deficient in α-ketoglutarate dehydrogenase activity or have a decreased α-ketoglutarate dehydrogenase activity, and they can be obtained as described above. Such strains include *Pantoea ananatis* AJ13356 (U.S. Patent No. 6,331,419). *Pantoea ananatis* AJ13356 was deposited at the National Institute of Bioscience and Human-Technology, Agency of Industrial Science and Technology (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 under an accession number of FERM P-16645. It was then converted to an international deposit under the provisions of Budapest Treaty on January 11, 1999 and assigned an accession number of FERM BP-6616. *Pantoea ananatis* AJ13356 is deficient in α-ketoglutarate dehydrogenase activity as a result of disruption of the αKGDH-E1 subunit gene (*sucA*). This strain was identified as *Enterobacter agglomerans* when it was isolated and deposited as the *Enterobacter agglomerans* AJ13356. However, it was recently re-classified as *Pantoea ananatis* on the basis of nucleotide sequencing of 16S rRNA and so forth. Although AJ13356 was deposited at the aforementioned depository as *Enterobacter agglomerans,* it is described as *Pantoea ananatis* in this specification.

### L-Phenylalanine-producing bacteria

Examples of L-phenylalanine-producing bacteria and parent strains which can be used to derive such bacteria include, but are not limited to, strains belonging to the genus *Escherichia,* such as *E. coli* AJ12739 (tyrA::Tn10, tyrR) (VKPM B-8197); *E. coli* HW1089 (ATCC 55371) harboring the mutant *pheA34* gene (U.S. Patent No. 5,354,672); *E. coli* MWEC101-b (KR 8903681); *E. coli* NRRL B-12141, NRRL B-12145, NRRL B-12146 and NRRL B-12147 (U.S. Patent No. 4,407,952). As parent strains, *E. coli* K-12 [W3110 (tyrA)/pPHAB] (FERM BP-3566), *E. coli* K-12 [W3110 (tyrA)/pPHAD] (FERM BP-12659), *E. coli* K-12 [W3110 (tyrA)/pPHATerm] (FERM BP-12662) and *E*. *coli* K-12 [W3110 (tyrA)/pBR-aroG4, pACMAB] named as AJ12604 (FERM BP-3579) may also be used (EP 488424 B1). Furthermore, L-phenylalanine-producing bacteria belonging to the genus *Escherichia* with an enhanced activity of the protein encoded by the *yedA* gene or the *yddG* gene can also be used (U.S. Patent Published Application Nos. 2003/0148473 A1 and 2003/0157667 A1).

### L-Tryptophan-producing bacteria

Examples of tryptophan-producing bacteria and parent strains which can be used to derive such bacteria include, but are not limited to, strains belonging to the genus *Escherichia,* such as *E. coli* JP4735/pMU3028 (DSM10122) and JP6015/pMU91 (DSM10123), which are deficient in the tryptophanyl-tRNA synthetase encoded by a mutant *trpS* gene (U.S. Patent No. 5,756,345); *E. coli* SV164 (pGH5) having a *serA* allele encoding phosphoglycerate dehydrogenase free from feedback inhibition by serine and a *trpE* allele encoding anthranilate synthase free from feedback inhibition by tryptophan (U.S. Patent No. 6,180,373); *E. coli* AGX17(pGX44) (NRRL B-12263) and AGX6(pGX50)aroP (NRRL B-12264) deficient in tryptophanase (U.S. Patent No. 4,371,614); and *E. coli* AGX17/pGX50,pACKG4-pps in which phosphoenolpyruvate-producing ability is enhanced (WO97/08333, U.S. Patent No. 6,319,696). L-Tryptophan-producing bacteria belonging to the genus *Escherichia* with an enhanced activity of the protein encoded by the *yedA* gene or the *yddG* gene may also be used (U.S. Patent Published Application Nos. 2003/0148473 A1 and 2003/0157667 A1).

Examples of L-tryptophan-producing bacteria and parent strains which can be used to derive such bacteria also include strains in which one or more activities of the enzymes selected from anthranilate synthase (*trpE*)*,* phosphoglycerate dehydrogenase (*serA*), and tryptophan synthase (*trpAB*) are increased. The anthranilate synthase and phosphoglycerate dehydrogenase both suffer from feedback inhibition by L-tryptophan and L-serine, and therefore a mutation desensitizing them to the feedback inhibition may be introduced into these enzymes. Specific examples of strains having such a mutation include *E. coli* SV164 which harbors desensitized anthranilate synthase and a transformant strain obtained by introducing the plasmid pGH5 (WO94/08031), which contains a mutant *serA* gene encoding feedback inhibition-desensitized phosphoglycerate dehydrogenase, into the *E. coli* SV164.

Examples of L-tryptophan-producing bacteria and parent strains which can be used to derive such bacteria also include strains into which the tryptophan operon containing a gene encoding inhibition-desensitized anthranilate synthase is introduced (Japanese Patent Laid-open Nos. 57-71397, 62-244382, U.S. Patent No. 4,371,614). Moreover, L-tryptophan-producing ability may be imparted by increasing expression of a gene which encodes tryptophan synthase in the tryptophan operon (*trpBA*)*.* The tryptophan synthase consists of α and β subunits which are encoded by the *trpA* and *trpB* genes, respectively. In addition, L-tryptophan-producing ability may also be improved by increasing expression of the isocitrate lyase-malate synthase operon (WO2005/103275).

### L-Proline-producing bacteria

Examples of L-proline-producing bacteria and parent strains which can be used to derive such bacteria include, but are not limited to, strains belonging to the genus *Escherichia,* such as *E. coli* 702ilvA (VKPM B-8012) which is deficient in the *ilvA* gene and is able to produce L-proline (EP 1172433).

The bacterium may be improved by increasing expression of one or more genes involved in L-proline biosynthesis. Such genes include the *proB* gene coding for glutamate kinase desensitized to feedback inhibition by L-proline (DE 3127361). In addition, the bacterium can be improved by increasing expression of one or more genes coding for proteins excreting L-amino acid from bacterial cells. Examples of such genes include the *b2682* and *b2683* genes (*ygaZH* genes) (EP 1239041 A2).

Examples of bacteria belonging to the genus *Escherichia* and having L-proline-producing ability include the following *E. coli* strains: NRRL B-12403 and NRRL B-12404 (British Patent No. 2075056), VKPM B-8012 (Russian Patent Application No. 2000124295), plasmid mutants described in German Patent No. 3127361, plasmid mutants described by Bloom F.R. et al. (The 15th Miami winter symposium, 1983, p. 34), and so forth.

### L-Arginine-producing bacteria

Examples of L-arginine-producing bacteria and parent strains which can be used to derive such bacteria include, but are not limited to, strains belonging to the genus *Escherichia,* such as *E. coli* strain 237 (VKPM B-7925) (U.S. Patent Published Application No. 2002/058315A1) and its derivative strains harboring a mutant N-acetylglutamate synthase (Russian Patent Application No. 2001112869), *E. coli* strain 382 (VKPM B-7926) (EP 1170358 A1), and an arginine-producing strain into which *argA* gene encoding N-acetylglutamate synthetase is introduced (EP 1170361 A1).

Examples of L-arginine-producing bacteria and parent strains which can be used to derive such bacteria also include strains in which expression of one or more genes encoding an L-arginine biosynthetic enzyme is increased. Examples of such genes include N-acetylglutamyl phosphate reductase gene (*argC*)*,* ornithine acetyl transferase gene (*argJ*)*,* N-acetylglutamate kinase gene (*argB*)*,* acetylornithine transaminase gene (*argD*)*,* ornithine carbamoyl transferase gene (*argF*)*,* argininosuccinic acid synthetase gene (*argG*)*,* argininosuccinic acid lyase gene (*argH*)*,* and carbamoyl phosphate synthetase gene *(carAB).*

### L-Valine-producing bacteria

Example of L-valine-producing bacteria and parent strains which can be used to derive such bacteria include, but are not limited to, strains which have been modified to overexpress the *ilvGMEDA* operon (U.S. Patent No. 5,998,178). It is desirable to remove the region of the *ilvGMEDA* operon which is required for attenuation so that expression of the operon is not attenuated by produced L-valine. Furthermore, the *ilvA* gene in the operon is preferably disrupted so that threonine deaminase activity is decreased.

Examples of L-valine-producing bacteria and parent strains which can be used to derive such bacteria also include mutants having a mutation of amino-acyl t-RNA synthetase (U.S. Patent No. 5,658,766). For example, *E. coli* VL1970, which has a mutation in the *ileS* gene encoding isoleucine tRNA synthetase, can be used. *E. coli* VL1970 was deposited at the Russian National Collection of Industrial Microorganisms (VKPM) (1 Dorozhny Proezd, 1 Moscow 117545, Russia) on June 24, 1988 under the accession number of VKPM B-4411.

Furthermore, mutants requiring lipoic acid for growth and/or lacking H⁺-ATPase (WO96/06926) can also be used as the parent strains.

### L-Isoleucine-producing bacteria

Examples of L-isoleucine-producing bacteria and parent strains which can be used to derive such bacteria include, but are not limited to, mutants having resistance to 6-dimethylaminopurine (Japanese Patent Laid-open No. 5-304969 A), mutants having resistance to an isoleucine analogue such as thiaisoleucine and isoleucine hydroxamate, and such mutants further having resistance to DL-ethionine and/or arginine hydroxamate (Japanese Patent Laid-open No. 5-130882). In addition, recombinant strains transformed with genes encoding proteins involved in L-isoleucine biosynthesis, such as threonine deaminase and acetohydroxate synthase, can also be used as the parent strains (Japanese Patent Laid-open No. 2-458, FR 0356739, and U.S. Patent No. 5,998,178).

### L-Asparagine-producing bacteria

L-Asparagine is produced by transferring amino group to aspartic acid (Boehlein, S.K., Richards, N.G.J., & Schuster, S.M. (1994a), J. Biol. Chem., 269, 7450-7457). Therefore, examples of L-asparagine-producing bacteria belonging to the genus *Escherichia* include L-asparatic acid-producing *Escherichia coli* strains in which asparagine synthetase is enhanced.

### Examples

Hereafter, the present invention will be more specifically explained with reference to examples.

### Reference Example 1: Construction of L-lysine-producing bacterium with enhanced fructose-6-phosphate aldolase, glycerol dehydrogenase, and dihydroxyacetone kinase activities (WO2008/102861)

### <1-1> Construction of plasmid for dakl gene expression

The total nucleotide sequence of *Saccharomyces cerevisiae* chromosome has already been determined (Science, 25 October, 274(5287), 546-567 (1996)). On the basis of the nucleotide sequence of the *dakl* gene reported in this literature, the synthetic oligonucleotide of SEQ ID NO: 14 was prepared as a 5' primer, and the synthetic oligonucleotide of SEQ ID NO: 15 was prepared as a 3' primer. PCR was performed by using these synthetic oligonucleotides and the chromosomal DNA of the *Saccharomyces cerevisiae* JCM7255 strain as a template. The PCR product was purified and ligated with the vector pMW119 (Takara Bio) digested with *Hin*dIII and *Sal*I to construct a *dαkI* expression plasmid pMW-dak1. The JCM7255 strain is stored in the independent administrative agency, RIKEN, "Japan Collection of Microorganisms", 2-1, Hirosawa, Wako-shi, Saitama-ken.

### <1-2> Construction of a strain with improved glycerol dehydrogenase activity

A strain containing the glycerol dehydrogenase gene (PtacMgldA::Cm) having the structure shown in SEQ ID NO: 11 was constructed from the WC196ΔcadAΔldcC strain (refer to International Patent Publication WO2006/038695). For the construction of the strain having this structure, the DNA of SEQ ID NO: 9 (PCR product) was used. In the sequence of the DNA of SEQ ID NO: 9, the nucleotide numbers 1 to 172 is the *attR* sequence of λ phage, the nucleotide numbers 324 to 983 is a chloramphenicol resistance gene (*cat*)*,* the nucleotide numbers 1540 to 1653 is the *attL* sequence of λ phage, and the nucleotide numbers 1654 to 1733 is the tacM promoter.

The tacM promoter (SEQ ID NO: 10) can be constructed by replacing the TTGACA sequence of the tac promoter (Gene, 25 (2-3), 167-178 (1983)) at the -35 region with TTCACA. The sequence of SEQ ID NO: 9 can be constructed by referring to the construction of pMW118-attL-Cm-attR (WO2005/010175).

The DNA of SEQ ID NO:9 was used as the template and was amplified by PCR using the primers of SEQ ID NOS: 12 and 13, and this amplification product was inserted into the chromosome of the WC196ΔcadAΔldcC strain by the λ-RED method (WO2005/010175) to construct a strain in which the promoter sequence upstream of the *gldA* was replaced. In this way, a strain with improved glycerol dehydrogenase activity, the WC196ΔcadAΔldcCPtacMgldA::Cm strain, was obtained.

### <1-3> Construction of L-lysine-producing bacterium with enhanced fructose-6-phosphate aldolase and glycerol dehydrogenase activities

A strain containing the fructose-6-phosphate aldolase gene and the glycerol dehydrogenase gene (PtacM fsaB-gldA::Cm sequence) having the structure shown in SEQ ID NO: 88 was constructed from the WC196ΔcadAΔldcC strain. For the construction of the strain having this structure, the DNA of SEQ ID NO: 9 (PCR product) was used. In the sequence of SEQ ID NO: 9, the nucleotide numbers 1 to 172 is the *attR* sequence of λ phage, the nucleotide numbers 324 to 983 is a chloramphenicol resistance gene (*cat*), the nucleotide numbers 1540 to 1653 is the *attL* sequence of λ phage, and the nucleotide numbers 1654 to 1733 is the tacM promoter.

The tacM promoter (SEQ ID NO: 10) can be constructed by replacing the TTGACA sequence of the tac promoter (Gene, 25 (2-3), 167-178 (1983)) at the -35 region with TTCACA. The DNA of SEQ ID NO: 9 can be constructed by referring to the construction of pMW118-attL-Cm-attR (WO2005/010175).

The DNA of SEQ ID NO: 9 was used as the template and was amplified by PCR using the primers of SEQ ID NOS: 89 and 90, and this amplification product was inserted into the chromosome of the WC196ΔcadAΔldcC strain (refer to International Publication WO2006/038695) by the λ-RED method (WO2005/010175) to construct a strain in which the promoter sequence upstream of the *fsaB-gldA* operon was replaced. In this way, a strain with improved fructose-6-phosphate aldolase and glycerol dehydrogenase activities, the WC196ΔcadAΔldcCPtacM fsaB-gldA::Cm strain, was obtained.

### <1-4> Construction of L-lysine-producing bacteria with enhanced fructose-6-phosphate aldolase, glycerol dehydrogenase, and dihydroxyacetone kinase activities

The WC196ΔcadAΔldcC strain (refer to International Publication WO2006/038695), the WC196ΔcadAΔldcCPtacMgldA::Cm strain, and the WC196ΔcadAΔ1dcCPtacM fsaB-gldA::Cm strain were transformed with the plasmid pCABD2 for Lys production carrying *dapA, dapB* and *lysC* genes (International Publication WO01/53459) in a conventional manner to obtain the WC196ΔcadAΔldcC/pCABD2 strain, the WC196ΔcadAΔldcCPtacMgldA::Cm/pCABD2 strain, and the WC196ΔcadAΔldcCPtacM fsaB-gldA::Cm/pCABD2 strain. Furthermore, the WC196ΔcadAΔldcC/pCABD2 strain, the WC196ΔcadAΔldcCPtacMgldA::Cm/pCABD2 strain, and the WC196ΔcadAΔldcCPtacM fsaB-gldA::Cm/pCABD2 strain were transformed with the *dakl* expression plasmid pMW-dak1 in a conventional manner to obtain the WC196ΔcadAΔldcC/pCABD2,pMW-dak1 strain, the WC196ΔcadAΔldcCPtacMgldA::Cm/pCABD2,pMW-dak1 strain and the WC196ΔcadAΔldcCPtacM fsaB-gldA::Cm/pCABD2,pMW-dak1 strain.

These strains were each cultured in L medium containing 20 mg/L of streptomycin, or 20 mg/L of streptomycin and 50 mg/L of ampicillin at 37°C until the final OD600 became about 0.6, then a 40% glycerol solution in a volume equal to the culture medium was added to each culture medium, and the mixture was stirred, then divided into appropriate volumes, and stored at -80°C. These are called glycerol stocks.

### Reference Example 2: Evaluation of L-lysine-producing bacteria with enhanced fructose-6-phosphate aldolase, glycerol dehydrogenase, and dihydroxyacetone kinase activities

The aforementioned glycerol stocks of the strains were thawed, 100 µL of each stock was uniformly applied to an L plate containing 20 mg/L of streptomycin, or 20 mg/L of streptomycin and 50 mg/L of ampicillin, and the culture was performed at 37°C for 24 hours. The obtained cells on the plate were suspended in 1 ml of physiological saline, the suspension was diluted 101 times, and the diluted suspension was inoculated in a volume corresponding to 50/n, wherein n is the absorbance of the diluted suspension at 600 nm, into 20 mL of a fermentation medium containing 20 mg/L of streptomycin, or 20 mg/L of streptomycin and 50 mg/L of ampicillin in a 500-mL Sakaguchi flask. The culture was performed at 37°C for 48 hours on a reciprocally shaking culture machine. After completion of the culture, the amount of lysine which had accumulated in the medium was measured by a known method (Biotec Analyzer AS210, SAKURA SEIKI).

The composition of the fermentation medium is shown below (unit: g/L):

| | |
|---|---|
| Glycerol | 40 |
| (NH₄)₂SO₄ | 24 |
| K₂HPO₄ | 1.0 |
| MgSO₄ · 7H₂O | 1.0 |
| FeSO₄ · 7H₂O | 0.01 |
| MnSO₄ · 5H₂O | 0.01 |
| Yeast extract | 2.0 |
| To a final volume of 1 L | |

The medium was adjusted to pH 7.0 with KOH, and autoclaved at 115°C for 10 minutes, provided that glycerol and MgSO₄ · 7H₂O were separately sterilized, and 30 g/L of CaCO₃ of Japanese Pharmacopoeia subjected to hot air sterilization at 180°C for 2 hours was added.

As antibiotics, 20 mg/L of streptomycin, or 20 mg/L of streptomycin and 50 mg/L of ampicillin were added. The culture was performed at a temperature of 37°C with stirring at 115 rpm for 48 hours.

As a result, the WC196ΔcadAΔldcCPtacMgldA::Cm/pCABD2,pMW-dak1 strain in which glycerol dehydrogenase and dihydroxyacetone kinase using ATP as a phosphate donor were both enhanced, and the WC196ΔcadAΔldcCPtacM fsaBgldA::Cm/pCABD2,pMW-dak1 strain in which fructose-6-phosphate aldolase was further enhanced, both produced larger amounts of L-lysine as compared to the non-modified strain.

Example 1: The aforementioned glycerol stock of the WC196ΔcadAΔldcCPtacM fsaB-gldA::Cm/pCABD2,pMW-dak1 strain was thawed, 100 µL of the stock was uniformly applied to an L plate containing 20 mg/L of streptomycin and 50 mg/L of ampicillin, and the culture was performed at 37°C for 18 hours. The obtained cells on the plate were suspended in 5 ml of physiological saline, the suspension was diluted 101 times, and the diluted suspension was inoculated in a volume corresponding to 146/n, wherein n is absorbance of the diluted suspension at 620 nm, into 300 mL of a fermentation medium (A medium) containing 20 mg/L of streptomycin and 50 mg/L of ampicillin in a 1-L volume jar fermenter, and a seed culture was performed at 37°C and pH 6.7 for 15 hours.

After the completion of the culture, to 300 mL of the A medium containing 20 mg/L of streptomycin and 50 mg/L of ampicillin in a 1-L volume jar fermenter, the seed culture medium was inoculated in a volume corresponding to 15% of the volume of the A medium, and the culture was started at 37°C and pH 6.7 and continued for 20 hours. After 8 hours of the culture, the glycerol concentration in the medium was controlled to be 0 to 5 g/L, 10 to 15 g/L, or 15 to 20 g/L by adding B medium dropwise. The glycerol concentration was measured with BF-5 produced by Oji Scientific Instruments.

**Composition of L plate medium:**

| | |
|---|---|
| Tryptone | 10 g/L |
| Yeast extract | 5 g/L |
| NaCl | 10 g/L |
| Agar | 20 g/L |

**Composition of A medium:**

| | |
|---|---|
| Glycerol | 40.0 g/L |
| Mameno^{*1)} | 11 mL/L |
| Ammonium sulfate | 8.5 g/L |
| KH₂PO₄ | 1.0 g/L |
| MgSO₄·7H₂O | 1.0 g/L |
| FeSO₄·7H₂O | 30 mg/L |
| MnSO₄·4H₂O | 30 mg/L |
| Threonine | 0.21 g/L |
| Methionine | 0.13 g/L |
| Betaine^{*2)} | 2 g/L |

| | |
|---|---|
| *1) Soy bean hydrolysate (total nitrogen: 0.35 g/L) *2) N,N,N-Trimethylglycine | |

**Composition of B medium:**

| | |
|---|---|
| Glycerol | 450 g/L |
| Threonine | 0.84 g/L |
| KH₂PO₄ | 3.37 g/L |
| Ammonium sulfate | 106 g/L |

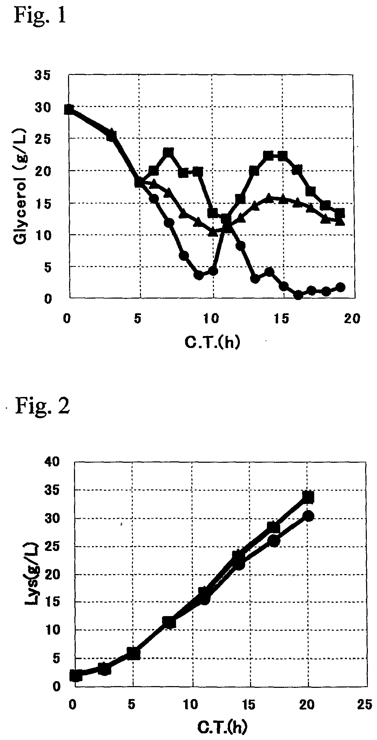

The results of the culture are shown in Table 5, and the change in the glycerol concentration over time and the change of amount of L-lysine which accumulated over time are shown in Figs. 1 to 3. When the glycerol concentration was controlled to be 10 to 15 g/L, or 15 to 20 g/L, the accumulation and productivity of L-lysine were improved, as compared to when the glycerol concentration was controlled to be 0 to 5 g/L.

**Table 5: Results of culture**

| Controlled concentration | Lys yield | Lys productivity | Consumed glycerol conc. | Lys production amount |
|---|---|---|---|---|
| | (%) | (g/L/h) | (g/fermenter) | (g/L) |
| 0-5(4.1±3.5) g/L | 35.3 | 1.58 | 26.8 | 30.5 |
| 10-15(13.3±1.8) g/L | 35.1 | 1.74 | 31.7 | 33.7 |
| 15-20(17.6±3.6) g/L | 36.2 | 1.75 | 31.0 | 33.9 |

| | | | | |
|---|---|---|---|---|
| *) Average glycerol concentrations after 8 hours and deviations thereof are in the parentheses in the table. | | | | |

### Reference Example 3: Construction of L-threonine-producing bacterium with enhanced glycerol dehydrogenase and dihydroxyacetone kinase activities

### <3-1> Construction of glycerol dehydrogenase activity-improved strain

A strain containing the *gldA* gene having the structure shown in SEQ ID NO: 91 was constructed from the *E. coli* VKPM B-5318 strain (EP 0593792 B). For the construction of this strain, the DNA of SEQ ID NO: 92 (PCR product) was used. In the DNA of SEQ ID NO: 92, the nucleotide numbers 1 to 72 is the *attR* sequence of λ phage, the nucleotide numbers 324 to 983 is chloramphenicol resistance gene (*cat*), the nucleotide numbers 1540 to 1653 is the *attL* sequences of λ phage, and the nucleotide numbers 1654 to 1733 is the tacM2 promoter.

The tacM2 promoter is a constitutive promoter which can be constructed by replacing the TTGACA sequence of the tac promoter (Gene, 25 (2-3), 167-178 (1983)) at the -35 region with TGTACA (Molecular Biology, 39 (5) 719-726 (2005)). The DNA of SEQ ID NO: 92 can be constructed by referring to the construction of pMW118-attL-Cm-attR (WO2005/010175).

The DNA of SEQ ID NO: 92 was used as the template and was amplified by PCR using the primers of SEQ ID NOS: 93 and 94, and this amplification product was inserted into chromosome of the B-5318 strain (VKPM B-5318) by the λ-RED method (WO2005/010175) to obtain a strain in which the promoter sequence upstream of the *gldA* was replaced. In this way, a strain with improved glycerol dehydrogenase activity, B5318PtacM2gldA::Cm strain, was obtained.

### <3-2> Construction of L-threonine-producing bacterium with enhanced glycerol dehydrogenase and dihydroxyacetone kinase activities

The B5318PtacM2gldA::Cm strain was transformed with the *dakl* expression plasmid pMW-dakl in a conventional manner to obtain B5318PtacM2gldA::Cm/pMW-dakl strain.

Example 2: A glycerol stock of the aforementioned strain was thawed, 100 µL of the stock was uniformly applied to an L plate containing 20 mg/L of streptomycin and 50 mg/L of ampicillin, and the culture was performed at 37°C for 18 hours. The obtained cells on the plate were suspended in 5 ml of physiological saline, the suspension was diluted 101 times, and the diluted suspension was inoculated in a volume (V) obtained by dividing a constant 146 with absorbance of the diluted suspension at 620 nm (n) (V = 146/n), into 300 mL of a fermentation medium (C medium) containing 20 mg/L of streptomycin and 50 mg/L of ampicillin in a 1-L volume jar fermenter, and the seed culture was performed at 37°C and pH 6.7 for 15 hours.

After completion of the culture, to 300 mL of C medium containing 20 mg/L of streptomycin and 50 mg/L of ampicillin in a 1-L volume jar fermenter, the seed culture medium was inoculated in a volume corresponding to 15% of the volume of the C medium, and the culture was started at 37°C and pH 6.7 and continued for 20 hours. After 6 hours of the culture, the glycerol concentration in the medium was controlled to be 0 to 5 g/L, 5 to 10 g/L, or 10 to 15 g/L by adding an aqueous solution of glycerol (700 g/L) dropwise.

**Composition of C medium:**

| | |
|---|---|
| Glycerol | 30 g/L |
| Mameno^{*1)} | 11 mL/L |
| KH₂PO₄ | 1.0 g/L |
| MgSO₄·7H₂O | 1.0 g/L |
| FeSO₄·7H₂O | 30 mg/L |
| MnSO₄·4H₂O | 30 mg/L |
| Betaine^{*2)} | 2 g/L |

| | |
|---|---|
| *1) Soy bean hydrolysate (total nitrogen: 0.35 g/L) *2) N,N,N-Trimethylglycine | |

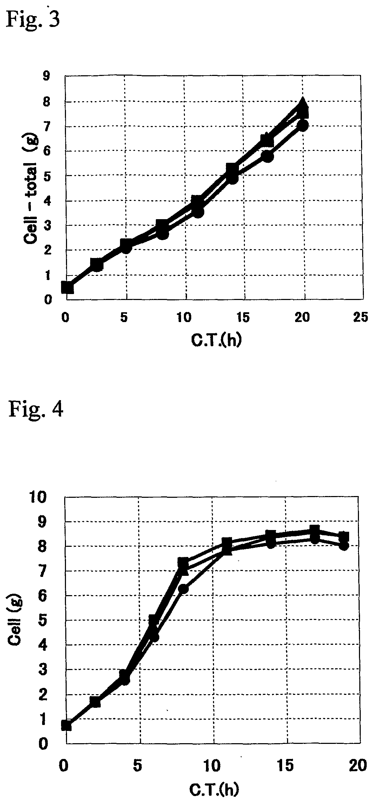

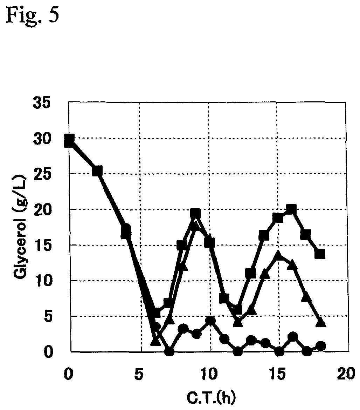

The results of the culture are shown in Table 6, and change in the glycerol concentration over time and the change in the cell growth over time are shown in Figs. 4 and 5.

When the glycerol concentration was controlled to be 5 to 10 g/L or 10 to 15 g/L, the accumulation and productivity of L-threonine were improved as compared to when the glycerol concentration was controlled to be 0 to 5 g/L.

**Table 6: Results of culture**

| Controlled concentration | Thr yield | Thr productivity | Consumed glycerol conc. | Thr production amount |
|---|---|---|---|---|
| | (%) | (g/L/h) | (g/fermenter) | (g/L) |
| 0-5(1.6±1.4) g/L | 32.9 | 2.23 | 37.7 | 41.2 |
| 5-10(9.1±5.3) g/L | 34.2 | 2.28 | 39.0 | 43.4 |
| 10-15(13.2±5.5) g/L | 34.6 | 2.31 | 40.6 | 43.9 |

| | | | | |
|---|---|---|---|---|
| *) Average glycerol concentrations after 6 hours and deviations thereof are mentioned in the parentheses in the table. | | | | |

### Explanation of Sequence Listing

SEQ ID NO: 1: *gldA* gene sequence of *Escherichia coli* (1104 bp)
SEQ ID NO: 2: GldA amino acid sequence of *Escherichia coli* (367 AA)
SEQ ID NO: 3: dakA1 gene sequence of *Saccharomyces cerevisiae* (1755 bp)
SEQ ID NO: 4: DakA amino acid sequence of *Saccharomyces cerevisiae* (584 AA)
SEQ ID NO: 5: *dhbK1* gene sequence of *Agrobacterium tumefaciens* (1695 bp)
SEQ ID NO: 6: Dhbkl amino acid sequence of *Agrobacterium tumefaciens* (564 AA)
SEQ ID NO: 7: *dhaK* gene sequence of *Citrobacter freundii* (1659 bp)
SEQ ID NO: 8: DhaK amino acid sequence of *Citrobacter freundii* (552 AA)
SEQ ID NO: 9: attR-cat-attL-ptacM-SD-spacer sequence (1740 bp)
SEQ ID NO: 10: tacM promoter (80 bp)
SEQ ID NO: 11: PtacMgldA::Cm sequence
SEQ ID NO: 12: atL-Ptac-gldA (PCR primer for enhancing *gldA* on chromosome)
SEQ ID NO: 13: atR-Ptac-fsaB1 (PCR primer for enhancing *gldA* on chromosome)
SEQ ID NO: 14: pMW-dak1F (primer for *dakA* cloning)
SEQ ID NO: 15: pMW-daklR (primer for *dakA* cloning)
SEQ ID NO: 16: *glpF* gene sequence of *Escherichia coli* (846 bp)
SEQ ID NO: 17: GlpF amino acid sequence of *Escherichia coli* (281 AA)
SEQ ID NO: 18: *tpiA* gene sequence of *Escherichia coli* (768 bp)
SEQ ID NO: 19: TpiA amino acid sequence of *Escherichia coli* (255 AA)
SEQ ID NO: 20: *fbaA* gene sequence of *Escherichia coli* (1080 bp)
SEQ ID NO: 21: FbaA amino acid sequence of *Escherichia coli* (359 AA)
SEQ ID NO: 22: *glpX* gene sequence of *Escherichia coli* (1011 bp)
SEQ ID NO: 23: GlpX amino acid sequence of *Escherichia coli* (336 AA)
SEQ ID NO: 24: *glpK* gene sequence of *Escherichia coli* (1509 bp)
SEQ ID NO: 25: GlpK amino acid sequence of *Escherichia coli* (502 AA)
SEQ ID NO: 26: *glpA* gene sequence of *Escherichia coli* (1629 bp)
SEQ ID NO: 27: GlpA amino acid sequence of *Escherichia coli* (542 AA)
SEQ ID NO: 28: *glpB* gene sequence of *Escherichia coli* (1260 bp)
SEQ ID NO: 29: GlpB amino acid sequence of *Escherichia coli* (419 AA)
SEQ ID NO: 30: *glpC* gene sequence of *Escherichia coli* (1191 bp)
SEQ ID NO: 31: GlpC amino acid sequence of *Escherichia coli* (396 AA)
SEQ ID NO: 32: *glpD* gene sequence of *Escherichia coli* (1506 bp)
SEQ ID NO: 33: GlpD amino acid sequence of *Escherichia coli* (501 AA)
SEQ ID NO: 34: *dhaK* gene sequence of *Escherichia coli* (1071 bp)
SEQ ID NO: 35: DhaK amino acid sequence of *Escherichia coli* (356 AA)
SEQ ID NO: 36: *dhaL* gene sequence of *Escherichia coli* (633 bp)
SEQ ID NO: 37: DhaL amino acid sequence of *Escherichia coli* (210 AA)
SEQ ID NO: 38: *dhaM* gene sequence of *Escherichia coli* (1419 bp)
SEQ ID NO: 39: DhaM amino acid sequence of *Escherichia coli* (472 AA)
SEQ ID NO: 40: Dihydroxyacetone kinase gene of *Schizosaccharomyces pombe* (1776 bp)
SEQ ID NO: 41: Dihydroxyacetone kinase of *Schizosaccharomyces pombe* (591 AA)
SEQ ID NO: 42: Dihydroxyacetone kinase gene of *Pichia angusta* (1830 bp)
SEQ ID NO: 43: Dihydroxyacetone kinase of *Pichia angusta* (609 AA)
SEQ ID NO: 44: Dihydroxyacetone kinase gene of *Pichia pastoris* (1827 bp)
SEQ ID NO: 45: Dihydroxyacetone kinase of *Pichia pastoris* (608 AA)
SEQ ID NO: 46: Dihydroxyacetone kinase gene of *Debaryomyces hansenii* (1824 bp)
SEQ ID NO: 47: Dihydroxyacetone kinase of *Debaryomyces hansenii* (607 AA)
SEQ ID NO: 48: Dihydroxyacetone kinase gene of *Escherichia blattae* (1752 bp)
SEQ ID NO: 49: Dihydroxyacetone kinase of *Escherichia blattae* (583 AA)
SEQ ID NO: 50: Dihydroxyacetone kinase gene of *Enterobacter* sp. 638 (1647 bp)
SEQ ID NO: 51: Dihydroxyacetone kinase of *Enterobacter* sp. 638 (548 AA)
SEQ ID NO: 52: Dihydroxyacetone kinase gene of *Psychromonas* sp. CNPT3 (1695 bp)
SEQ ID NO: 53: Dihydroxyacetone kinase of *Psychromonas* sp. CNPT3 (564 AA)
SEQ ID NO: 54: Dihydroxyacetone kinase gene of *Stappia aggregata* (1647 bp)
SEQ ID NO: 55: Dihydroxyacetone kinase of *Stappia aggregata* (548 AA)
SEQ ID NO: 56: Dihydroxyacetone kinase gene of *Rhizobium leguminosarum* bv. *viciae* 3841 (1641 bp)
SEQ ID NO: 57: Dihydroxyacetone kinase of *Rhizobium leguminosarum* bv. *viciae* 3841 (546 AA)
SEQ ID NO: 58: Dihydroxyacetone kinase gene of *Myxococcus xanthus* DK 1622 (1701 bp)
SEQ ID NO: 59: Dihydroxyacetone kinase of *Myxococcus xanthus* DK 1622 (566 AA)
SEQ ID NO: 60: Dihydroxyacetone kinase gene of *Burkholderia* sp. 383 (1701 bp)
SEQ ID NO: 61: Dihydroxyacetone kinase of *Burkholderia* sp. 383 (566 AA)
SEQ ID NO: 62: Dihydroxyacetone kinase gene of *Burkholderia thailandensis* E264 (1704 bp)
SEQ ID NO: 63: Dihydroxyacetone kinase of *Burkholderia thailandensis* E264 (567 AA)
SEQ ID NO: 64: Dihydroxyacetone kinase gene of *Burkholderia multivorans* ATCC 17616 (1851 bp)
SEQ ID NO: 65: Dihydroxyacetone kinase of *Burkholderia multivorans* ATCC 17616 (616 AA)
SEQ ID NO: 66: *dhaR* gene of *Escherichia coli* (1920 bp)
SEQ ID NO: 67: DhaR amino acid sequence of *Escherichia coli* (639 AA)
SEQ ID NO: *68:fsaA* gene of *Escherichia coli* (663 bp)
SEQ ID NO: 69: FsaA amino acid sequence of *Escherichia coli* (220 AA)
SEQ ID NO: 70: *fsaB* gene of *Escherichia coli* (663 bp)
SEQ ID NO: 71: FsaB amino acid sequence of *Escherichia coli* (220 AA)
SEQ ID NO: 72: *fbaB* gene of *Escherichia coli* (1053 bp)
SEQ ID NO: 73 : FbaB amino acid sequence of *Escherichia coli* (350 AA)
SEQ ID NO: 74: *gldA* gene of *Shigella dysenteriae* Sd197 (1143 bp)
SEQ ID NO: 75: GldA amino acid sequence of *Shigella dysenteriae* Sd197 (380 AA)
SEQ ID NO: 76: *gldA* gene of *Salmonella typhimurium* LT2 (1104 bp)
SEQ ID NO: 77: GldA amino acid sequence of *Salmonella typhimurium* LT2 (367 AA)
SEQ ID NO: 78: *gldA* gene of *Pseudomonas putida* (1098 bp)
SEQ ID NO: 79: GldA amino acid sequence of *Pseudomonas putida* (365 AA)
SEQ ID NO: 80: *gldA* gene of *Bacillus coagulans* 36D1 (1104 bp)
SEQ ID NO: 81: GldA amino acid sequence of *Bacillus coagulans* 36D1 (367 AA)
SEQ ID NO: 82: *fbp* gene of *Escherichia coli* (999 bp)
SEQ ID NO: 83: Fbp amino acid sequence of *Escherichia coli* (322 AA)
SEQ ID NO: 84: *ybhA* gene of *Escherichia coli* (819 bp)
SEQ ID NO: 85: YbhA amino acid sequence of *Escherichia coli*
   (272 AA)
SEQ ID NO: 86: *ptsI* gene of *Escherichia coli* (1782 bp)
SEQ ID NO: 87: PtsI amino acid sequence of *Escherichia coli* (575 AA)
SEQ ID NO: 88: PtacM fsaB-gldA::Cm sequence
SEQ ID NO: 89: atL-Ptac-fsaB (PCR primer for enhancing *fsaB* + *gldA* on chromosome)
SEQ ID NO: 90: atR-Ptac-fsaB (PCR primer for enhancing *fsaB* + *gldA* on chromosome)
SEQ ID NO: 91: PtacM2gldA::Cm sequence
SEQ ID NO: 92: attR-cat-attL-PtacM2-SD-spacer sequence
SEQ ID NO: 93: atL-Ptac-gldA (PCR primer for enhancing *gldA* on chromosome)
SEQ ID NO: 94: atR-Ptac-fsaB 1 (PCR primer for enhancing *gldA* on chromosome)

### Industrial Applicability

According to the present invention, in a method for producing an L-amino acid from glycerol by fermentation using a microorganism belonging to the family *Enterobacteriaceae* and having an L-amino acid producing ability, productivity of L-amino acid fermentation can be improved.

## Claims

1. A method for producing an L-amino acid comprising:
A) culturing a bacterium, which belongs to the family *Enterobacteriaceae* and has an L-amino acid-producing ability, in a medium containing glycerol to cause accumulation of the L-amino acid in the medium, and
B) collecting the L-amino acid from the medium,
wherein the culturing is performed as a fed-batch culture or a continuous culture, and a feed medium containing glycerol is added to the fermentation medium so that the glycerol concentration of the fermentation medium is 5 g/L or higher.

2. The method according to claim 1, wherein the glycerol concentration is 5 g/L or higher for a period which is at least 50% or more of the entire culture period.

3. The method according to claim 1, wherein the culturing comprises a proliferation phase during which the bacterium proliferates, and a production phase during which the L-amino acid is produced, wherein the production phase follows the proliferation phase, and wherein the production phase is at least 70% or more of the entire culture period, and the glycerol concentration is 5 g/L or higher for a period which is at least 70% or more of the production phase.

4. The method according to any one of claims 1 to 3, wherein an activity of an enzyme of the dihydroxyacetone pathway has been enhanced in the bacterium .

5. The method according to claim 4, wherein the enzyme is selected from the group consisting of glycerol dehydrogenase, dihydroxyacetone kinase, fructose-6-phosphate aldolase, and combinations thereof.

6. The method according to claim 5, wherein the enzyme comprises glycerol dehydrogenase, dihydroxyacetone kinase, and fructose-6-phosphate aldolase.

7. The method according to any one of claims 4 to 6, wherein, the activity of the enzyme has been enhanced by increasing copy number of a gene coding for the enzyme, or by modifying an expression control sequence of the gene .

8. The method according to any one of claims 1 to 7, wherein the bacterium is an *Escherichia* bacterium.

9. The method according to claim 8, wherein the bacterium is *Escherichia coli.*

10. The method according to any one of claims 1 to 9, wherein the L-amino acid is L-lysine or L-threonine.

## Patentansprüche

1. Verfahren zum Produzieren einer L-Aminosäure, umfassend:
A) Kultivieren eines Bakteriums, welches zur Familie der *Enterobacteriacaea* gehört, und die Fähigkeit hat, eine L-Aminosäurezu produzieren, und
B) Gewinnen der L-Aminosäure aus dem Medium,
wobei das Kultivieren als Fed-batch-Kultur oder als kontinuierliche Kultur ausgeführt wird, und ein Feed-Medium, welches Glycerol enthält, dem Fermentationsmedium hinzugefügt wird, so dass die Glycerolkonzentration des Fermentationsmediums 5 g/L oder höher ist.

2. Verfahren gemäß Anspruch 1, wobei für eine Dauer, welche mindestens 50% oder mehr der gesamten Kulturdauer ausmacht, die Glycerolkonzentration 5 g/L oder höher ist.

3. Verfahren gemäß Anspruch 1, wobei das Kultivieren eine Proliferationsphase, während welcher das Bakterium proliferiert, und eine Produktionsphase, während welcher die L-Aminosäure produziert wird, umfasst, wobei die Produktionsphase auf die Proliferationsphase folgt, und wobei die Produktionsphase mindestens 70% oder mehr der gesamten Kulturdauer ausmacht, und für eine Dauer, welche mindestens 70% oder mehr der Produktionphase ausmacht, die Glycerolkonzentration 5 g/L oder höher ist.

4. Verfahren gemäß irgendeinem der Ansprüche 1 bis 3, wobei eine Aktivität eines Enzyms des Dihydroxyaceton-Stoffwechselwegs im Bakterium gesteigert wurde.

5. Verfahren gemäß Anspruch 4, wobei das Enzym aus der Gruppe bestehend aus Glyceroldehydrogenase, Dihydroxyacetonkinase, Fructose-6-Phosphataldolase, und Kombinationen davon ausgewählt ist.

6. Verfahren gemäß Anspruch 5, wobei das Enzym Glyceroldehydrogenase, Dihydroxyacetonkinase, und Fructose-6-Phosphataldolase umfasst.

7. Verfahren gemäß irgendeinem der Ansprüche 4 bis 6, wobei die Aktivität des Enzyms durch Erhöhen der Kopienzahl eines Gens, welches das Enzym codiert, oder durch Modifizieren einer Expressionskontrollsequenz des Gens gesteigert wurde.

8. Verfahren gemäß irgendeinem der Ansprüche 1 bis 7, wobei das Bakterium ein Escherichia-Bakterium ist.

9. Verfahren gemäß Anspruch 8, wobei das Bakterium *Escherichia coli* ist.

10. Verfahren gemäß irgendeinem der Ansprüche 1 bis 9, wobei die L-Aminosäure L-Lysin oder L-Threonin ist.

## Revendications

1. Procédé de production d'un acide L-aminé comprenant :
A) la culture d'une bactérie, qui appartient à la famille des *Enterobacteriaceae* et possède une capacité de production d'acide L-aminé, dans un milieu contenant du glycérol pour provoquer l'accumulation de l'acide L-aminé dans le milieu, et
B) le recueil de l'acide L-aminé à partir du milieu,
dans lequel la culture est réalisée comme une culture à alimentation discontinue ou une culture continue, et un milieu d'alimentation contenant du glycérol est ajouté au milieu de fermentation de sorte que la concentration de glycérol du milieu de fermentation est de 5 g/L ou plus.

2. Procédé selon la revendication 1, dans lequel la concentration de glycérol est de 5 g/L ou plus pendant une période qui représente au moins 50% ou plus de toute la période de culture.

3. Procédé selon la revendication 1, dans lequel la culture comprend une phase de prolifération durant laquelle la bactérie prolifère, et une phase de production durant laquelle l'acide L-aminé est produit, dans lequel la phase de production suit la phase de prolifération, et dans lequel la phase de production représente au moins 70% ou plus de toute la période de culture, et la concentration de glycérol est de 5 g/L ou plus pendant une période qui représente au moins 70 % ou plus de la phase de production.

4. Procédé selon l'une quelconque des revendications 1 à 3, dans lequel une activité d'une enzyme de la voie de la dihydroxyacétone a été améliorée dans la bactérie.

5. Procédé selon la revendication 4, dans lequel l'enzyme est sélectionnée dans le groupe constitué de la glycérol déshydrogénase, la dihydroxyacétone kinase, la fructose-6-phosphate aldolase, et des combinaisons de celles-ci.

6. Procédé selon la revendication 5, dans lequel l'enzyme comprend de la glycérol déshydrogénase, de la dihydroxyacétone kinase, et de la fructose-6-phosphate aldolase.

7. Procédé selon l'une quelconque des revendications 4 à 6, dans lequel, l'activité de l'enzyme a été améliorée en augmentant le nombre de copies d'un gène codant pour l'enzyme, ou en modifiant une séquence de commande d'expression du gène.

8. Procédé selon l'une quelconque des revendications 1 à 7, dans lequel la bactérie est une bactérie *Escherichia.*

9. Procédé selon la revendication 8, dans lequel la bactérie est *Escherichia coli.*

10. Procédé selon l'une quelconque des revendications 1 à 9, dans lequel l'acide L-aminé est la L-lysine ou la L-thréonine.
